# EUROPEAN PATENT APPLICATION

(11) **EP 4 749 364 A1**
(43) Date of publication of application: **27.05.2026**
(21) Application number: 25217526.0
(22) Date of filing: 21.11.2025
(51) Int. Cl.: G03F 7/004, G03F 7/039

(54) **ONIUM SALT, CHEMICALLY AMPLIFIED POSITIVE RESIST COMPOSITION AND RESIST PATTERN FORMING PROCESS**

(30) Priority: 22.11.2024 JP 2024203635
(71) Applicant: Shin-Etsu Chemical Co., Ltd., Tokyo 100-0005 (JP)
(72) Inventor: FUKUSHIMA, Masahiro, Joetsu-shi (JP)
(74) Representative: Ter Meer Steinmeister & Partner

(57) **Abstract**

An onium salt in which an aromatic ring having a sulfo group bonded thereto and another aromatic ring structure containing a bulky substituent group are linked via a sulfonamide bond generates an aromatic sulfonic acid with adequate acidity and controlled diffusion. A chemically amplified positive resist composition comprising the onium salt has a high resolution and forms a pattern of rectangular profile with reduced LER.

## Description

### TECHNICAL FIELD

This invention relates to an onium salt, a chemically amplified positive resist composition, and a resist pattern forming process.

### BACKGROUND ART

Pattern formation to a smaller feature size is required to meet the recent demand for higher integration in integrated circuits. Acid-catalyzed chemically amplified resist compositions are most often used in forming resist patterns with a feature size of 0.2 µm or less. High-energy radiation such as UV, deep-UV or EB is used as the energy source for exposure of these resist compositions. In particular, the EB lithography, which is utilized as the ultra-fine microfabrication technique, is also indispensable in processing a photomask blank into a photomask for use in the fabrication of semiconductor devices. Resist compositions for use in the EB lithography include positive ones wherein exposed regions are dissolved away to form a pattern and negative ones wherein exposed regions are retained to form a pattern. Either one which is easier to use is chosen in accordance with the morphology of the necessary resist pattern.

In general, the EB lithography is by writing an image with EB, without using a mask. In the case of positive resist, those regions of a resist film other than the regions to be retained are successively irradiated with EB having a minute area. In the case of negative resist, those regions of a resist film to be retained are successively irradiated with EB. The operation of successively scanning all finely divided regions on the work surface takes a long time as compared with full wafer exposure through a photomask. To prevent any throughput decline, a resist film having a high sensitivity is required. Because of a long image writing time, it is likely that a difference arises between an initially imaged portion and a lately imaged portion. The stability with time of the exposed portion in vacuum is one of the important performance factors. One of the important applications of chemically amplified resist material resides in processing of photomask blanks. Some photomask blanks have a surface material that can have an impact on the pattern profile of the overlying chemically amplified resist film, for example, a layer of a chromium compound, typically chromium oxide deposited on a photomask substrate. For high resolution and profile retention after etching, it is one important performance factor to maintain the profile of a resist film pattern rectangular independent of the type of substrate.

Attempts were made to ameliorate resist sensitivity and pattern profile in a controlled way by properly selecting and combining components used in resist compositions and adjusting processing conditions. One outstanding problem is the diffusion of acid, which has a significant impact on the resolution of a chemically amplified resist film. In the processing of photomasks, it is required that the profile of the resist pattern resulting from exposure does not change depending on the time taken until PEB. The major cause for time-dependent changes is the diffusion of acid generated upon exposure. Since the problem of acid diffusion has large impacts on sensitivity and resolution not only in the photomask processing, but also in general resist compositions, many studies are made thereon.

Patent Documents 1 and 2 describe acid generators capable of generating bulky acids upon exposure, for thereby controlling acid diffusion and reducing roughness. Since these acid generators are still insufficient to control acid diffusion, it is desired to have an acid generator with more controlled diffusion.

Patent Document 3 discloses a resist composition comprising a base polymer having bound thereto an acid generator capable of generating a sulfonic acid upon light exposure whereby acid diffusion is controlled. This approach of controlling acid diffusion by binding repeat units capable of generating acid upon exposure to a base polymer is effective in forming a pattern with reduced LER. However, the base polymer having bound therein repeat units capable of generating acid upon exposure encounters a problem with respect to its solubility in organic solvent, depending on the structure and proportion of the relevant units.

Polymers comprising a major proportion of aromatic structure having an acidic side chain, for example, polyhydroxystyrene are useful in resist materials for the KrF lithography. These polymers are not used in resist materials for the ArF lithography since they exhibit strong absorption at a wavelength of around 200 nm. These polymers, however, are expected to form useful resist materials for the EB and EUV lithography for forming patterns of smaller size than the processing limit of ArF lithography because they offer high etching resistance.

Often used as the base polymer in positive resist compositions for EB and EUV lithography is a polymer having an acidic functional group on phenol side chain masked with an acid labile group (or acid-decomposable protective group). Upon exposure to high-energy radiation, the acid labile group is deprotected by the catalysis of an acid generated from a photoacid generator so that the polymer may turn soluble in alkaline developer. Typical of the acid labile group are tertiary alkyl, tert-butoxycarbonyl, and acetal groups. The use of protective groups (e.g., acetal groups) requiring a relatively low level of activation energy for deprotection offers the advantage that a resist film having a high sensitivity is obtainable. However, if the diffusion of generated acid is not fully controlled, deprotection reaction can occur even in the unexposed region of the resist film, giving rise to problems like a degradation of LER and a lowering of in-plane uniformity (CDU) of pattern line width.

Patent Document 4 describes a resist composition comprising a sulfonium salt capable of generating an acid having a low pKa and a high acid strength, typically a fluorinated alkane sulfonic acid and a resin comprising repeat units having an acetal group. This raises the problem of forming a pattern having increased LER. The acid strength of the fluorinated alkane sulfonic acid is too high for deprotection of an acetal group which requires a relatively low level of activation energy for deprotection. Then, even though acid diffusion is restrained, deprotection reaction can take place with a minor amount of acid which has diffused into the unexposed region.

Patent Documents 5 and 6 describe photoacid generators capable of generating a non-fluorinated aromatic sulfonic acid having a plurality of bulky alkyl substituents. Patent Document 7 proposes a photoacid generator of triarylbenzene sulfonic acid anion structure. Patent Document 8 proposes a photoacid generator capable of generating a non-fluorinated aromatic sulfonic acid having an iodized aromatic ring introduced therein. They intend to control acid diffusion by introducing a plurality of alkyl substituents, aromatic rings or iodine atoms to increase the molecular weight of the generated acid. The control of acid diffusion is still insufficient for the purpose of forming small-size patterns. There remains room for further improvement.

### Citation List

| | |
|---|---|
| Patent Document 1: | JP-A 2009-053518 |
| Patent Document 2: | JP-A 2010-100604 |
| Patent Document 3: | JP-A 2011-022564 |
| Patent Document 4: | JP 5083528 |
| Patent Document 5: | JP 6248882 |
| Patent Document 6: | JP 7067271 |
| Patent Document 7: | JP 7032549 |
| Patent Document 8: | JP-A 2023-177038 |

### SUMMARY OF THE INVENTION

Resist compositions are recently demanded which are capable of forming not only line-and-space (LS), isolated line (IL) and isolated space (IS) patterns of satisfactory profile, but also hole patterns of satisfactory profile. The acid generator described in Patent Document 5 generates a bulky acid, indicating that acid diffusion is controlled and a pattern with satisfactory resolution and roughness is formed. However, the problem of corner rounding arises when a hole pattern is formed.

An object of the invention is to provide an onium salt capable of generating an acid having an adequate strength and controlled diffusion, a chemically amplified positive resist composition comprising the same, and a resist pattern forming process using the composition.

The inventors have found that when an onium salt in which an aromatic ring having a sulfo group bonded thereto and another aromatic ring structure containing a bulky substituent group are linked via a sulfonamide bond to restrain the degree of freedom of rotation is introduced into a resist composition as an acid generator, a pattern with satisfactory resolution and reduced LER is obtained because the generated aromatic sulfonic acid has an adequate acidity and excessive acid diffusion is restrained. By virtue of proper dissolution inhibition, a pattern of rectangular profile is obtained.

In one aspect, the invention provides an onium salt having the formula (A).

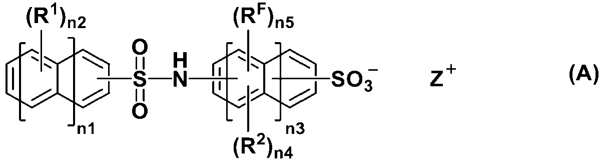

Herein n1 is 0 or 1, n2 is 1, 2, 3, 4 or 5 when n1=0, n2 is 1, 2, 3, 4, 5, 6 or 7 when n1=1, n3 is 0 or 1, n4 is 0, 1, 2, 3 or 4, n5 is 0, 1, 2, 3 or 4, n3 to n5 are in the range: 0 ≤ n4+n5 ≤ 4 when n3=0, and 0 ≤ n4+n5 ≤ 6 when n3=1,
R¹ is each independently iodine or a C₃-C₂₀ branched or cyclic hydrocarbyl group which may contain a heteroatom, at least one R¹ is bonded to a carbon atom adjoining the carbon atom to which S is bonded; when n2 is 2 or more, a plurality of R¹ may be identical or different and a plurality of R¹ may bond together to form a ring with the carbon atoms to which they are attached,
R² is halogen exclusive of fluorine, or a C₁-C₂₀ hydrocarbyl group which may contain a heteroatom exclusive of fluorine; when n4 is 2, 3 or 4, a plurality of R² may be identical or different and a plurality of R² may bond together to form a ring with the carbon atoms to which they are attached,
R^{F} is fluorine, a C₁-C₆ fluorinated alkyl group, C₁-C₆ fluorinated alkoxy group or C₁-C₆ fluorinated alkylthio group; when n5 is 2, 3 or 4, a plurality of R^{F} may be identical or different, and
Z⁺ is an onium cation.

Preferably, the onium salt has the formula (A1): 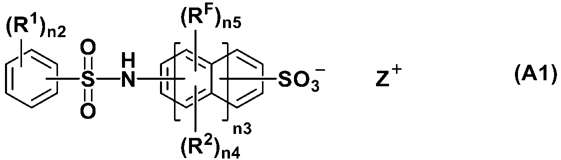 wherein n2, n3, n4, n₅, R¹, R², R^{F}, and Z⁺ are as defined above.

More preferably, the onium salt has the formula (A2): 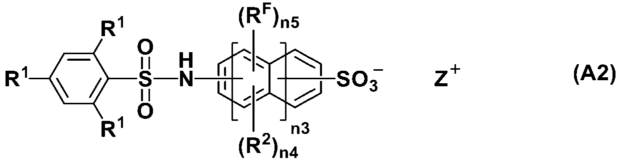 wherein n3, n4, n5, R¹, R², R^{F} and Z⁺ are as defined above.

In a preferred embodiment, Z⁺ is a sulfonium cation having the formula (Z-1) or iodonium cation having the formula (Z-2).

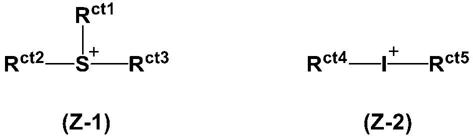

Herein R^{ct1} to R^{ct5} are each independently halogen or a C₁-C₃₀ hydrocarbyl group which may contain a heteroatom, R^{ct1} and R^{ct2} may bond together to form a ring with the sulfur atom to which they are attached.

In another preferred embodiment, Z⁺ is a sulfonium cation having the formula (Z-3).

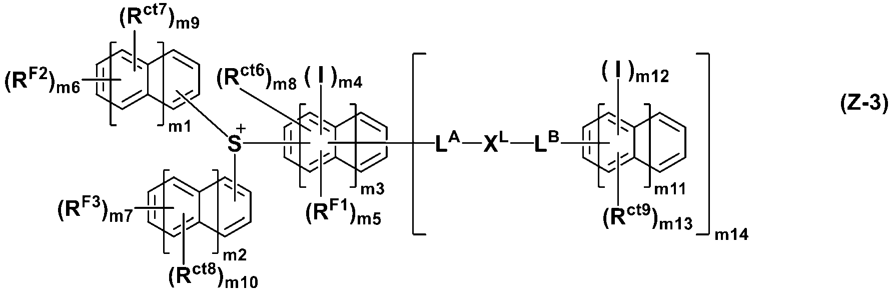

Herein m1 is 0 or 1, m2 is 0 or 1, m3 is 0 or 1, m4 is 0, 1,2,3 or 4, m5 is 0, 1,2,3 or 4, m6 is 0, 1, 2, 3, 4, 5 or 6, m7 is 0, 1, 2, 3, 4, 5 or 6, m8 is 0, 1 or 2, m9 is 0, 1 or 2, m10 is 0, 1 or 2, m11 is 0 or 1, m12 is 0, 1, 2, 3 or 4, m13 is 0, 1 or 2, m14 is 0, 1 or 2, meeting 0 ≤ m6+m9 ≤ 4 when m1=0, 0 ≤ m6+m9 ≤ 6 when ml=1, 0 ≤ m7+m10 ≤ 4 when m2=0, 0 ≤ m7+m10 ≤ 6 when m2=1, 1 ≤ m4+m5+m8+m14 ≤ 4 when m3=0, 1 ≤ m4+m5+m8+m14 ≤ 6 when m3=1, 0 ≤ m12+m13 ≤ 4 when m11=0, 0 ≤ m12+m13 ≤ 6 when m11=1, and m4+m12 ≥ 1,
R^{F1} to R^{F3} are each independently fluorine, a C₁-C₆ fluorinated saturated hydrocarbyl group, C₁-C₆ fluorinated saturated hydrocarbyloxy group, or C₁-C₆ fluorinated saturated hydrocarbylthio group, with the proviso that a plurality of R^{F1} may be identical or different when m5 is 2,3 or 4, a plurality of R^{F2} may be identical or different when m6 is 2, 3, 4, 5 or 6, and a plurality of R^{F3} may be identical or different when m7 is 2, 3, 4, 5 or 6,
R^{ct6} to R^{ct9} are each independently halogen exclusive of iodine and fluorine, nitro, cyano, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom, with the proviso that two R^{ct6} may be identical or different and two R^{ct6} may bond together to form a ring with the carbon atoms to which they are attached when m8=2, two R^{ct7} may be identical or different and two R^{ct7} may bond together to form a ring with the carbon atoms to which they are attached when m9=2, two R^{ct8} may be identical or different and two R^{ct8} may bond together to form a ring with the carbon atoms to which they are attached when m10=2, two R^{ct9} may be identical or different and two R^{ct9} may bond together to form a ring with the carbon atoms to which they are attached when m13=2,
the aromatic rings directly bonded to S⁺ in the sulfonium cation may bond together to form a ring with S⁺,
L^{A} and L^{B} are each independently a single bond, ether bond, ester bond, amide bond, sulfonate ester bond, sulfonamide bond, carbonate bond or carbamate bond, and
X^{L} is a single bond or a C₁-C₄₀ hydrocarbylene group which may contain a heteroatom.

In another aspect, the invention provides a photoacid generator in the form of the onium salt defined herein.

In a further aspect, the invention provides a chemically amplified positive resist composition comprising the photoacid generator defined above.

In a preferred embodiment, the resist composition further comprises a base polymer containing a polymer which is decomposed under the action of acid to increase its solubility in alkaline developer.

Preferably, the polymer comprises repeat units having the formula (B1).

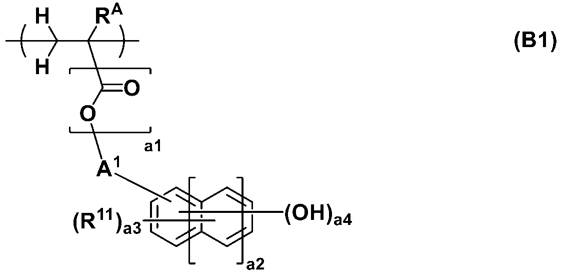

Herein a1 is 0 or 1, a2 is 0, 1 or 2, a3 is an integer meeting 0 ≤ a3 ≤ 5+2(a2)-a4, a4 is 1, 2 or 3,
R^{A} is hydrogen, fluorine, methyl or trifluoromethyl,
R¹¹ is halogen, nitro group, carboxy group, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, a plurality of R¹¹ may be identical or different when a3 is 2 or more, and

A¹ is a single bond or C₁-C₁₀ saturated hydrocarbylene group in which some -CH₂-may be replaced by -O-.

In a preferred embodiment, the polymer comprises repeat units having the formula (B2-1).

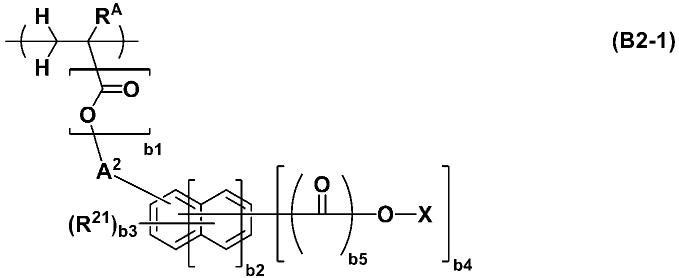

Herein b1 is 0 or 1, b2 is 0, 1 or 2, b3 is an integer meeting 0 ≤ b3 ≤ 5+2(b2)-b4, b4 is 1, 2 or 3, b5 is 0 or 1,
R^{A} is hydrogen, fluorine, methyl or trifluoromethyl,
R²¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
A² is a single bond or C₁-C₁₀ saturated hydrocarbylene group in which some -CH₂-may be replaced by -O-,
X is an acid labile group when b4=1, and X is each independently hydrogen or an acid labile group, at least one being an acid labile group, when b4=2 or 3.

In a preferred embodiment, the polymer comprises repeat units having the formula (B2-2).

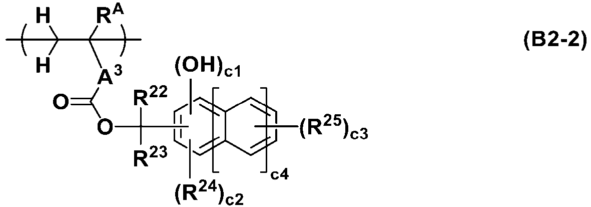

Herein c1 is 0, 1 or 2, c2 is 0, 1 or 2, c3 is 0, 1, 2, 3, 4 or 5, c4 is 0, 1 or 2,
R^{A} is hydrogen, fluorine, methyl or trifluoromethyl,
R²² and R²³ are each independently a C₁-C₁₀ hydrocarbyl group which may contain a heteroatom, R²² and R²³ may bond together to form a ring with the carbon atom to which they are attached,
R²⁴ is each independently fluorine, a C₁-C₅ fluorinated alkyl group or C₁-C₅ fluorinated alkoxy group, a plurality of R²⁴ may be identical or different when c2=2,
R²⁵ is each independently a C₁-C₁₀ hydrocarbyl group which may contain a heteroatom, a plurality of R²⁵ may be identical or different when c3 is 2, 2, 4 or 5, and
A³ is a single bond, phenylene, naphthylene or *-C(=O)-O-A³¹-, A³¹ is a C₁-C₂₀ aliphatic hydrocarbylene group which may contain a hydroxy moiety, ether bond, ester bond or lactone ring, a phenylene group or a naphthylene group, * designates a point of attachment to the carbon atom in the backbone.

In a preferred embodiment, the polymer comprises repeat units of at least one type selected from repeat units having the formula (B3), repeat units having the formula (B4), and repeat units having the formula (B5).

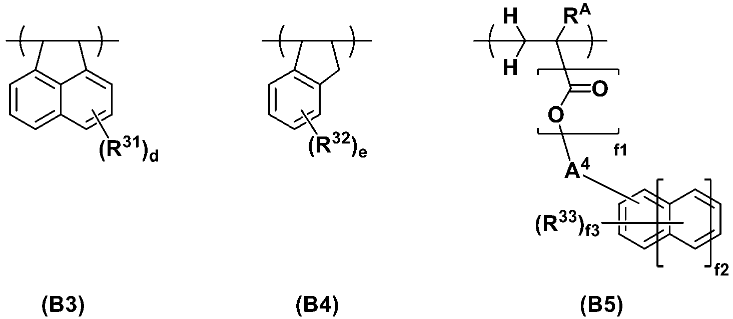

Herein d is 0, 1, 2, 3, 4, 5 or 6, e is 0, 1, 2, 3 or 4, f1 is 0 or 1, f2 is 0, 1 or 2, f3 is 0, 1, 2, 3, 4 or 5,
R^{A} is hydrogen, fluorine, methyl or trifluoromethyl,
R³¹ and R³² are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group; a plurality of R³¹ may be identical or different when d is 2, 3, 4, 5 or 6, and a plurality of R³² may be identical or different when e is 2, 3 or 4,
R³³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro, or cyano, R³³ may also be hydroxy when f2=1 or 2, a plurality of R³³ may be identical or different when f3 is 2, 3, 4 or 5, and
A⁴ is a single bond or C₁-C₁₀ saturated hydrocarbylene group in which some -CH₂-may be replaced by -O-.

In a preferred embodiment, the polymer comprises repeat units of at least one type selected from repeat units having the formula (B6), repeat units having the formula (B7), repeat units having the formula (B8), repeat units having the formula (B9), and repeat units having the formula (B10).

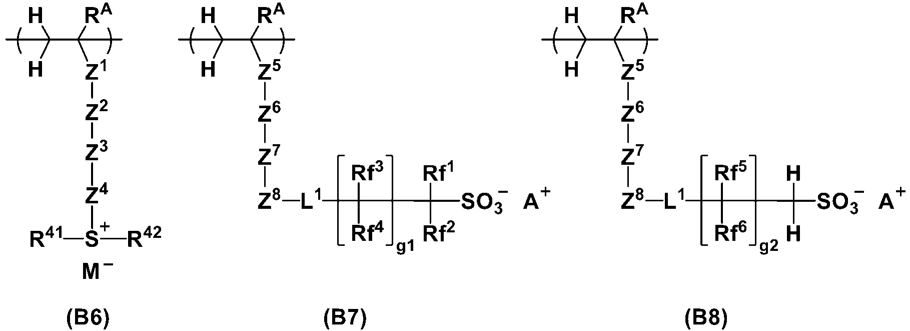

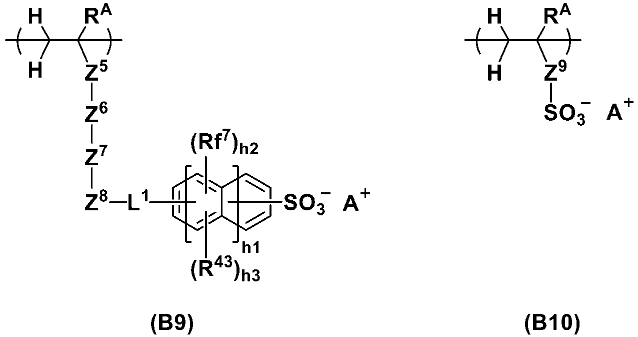

Herein g1 and g2 are each independently 0, 1, 2 or 3, h1 is 0 or 1, h2 is 0, 1, 2, 3 or 4, h3 is 0, 1, 2, 3 or 4, h1 to h3 are in the range: 0 ≤ h2+h3 ≤ 4 when h1=0, and 0 ≤ h2+h3 ≤ 6 when h1=1,
R^{A} is hydrogen, fluorine, methyl or trifluoromethyl,
Z¹ is a single bond or an optionally substituted phenylene group,
Z² is a single bond, **-C(=O)-O-Z²¹-, **-C(=O)-NH-Z²¹-, or **-O-Z²¹-, Z²¹ is a C₁-C₆ aliphatic hydrocarbylene group, phenylene group or a divalent group obtained by combining the foregoing, which may contain halogen, carbonyl moiety, ester bond, ether bond or hydroxy moiety,
Z³ is a single bond, ether bond, ester bond, amide bond, sulfonate ester bond, sulfonamide bond, carbonate bond or carbamate bond,
Z⁴ is a single bond, or a C₁-C₆ aliphatic hydrocarbylene group, phenylene group or a divalent group obtained by combining the foregoing, which may contain halogen, carbonyl moiety, ester bond, ether bond or hydroxy moiety,
Z⁵ is each independently a single bond, an optionally substituted phenylene, naphthylene, or *-C(=O)-O-Z⁵¹-, Z⁵¹ is a C₁-C₁₀ aliphatic hydrocarbylene group which may contain halogen, hydroxy moiety, ether bond, ester bond or lactone ring, or phenylene or naphthylene group,
Z⁶ is a single bond, ether bond, ester bond, amide bond, sulfonate ester bond, sulfonamide bond, carbonate bond or carbamate bond,
Z⁷ is each independently a single bond, ***-Z⁷¹-C(=O)-O-, ***-C(=O)-NH-Z⁷¹-, or ***-O-Z⁷¹-, Z⁷¹ is a C₁-C₂₀ hydrocarbylene group which may contain a heteroatom,
Z⁸ is each independently a single bond, ****-Z⁸¹-C(=O)-O-, ****-C(=O)-NH-Z⁸¹-, or ****-O-Z⁸¹-, Z⁸¹ is a C₁-C₂₀ hydrocarbylene group which may contain a heteroatom,
Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, Z⁹¹ is a C₁-C₆ aliphatic hydrocarbylene group, phenylene group, fluorinated phenylene group or trifluoromethyl-substituted phenylene group, which may contain a carbonyl moiety, ester bond, ether bond or hydroxy moiety,
* is a point of attachment to the carbon atom in the backbone, ** is a point of attachment to Z¹, *** is a point of attachment to Z⁶, **** is a point of attachment to Z⁷,
L¹ is a single bond, ether bond, ester bond, carbonyl group, sulfonate ester bond, sulfonamide bond, carbonate bond or carbamate bond,
Rf¹ and Rf² are each independently fluorine or a C₁-C₆ fluorinated saturated hydrocarbyl group,
Rf³ and Rf⁴ are each independently hydrogen, fluorine, or a C₁-C₆ fluorinated saturated hydrocarbyl group,
Rf⁵ and Rf⁶ are each independently hydrogen, fluorine, or a C₁-C₆ fluorinated saturated hydrocarbyl group, excluding that all Rf⁵ and Rf⁶ are hydrogen at the same time,
Rf⁷ is fluorine, a C₁-C₆ fluorinated alkyl group, C₁-C₆ fluorinated alkoxy group, or C₁-C₆ fluorinated alkylthio group, a plurality of Rf⁷ may be identical or different when h2 is 2, 3 or 4,
R⁴¹ and R⁴² are each independently a C₁-C₂₀ hydrocarbyl group which may contain a heteroatom, R⁴¹ and R⁴² may bond together to form a ring with the sulfur atom to which they are attached,
R⁴³ is halogen exclusive of fluorine, or a C₁-C₂₀ hydrocarbyl group which may contain a heteroatom; when h3 is 2, 3 or 4, a plurality of R⁴³ may be identical or different and a plurality of R⁴³ may bond together to form a ring with the carbon atoms to which they are attached,
M⁻ is a non-nucleophilic counter ion, and
A⁺ is an onium cation.

In a preferred embodiment, repeat units having an aromatic ring structure account for at least 60 mol% of the overall repeat units of the polymer in the base polymer.

The resist composition may further comprise an organic solvent and/or a quencher.

In a preferred embodiment, the resist composition further comprise (E) a fluorinated polymer comprising repeat units of at least one type selected from repeat units having the formula (E1), repeat units having the formula (E2), repeat units having the formula (E3) and repeat units having the formula (E4) and optionally repeat units of at least one type selected from repeat units having the formula (E5) and repeat units having the formula (E6).

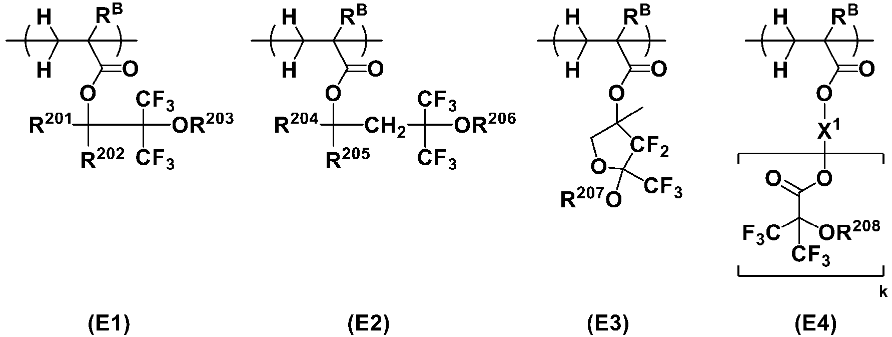

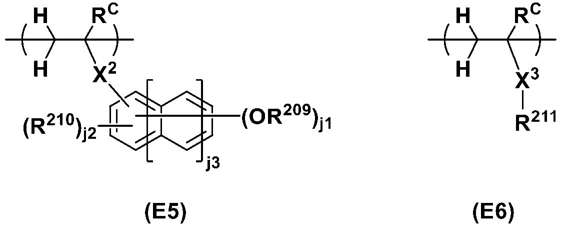

Herein j1 is 1, 2 or 3, j2 is an integer meeting 0 ≤ j2 ≤ 5+2(j3)-j1, j3 is 0 or 1, k is 1, 2 or 3,
R^{B} is each independently hydrogen, fluorine, methyl or trifluoromethyl,
R^{C} is each independently hydrogen or methyl,
R²⁰¹, R²⁰², R²⁰⁴ and R²⁰⁵ are each independently hydrogen or a C₁-C₁₀ saturated hydrocarbyl group,
R²⁰³, R²⁰⁶, R²⁰⁷ and R²⁰⁸ are each independently hydrogen, a C₁-C₁₅ hydrocarbyl group, C₁-C₁₅ fluorinated hydrocarbyl group, or acid labile group, and when R²⁰³, R²⁰⁶, R²⁰⁷ and R²⁰⁸ each are a hydrocarbyl or fluorinated hydrocarbyl group, an ether bond or carbonyl moiety may intervene in a carbon-carbon bond,
R²⁰⁹ is hydrogen or a C₁-C₅ straight or branched hydrocarbyl group in which a heteroatom-containing moiety may intervene in a carbon-carbon bond, a plurality of R²⁰⁹ may be identical or different when j1 is 2 or 3,
R²¹⁰ is a C₁-C₅ straight or branched hydrocarbyl group in which a heteroatom-containing moiety may intervene in a carbon-carbon bond, a plurality of R²¹⁰ may be identical or different when j2 is 2 or more,
R²¹¹ is a C₁-C₂₀ saturated hydrocarbyl group in which at least one hydrogen is substituted by fluorine, and in which some constituent -CH₂- may be replaced by an ester bond or ether bond,
X¹ is a C₁-C₂₀ (k+1)-valent hydrocarbon group or C₁-C₂₀ (k+1)-valent fluorinated hydrocarbon group,
X² is a single bond, *-C(=O)-O- or *-C(=O)-NH-, * designates a point of attachment to the carbon atom in the backbone,
X³ is a single bond, -O-, *-C(=O)=O-X³¹-X³²- or *-C(=O)-NH-X³¹-X³²-, X³¹ is a single bond or C₁-C₁₀ saturated hydrocarbylene group, X³² is a single bond, ester bond, ether bond, or sulfonamide bond, and * designates a point of attachment to the carbon atom in the backbone.

The resist composition may further comprise a photoacid generator other than the photoacid generator set forth above.

In a further aspect, the invention provides a resist pattern forming process comprising the steps of:
applying the chemically amplified positive resist composition defined herein onto a substrate to form a resist film thereon,
exposing the resist film patternwise to high-energy radiation, and
developing the exposed resist film in an alkaline developer.

Typically, the high-energy radiation is EUV of wavelength 3 to 15 nm or EB.

In one embodiment, the substrate has the outermost surface of a chromium-containing material. Typically, the substrate is a photomask blank.

### ADVANTAGEOUS EFFECTS OF THE INVENTION

When processed by the microfabrication technology, especially EB and EUV lithography processes, a chemically amplified positive resist composition comprising the inventive onium salt as a photoacid generator can form a resist pattern having a very high resolution and reduced LER. A pattern of rectangular profile is obtainable by virtue of adequate dissolution inhibition.

### DESCRIPTION OF THE PREFERRED EMBODIMENT

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group. In chemical formulae, the broken line (---) and asterisk (*) each designate a point of attachment, namely valence bond. Me stands for methyl and Ac for acetyl. As used herein, the term "halogenated" refers to a halogen-substituted or halogen-containing compound or group. For example, "fluorinated" refers to a fluorine-substituted or fluorine-containing compound or group. The terms "group" and "moiety" are interchangeable.

The abbreviations and acronyms have the following meaning.
- PAG:: photoacid generator
- Mw:: weight average molecular weight
- Mn:: number average molecular weight
- Mw/Mn:: molecular weight distribution or dispersity
- GPC:: gel permeation chromatography
- PEB:: post-exposure baking
- LER:: line edge roughness
- CDU:: critical dimension uniformity

It is understood that for some structures represented by chemical formulae, there can exist enantiomers and diastereomers because of the presence of asymmetric carbon atoms. In such a case, a single formula collectively represents all such isomers. The isomers may be used alone or in admixture.

### Onium salt

One embodiment of the invention is an onium salt having the formula (A).

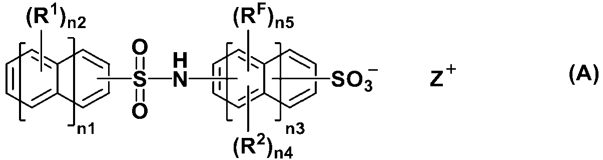

In formula (A), n1 is 0 or 1. The relevant structure is a benzene ring when n1=0, and a naphthalene ring when n1=1. The benzene ring corresponding to n1=0 is preferred from the aspect of solvent solubility. The subscript n2 is 1, 2, 3, 4 or 5 when n1=0, and n2 is 1, 2, 3, 4, 5, 6 or 7 when n1=1. It is preferred from the aspect of reactant availability that n2 be 1, 2, 3 or 4, more preferably 2 or 3. The subscript n3 is 0 or 1. The relevant structure is a benzene ring when n3=0, and a naphthalene ring when n3=1. The benzene ring corresponding to n3=0 is preferred from the aspect of solvent solubility. The subscript n4 is 0, 1, 2, 3 or 4, and n5 is 0, 1, 2, 3 or 4. It is noted that n3 to n5 are in the range: 0 ≤ n4+n5 ≤ 4 when n3=0, and 0 ≤ n4+n5 ≤ 6 when n3=1.

In formula (A), R¹ is each independently iodine or a C₃-C₂₀ branched or cyclic hydrocarbyl group which may contain a heteroatom. At least one R¹ is bonded to a carbon atom adjoining the carbon atom to which S is bonded. Non-limiting examples of the hydrocarbyl group include branched alkyl groups such as isopropyl, sec-butyl, tert-butyl, tert-pentyl and 2-ethylhexyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, oxanorbornyl, tricyclo[5.2.1.0^{2,6}]decyl, and adamantyl; C₆-C₂₀ aryl groups such as phenyl, naphthyl and anthracenyl; and combinations thereof. In the hydrocarbyl group, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy, cyano, halogen, carbonyl, ether bond, ester bond, sulfonate ester bond, carbonate bond, lactone ring, sultone ring, carboxylic anhydride (-C(=O)-O-C(=O)-) or haloalkyl moiety. Suitable halogen atoms include fluorine, chlorine, bromine and iodine, with fluorine and iodine being preferred. Preferably, R¹ is selected from C₃-C₂₀ branched alkyl groups such as isopropyl, sec-butyl, tert-butyl, tert-pentyl and 2-ethylhexyl; C₃-C₂₀ aliphatic cyclic hydrocarbyl groups such as cyclopentyl, cyclohexyl, norbornyl, oxanorbornyl, and adamantyl; and optionally substituted C₆-C₂₀ aryl groups such as phenyl and naphthyl. Preferred examples of the optional substituent on the aryl group include fluorine, chlorine, bromine, iodine, C₁-C₁₀ hydrocarbyl moieties which may contain a heteroatom, C₁-C₁₀ hydrocarbyloxy moieties which may contain a heteroatom, and C₂-C₁₀ hydrocarbyloxycarbonyl moieties which may contain a heteroatom. When n2 is 2 or more, a plurality of R¹ may be identical or different.

When n2 is 2 or more, a plurality of R¹ may bond together to form a ring with the carbon atoms to which they are attached. The ring is preferably 5 to 8-membered.

Examples of the structure of an aromatic ring having R¹ bonded thereto are shown below, but not limited thereto. Herein, the broken line (---) designates a point of attachment to S.

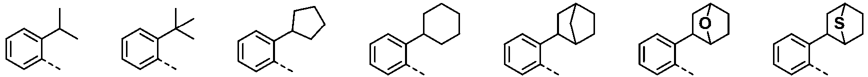

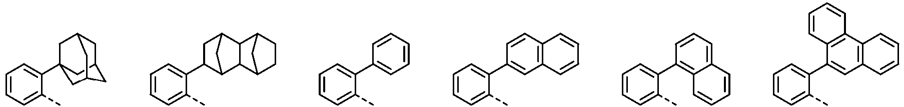

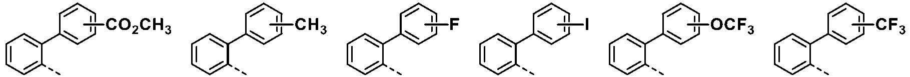

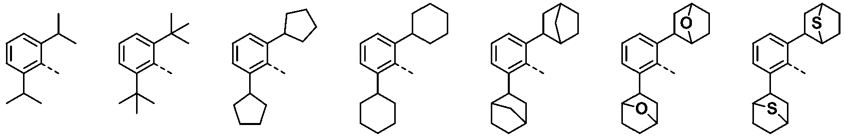

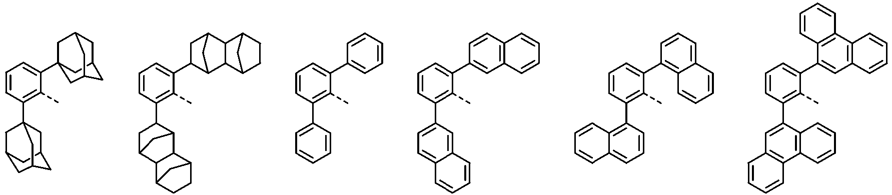

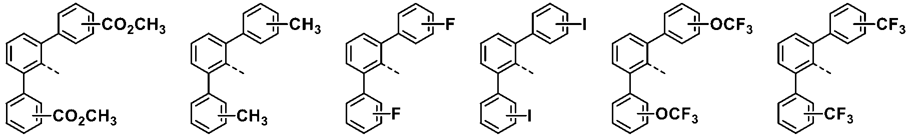

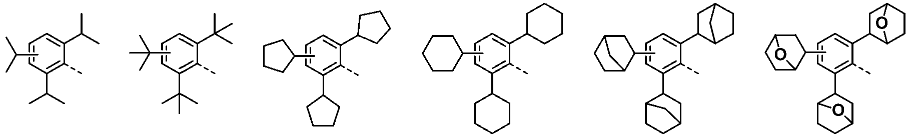

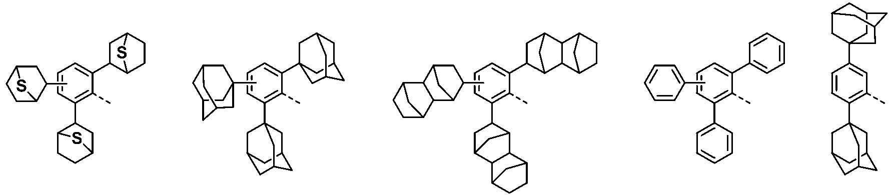

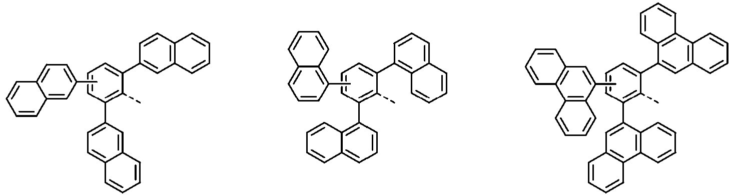

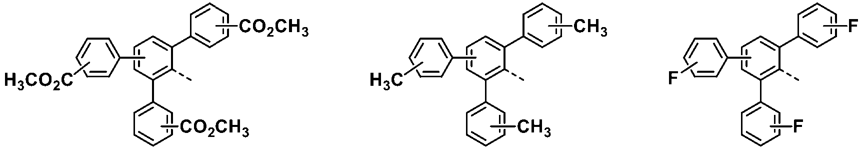

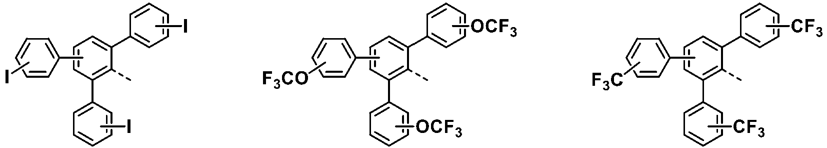

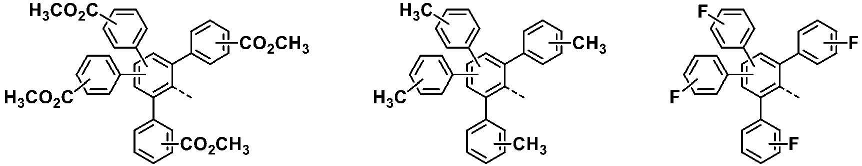

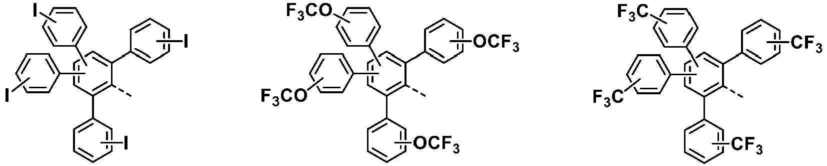

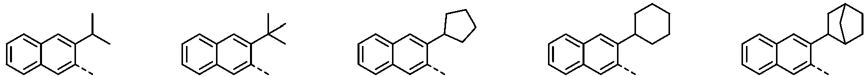

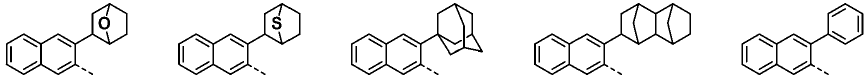

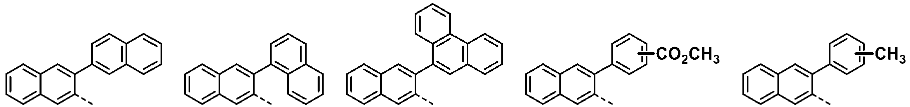

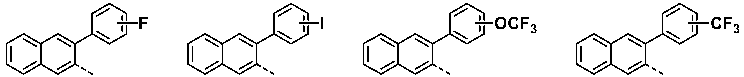

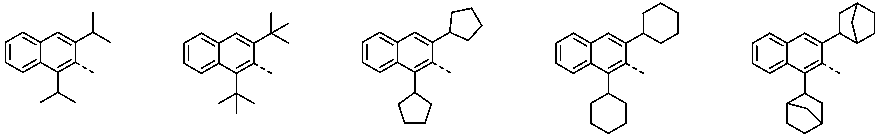

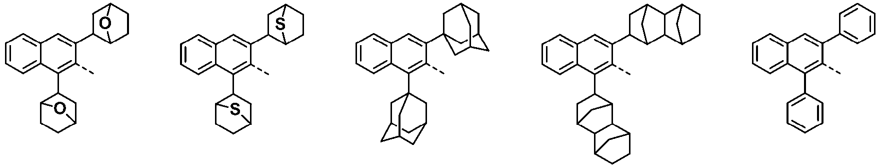

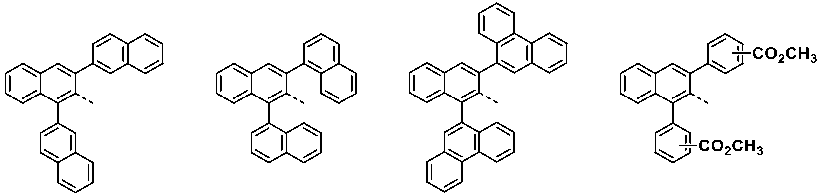

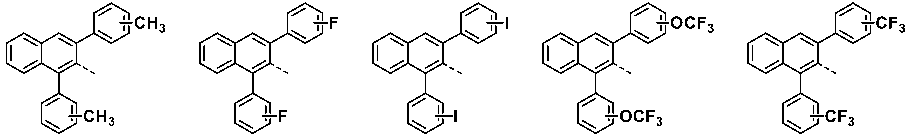

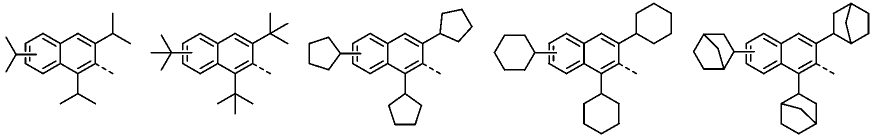

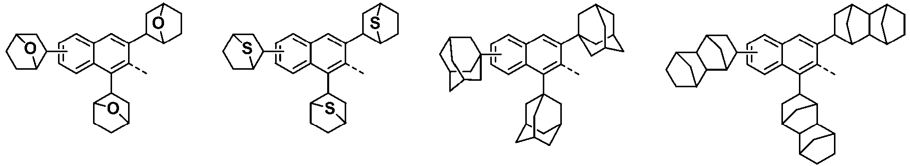

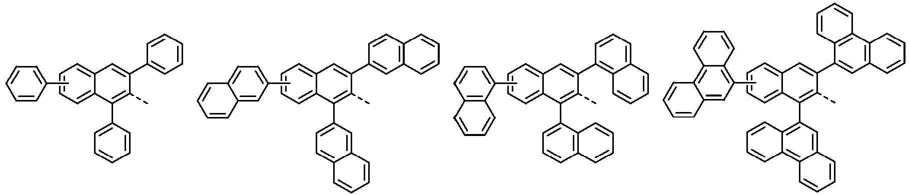

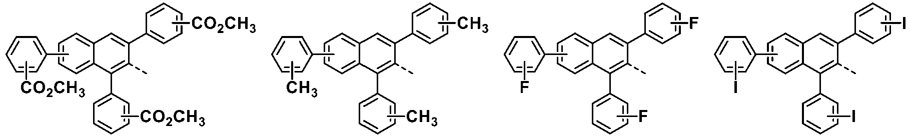

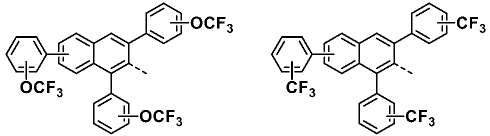

In formula (A), at least one R¹ is bonded to a carbon atom adjoining the carbon atom to which S is bonded. This means that R¹ and the other aromatic ring having the sulfo group bonded thereto are positioned spatially close to bring about steric hindrance. Then the rotation of the sulfonamide bond is restrained and acid diffusion is substantially suppressed.

In formula (A), R² is halogen exclusive of fluorine, or a C₁-C₂₀ hydrocarbyl group which may contain a heteroatom exclusive of fluorine. Suitable halogen atoms exclusive of fluorine include chlorine, bromine and iodine. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, tert-butyl, n-pentyl, tert-pentyl, n-hexyl, n-octyl, 2-ethylhexyl, n-nonyl, and n-decyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl and adamantylmethyl; and C₆-C₂₀ aryl groups such as phenyl, naphthyl and anthracenyl. In the hydrocarbyl group, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy, cyano, chlorine, bromine, iodine, carbonyl, ether bond, ester bond, sulfonate ester bond, carbonate bond, lactone ring, sultone ring, carboxylic anhydride (-C(=O)-O-C(=O)-) or haloalkyl moiety. When n4 is 2, 3 or 4, a plurality of R² may be identical or different. When n4 is 2, 3 or 4, a plurality of R² may bond together to form a ring with the carbon atoms to which they are attached. The ring is preferably 5- to 8-membered.

In formula (A), R^{F} is fluorine, a C₁-C₆ fluorinated alkyl group, C₁-C₆ fluorinated alkoxy group, or C₁-C₆ fluorinated alkylthio group. R^{F} is preferably fluorine, trifluoromethyl, difluoromethyl, trifluoromethoxy, difluoromethoxy, trifluoromethylthio or difluoromethylthio, more preferably fluorine, trifluoromethyl or trifluoromethoxy. Since fluorine or fluorine-containing substituents exert an electron-withdrawing effect, the strength of generated acid is so increased that the deprotection reaction of acid labile groups such as tertiary ester or tertiary ether groups may smoothly take place. When n5 is 2, 3 or 4, a plurality of R^{F} may be identical or different.

Of the onium salts having formula (A), those having the formula (A1) are preferred.

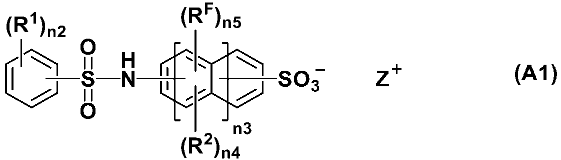

Herein n2, n3, n4, n5, R¹, R², R^{F}, and Z⁺ are as defined above.

Of the onium salts having formula (A1), those having the formula (A2) are preferred.

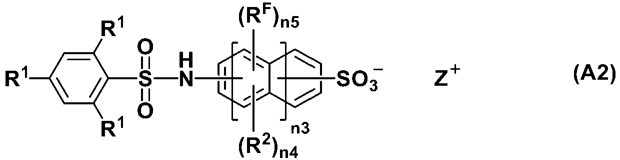

Herein n3, n4, n5, R¹, R², R^{F}, and Z⁺ are as defined above.

Preferred examples of the anion in the onium salt having formula (A) are shown below, but not limited thereto.

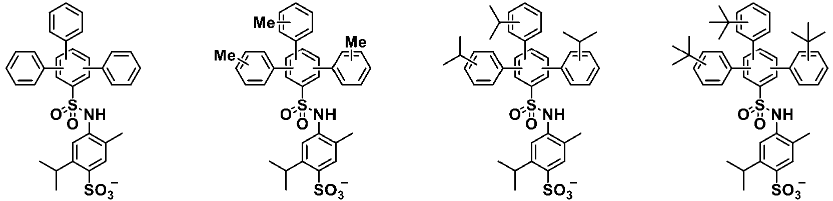

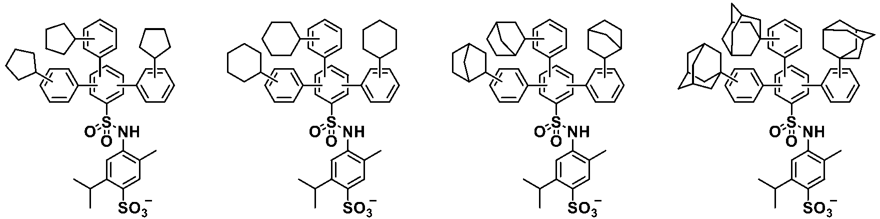

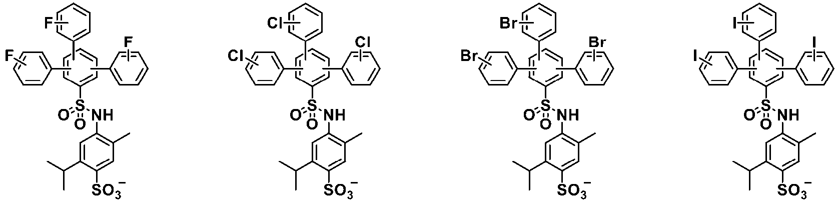

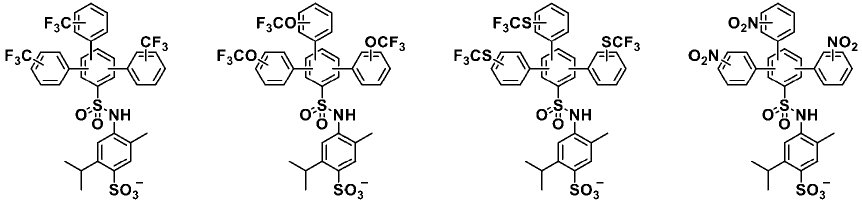

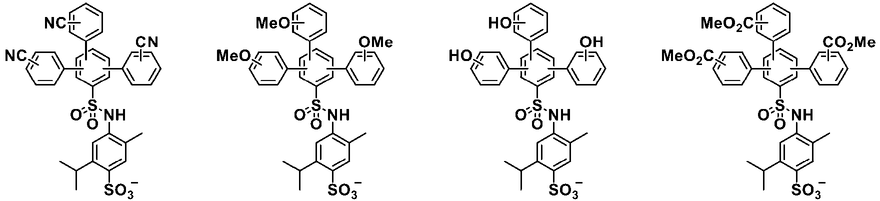

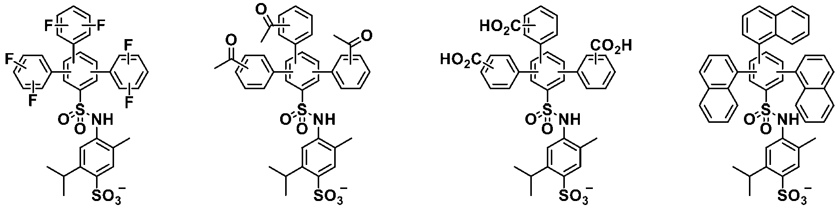

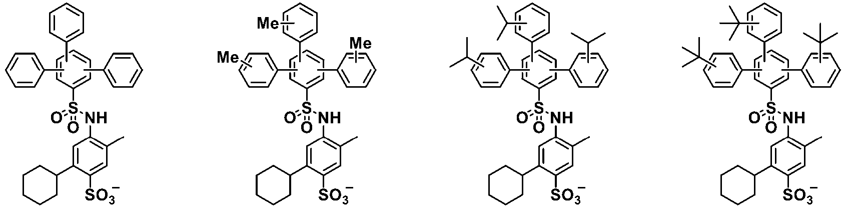

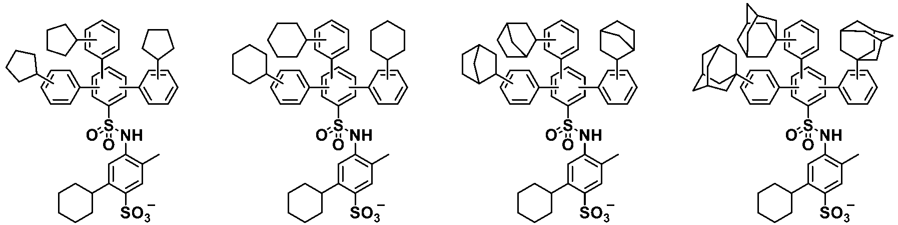

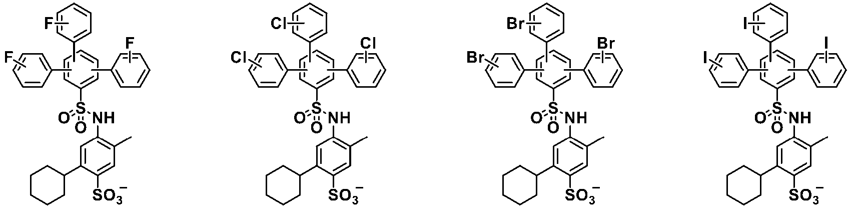

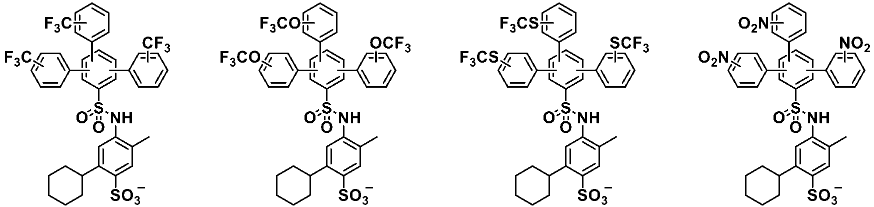

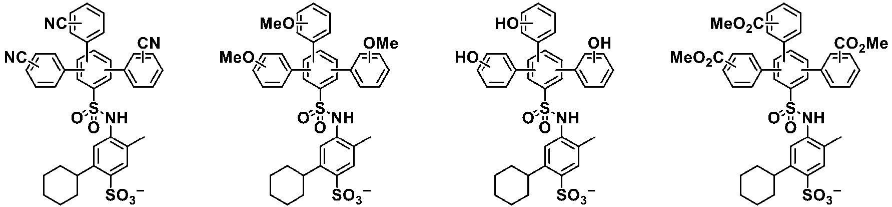

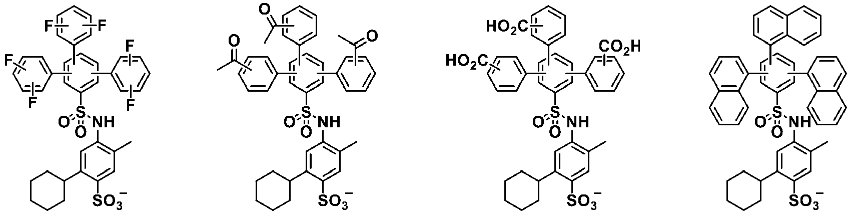

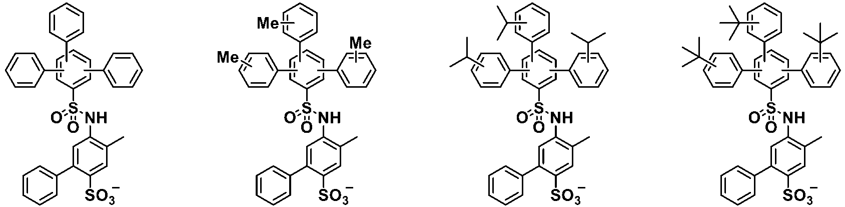

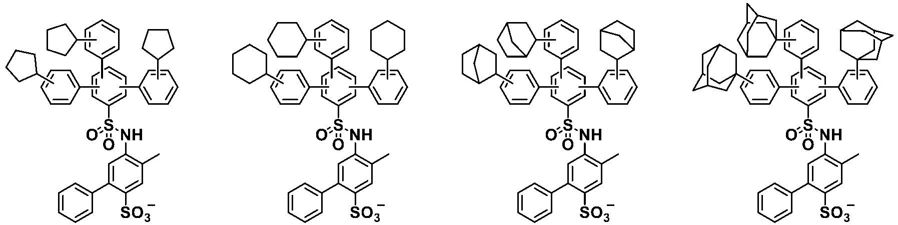

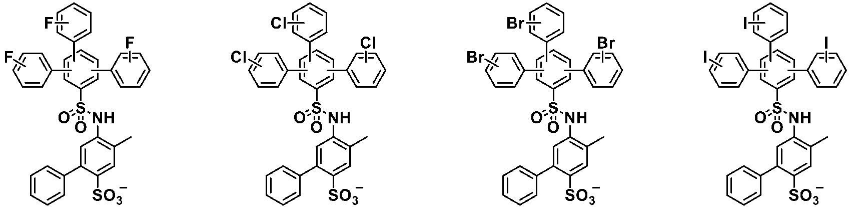

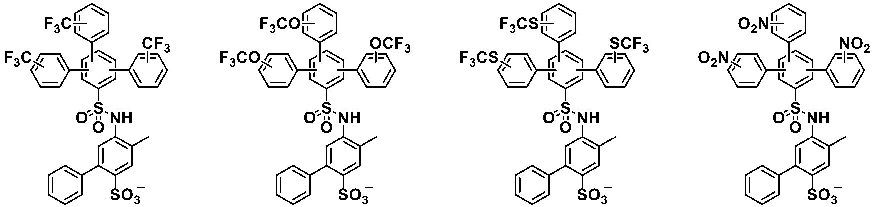

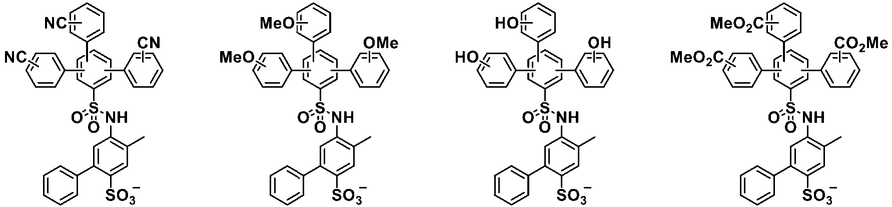

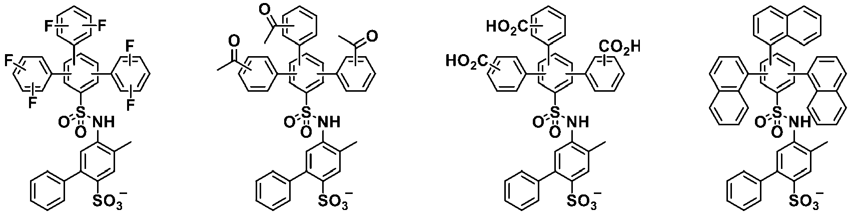

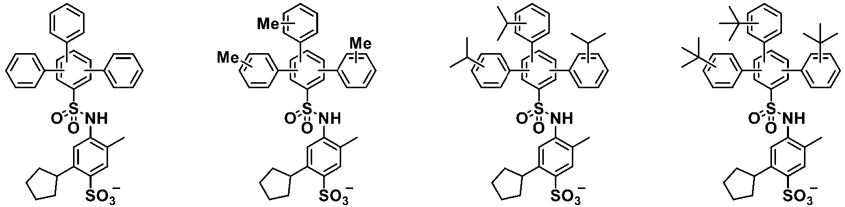

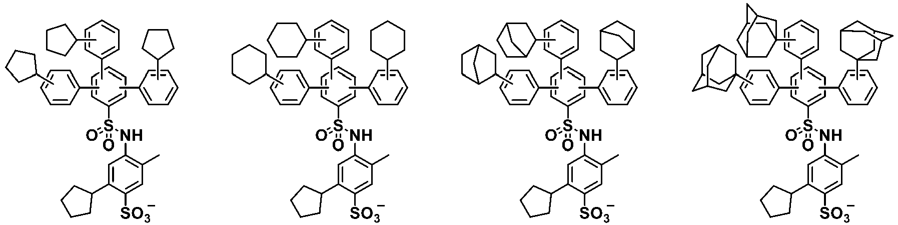

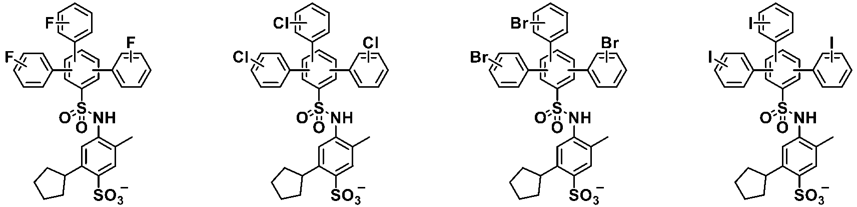

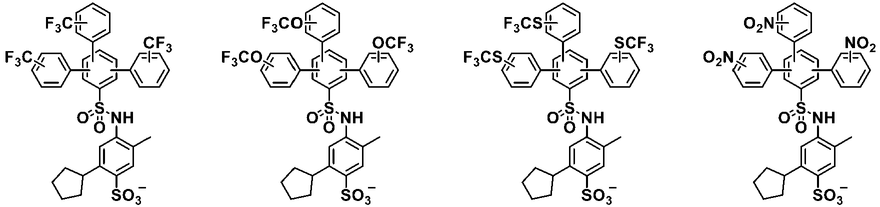

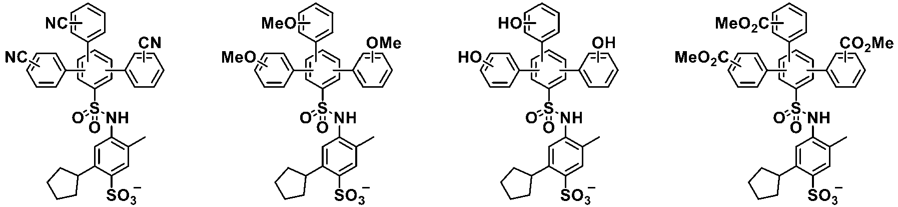

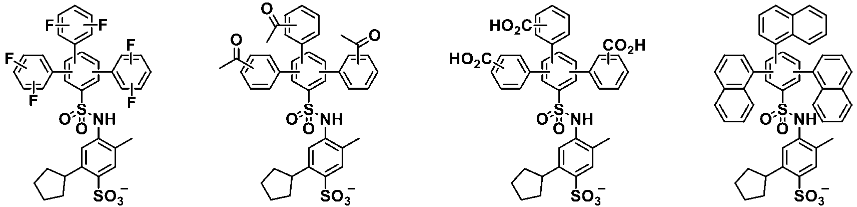

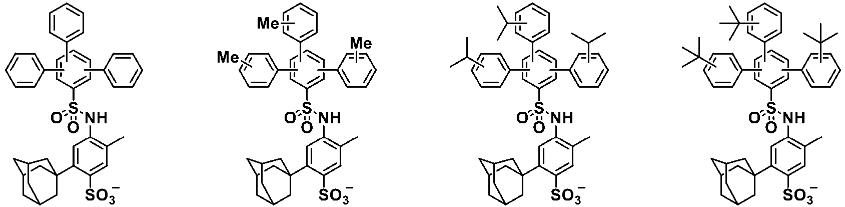

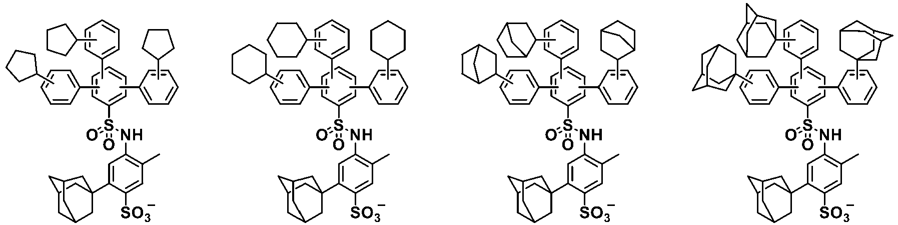

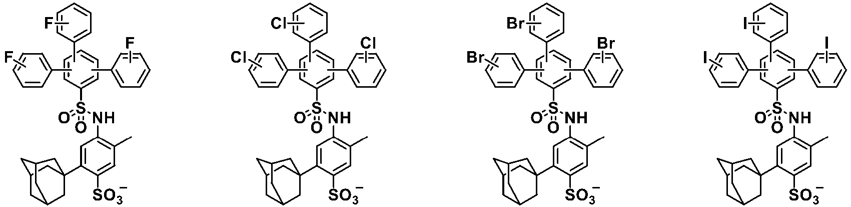

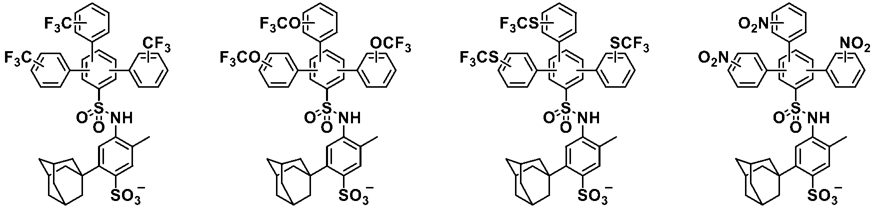

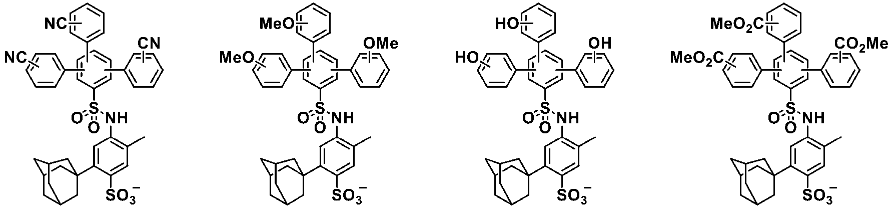

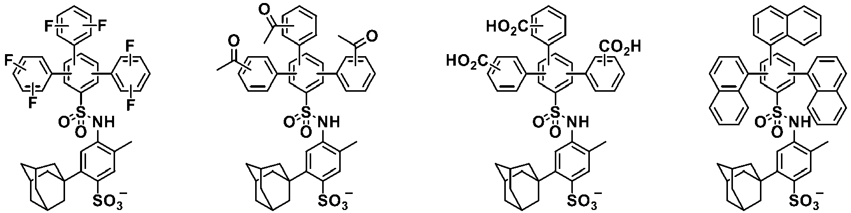

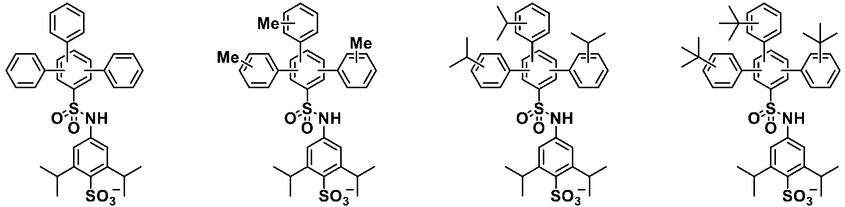

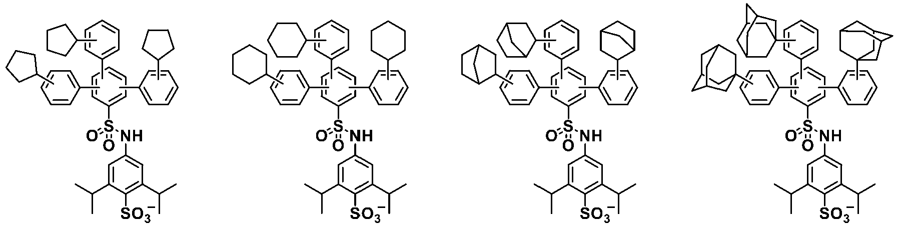

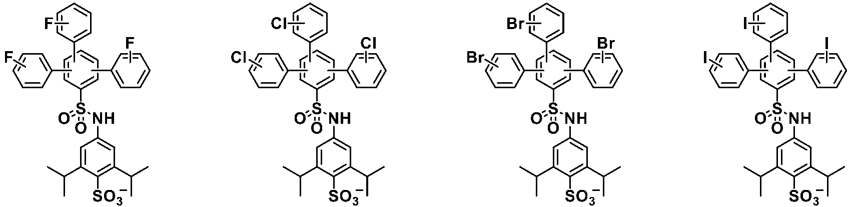

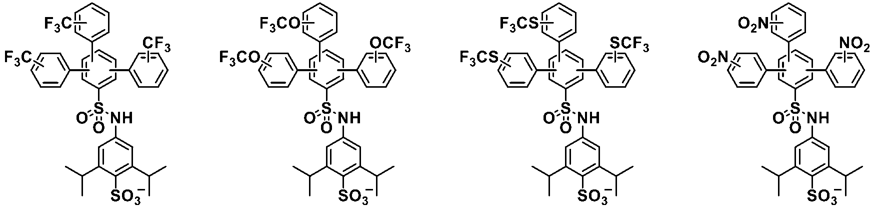

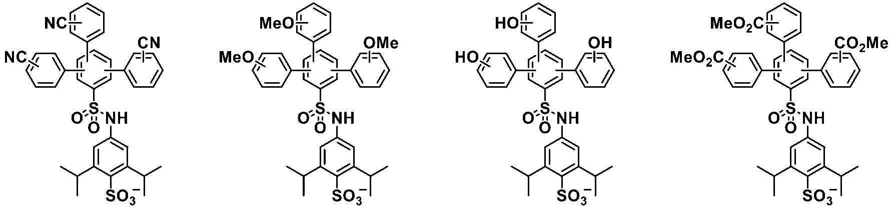

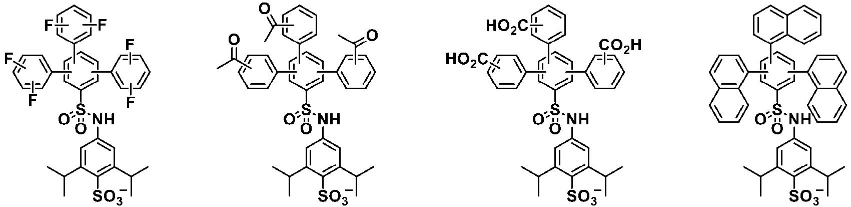

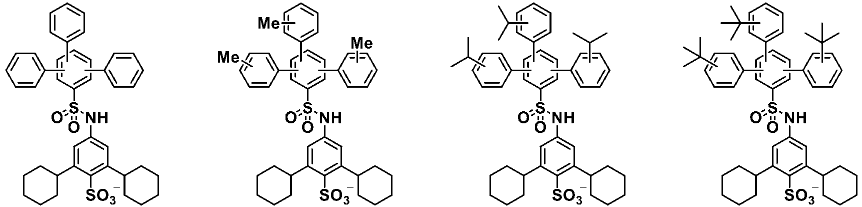

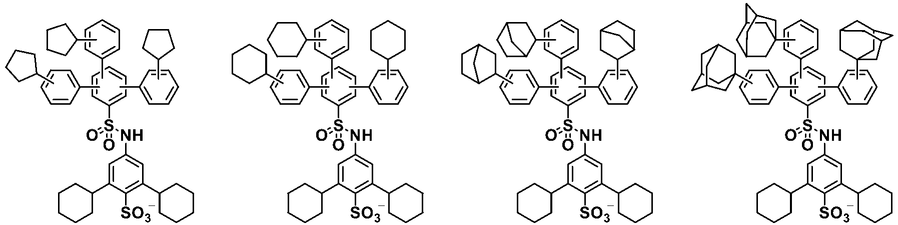

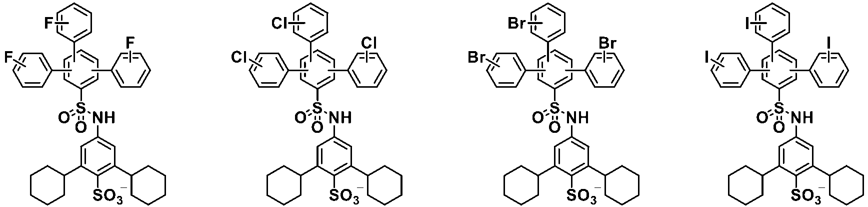

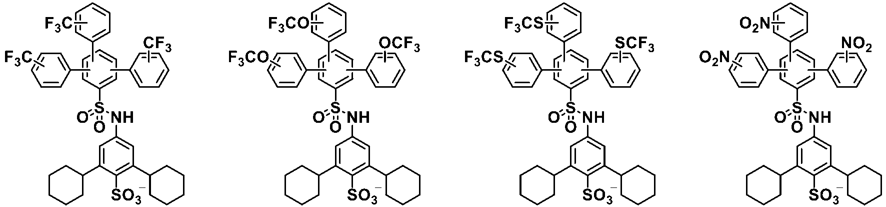

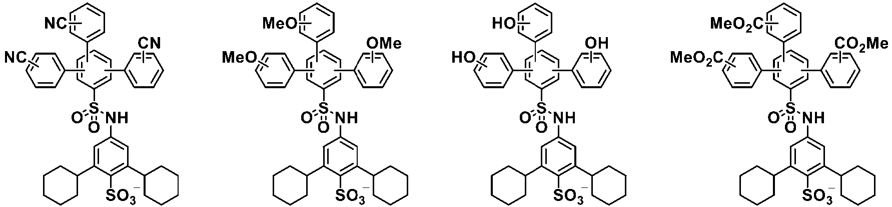

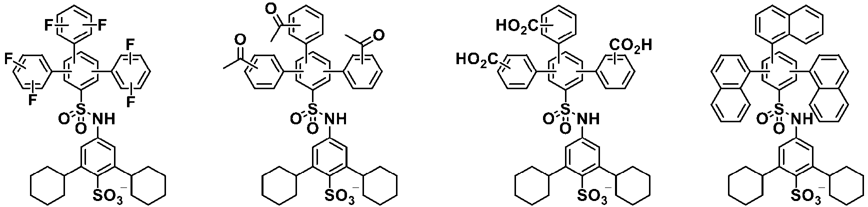

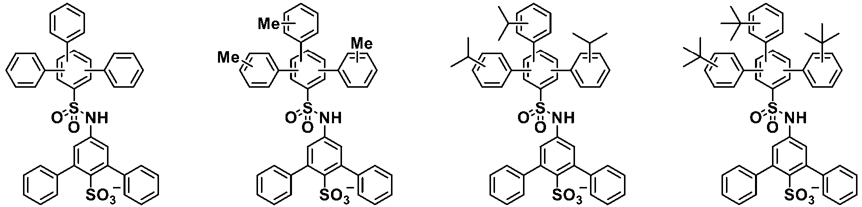

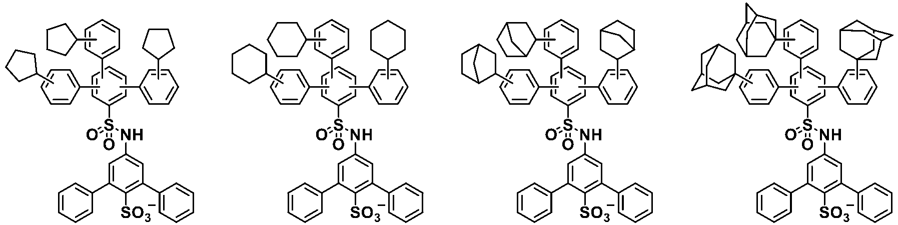

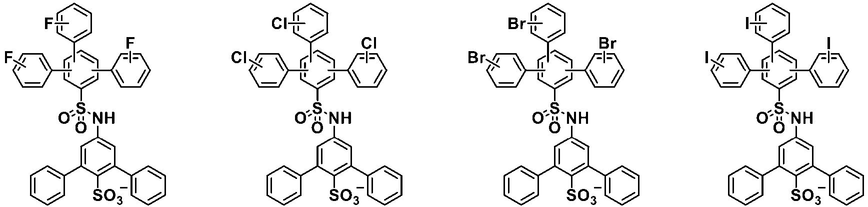

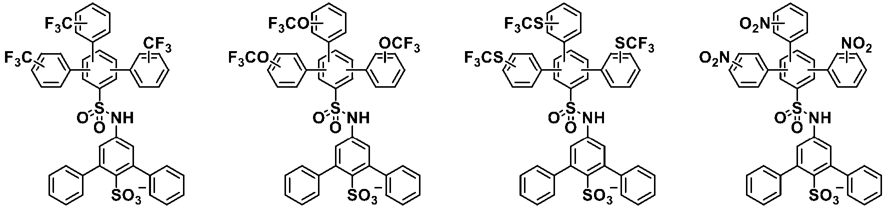

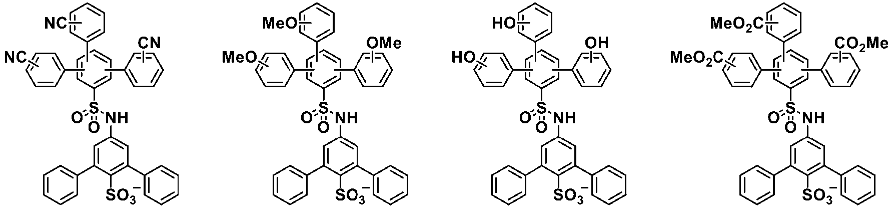

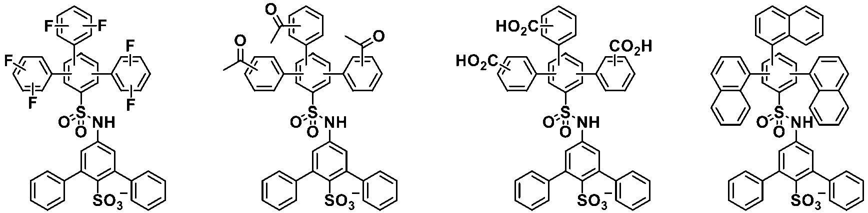

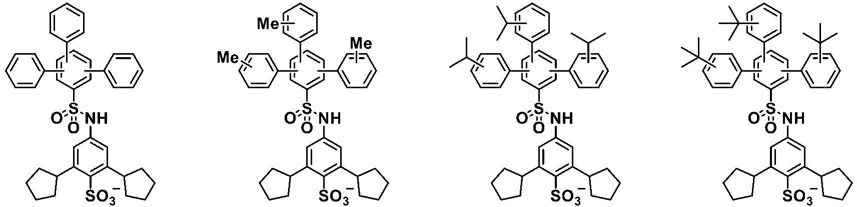

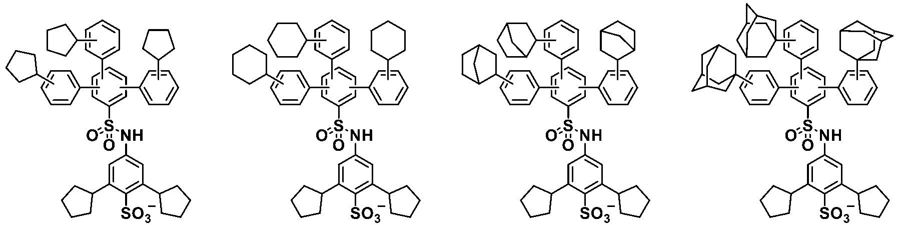

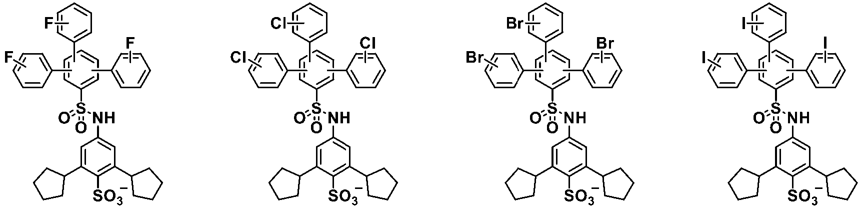

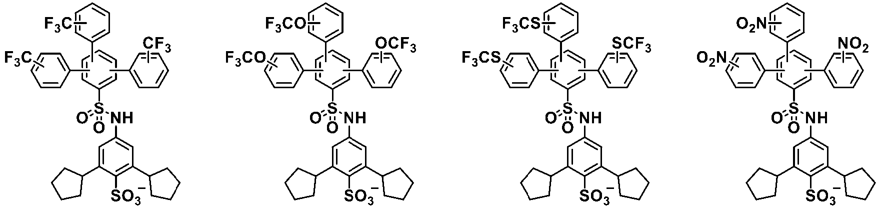

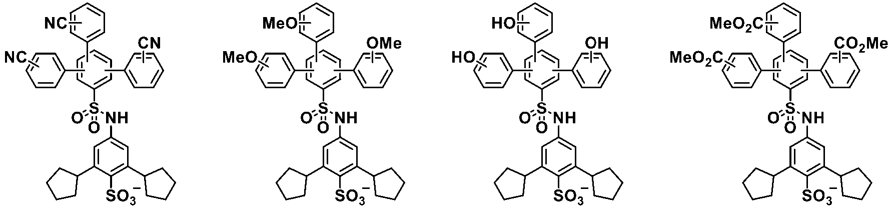

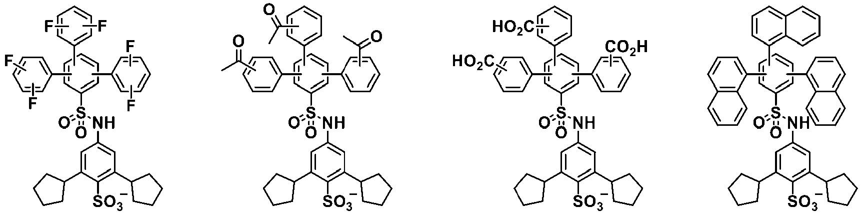

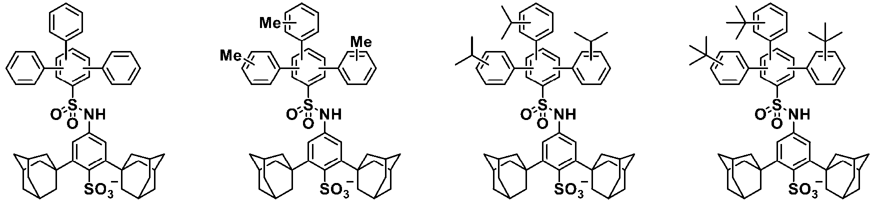

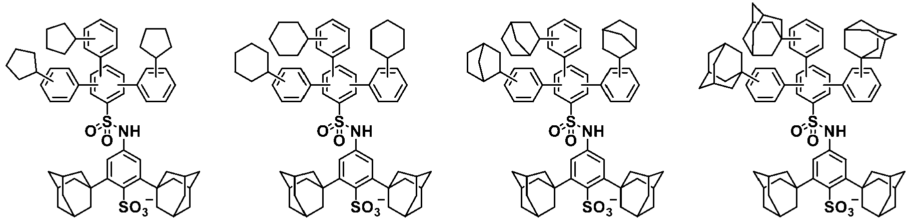

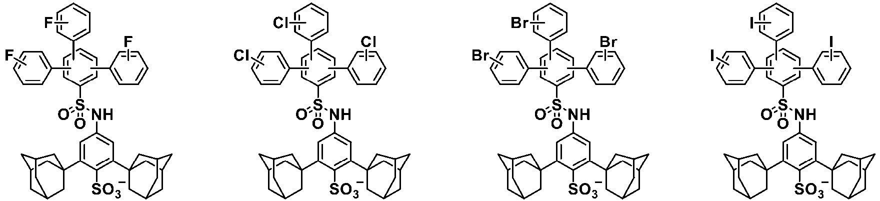

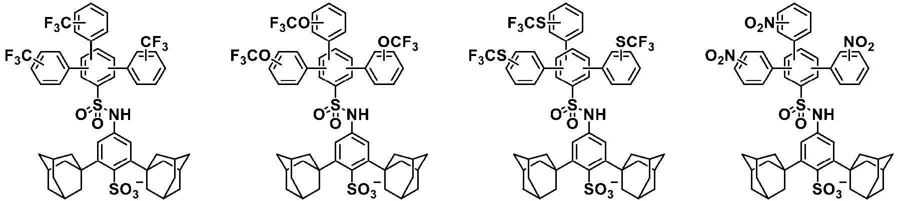

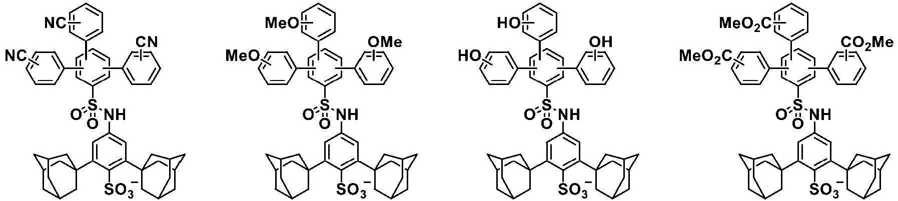

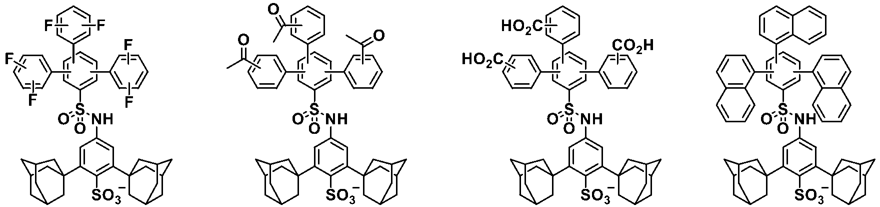

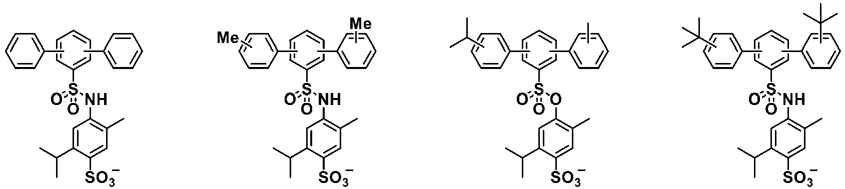

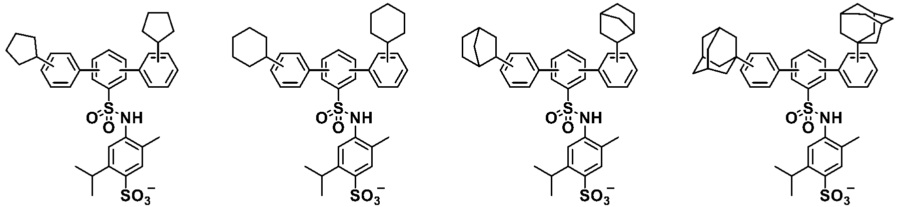

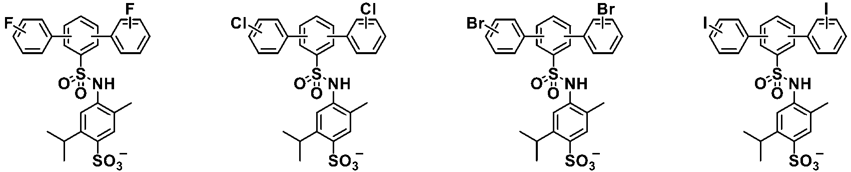

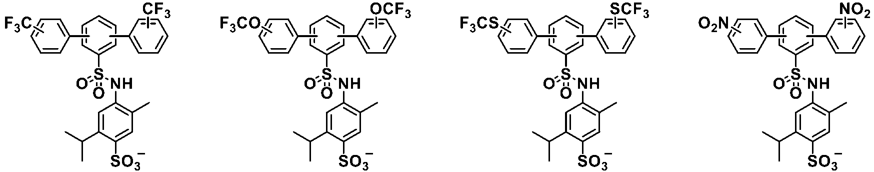

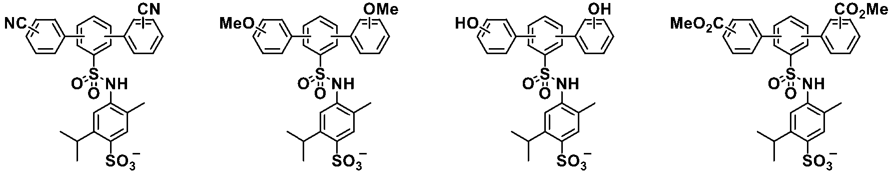

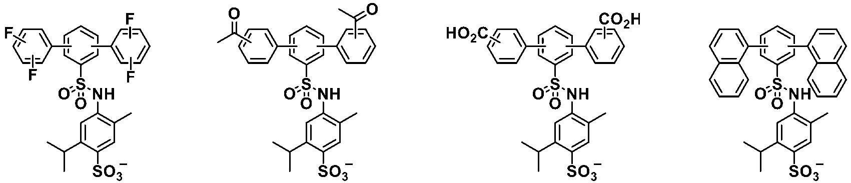

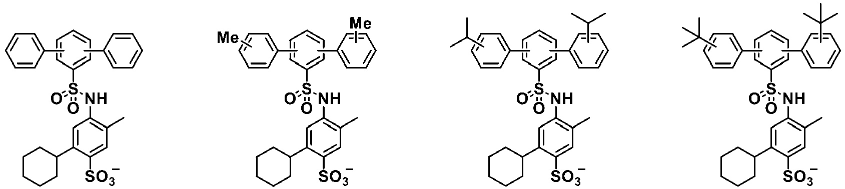

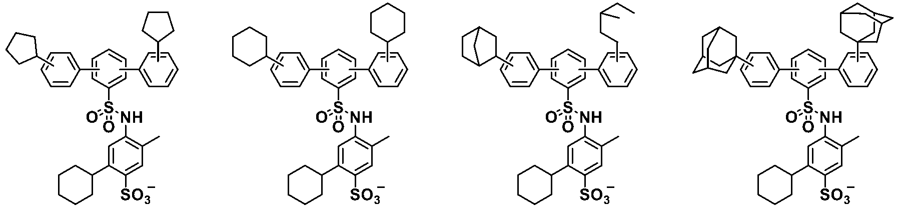

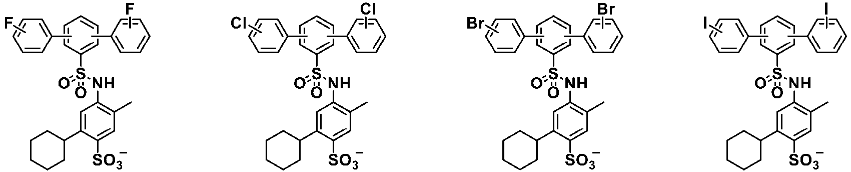

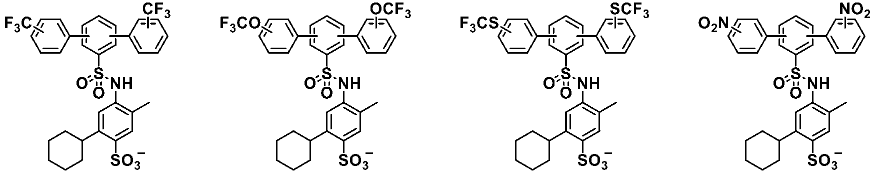

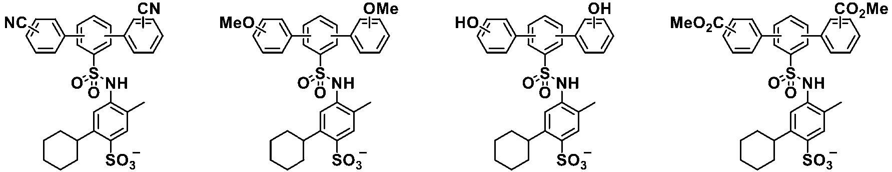

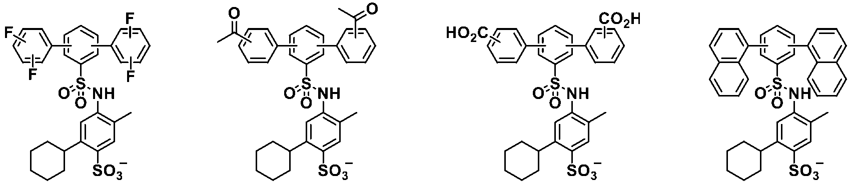

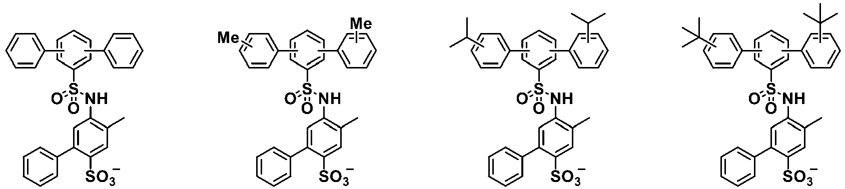

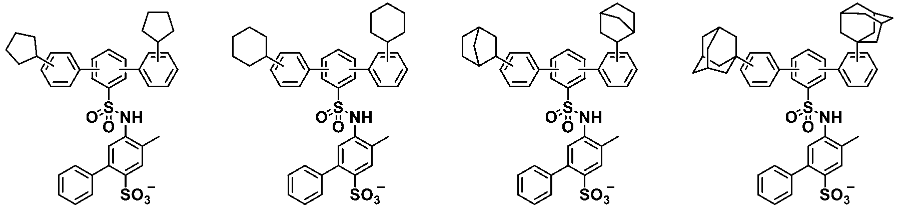

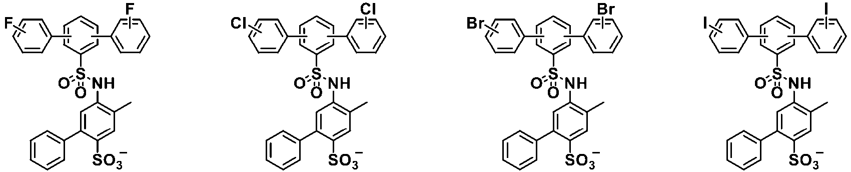

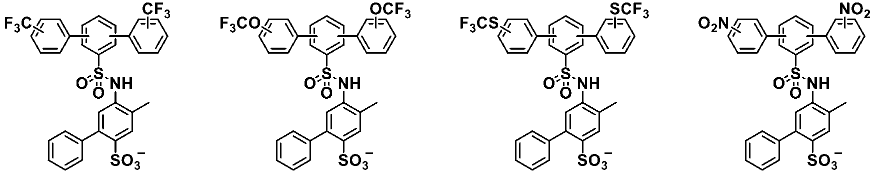

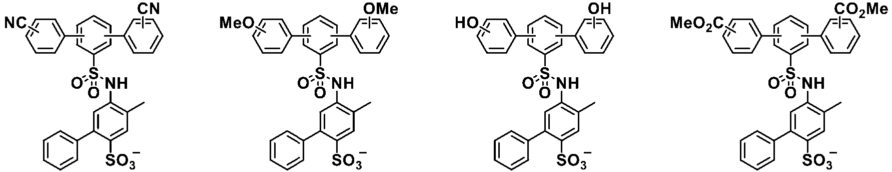

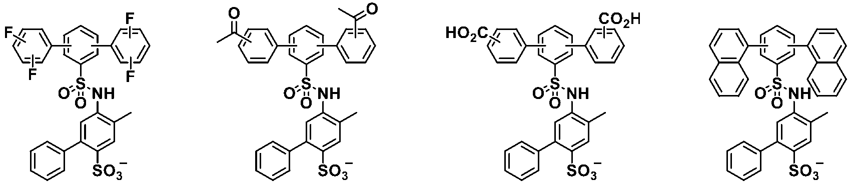

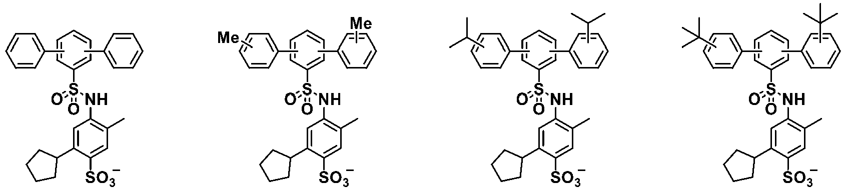

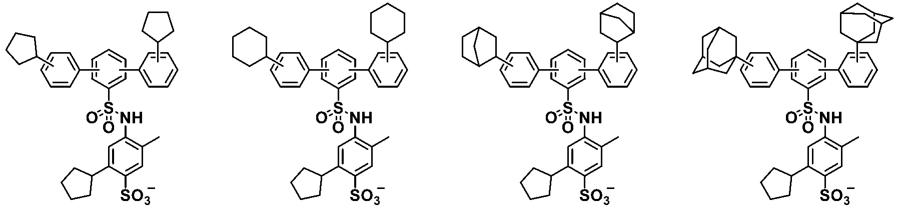

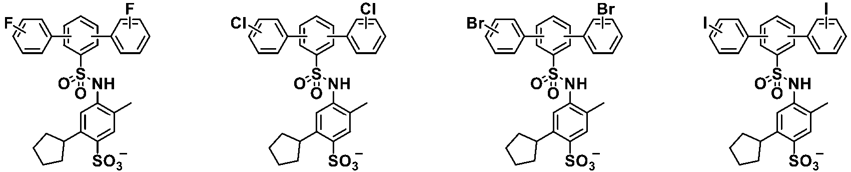

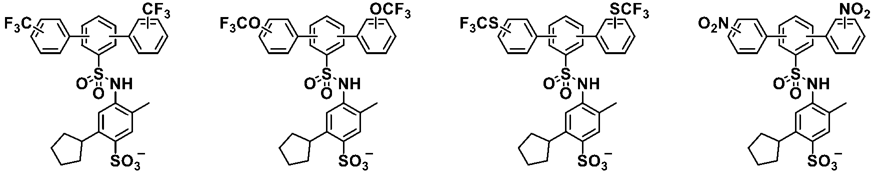

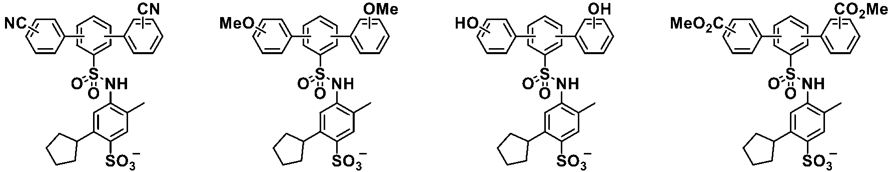

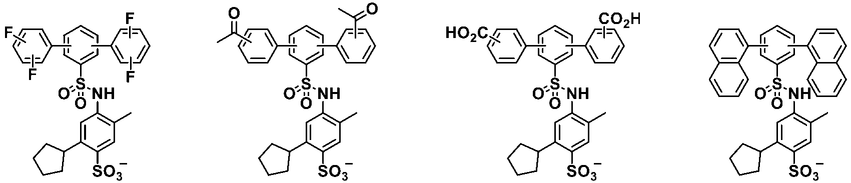

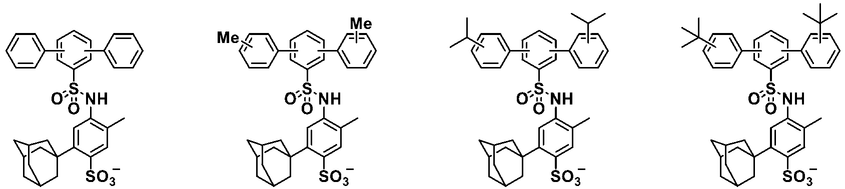

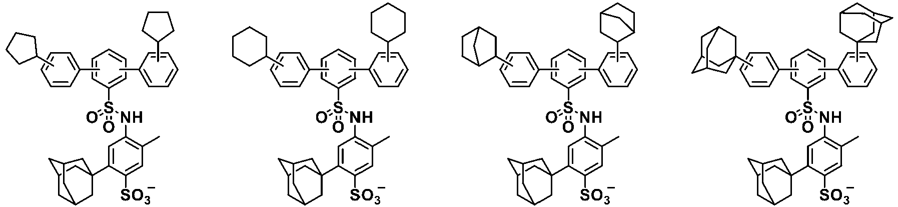

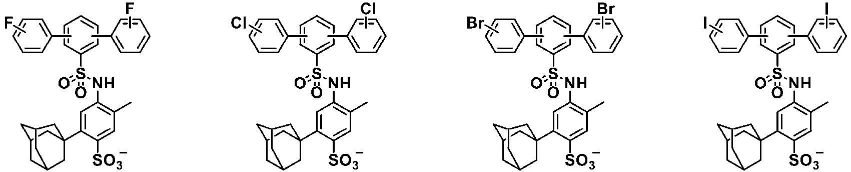

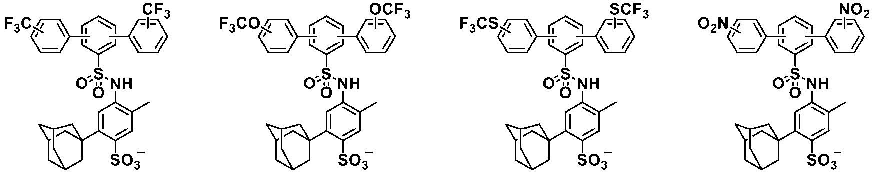

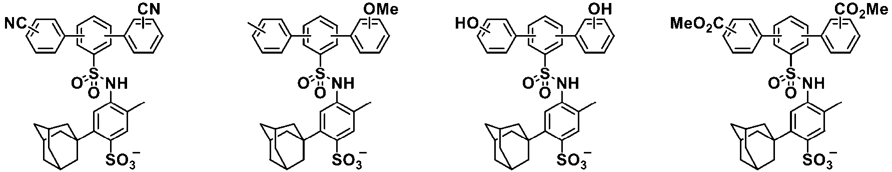

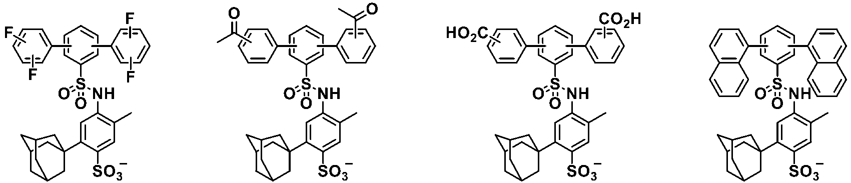

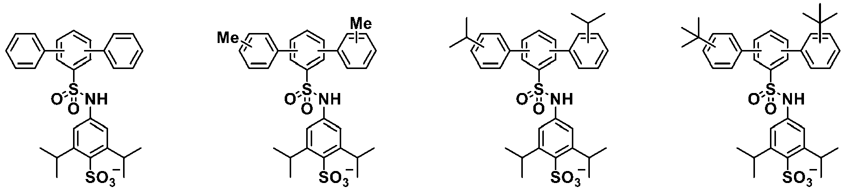

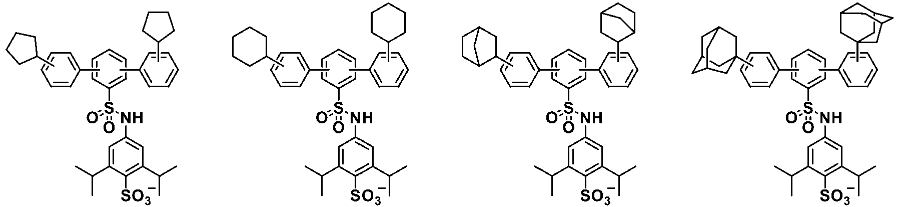

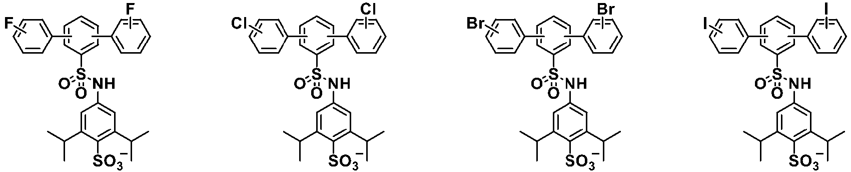

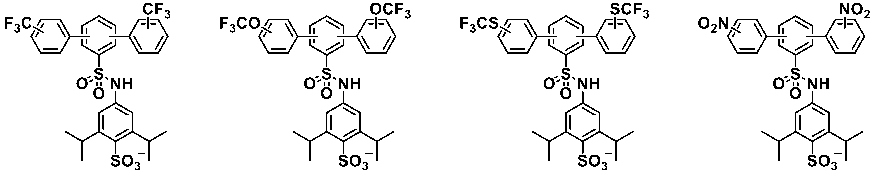

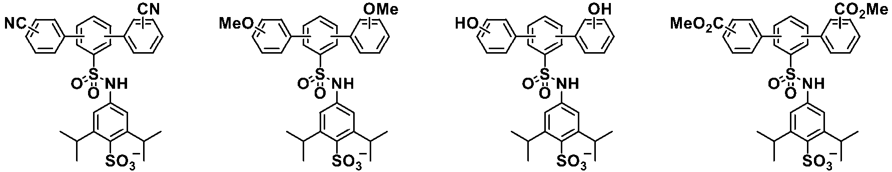

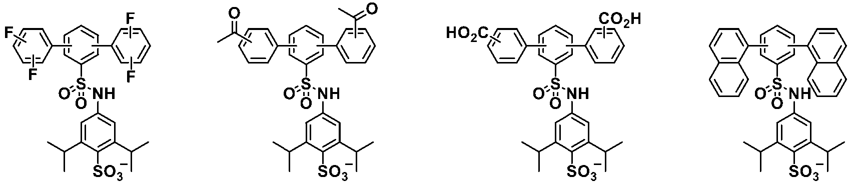

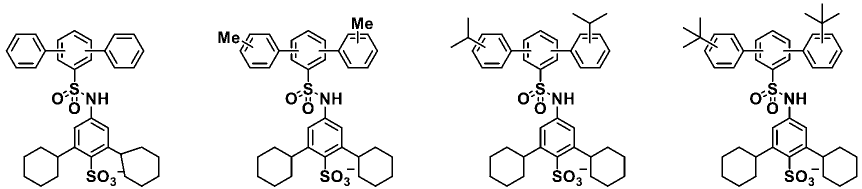

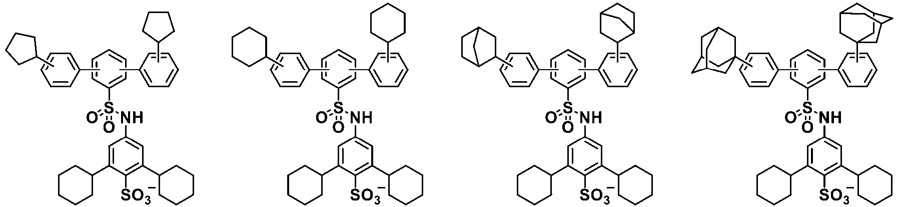

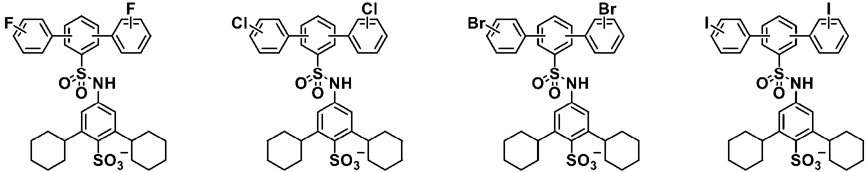

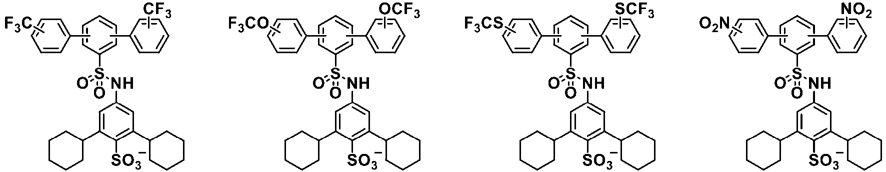

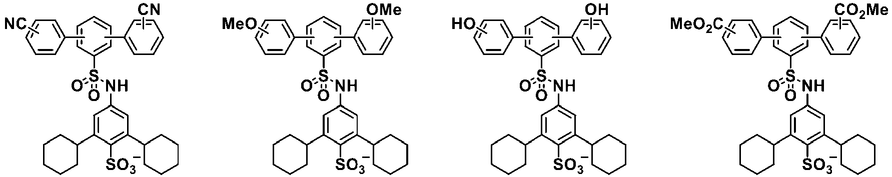

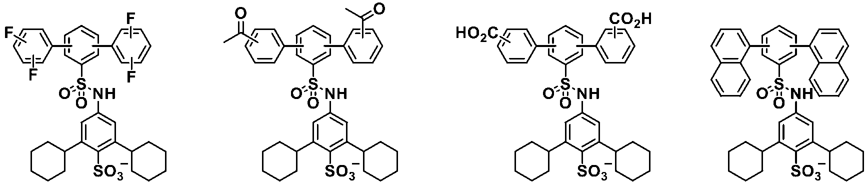

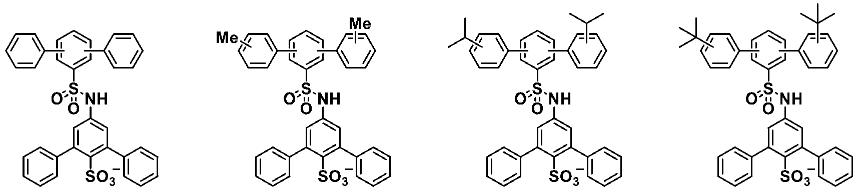

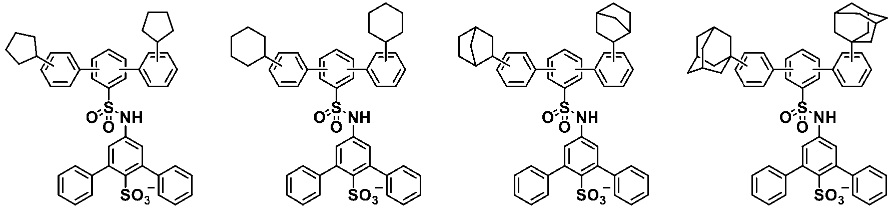

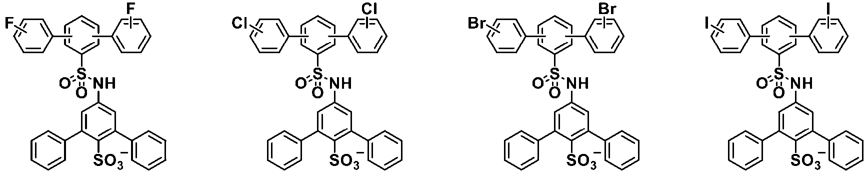

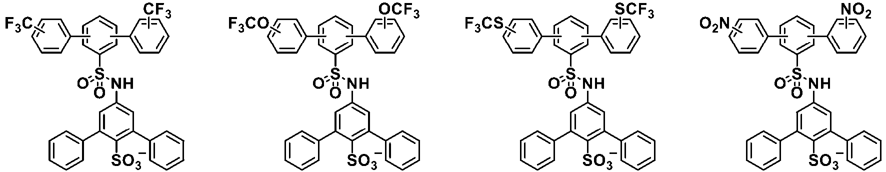

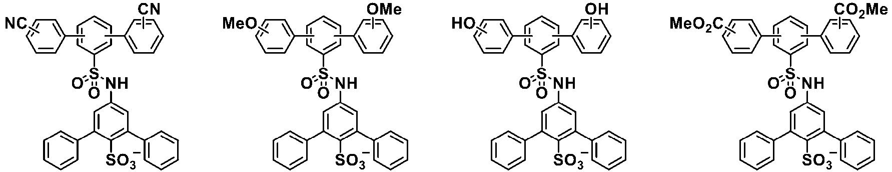

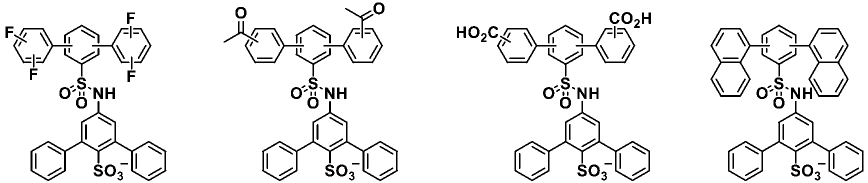

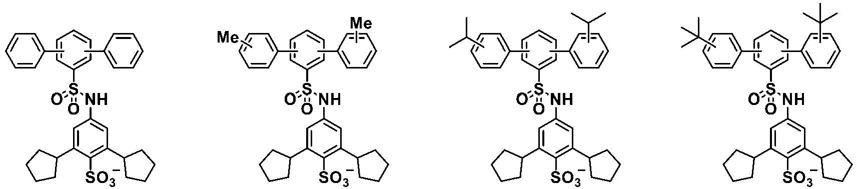

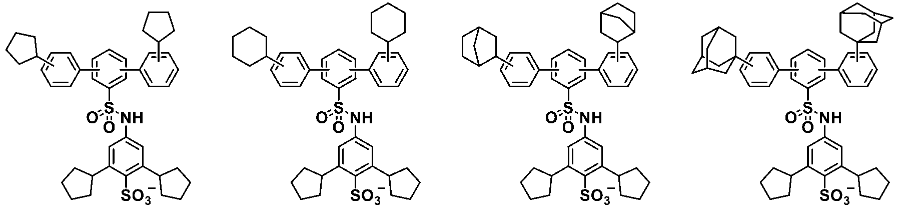

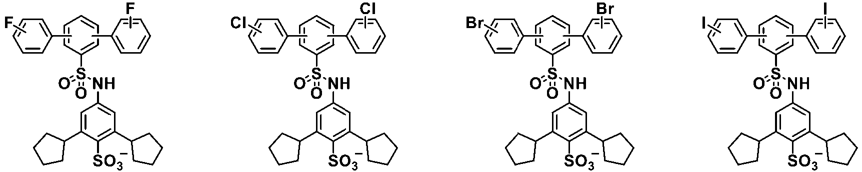

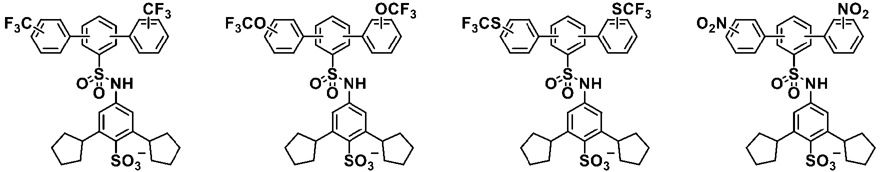

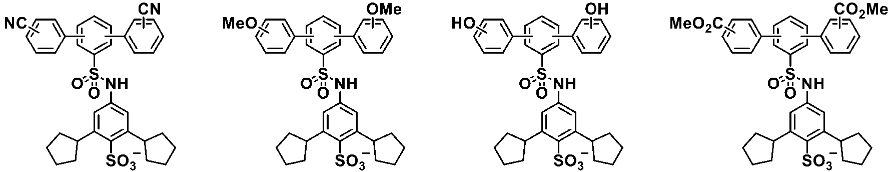

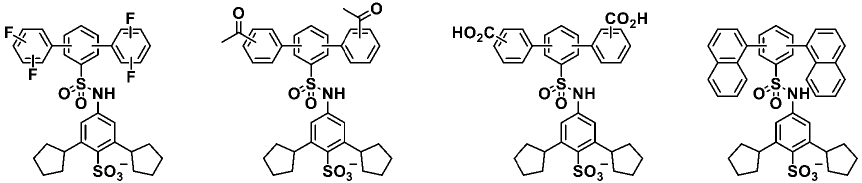

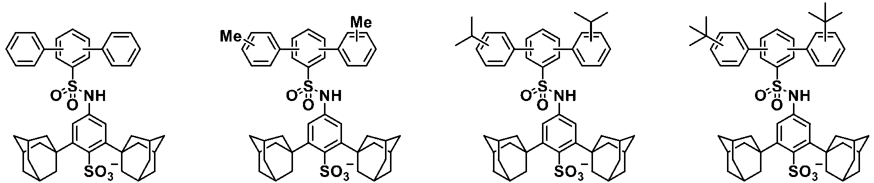

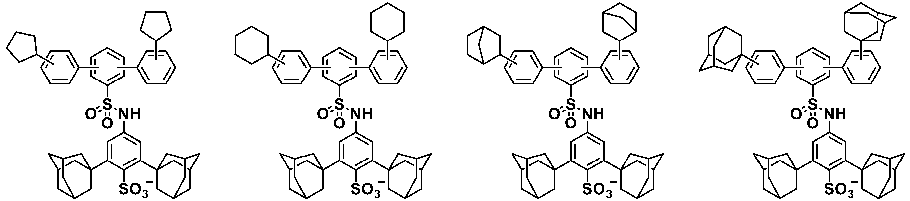

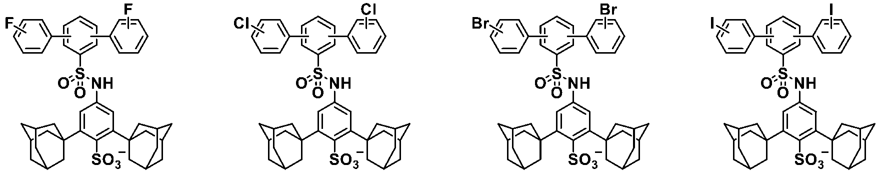

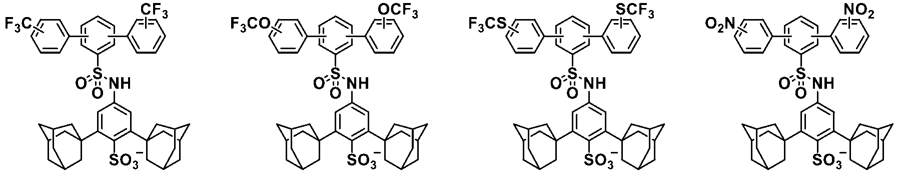

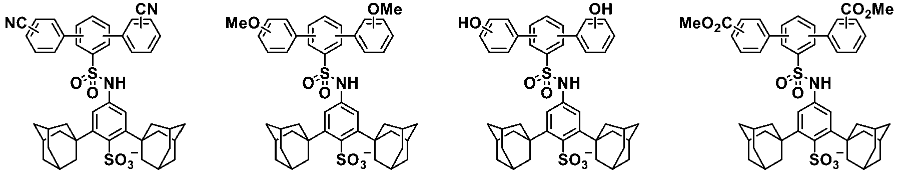

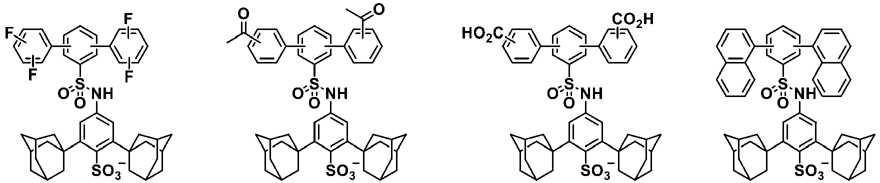

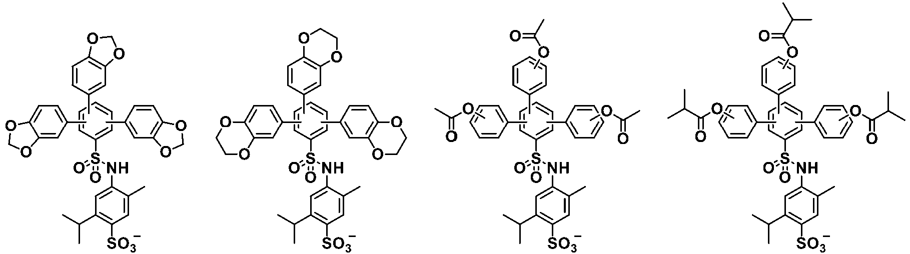

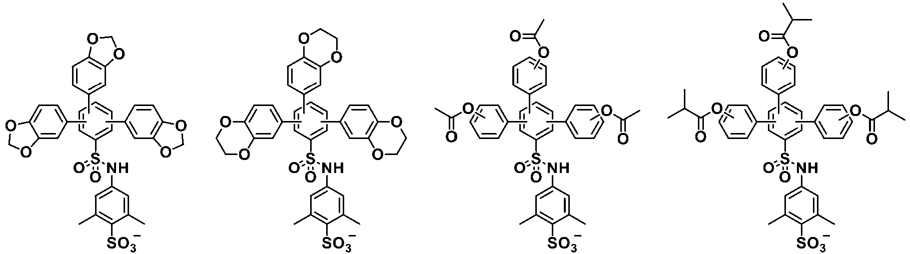

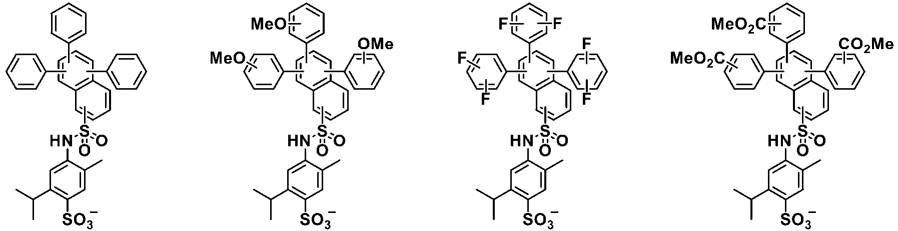

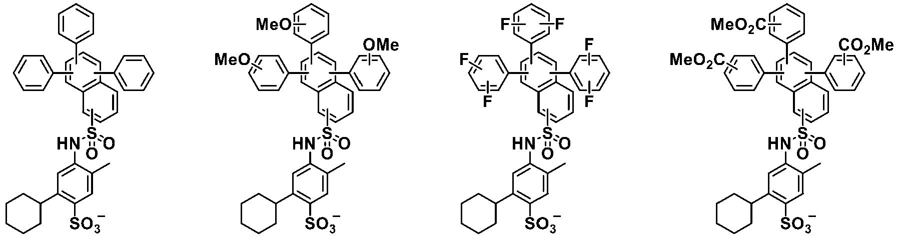

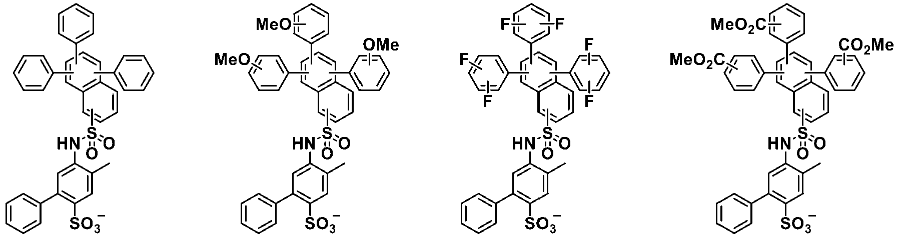

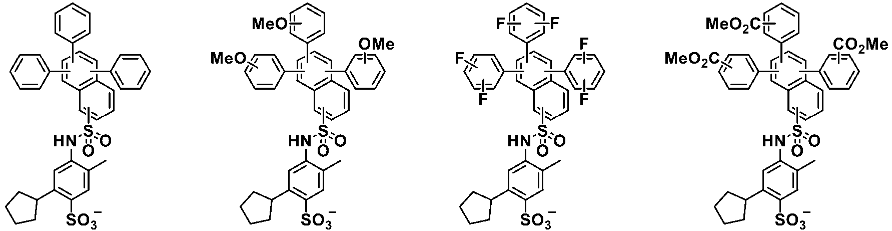

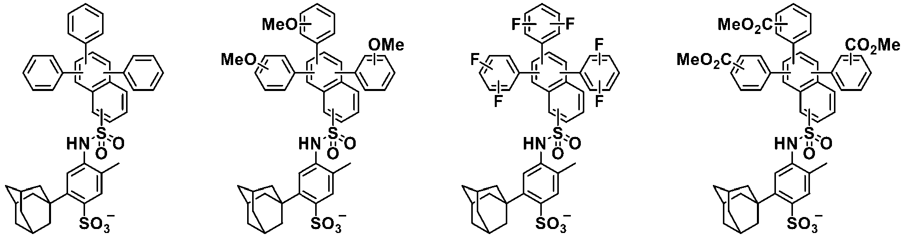

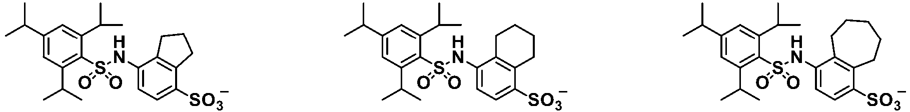

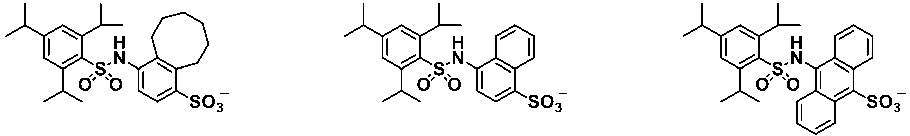

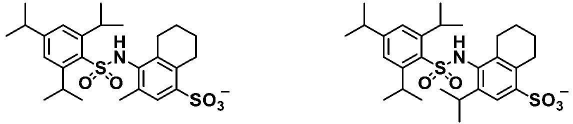

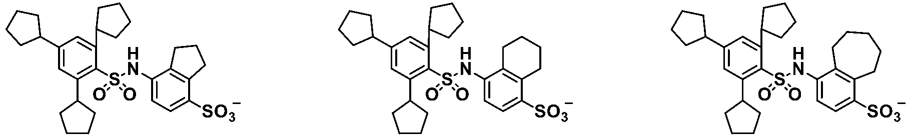

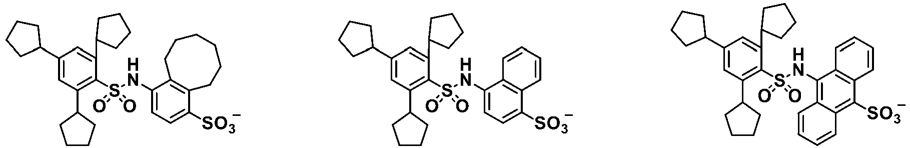

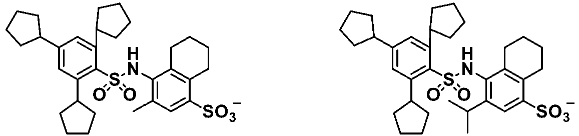

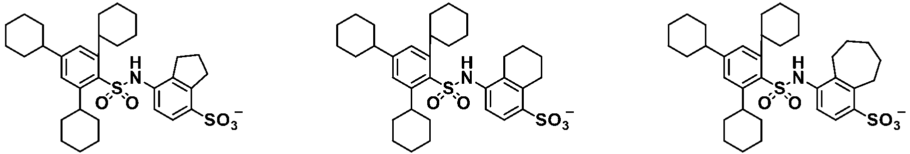

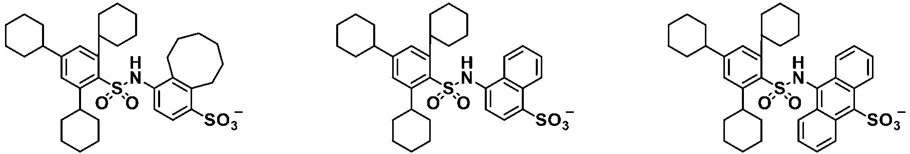

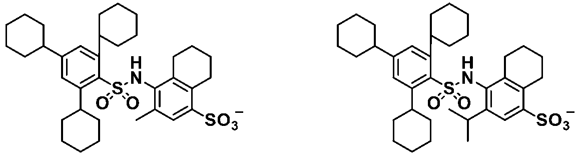

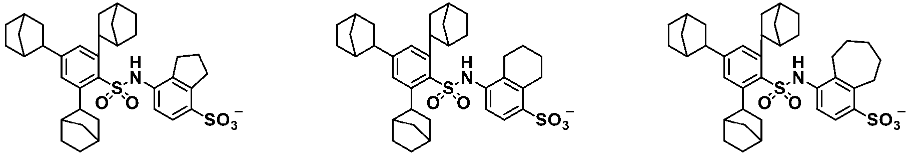

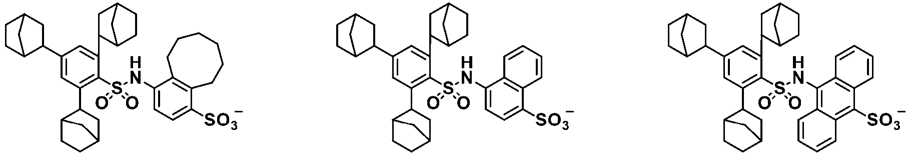

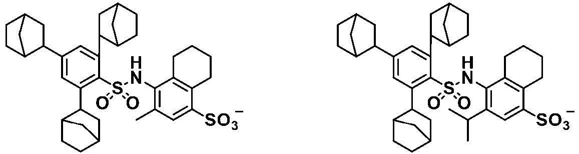

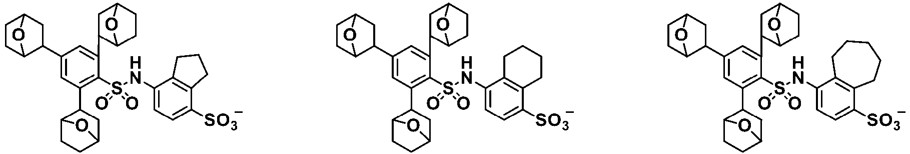

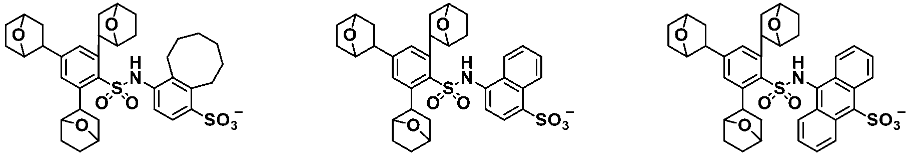

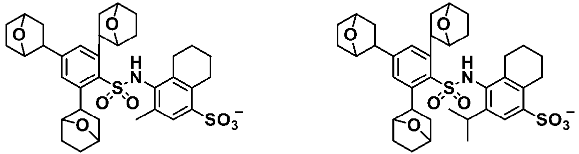

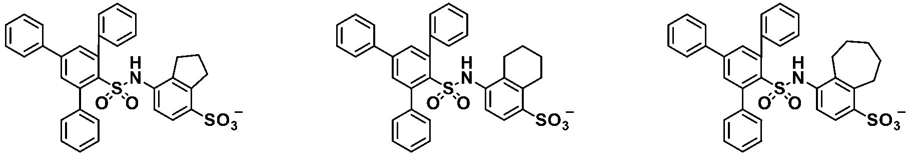

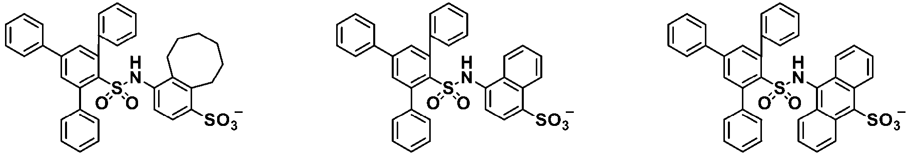

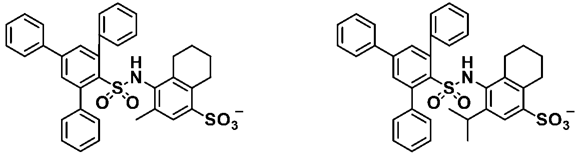

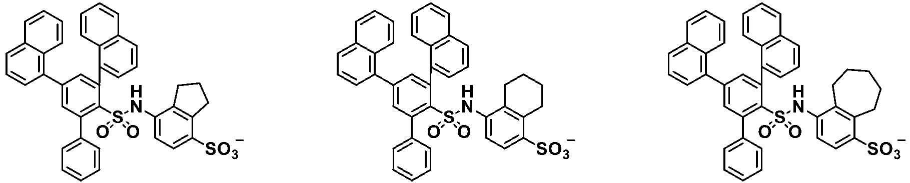

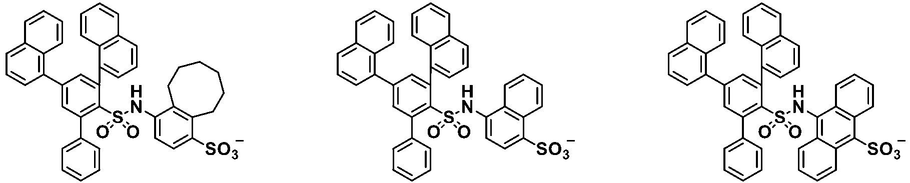

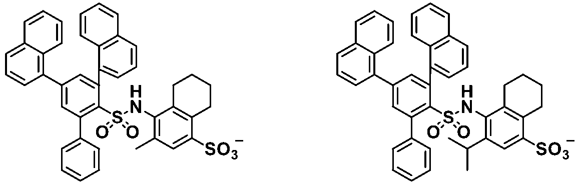

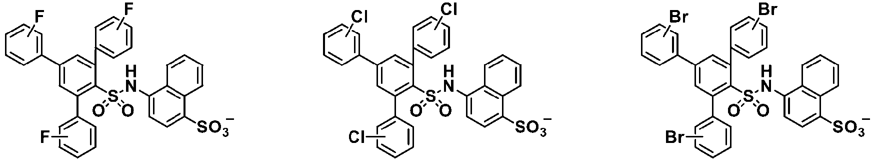

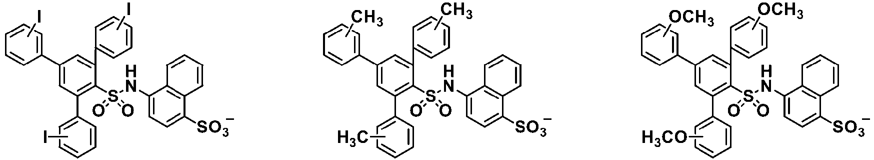

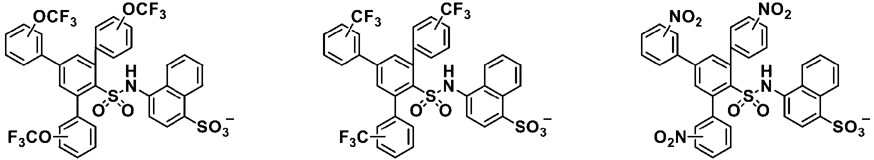

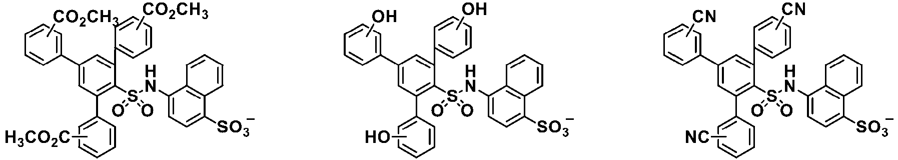

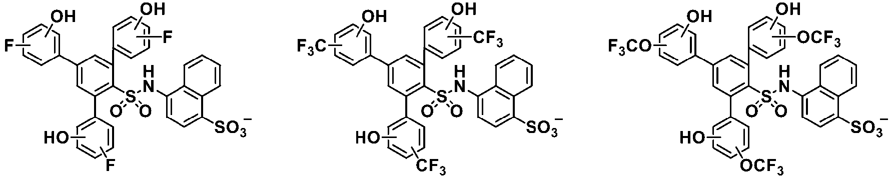

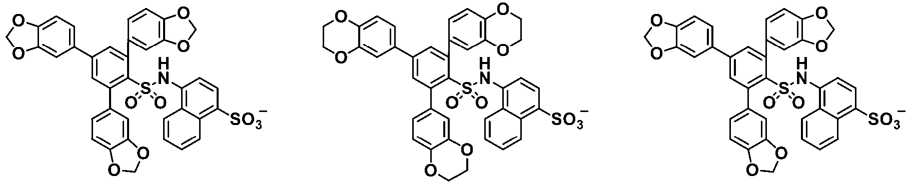

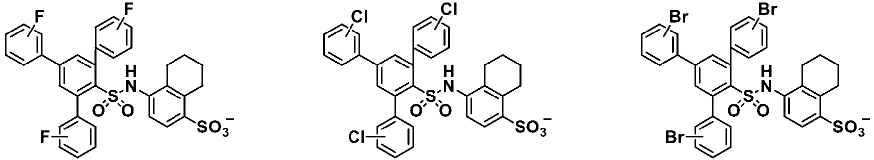

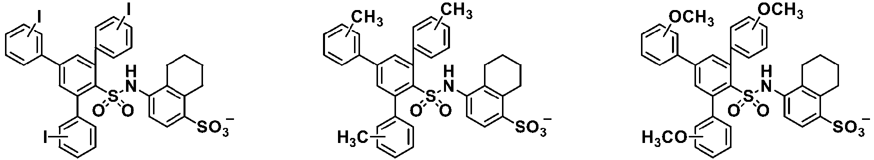

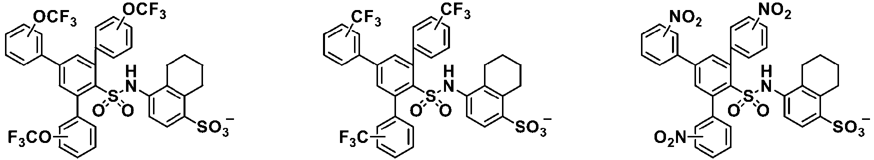

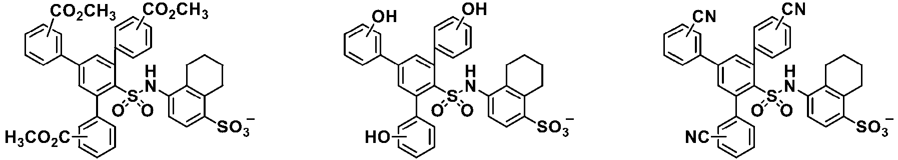

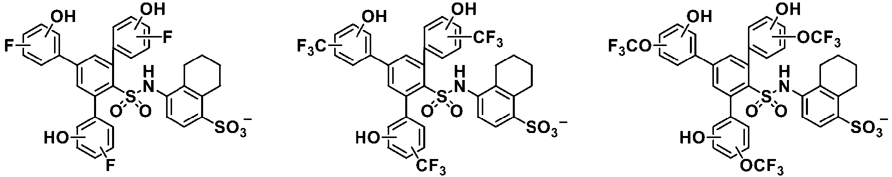

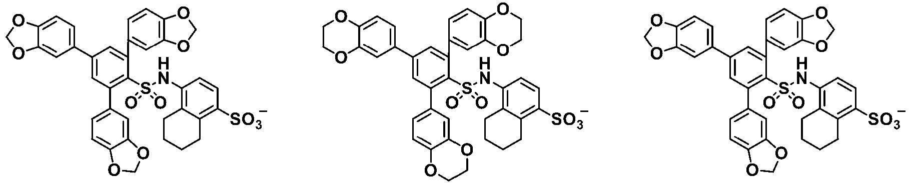

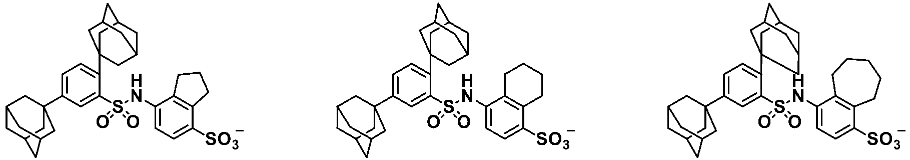

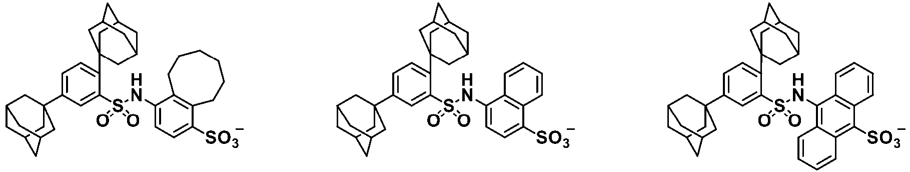

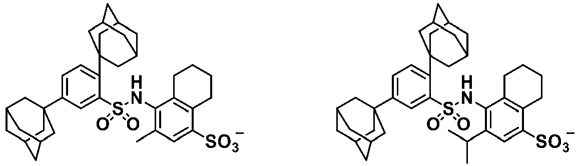

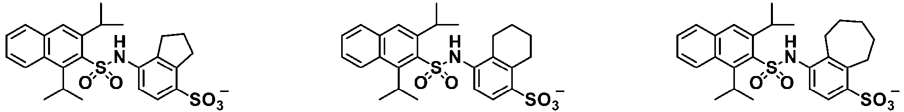

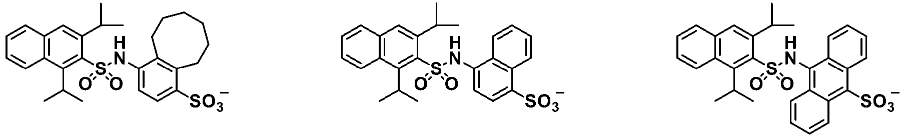

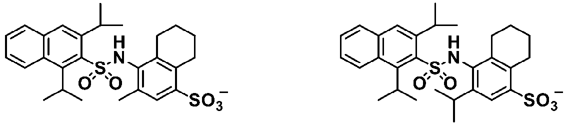

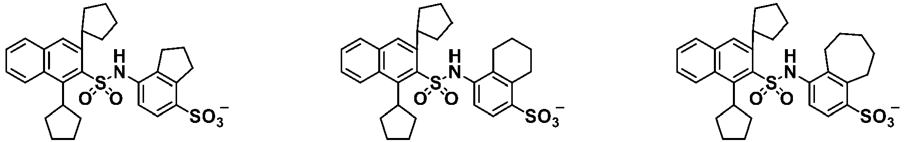

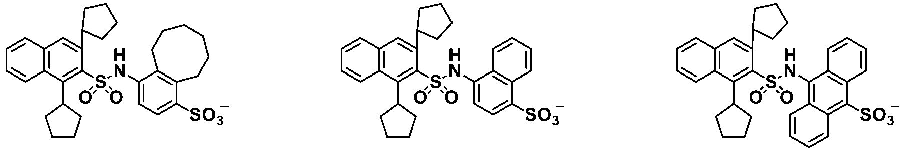

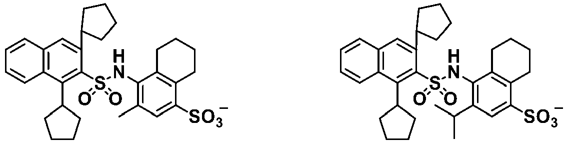

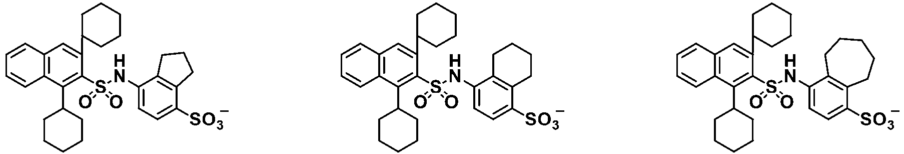

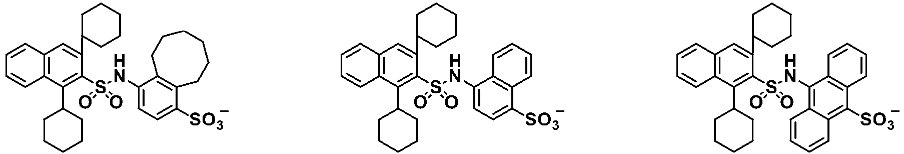

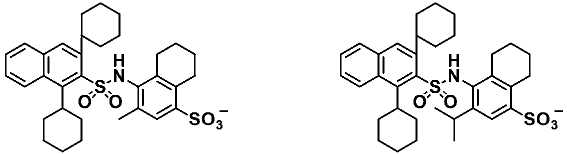

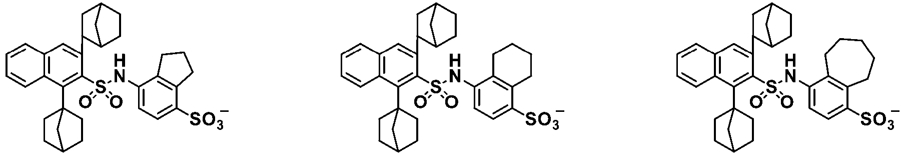

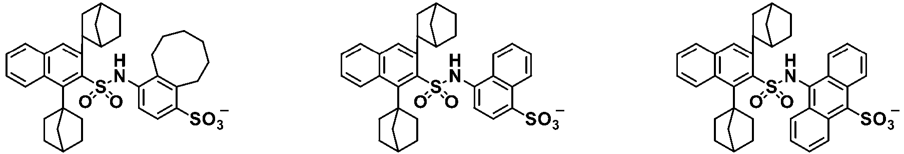

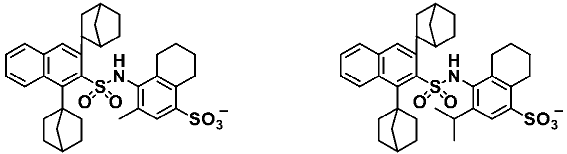

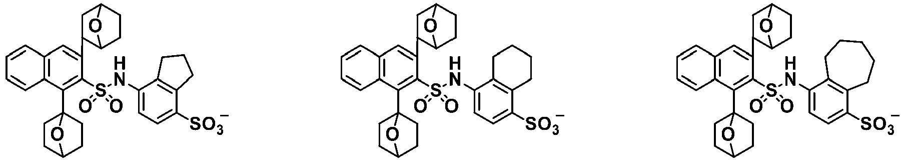

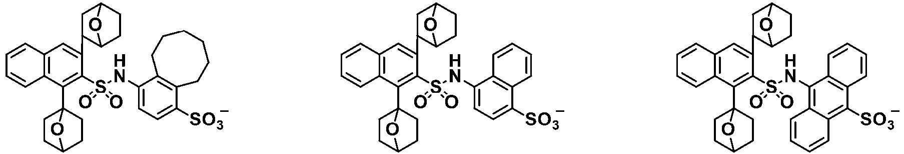

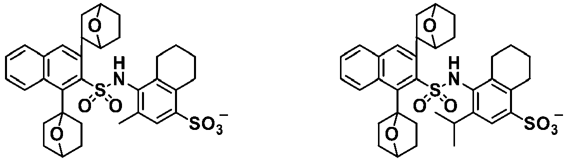

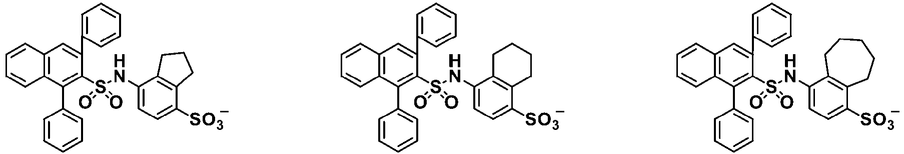

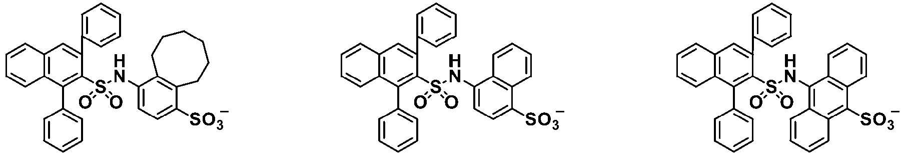

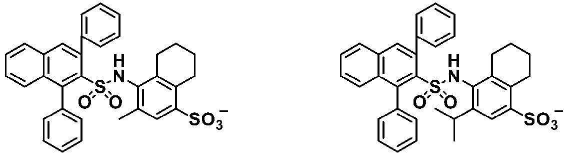

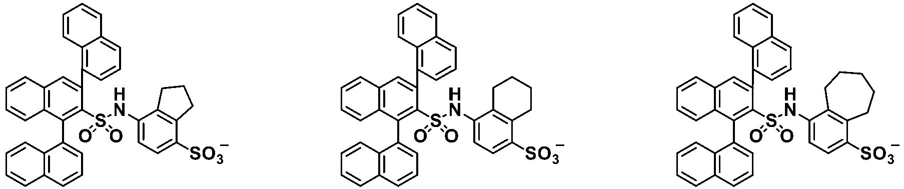

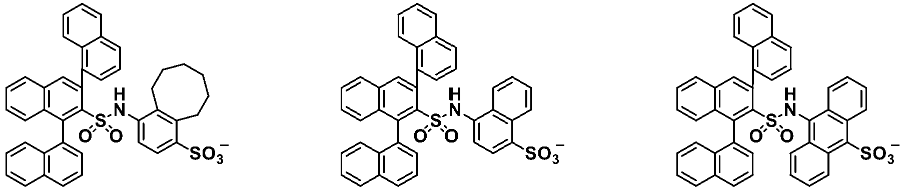

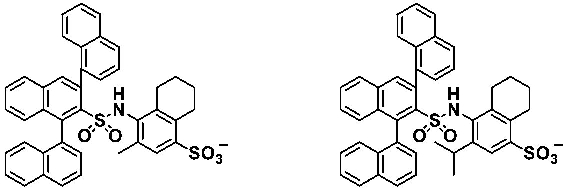

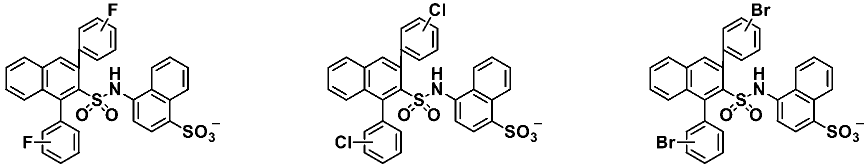

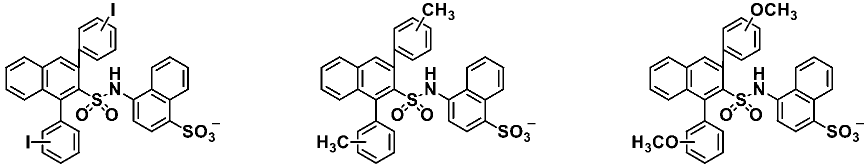

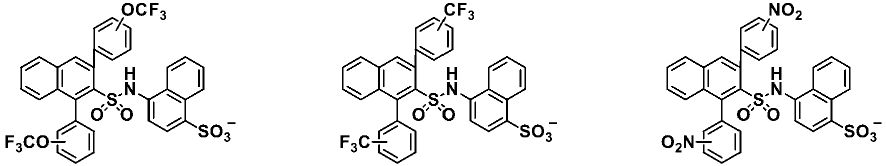

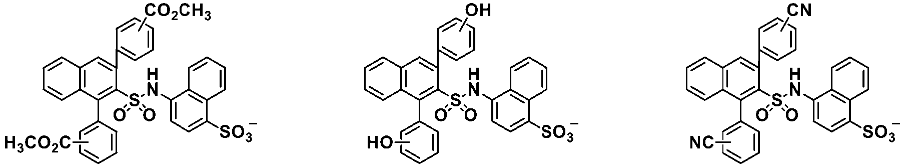

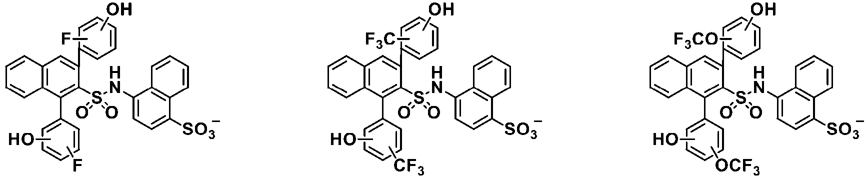

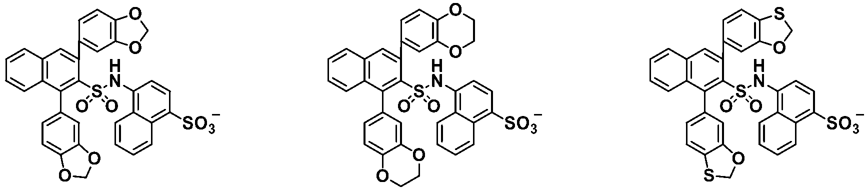

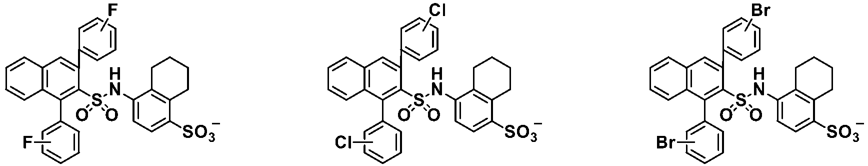

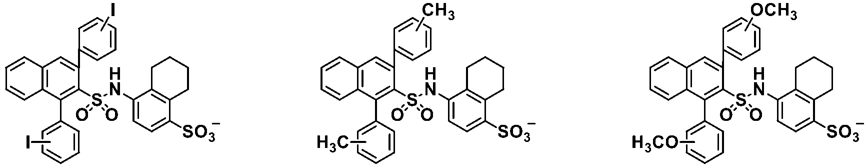

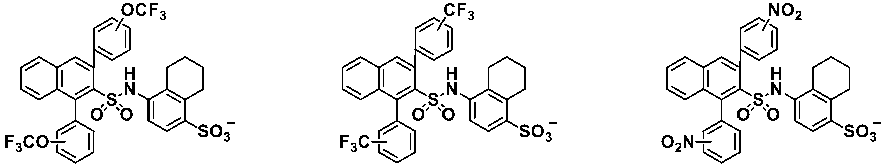

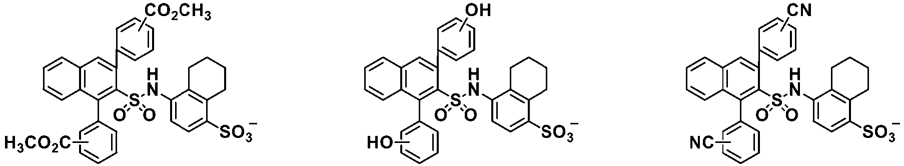

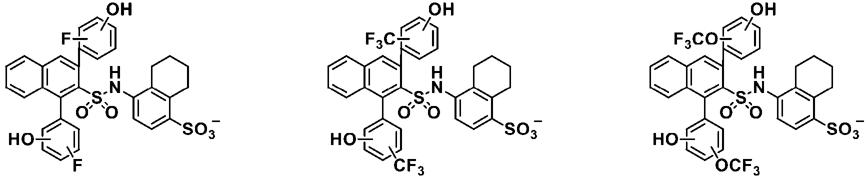

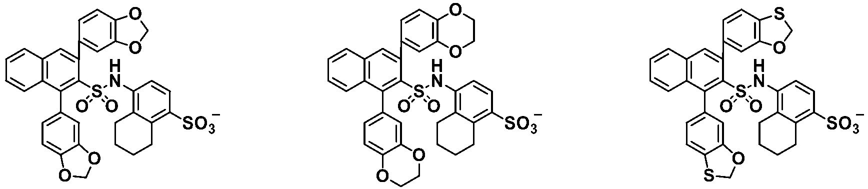

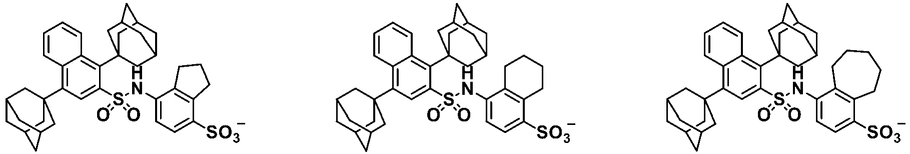

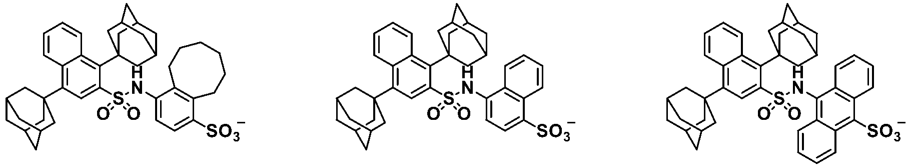

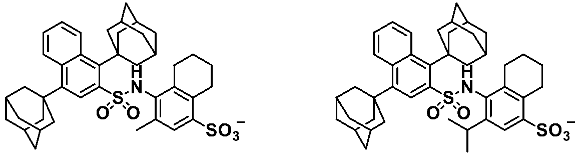

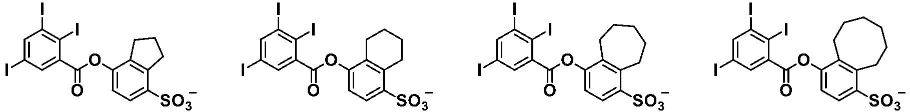

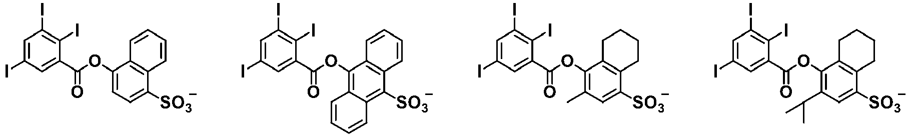

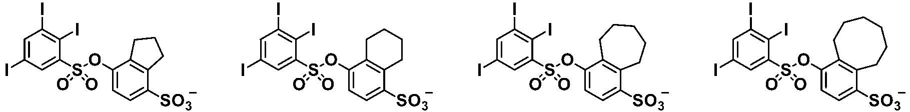

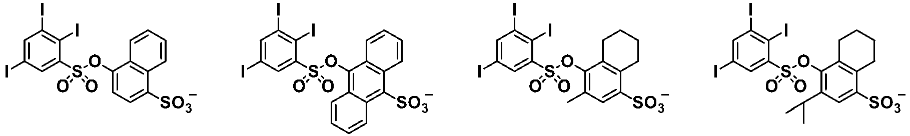

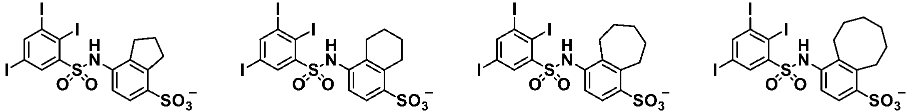

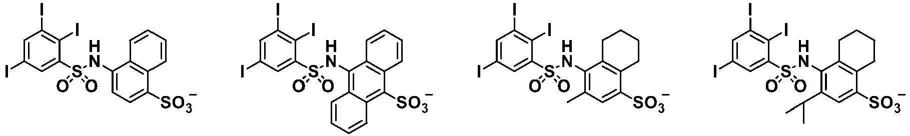

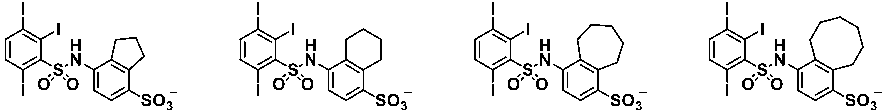

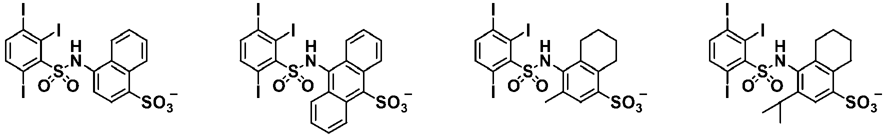

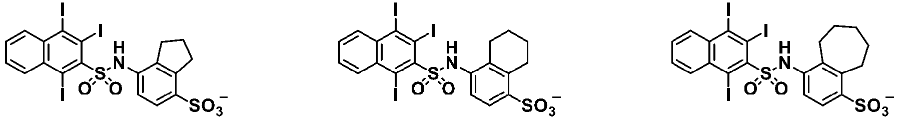

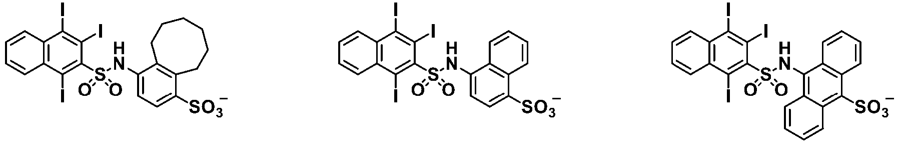

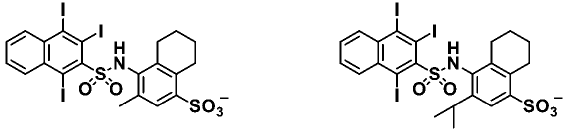

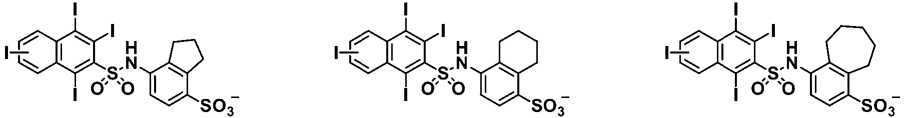

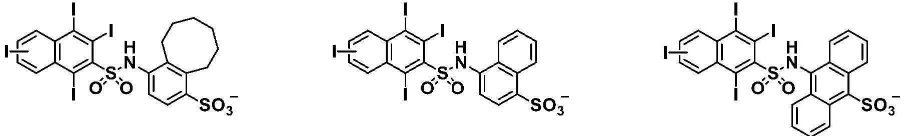

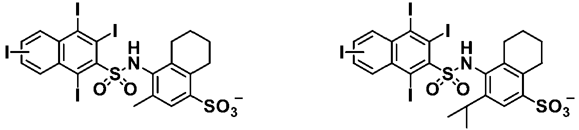

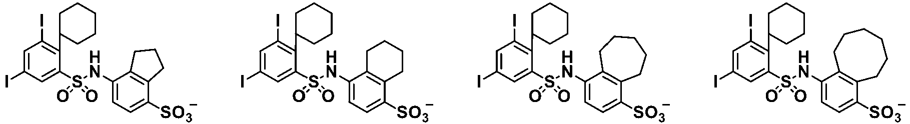

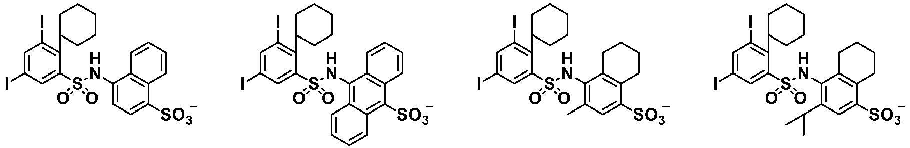

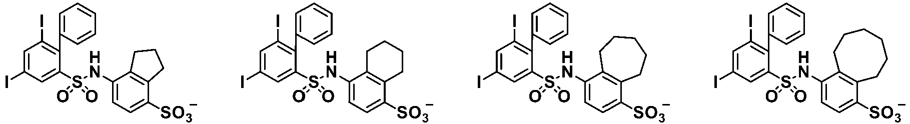

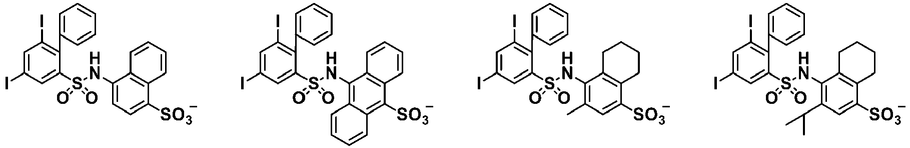

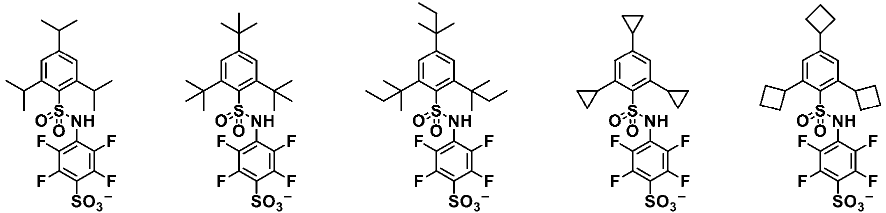

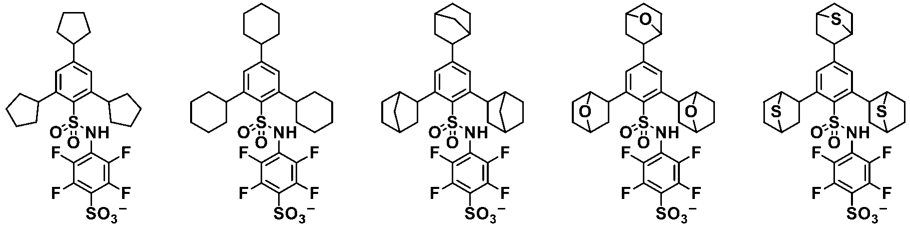

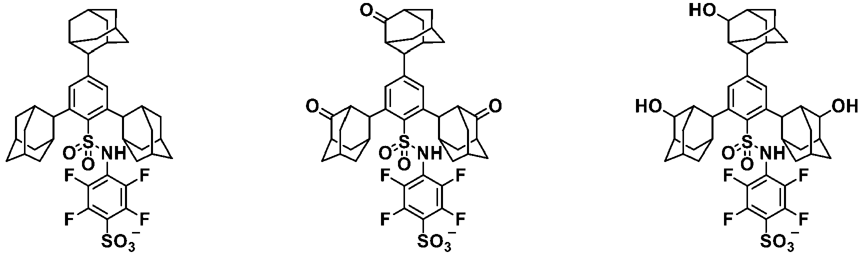

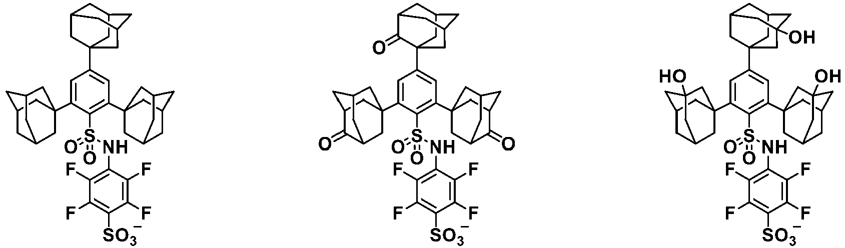

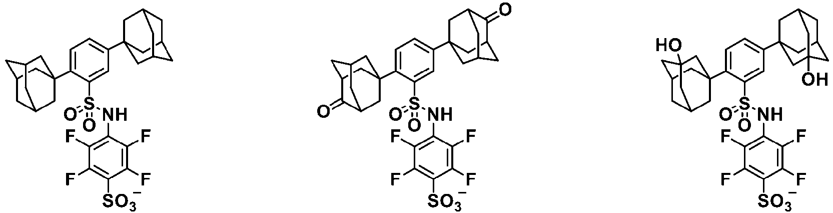

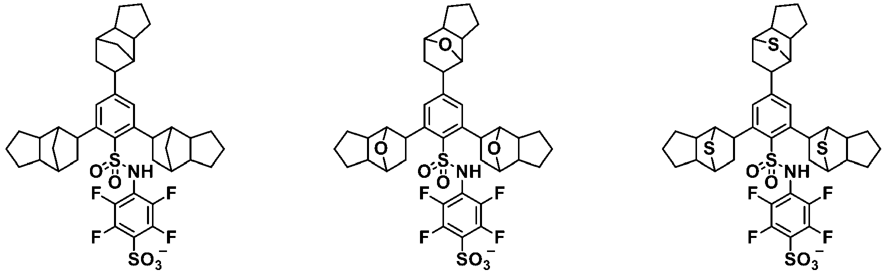

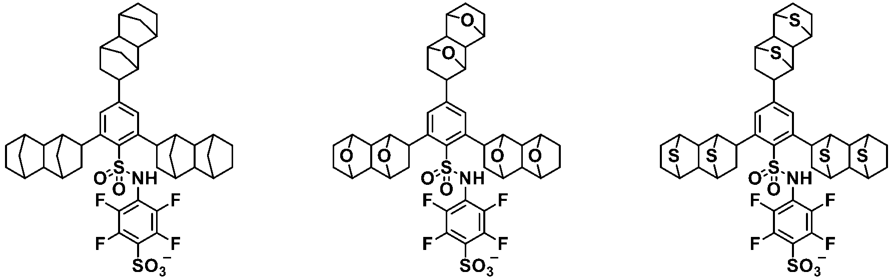

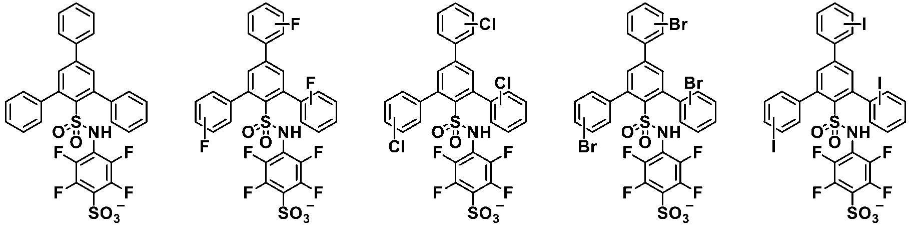

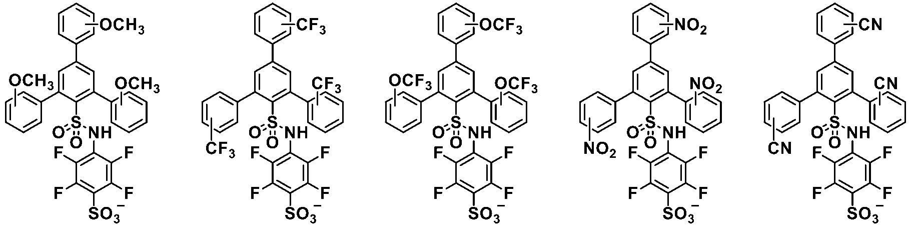

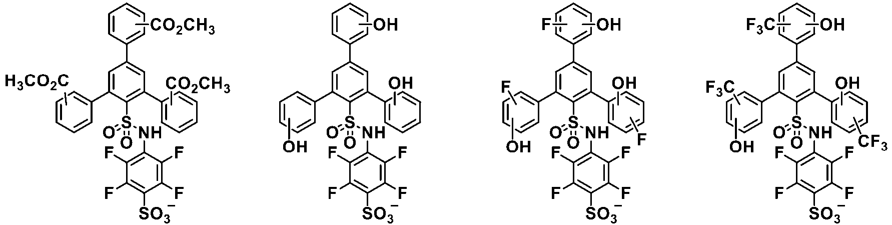

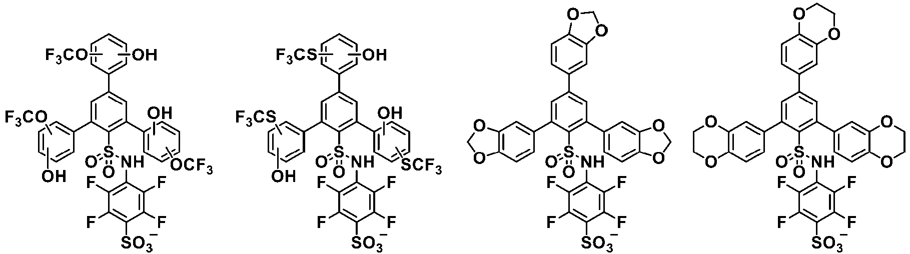

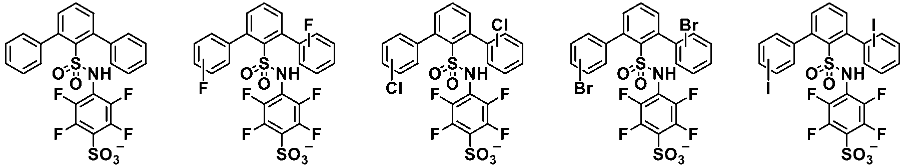

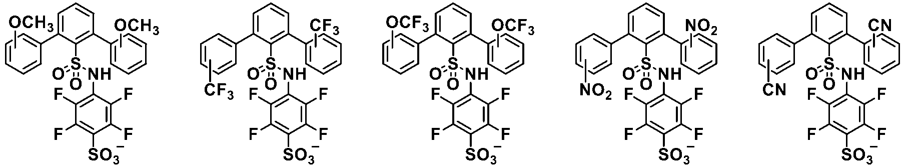

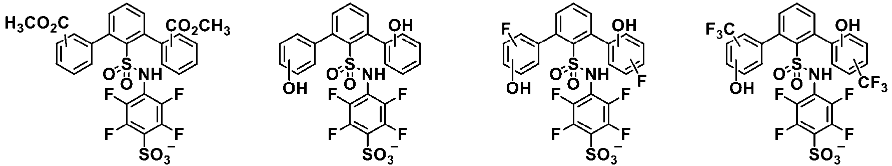

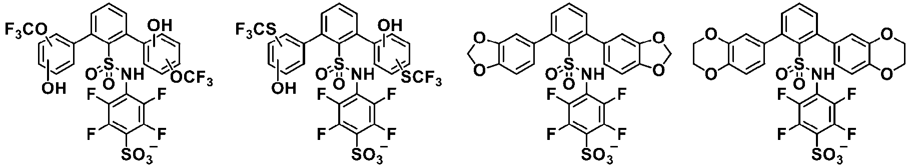

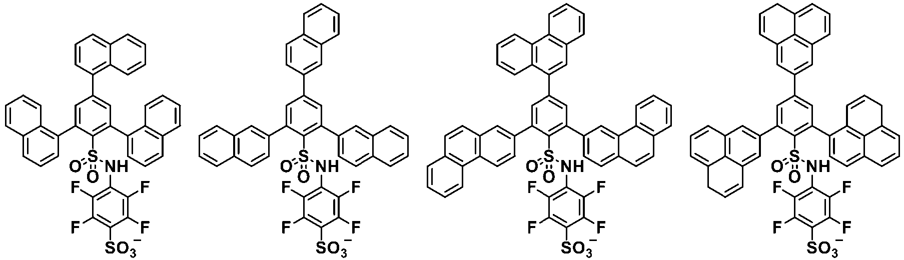

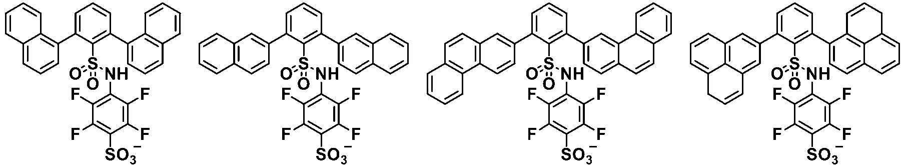

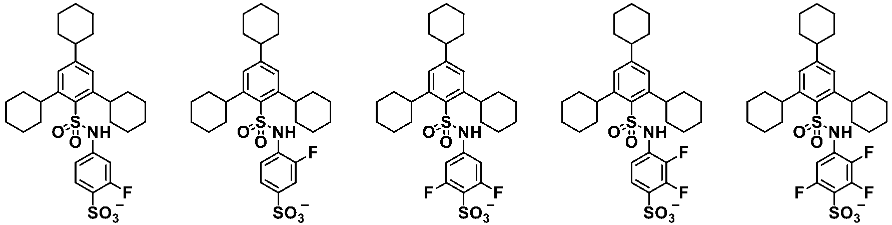

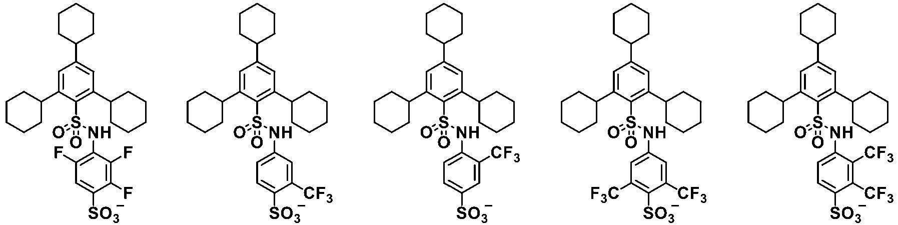

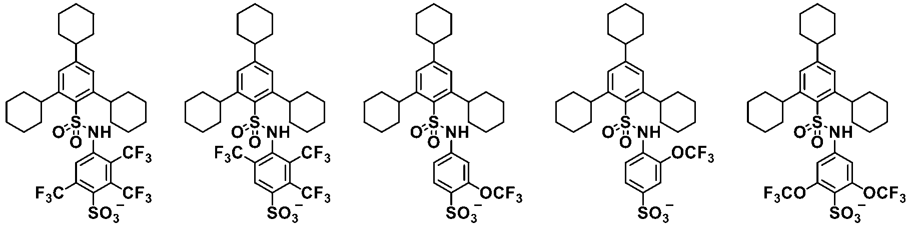

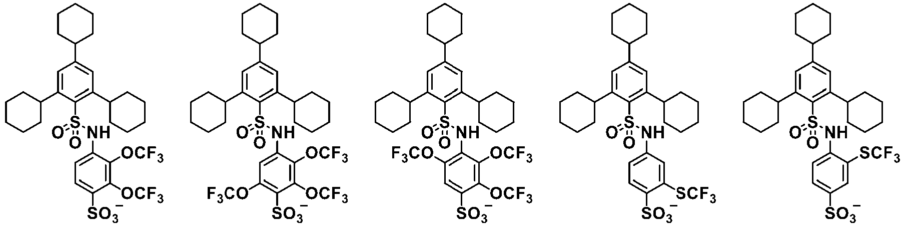

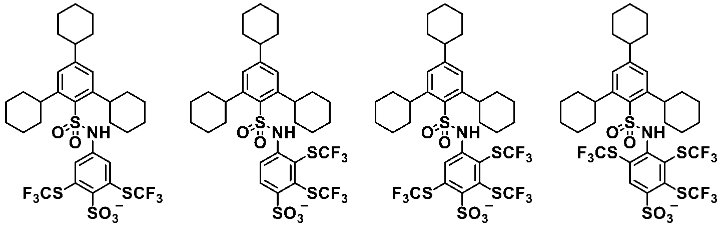

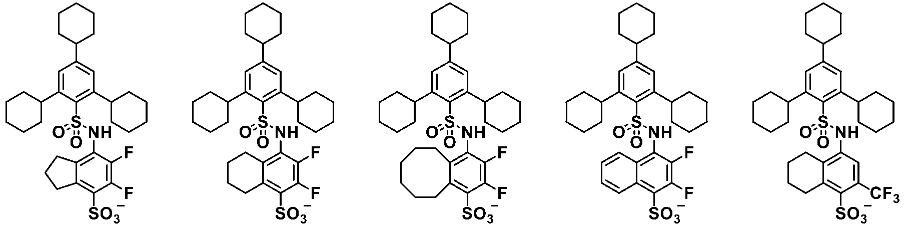

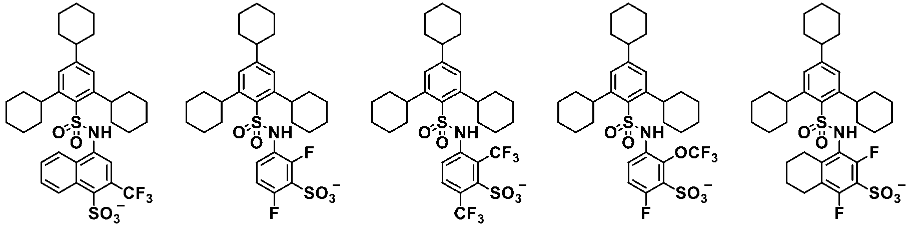

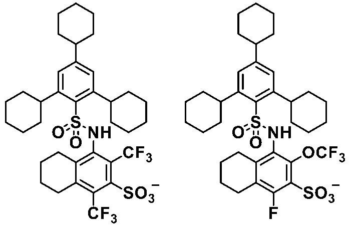

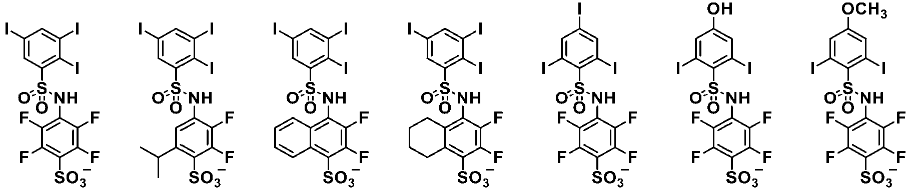

In formula (A), Z⁺ is an onium cation, preferably sulfonium cation having the formula (Z-1) or iodonium cation having the formula (Z-2).

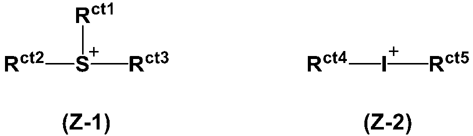

In formulae (Z-1) and (Z-2), R^{ct1} to R^{ct5} are each independently halogen or a C₁-C₃₀ hydrocarbyl group which may contain a heteroatom. Suitable halogen atoms include fluorine, chlorine, bromine and iodine. The hydrocarbyl group may be saturated or unsaturated and straight, branched or cyclic. Examples thereof include C₁-C₃₀ alkyl groups such as methyl, ethyl, n-propyl, isopropyl, n-butyl, isobutyl, sec-butyl, tert-butyl; C₃-C₃₀ cyclic saturated hydrocarbyl groups such as cyclopropyl, cyclopentyl, cyclohexyl, cyclopropylmethyl, 4-methylcyclohexyl, cyclohexylmethyl, norbornyl, adamantyl; C₂-C₃₀ alkenyl groups such as vinyl, 1-propenyl, 2-propenyl, butenyl, hexenyl; C₃-C₃₀ cyclic unsaturated hydrocarbyl groups such as cyclohexenyl; C₆-C₃₀ aryl groups such as phenyl, naphthyl, thienyl; C₇-C₃₀ aralkyl groups such as benzyl, 1-phenylethyl, 2-phenylethyl, and combinations thereof. Inter alia, the aryl groups are preferred. In the hydrocarbyl groups, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy, fluorine, chlorine, bromine, iodine, cyano, nitro, carbonyl, ether bond, ester bond, sulfonate ester bond, carbonate bond, lactone ring, sultone ring, carboxylic anhydride (-C(=O)-O-C(=O)-) or haloalkyl moiety.

Also, R^{ct1} and R^{ct2} may bond together to form a ring with the sulfur atom to which they are attached. Exemplary structures of the ring are shown below.

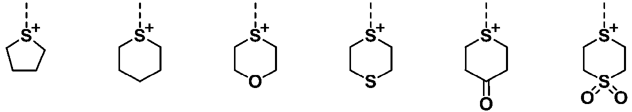

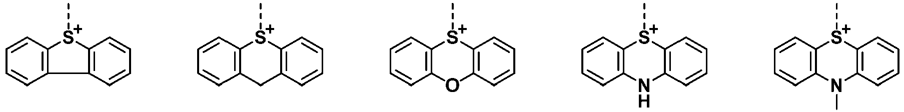

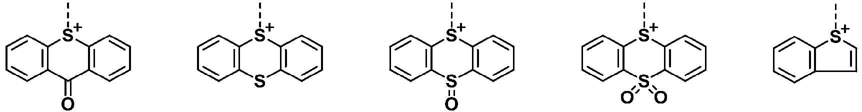

The broken line designates a point of attachment to R^{ct3}.

Examples of the sulfonium cation having formula (Z-1) include the cations described in JP-A 2024-003744, paragraphs [0102]-[0125] and JP-A 2023-169812, paragraphs [0070]-[0085], but are not limited thereto.

Examples of the iodonium cation having formula (Z-2) include the cations described in JP-A 2024-000259, paragraph [0181], but are not limited thereto.

A sulfonium cation having the formula (Z-3) is also preferable as the onium cation Z⁺.

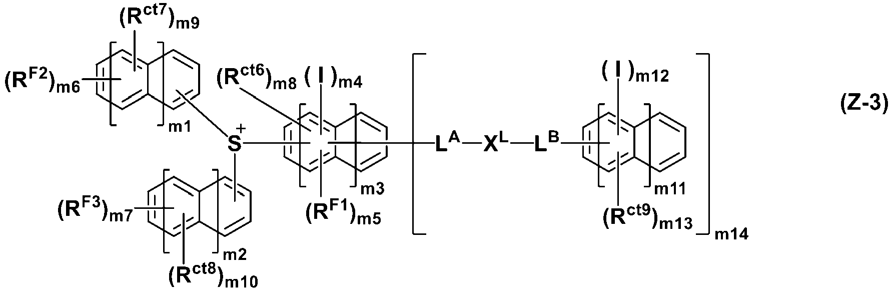

In formula (Z-3), m1 is 0 or 1. The relevant structure is a benzene ring when m1=0, and a naphthalene ring when m1=1. The benzene ring corresponding to m1=0 is preferred from the aspect of solvent solubility. The subscript m2 is 0 or 1. The relevant structure is a benzene ring when m2=0, and a naphthalene ring when m2=1. The benzene ring corresponding to m2=0 is preferred from the aspect of solvent solubility. The subscript m3 is 0 or 1. The relevant structure is a benzene ring when m3=0, and a naphthalene ring when m3=1. The benzene ring corresponding to m3=0 is preferred from the aspect of solvent solubility.

In formula (Z-3), m4 is 0, 1, 2, 3 or 4. As the number of iodine atoms in the cation structure increases, the compound becomes more absorptive to EUV, but so poor in solvent solubility that it may precipitate in a resist composition. For this reason, m4 is preferably 0, 1, 2 or 3, more preferably 0, 1 or 2.

In formula (Z-3), m5 is 0, 1, 2, 3 or 4. From the aspect of reactant availability, m5 is preferably 0, 1, 2 or 3, more preferably 0, 1 or 2. The subscript m6 is 0, 1, 2, 3, 4, 5 or 6. From the aspect of reactant availability, m6 is preferably 0, 1, 2 or 3, more preferably 0, 1 or 2. The subscript m7 is 0, 1, 2, 3, 4, 5 or 6. From the aspect of reactant availability, m7 is preferably 0, 1, 2 or 3, more preferably 0, 1 or 2.

In formula (Z-3), m8 is 0, 1 or 2. From the aspect of reactant availability, m8 is preferably 0 or 1. The subscript m9 is 0, 1 or 2. From the aspect of reactant availability, m9 is preferably 0 or 1. The subscript m10 is 0, 1 or 2. From the aspect of reactant availability, m10 is preferably 0 or 1.

In formula (Z-3), m11 is 0 or 1. The relevant structure is a benzene ring when m11=0, and a naphthalene ring when m11=1. The benzene ring corresponding to m11=0 is preferred from the aspect of solvent solubility.

In formula (Z-3), m12 is 0, 1, 2, 3 or 4. As the number of iodine atoms in the cation structure increases, the compound becomes more absorptive to EUV, but so poor in solvent solubility that it may precipitate in a resist composition. For this reason, m12 is preferably 0, 1, 2 or 3, more preferably 0, 1 or 2.

In formula (Z-3), m13 is 0, 1 or 2. From the aspect of reactant availability, m13 is preferably 0 or 1. The subscript m14 is 0, 1 or 2. From the aspect of synthesis, m14 is preferably 0 or 1.

The subscripts m1 to m14 are in the range: 0 ≤ m6+m9 ≤ 4 when m1=0, 0 ≤ m6+m9 ≤ 6 when m1=1; 0 ≤ m7+m10 ≤ 4 when m2=0, 0 ≤ m7+m10 ≤ 6 when m2=1; 1 ≤ m4+m5+m8+m14 ≤ 4 when m3=0, 1 ≤ m4+m5+m8+m14 ≤ 6 when m3=1; 0 ≤ m12+m13 ≤ 4 when m11=0, 0 ≤ m12+m13 ≤ 6 when m11=1; and m4+m12 ≥ 1.

In formula (Z-3), R^{F1} to R^{F3} are each independently fluorine, a C₁-C₆ fluorinated saturated hydrocarbyl group, C₁-C₆ fluorinated saturated hydrocarbyloxy group, or C₁-C₆ fluorinated saturated hydrocarbylthio group. Of these, trifluoromethyl, trifluoromethoxy, and trifluorothiomethoxy are preferred. A plurality of R^{F1} may be identical or different when m5 is 2, 3 or 4, a plurality of R^{F2} may be identical or different when m6 is 2, 3, 4, 5 or 6, and a plurality of R^{F3} may be identical or different when m7 is 2, 3, 4, 5 or 6.

In formula (Z-3), R^{ct6} to R^{ct9} are each independently halogen exclusive of iodine and fluorine, nitro, cyano, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom. The hydrocarbyl group and hydrocarbyl moiety in the hydrocarbyloxy and hydrocarbylthio groups may be saturated or unsaturated and straight, branched or cyclic. Examples thereof are as exemplified above for the hydrocarbyl group R² in formula (A). In the hydrocarbyl group and hydrocarbyl moiety in the hydrocarbyloxy and hydrocarbylthio groups, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, fluorine, chlorine, bromine, iodine, carbonyl moiety, ether bond, ester bond, sulfonate ester bond, carbonate bond, lactone ring, sultone ring, carboxylic anhydride (-C(=O)-O-C(=O)-) or haloalkyl moiety.

When m8=2, two R^{ct6} may be identical or different and two R^{ct6} may bond together to form a ring with the carbon atoms to which they are attached. When m9=2, two R^{ct7} may be identical or different and two R^{ct7} may bond together to form a ring with the carbon atoms to which they are attached. When m10=2, two R^{ct8} may be identical or different and two R^{ct8} may bond together to form a ring with the carbon atoms to which they are attached. When m13=2, two R^{ct9} may be identical or different and two R^{ct9} may bond together to form a ring with the carbon atoms to which they are attached. Examples of the ring thus formed include cyclopropane, cyclobutane, cyclopentane, cyclohexane, norbornane, and adamantane rings. In the ring, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the ring may contain a hydroxy moiety, fluorine, chlorine, bromine, iodine, cyano moiety, carbonyl moiety, ether bond, ester bond, sulfonate ester bond, carbonate bond, lactone ring, sultone ring, carboxylic anhydride (-C(=O)-O-C(=O)-) or haloalkyl moiety.

The aromatic rings directly bonded to S⁺ in the sulfonium cation having formula (Z-3) may bond together to form a ring with S⁺. Exemplary structures of the ring are shown below.

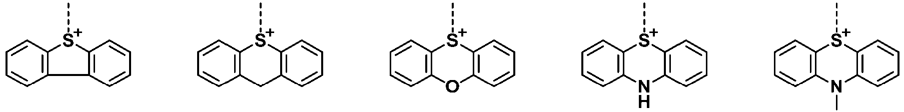

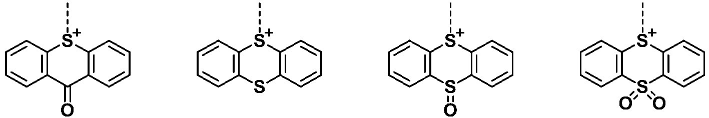

In formula (Z-3), L^{A} and L^{B} are each independently a single bond, ether bond, ester bond, sulfonate ester bond, amide bond, sulfonamide bond, carbonate bond or carbamate bond. L^{A} is preferably a single bond, ether bond, ester bond or sulfonate ester bond, more preferably an ester bond or sulfonate ester bond. L^{B} is preferably a single bond, ether bond or ester bond, more preferably a single bond.

In formula (Z-3), X^{L} is a single bond or a C₁-C₄₀ hydrocarbylene group which may contain a heteroatom. The hydrocarbylene group may be straight, branched or cyclic and examples thereof include alkanediyl and cyclic saturated hydrocarbylene groups. Exemplary heteroatoms include oxygen, nitrogen and sulfur.

Examples of the C₁-C₄₀ hydrocarbylene group which may contain a heteroatom, represented by X^{L}, are shown below, but not limited thereto. Herein, * designates a point of attachment to L^{A} or L^{B}.

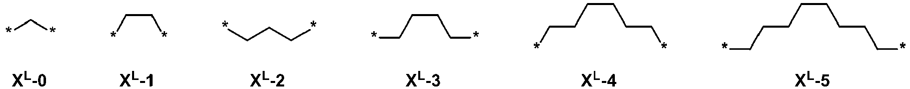

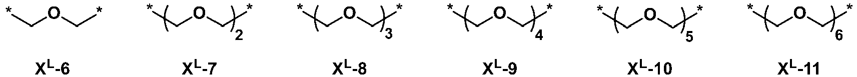

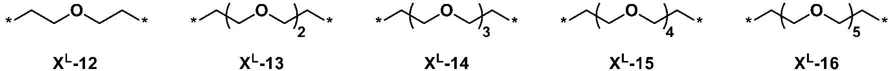

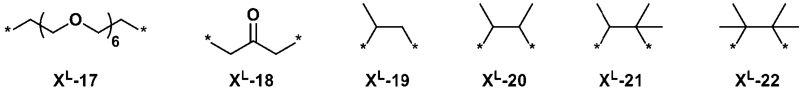

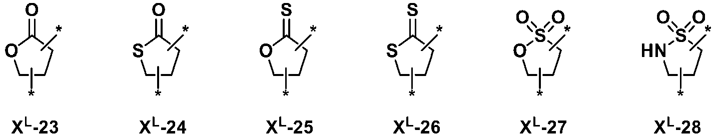

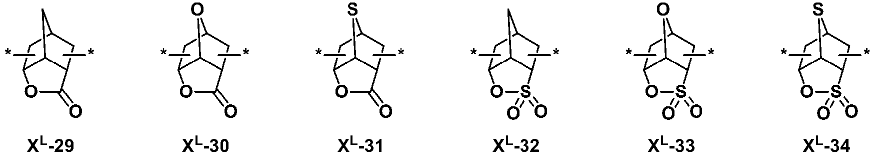

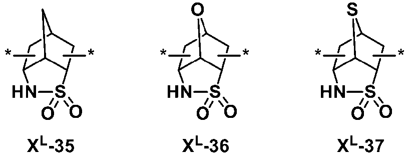

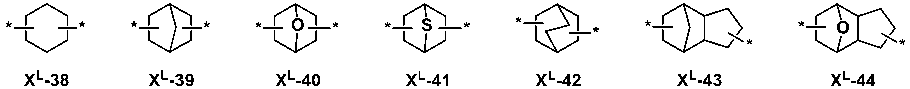

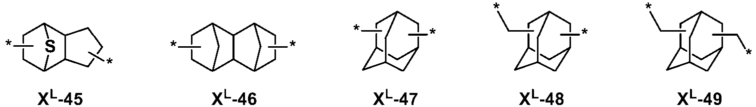

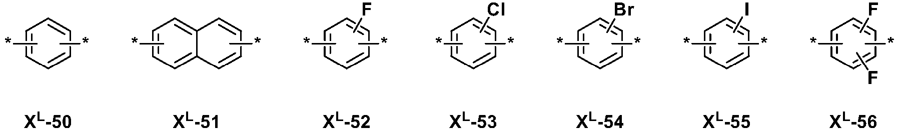

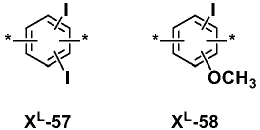

Of these, X^{L}-0 to X^{L}-22 and X^{L}-47 to X^{L}-58 are preferred.

Of the sulfonium cations having formula (Z-3), those having the formula (Z-3-1) are preferred.

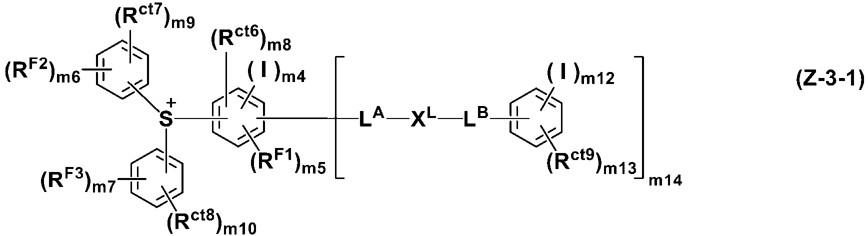

Herein m4 to m10, m12 to m14, R^{F1} to R^{F3}, R^{ct6} to R^{ct9}, L^{A}, L^{B}, and X^{L} are as defined above.

Of the sulfonium cations having formula (Z-3-1), those having the formula (Z-3-2) are preferred.

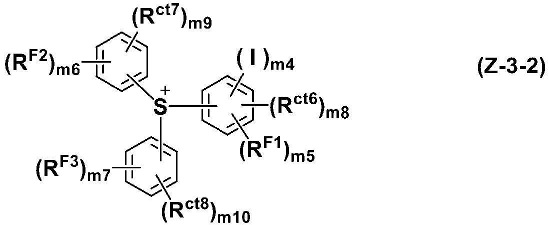

Herein m4 to m10, R^{F1} to R^{F3}, and R^{ct6} to R^{ct8} are as defined above.

Examples of the sulfonium cation having formula (Z-3) are shown below, but not limited thereto.

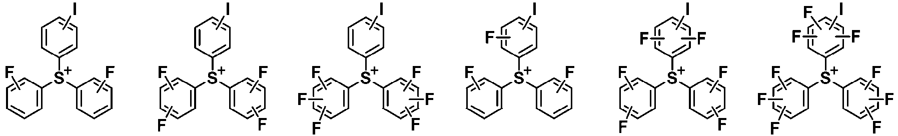

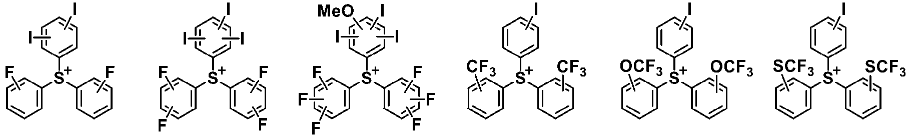

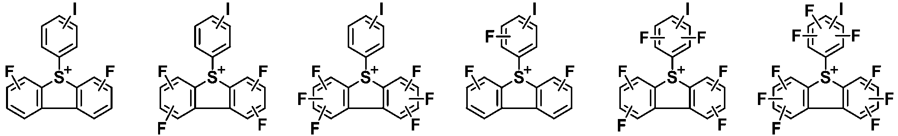

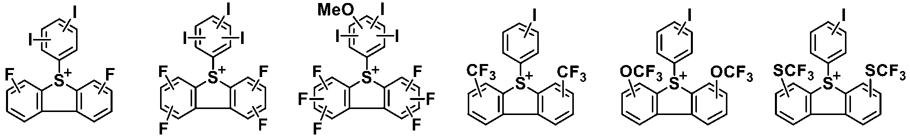

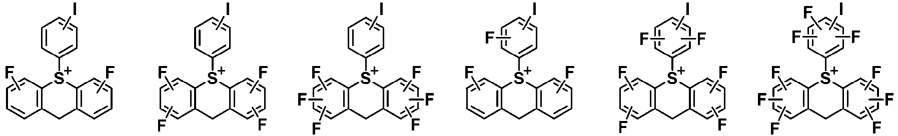

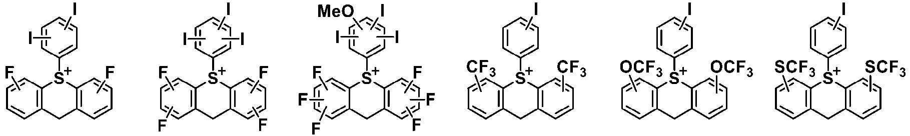

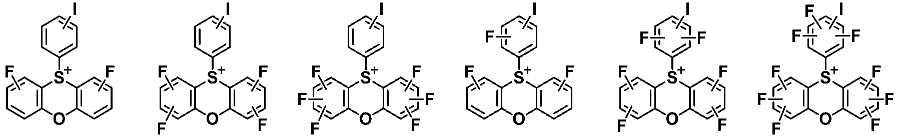

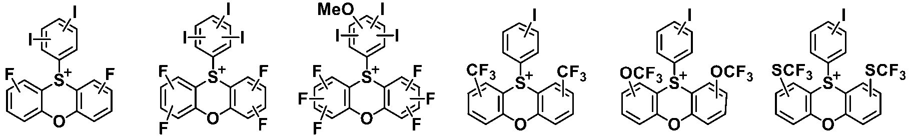

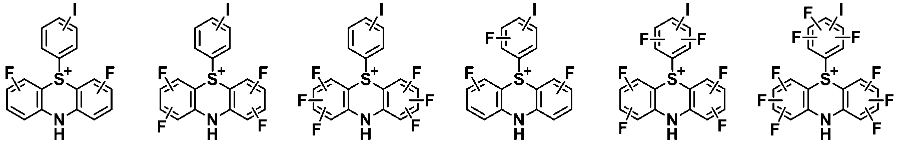

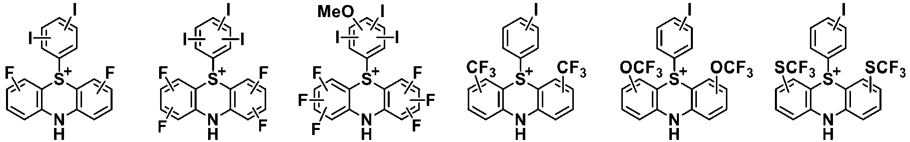

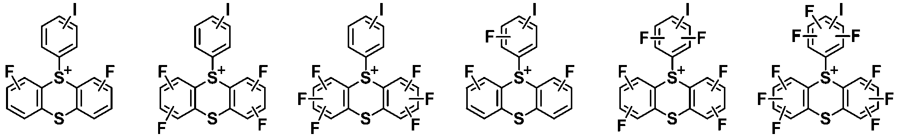

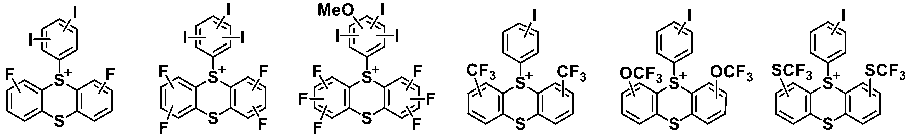

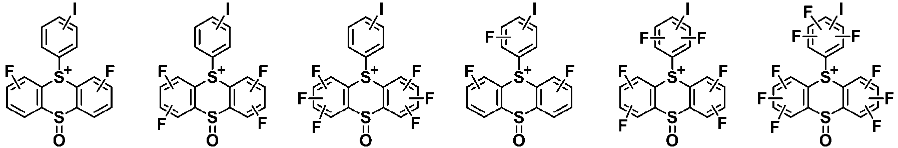

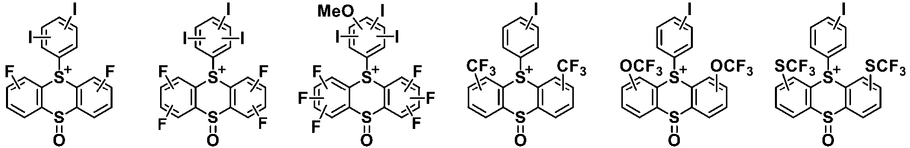

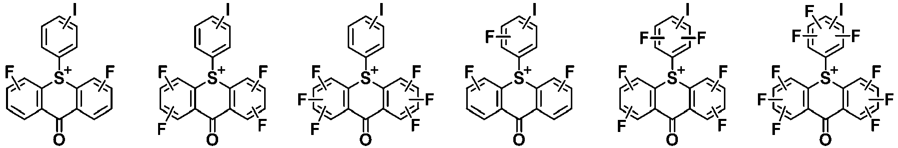

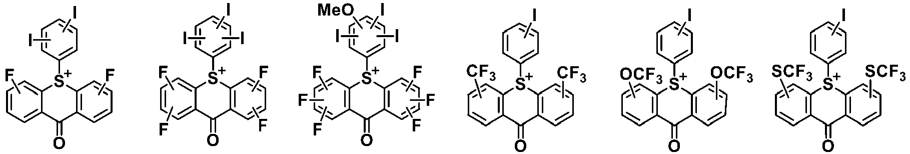

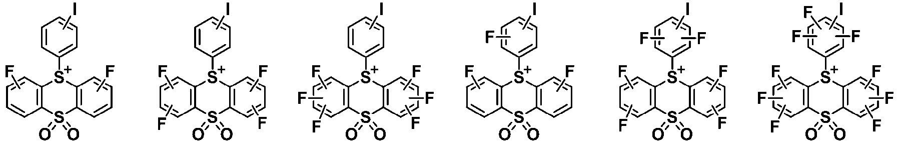

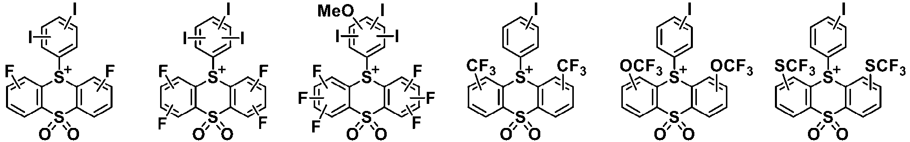

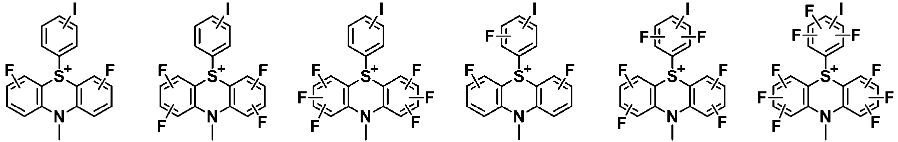

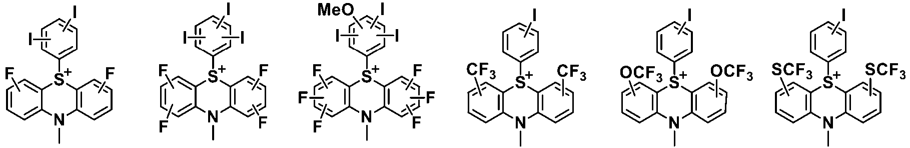

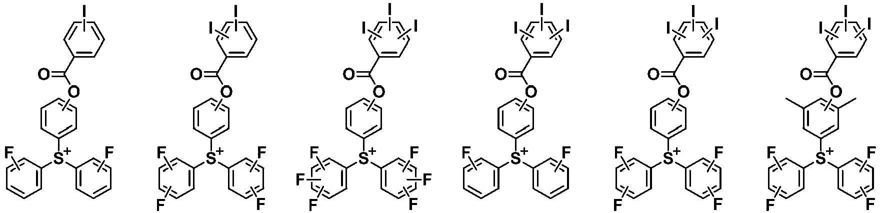

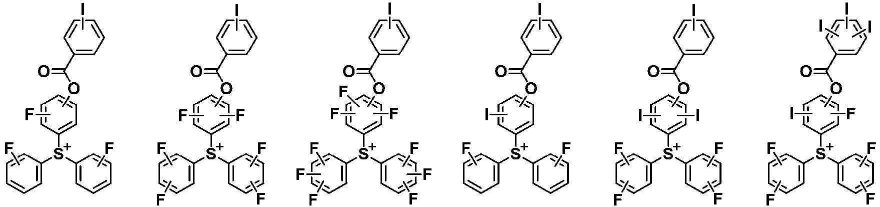

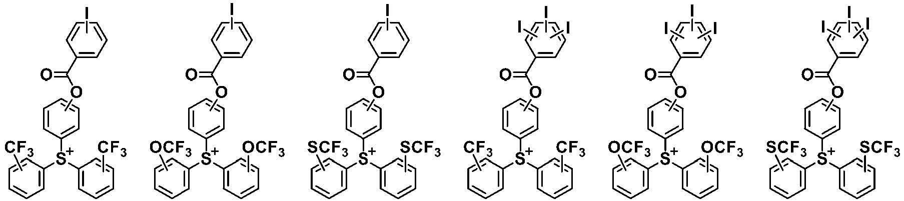

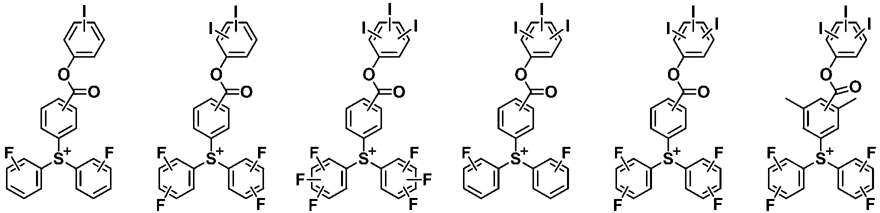

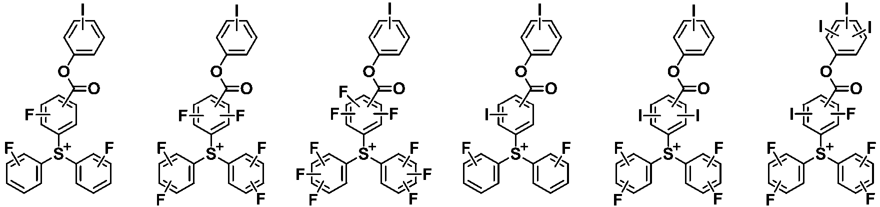

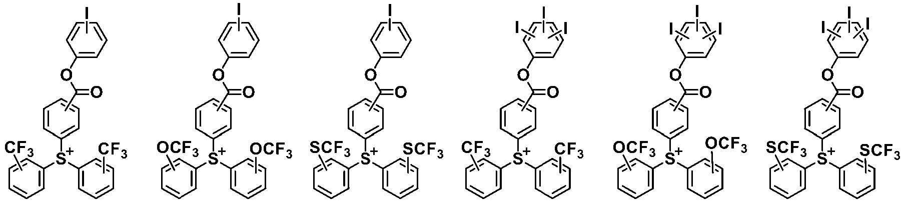

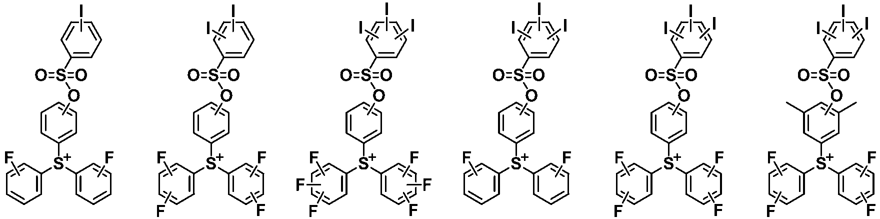

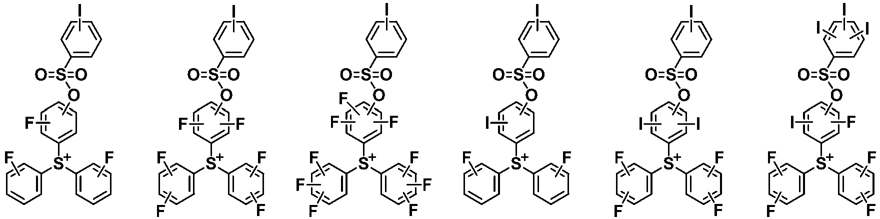

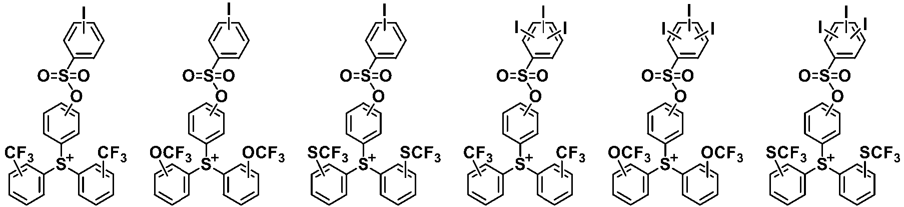

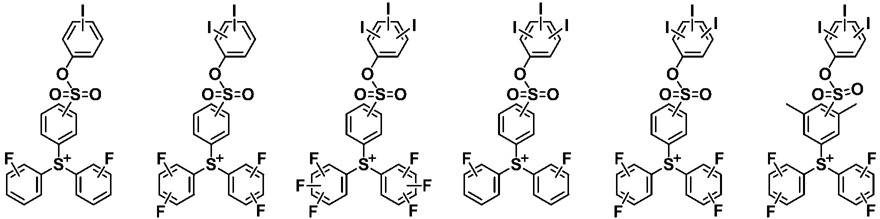

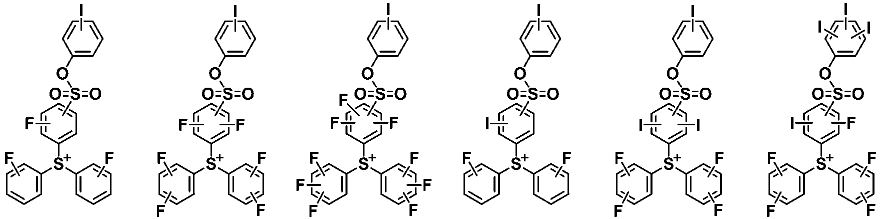

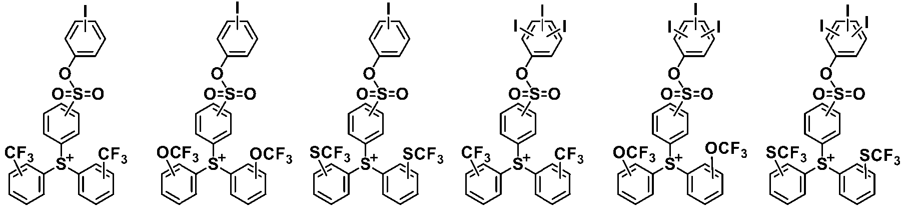

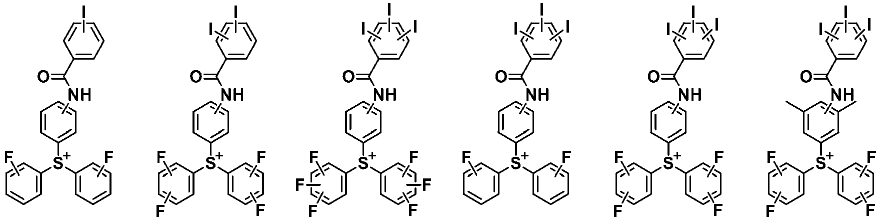

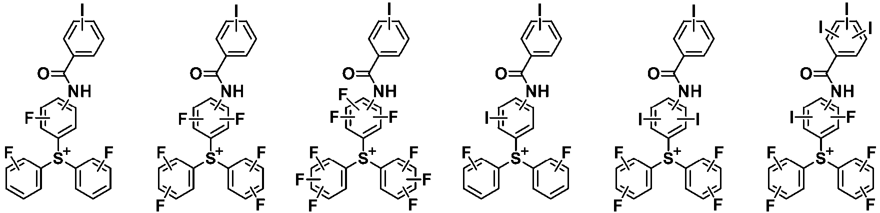

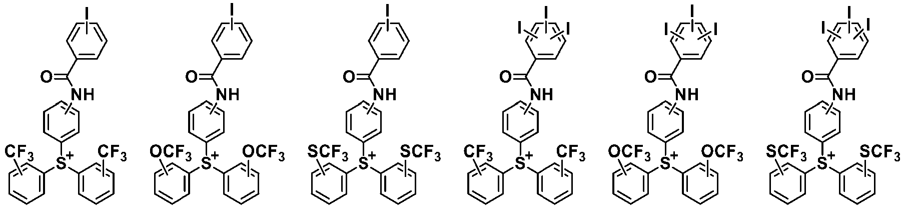

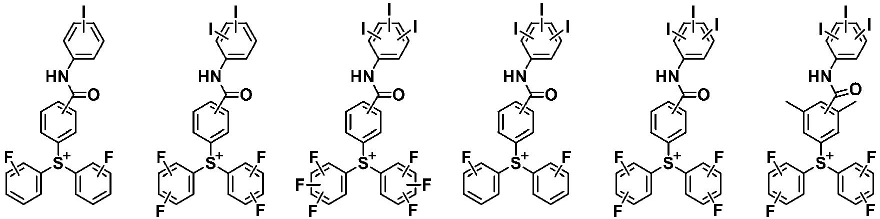

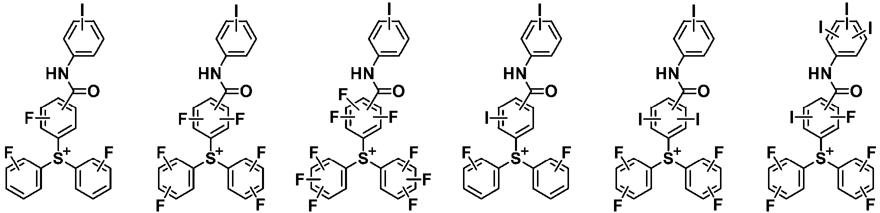

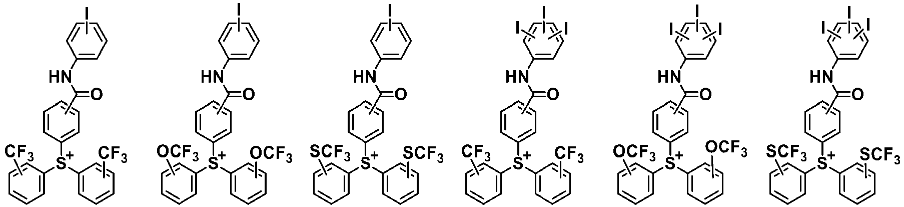

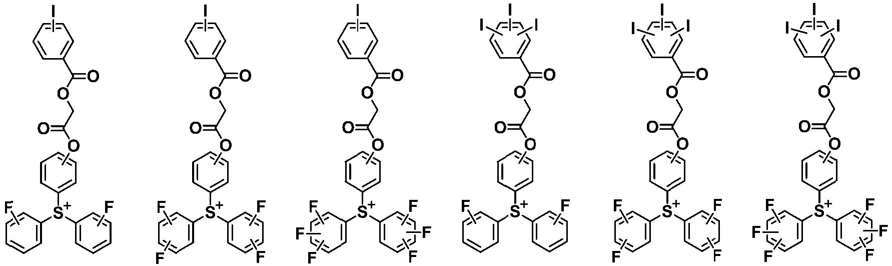

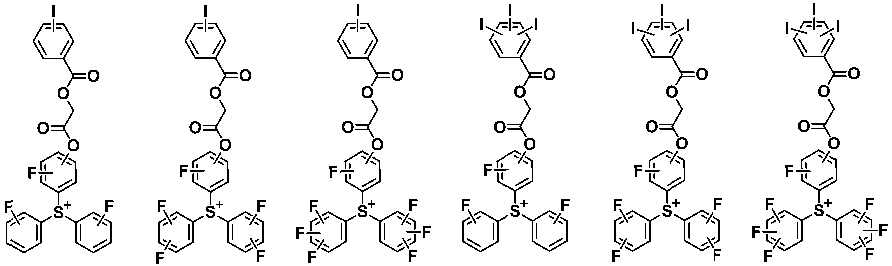

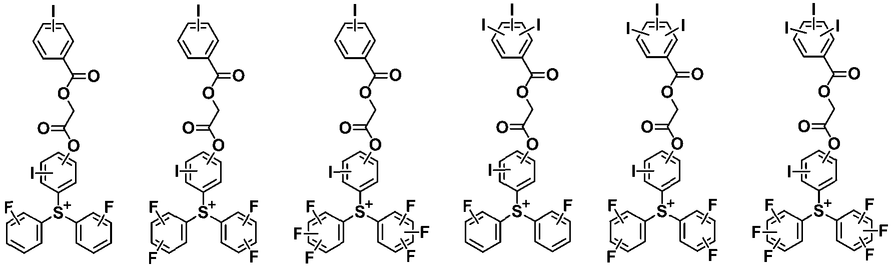

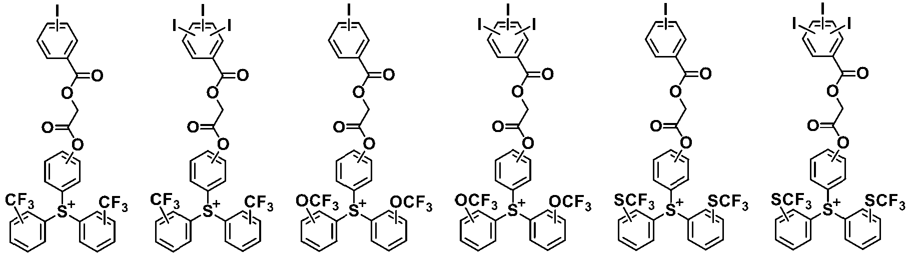

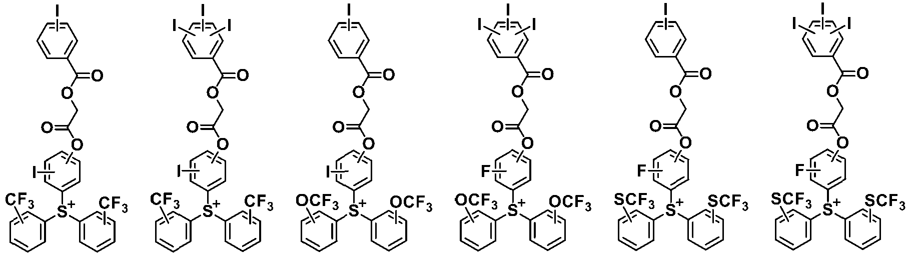

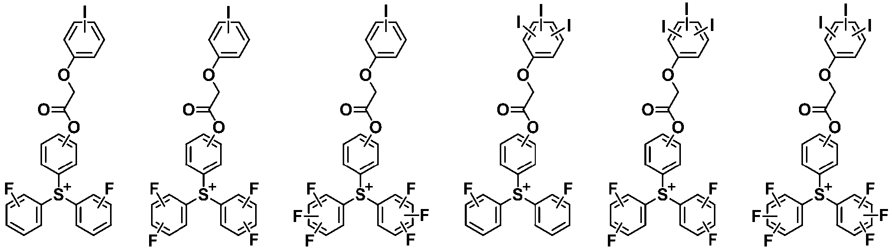

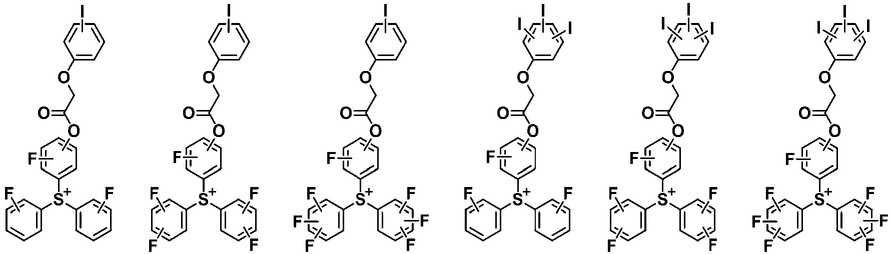

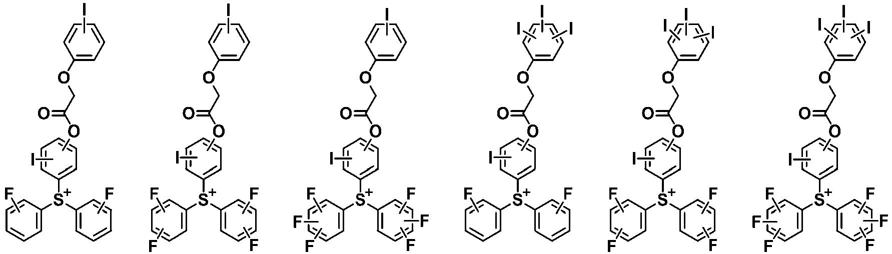

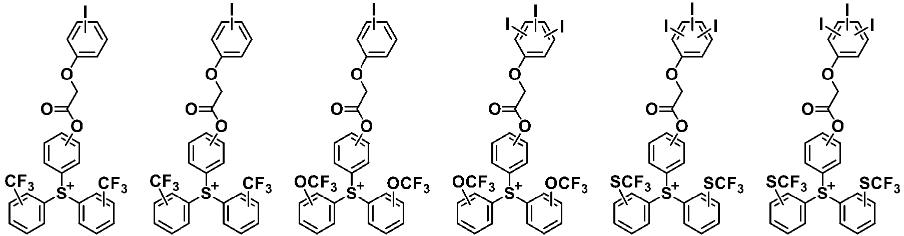

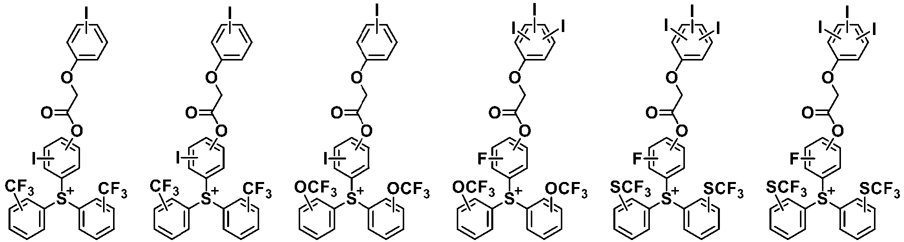

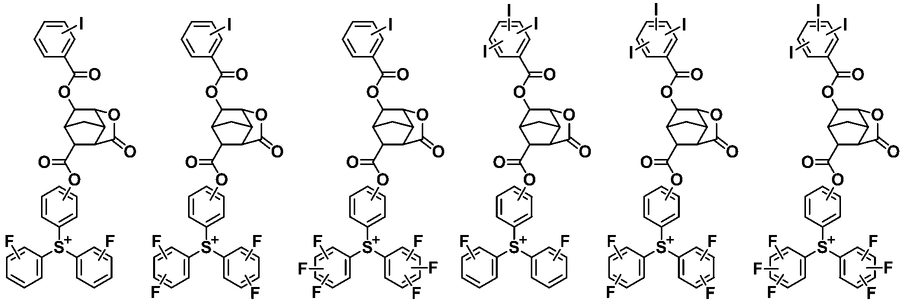

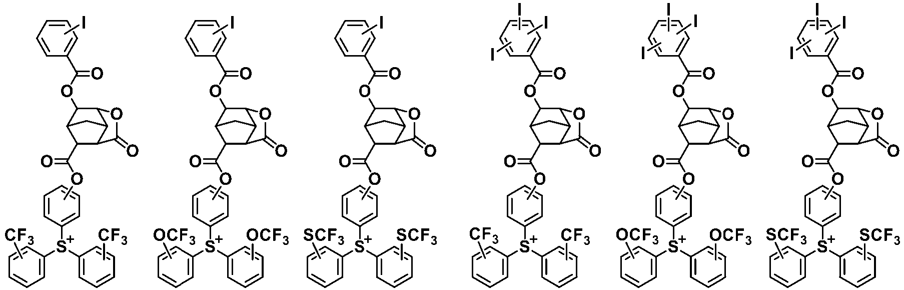

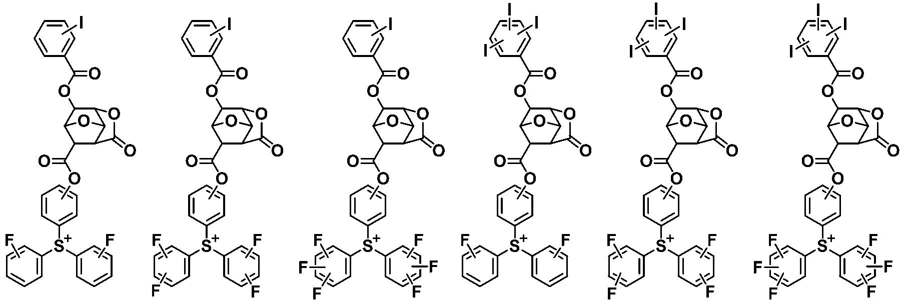

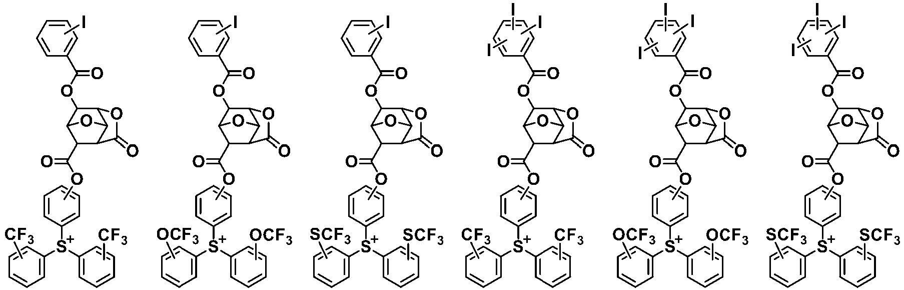

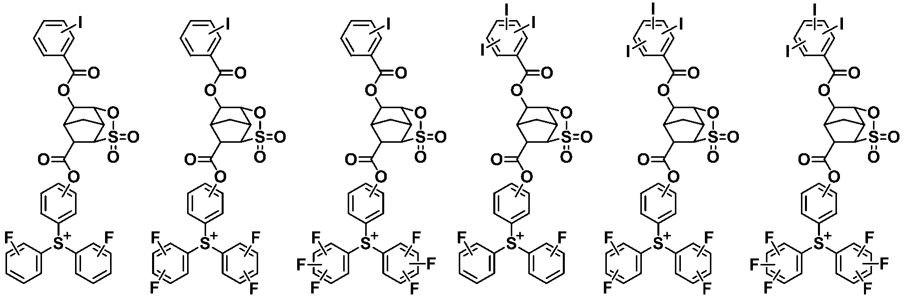

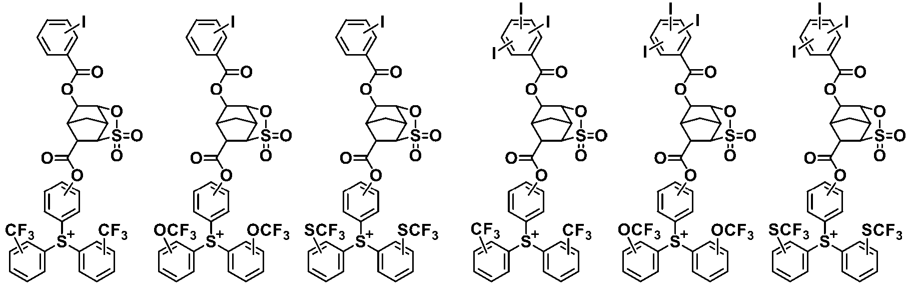

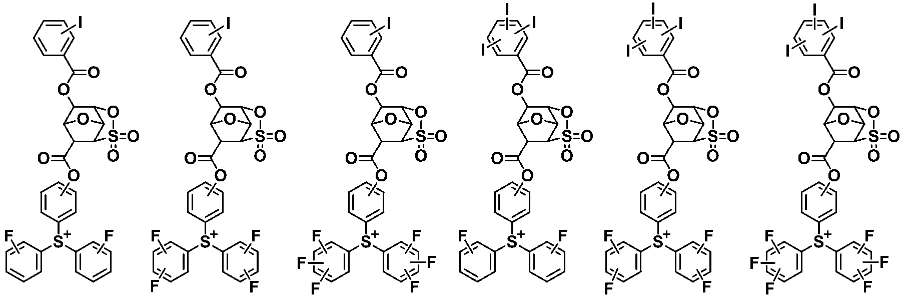

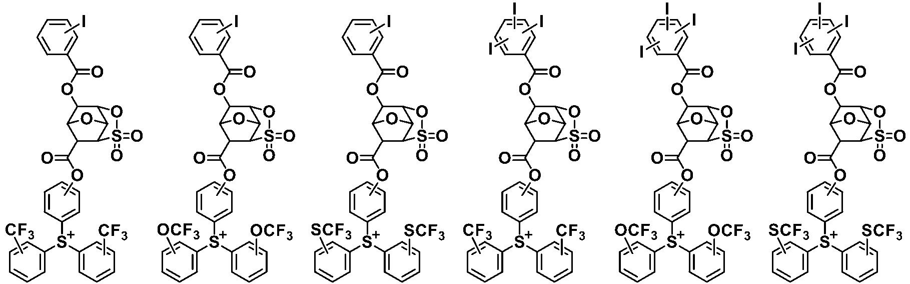

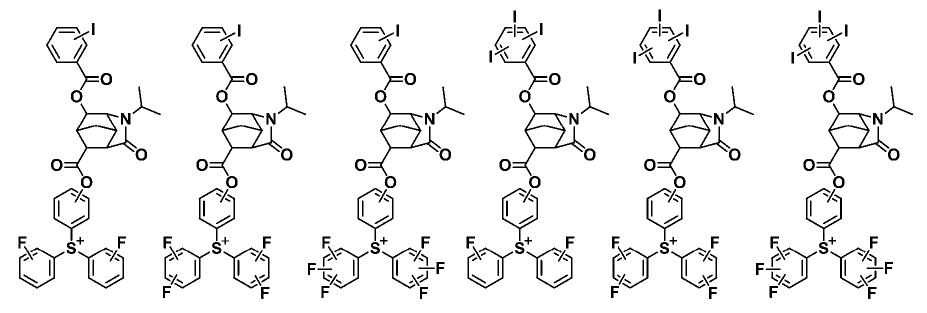

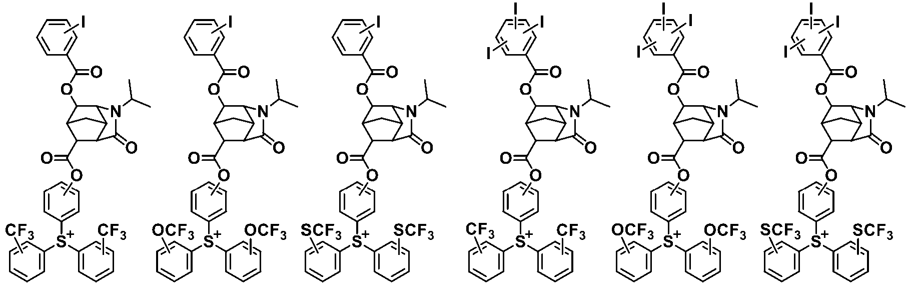

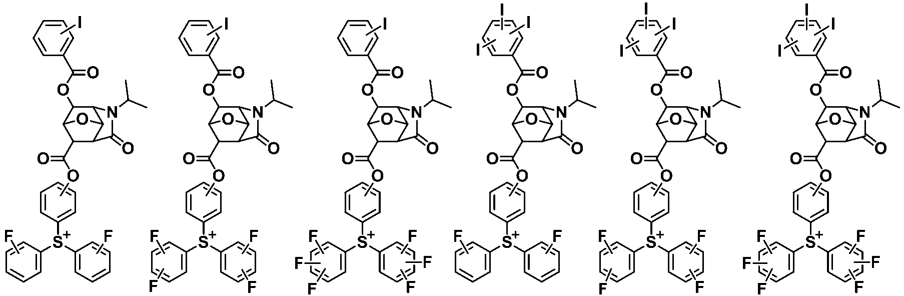

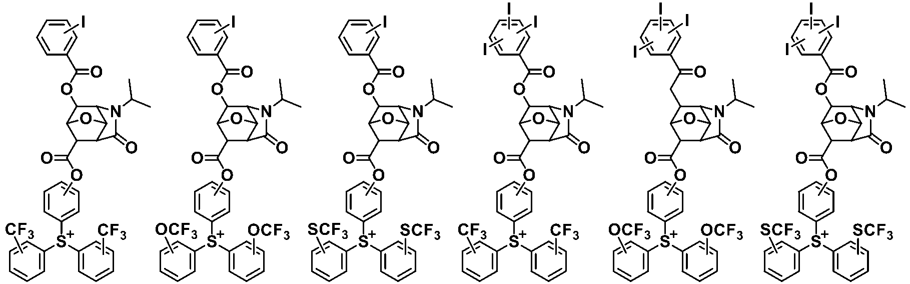

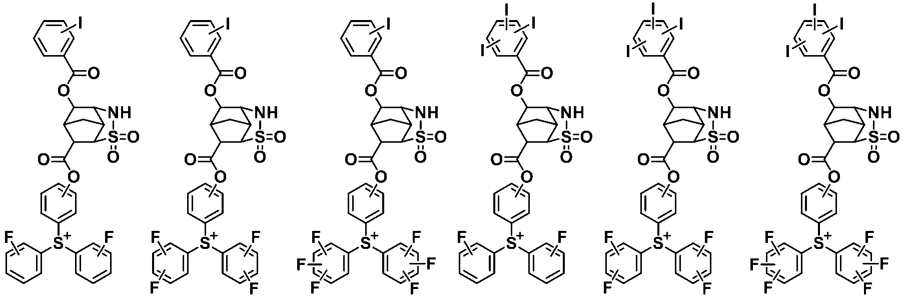

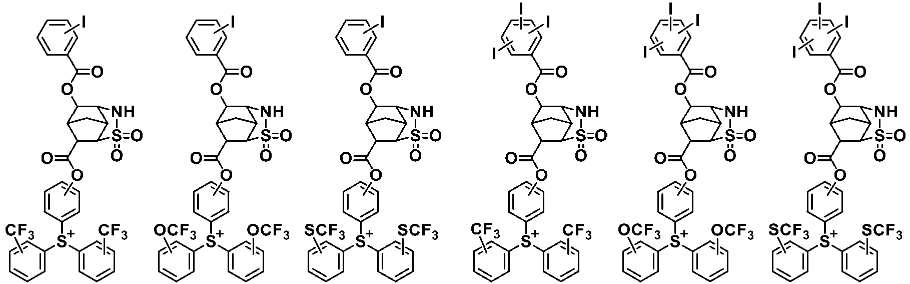

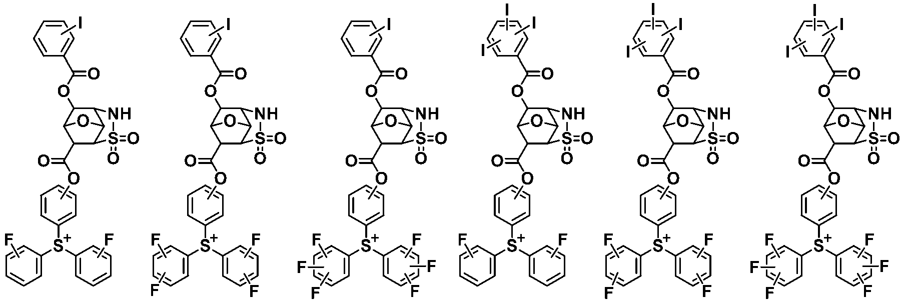

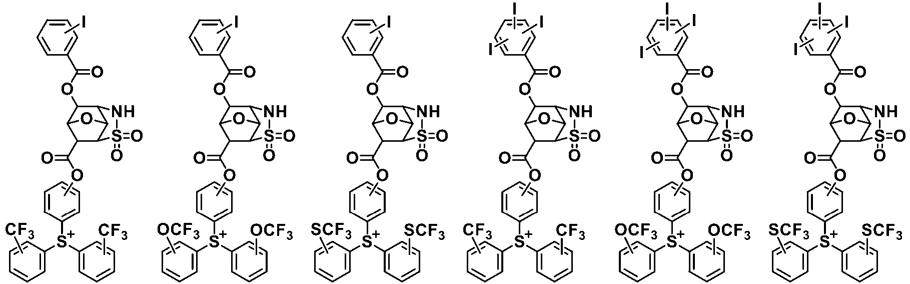

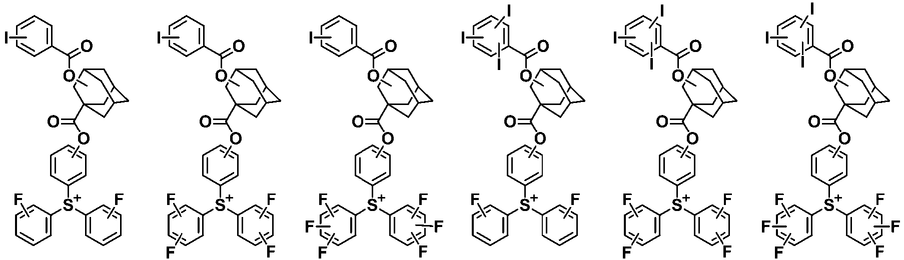

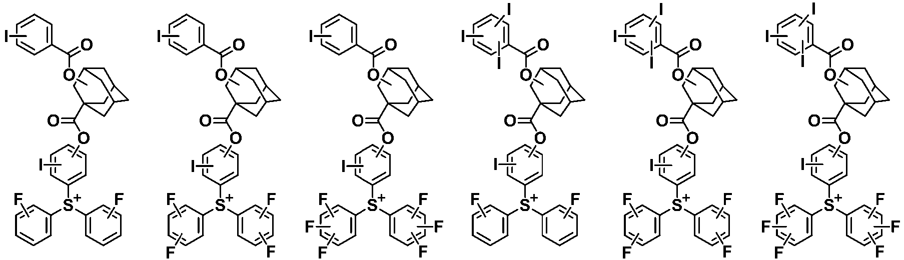

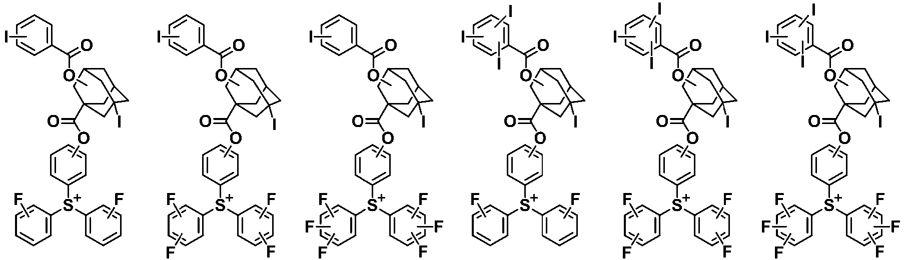

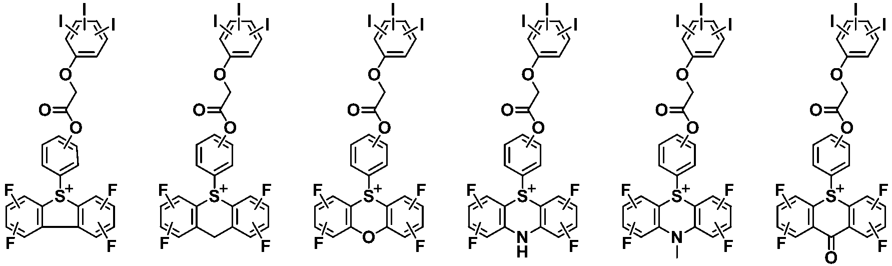

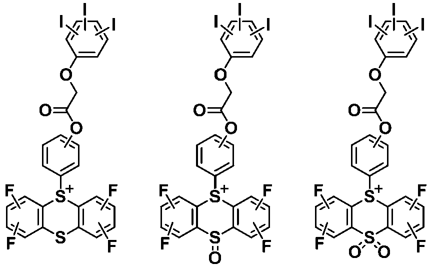

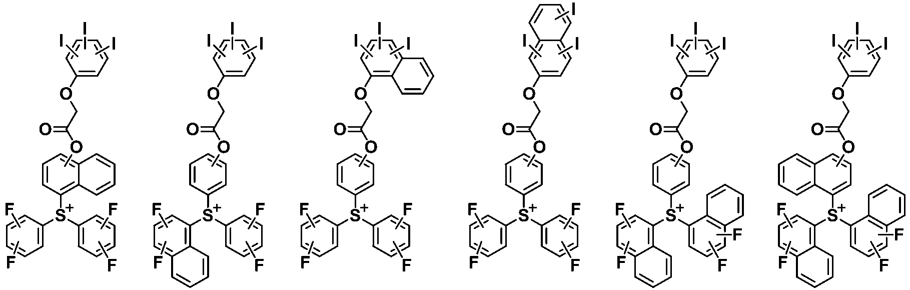

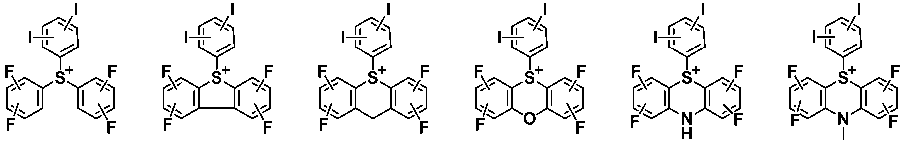

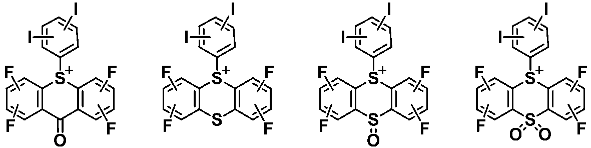

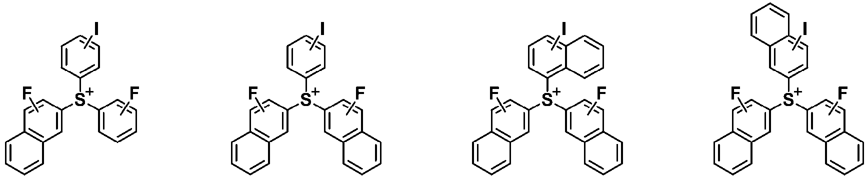

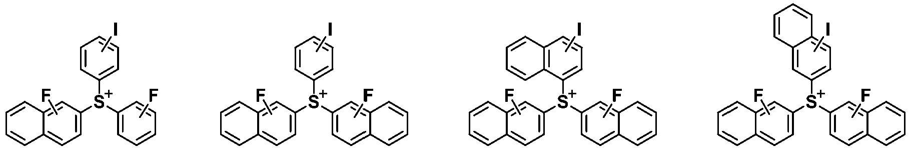

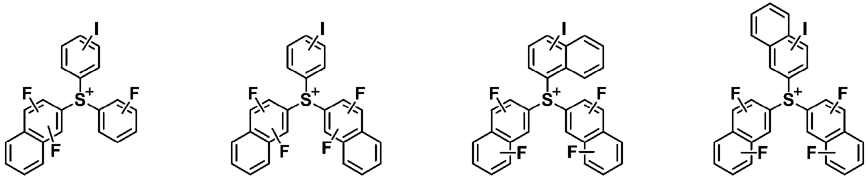

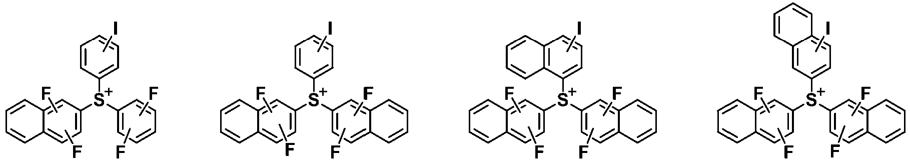

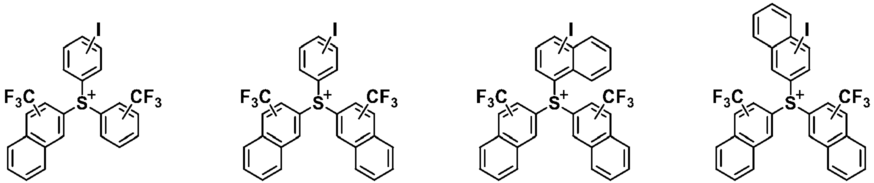

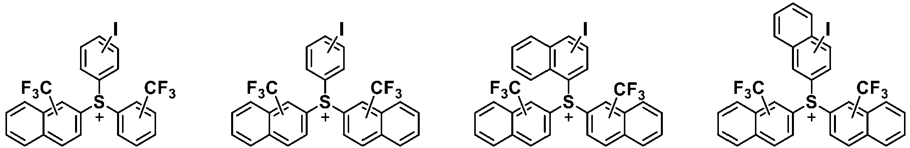

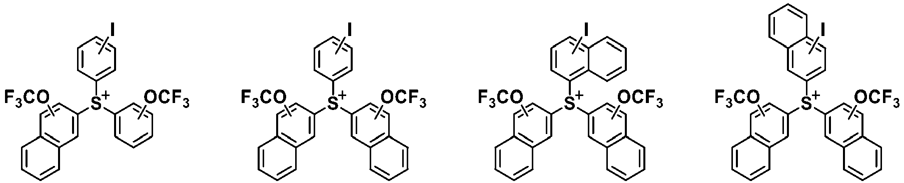

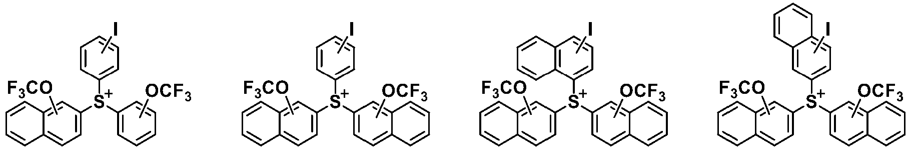

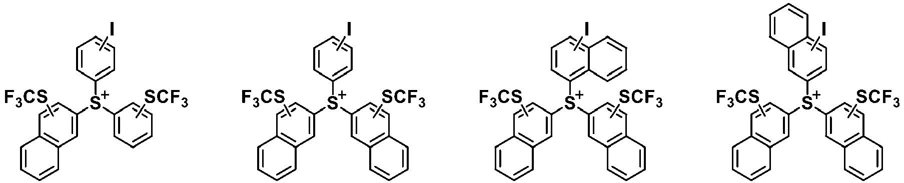

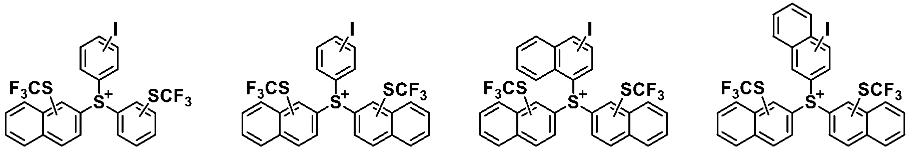

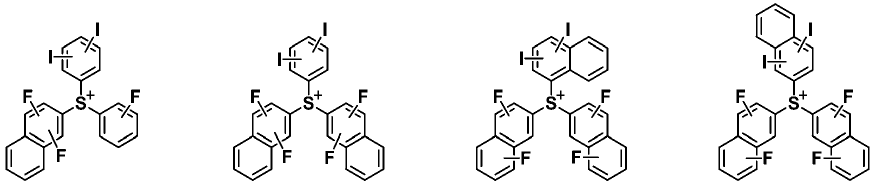

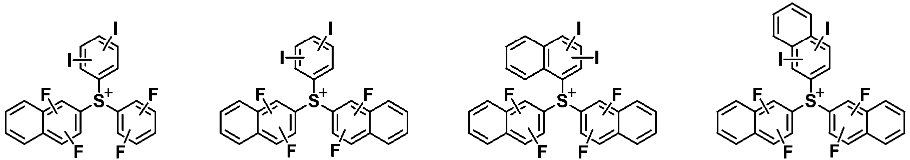

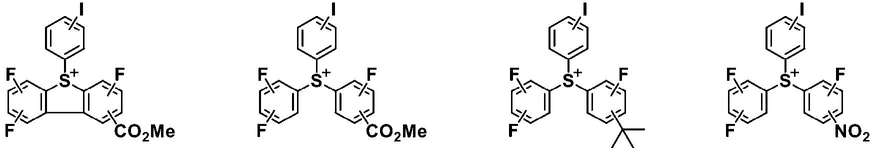

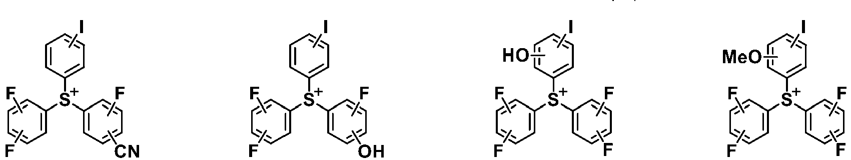

Examples of the onium salt include arbitrary combinations of anions with cations, both as exemplified above.

The onium salt having formula (A) can be synthesized by well-known methods. For example, a method of preparing an onium salt having formula (PAG-1-ex) is described.

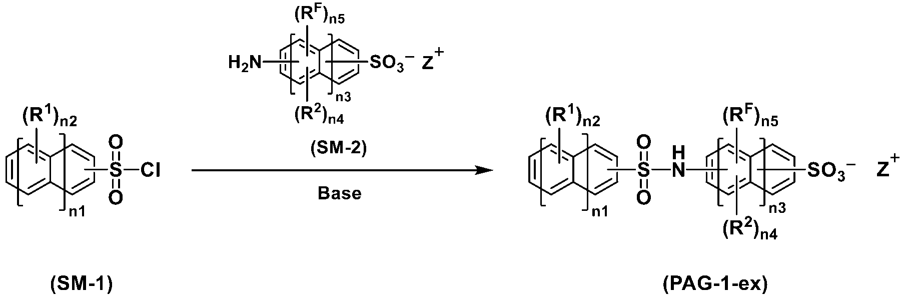

Herein n1 to n5, R¹, R², R^{F}, and Z⁺ are as defined above.

The reaction scheme shown herein is to prepare an aromatic sulfonic acid onium salt PAG-1-ex by sulfon-amidation reaction of a reactant SM-1 with an amino aromatic sulfonic acid salt SM-2. The reaction can be carried out by the standard method. A solvent is typically used in the reaction. Examples of the solvent include ethers such as tetrahydrofuran (THF), diethyl ether, diisopropyl ether, di-n-butyl ether, and 1,4-dioxane, hydrocarbons such as n-hexane, n-heptane, benzene, toluene, and xylene, aprotic polar solvents such as acetonitrile, dimethyl sulfoxide (DMSO) and N,N-dimethylformamide (DMF), and chlorine organic solvents such as methylene chloride, chloroform and carbon tetrachloride. Any of these solvents may be chosen depending on reaction conditions while they may be used alone or in admixture of two or more. The preferred procedure includes adding reactant SM-1, amino aromatic sulfonic acid salt SM-2, and a base to the solvent sequentially or simultaneously and heating or cooling if necessary. Examples of the base used in the reaction include ammonia, amines such as triethylamine, pyridine, lutidine, collidine, and N,N-dimethylaniline, hydroxides such as sodium hydroxide, potassium hydroxide, and tetramethylammonium hydroxide; and carbonate salts such as potassium carbonate and sodium hydrogencarbonate. The bases may be used alone or in admixture. It is preferred from the yield aspect to monitor the progress of reaction by silica gel thin-layer chromatography (TLC). By ordinary aqueous work-up, the onium salt PAG-1-ex is recovered from the reaction mixture. The onium salt PAG-1-ex may be purified by a standard technique such as chromatography or re-crystallization if necessary.

Since the onium salt having formula (A) is an onium salt of aromatic sulfonic acid, it generates an acid having an adequate strength upon exposure to high-energy radiation. At least one group selected from branched alkyl groups, alicyclic groups and aryl groups is included as R¹. A variety of functional groups can be introduced into the aryl group. When R¹ is an aryl group, the aryl group having a functional group is preferred. When R¹ is an aryl group which has a hydrocarbyloxycarbonyl group as the functional group, the inclusion of plural heteroatoms in the form of ester bonds is effective for reducing acid diffusion. Also when R¹ is an aryl group which has a functional group containing halogen, especially fluorine, the onium salt itself has an increased solvent solubility, contributing to an improvement in uniform dispersion. In addition, the acidity of generated sulfonic acid is increased, promoting deprotection reaction of protective groups on the base polymer, and an improvement in resolution is expectable. When R¹ is an aryl group having a branched alkyl group or alicyclic group, the anion has a large excluded volume, takes a bulky structure, and generates an acid with low diffusivity, contributing to an improvement in LER. Also, a plurality of aromatic rings in the anion contribute to improved compatibility due to their interaction (π-π stacking interaction) with aromatic rings in the base polymer, and an effect of restraining excessive acid diffusion is expectable. Since the nitrogen atom inherent to the sulfonamide bond is reduced in basicity by the electron withdrawing effect of the adjoining sulfone bond, it is insufficient to trap protons of the generated acid. Since the sulfonamide bond has a plurality of heteroatoms, a lone pair and proton form a hydrogen bond, restraining excessive acid diffusion. Since the N-H bond of the sulfonamide bond bears a weak acidity, a resist film, when developed in an alkaline developer, is so improved in affinity to the alkaline developer that few development residues may be left in the exposed region. Due to the synergy of these effects, an acid with adequate strength and controlled diffusion is generated and distributed in the resist film uniformly. Then, a chemically amplified positive resist composition comprising the inventive onium salt can form small-size patterns with satisfactory resolution and reduced LWR. When the positive resist composition is developed in an alkaline developer, patterns of rectangular profile are formed due to adequate dissolution inhibition in the unexposed region and minimal development residues in the exposed region.

The inventive onium salt is advantageously used as a PAG.

### Chemically amplified positive resist composition

### (A) Photoacid generator

Another embodiment of the invention is a chemically amplified positive resist composition essentially comprising (A) a photoacid generator in the form of the onium salt having formula (A).

In the chemically amplified positive resist composition, the amount of the PAG in the form of the onium salt as component (A) is preferably 0.1 to 40 parts by weight, more preferably 1 to 20 parts by weight per 80 parts by weight of a base polymer to be described just below. As long as the amount of component (A) is in the range, the acid is generated in a necessary amount to deprotect acid labile groups and the resist composition has shelf stability. The PAG may be used alone or in admixture as component (A).

### (B) Base polymer

The chemically amplified positive resist composition may comprise a base polymer as component (B). The base polymer contains a polymer which is decomposed under the action of acid to increase its solubility in alkaline developer.

The polymer preferably contains repeat units having the formula (B1), which are also referred to as repeat units B1.

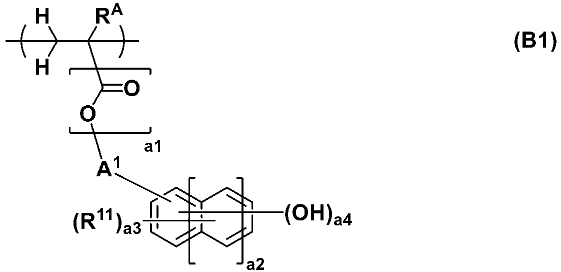

In formula (B1), a1 is 0 or 1. The subscript a2 is 0, 1 or 2. The relevant structure is a benzene ring when a2=0, a naphthalene ring when a2=1, and an anthracene ring when a2=2. The subscript a3 is an integer meeting 0 ≤ a3 ≤ 5+2(a2)-a4, and a4 is 1, 2 or 3. When a2 is 0, preferably a3 is 0, 1, 2 or 3 and a4 is 1, 2 or 3. When a2 is 1 or 2, preferably a3 is 0, 1, 2, 3 or 4 and a4 is 1, 2 or 3.

In formula (B1), R^{A} is hydrogen, fluorine, methyl or trifluoromethyl.

In formula (B1), R¹¹ is halogen, nitro, carboxy, an optionally halogenated C₁-C₆ saturated hydrocarbyl group, optionally halogenated C₁-C₆ saturated hydrocarbyloxy group or optionally halogenated C₂-C₈ saturated hydrocarbylcarbonyloxy group. The saturated hydrocarbyl group and saturated hydrocarbyl moiety in the saturated hydrocarbyloxy and saturated hydrocarbylcarbonyloxy groups may be straight, branched or cyclic. Examples thereof include alkyl groups such as methyl, ethyl, n-propyl, isopropyl, butyl, pentyl, hexyl, and structural isomers thereof; cycloalkyl groups such as cyclopropyl, cyclobutyl, cyclopentyl and cyclohexyl; and combinations thereof. A carbon count within the upper limit ensures a satisfactory solubility in alkaline developer. A plurality of R¹¹ may be identical or different when a3 is 2 or more.

In formula (B1), A¹ is a single bond or C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of a1=1 in formula (B1), the ether bond may be incorporated at any position excluding the position between the α- and β-carbons relative to the ester oxygen. In case of a1=0, the atom bonding to the backbone becomes an ether oxygen atom, and a second ether bond may be incorporated at any position excluding the position between the α- and β-carbons relative to the ether oxygen. Saturated hydrocarbylene groups having no more than 10 carbon atoms are desirable because of a sufficient solubility in alkaline developer.

Preferred examples of the repeat units B1 wherein a1=0 and A¹ is a single bond (meaning that the aromatic ring is directly bonded to the backbone of the polymer), that is, repeat units free of a linker: -C(=O)-O-A¹- include units derived from 3-hydroxystyrene, 4-hydroxystyrene, 5-hydroxy-2-vinylnaphthalene, and 6-hydroxy-2-vinylnaphthalene. Exemplary units are shown below, but not limited thereto. Herein R^{A} is as defined above.

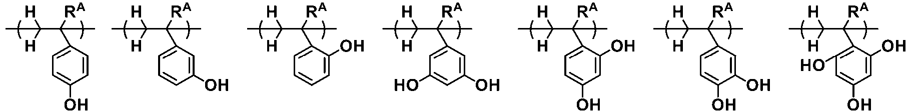

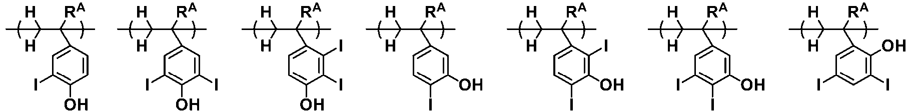

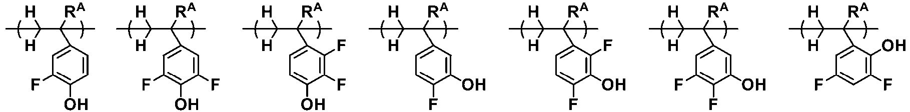

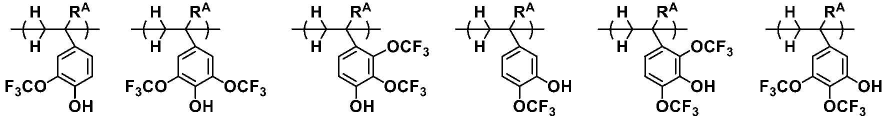

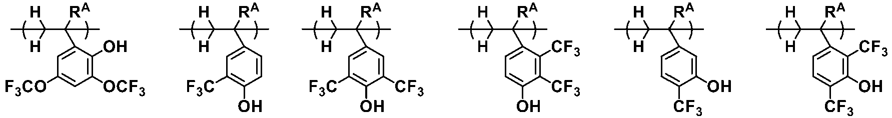

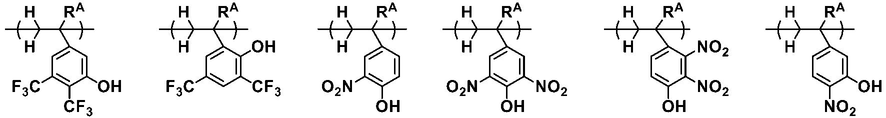

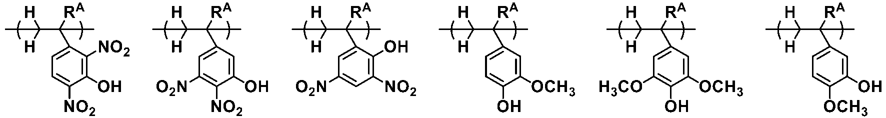

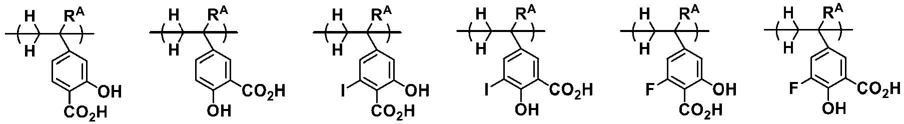

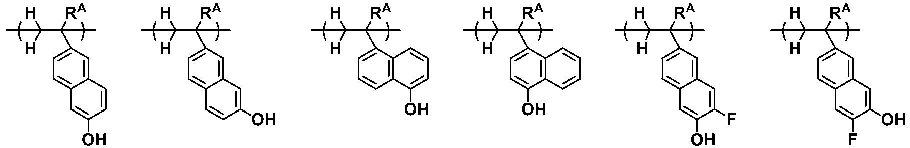

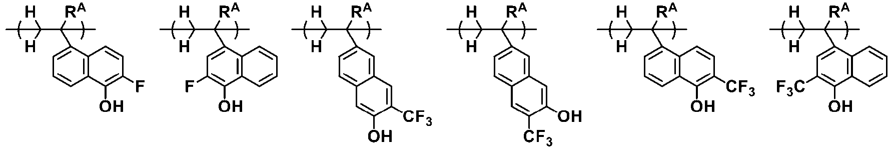

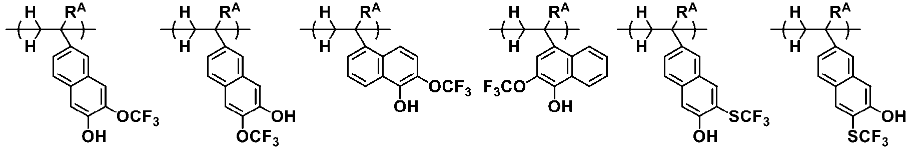

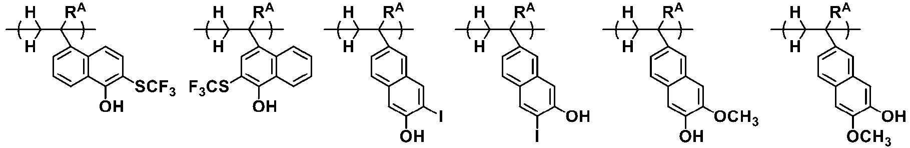

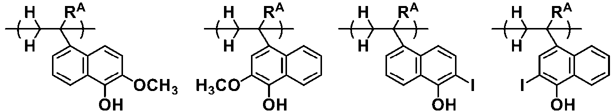

Preferred examples of the repeat units B1 wherein a1=1, that is, having a linker: -C(=O)-O-A¹- are shown below, but not limited thereto. Herein R^{A} is as defined above.

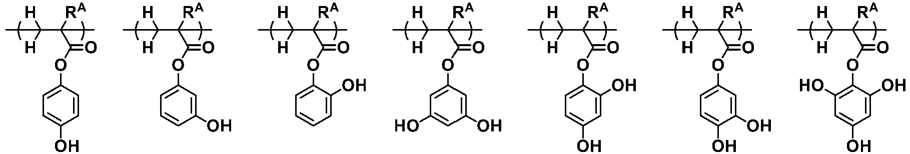

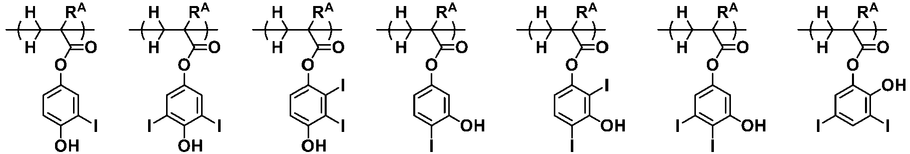

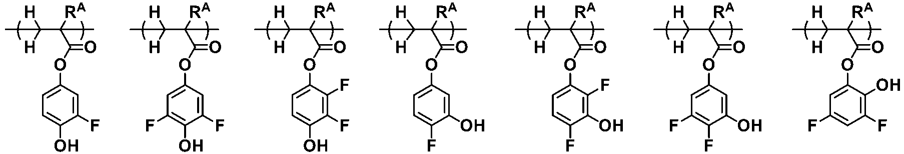

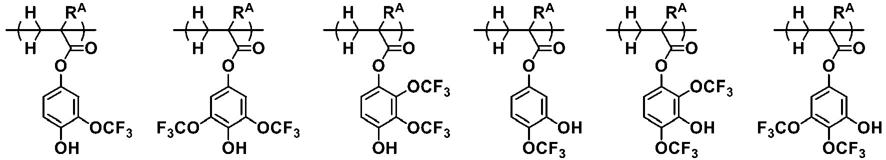

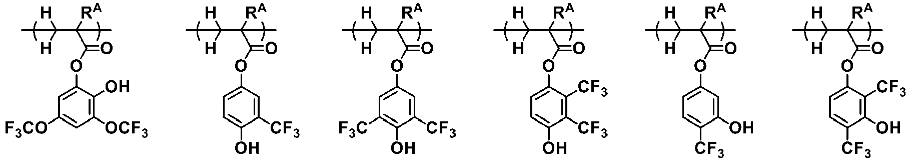

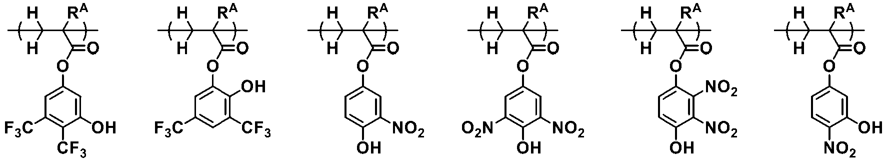

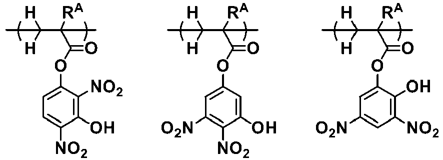

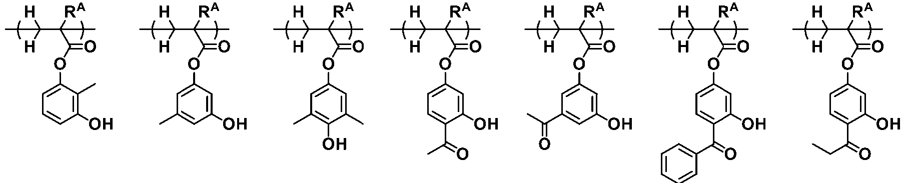

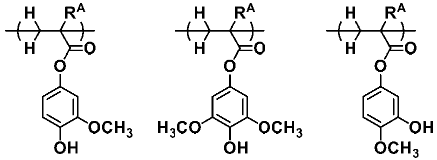

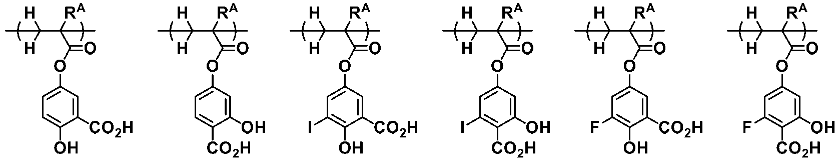

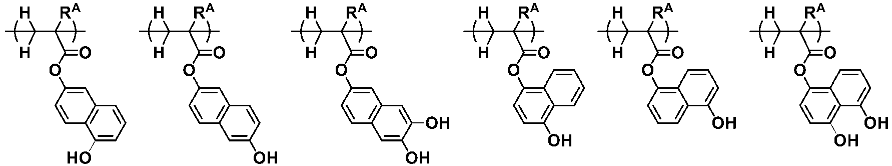

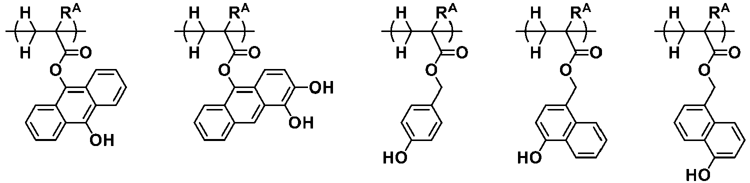

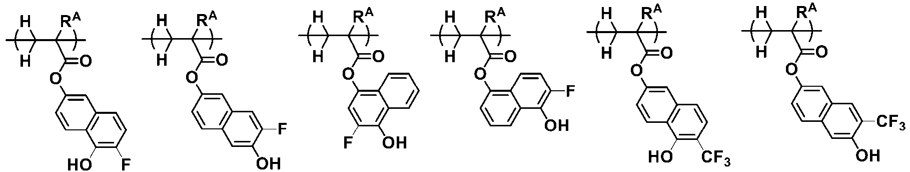

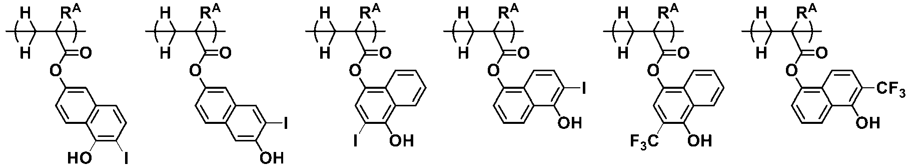

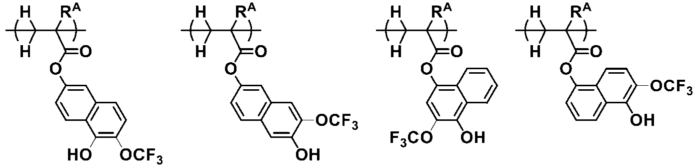

The content of repeat units B1 is preferably 15 to 90 mol%, more preferably 15 to 80 mol% of the overall units of the polymer. When the polymer further comprises repeat units of at least one type selected from repeat units having formula (B3) and repeat units having formula (B4), which provide the polymer with higher etch resistance, the repeat units containing a phenolic hydroxy group as a substituent, the total content of repeat units B1 and repeat units B3 and/or B4 should preferably fall in the range. The repeat units B1 may be of one type or a combination of plural types.

In a preferred embodiment, the polymer further contains repeat units B2 having an acidic functional group protected with an acid labile group (i.e., repeat units protected with an acid labile group and adapted to turn alkali soluble under the action of acid) in order that the positive resist composition in an exposed region turn soluble in alkaline developer.

Typical of the repeat unit B2 is a unit having the formula (B2-1), also referred to as repeat unit B2-1.

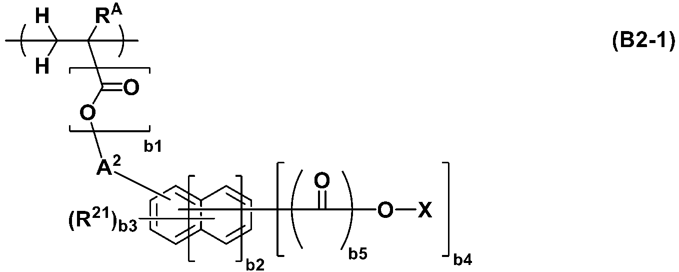

In formula (B2-1), b1 is 0 or 1. The subscript b2 is 0, 1 or 2. The structure represents a benzene skeleton when b2=0, a naphthalene skeleton when b2=1, and an anthracene skeleton when b2=2. The subscript b3 is an integer meeting 0 ≤ b3 ≤ 5+2(b2)-b4. The subscript b4 is 1, 2 or 3, and b5 is 0 or 1. When b2=0, preferably b3 is 0, 1, 2 or 3 and b4 is 1, 2 or 3. When b2=1 or 2, preferably b3 is 0, 1, 2, 3 or 4 and b4 is 1, 2 or 3.

In formula (B2-1), R^{A} is hydrogen, fluorine, methyl or trifluoromethyl.

In formula (B2-1), R²¹ is halogen, an optionally halogenated C₁-C₆ saturated hydrocarbyl group, optionally halogenated C₁-C₆ saturated hydrocarbyloxy group or optionally halogenated C₂-C₈ saturated hydrocarbylcarbonyloxy group. The saturated hydrocarbyl group and saturated hydrocarbyl moiety in the saturated hydrocarbyloxy group and saturated hydrocarbylcarbonyloxy group may be straight, branched or cyclic, and examples thereof include alkyl groups such as methyl, ethyl, n-propyl, isopropyl, butyl, pentyl, hexyl, and structural isomers thereof, cycloalkyl groups such as cyclopropyl, cyclobutyl, cyclopentyl and cyclohexyl, and combinations thereof. A carbon count within the upper limit ensures good solubility in alkaline developer. A plurality of R²¹ may be identical or different when b3 is 2 or more.

In formula (B2-1), A² is a single bond or a C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of b1=1 in formula (B2-1), the ether bond may be incorporated at any position excluding the position between the α-carbon and β-carbon relative to the ester oxygen. In case of b1=0, the atom that bonds with the backbone becomes an ethereal oxygen, and a second ether bond may be incorporated at any position excluding the position between the α-carbon and β-carbon relative to that ethereal oxygen. Saturated hydrocarbylene groups having no more than 10 carbon atoms are desirable because of a sufficient solubility in alkaline developer.

In formula (B2-1), X is an acid labile group when b4=1. X is hydrogen or an acid labile group, at least one X being an acid labile group, when b4=2 or 3. That is, repeat units B2-1 have phenolic hydroxy groups bonded to an aromatic ring, at least one of which is protected with an acid labile group, or repeat units B2-1 have a carboxy group bonded to an aromatic ring, which is protected with an acid labile group. The acid labile group used herein is not particularly limited as long as it is commonly used in a number of well-known chemically amplified resist compositions and eliminated under the action of acid to release an acidic group.

Typical of the acid labile group is a tertiary saturated hydrocarbyl group. The tertiary saturated hydrocarbyl group is preferably of 4 to 18 carbon atoms because a monomer for use in polymerization is recoverable by distillation.

The saturated hydrocarbyl group bonded to the tertiary carbon atom in the tertiary saturated hydrocarbyl group is preferably of 1 to 15 carbon atoms. The C₁-C₁₅ saturated hydrocarbyl group may be straight, branched or cyclic and contain an oxygen-containing functional group such as an ether bond or carbonyl group in its carbon-carbon bond. The saturated hydrocarbyl groups bonded to the tertiary carbon atom may bond together to form a ring with the tertiary carbon atom to which they are attached.

Examples of the alkyl substituent include methyl, ethyl, propyl, adamantyl, norbornyl, tetrahydrofuran-2-yl, 7-oxanorbornan-2-yl, cyclopentyl, 2-tetrahydrofuryl, tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, tetracyclo[4.4.0.1^{2,5}.1^{7,10}]dodecyl, and 3-oxo-1-cyclohexyl.

Examples of the tertiary saturated hydrocarbyl group include, but are not limited to, tert-butyl, tert-pentyl, 1-ethyl-1-methylpropyl, 1,1-diethylpropyl, 1,1,2-trimethylpropyl, 1-adamantyl-1-methylethyl, 1-methyl-1-(2-norbornyl)ethyl, 1-methyl-1-(tetrahydrofuran-2-yl)ethyl, 1-methyl-1-(7-oxanorbornan-2-yl)ethyl, 1-methylcyclopentyl, 1-ethylcyclopentyl, 1-propylcyclopentyl, 1-cyclopentylcyclopentyl, 1-cyclohexylcyclopentyl, 1-(2-tetrahydrofuryl)cyclopentyl, 1-(7-oxanorbornan-2-yl)cyclopentyl, 1-methylcyclohexyl, 1-ethylcyclohexyl, 1-cyclopentylcyclohexyl, 1-cyclohexylcyclohexyl, 2-methyl-2-norbornyl, 2-ethyl-2-norbornyl, 8-methyl-8-tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, 3-ethyl-3-tetracyclo[4.4.0.1^{2,5}.1^{7,10}]dodecyl, 2-methyl-2-adamantyl, 2-ethyl-2-adamantyl, 1-methyl-3-oxo-1-cyclohexyl, 1-methyl-1-(tetrahydrofuran-2-yl)ethyl, 5-hydroxy-2-methyl-2-adamantyl, and 5-hydroxy-2-ethyl-2-adamantyl.

A group having the following formula (B2-1-1) is also suitable as the acid labile group. The group having formula (B2-1-1) is often used as the acid labile group. It is a good choice of the acid labile group that ensures to form a pattern having a relatively rectangular pattern-substrate interface in a consistent manner. An acetal structure is formed when X is a group having formula (B2-1-1).

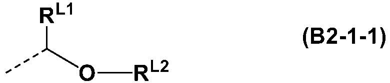

In formula (B2-1-1), R^{L1} is hydrogen or a C₁-C₁₀ saturated hydrocarbyl group. The saturated hydrocarbyl group may be straight, branched or cyclic.

A choice of R^{L1} may depend on the designed sensitivity of labile group to acid. For example, hydrogen or a group in which the carbon atom bonded to acetal carbon is tertiary is selected when the acid labile group is designed to ensure relatively high stability and to be decomposed with strong acid. Examples of R^{L1} bonded to acetal carbon via tertiary carbon include tert-butyl, tert-pentyl, and 1-adamantyl, but are not limited thereto. A straight alkyl group is selected when the acid labile group is designed to have relatively high reactivity and high sensitivity to pH changes. Although the choice varies with a particular combination of acid generator and quencher in the resist composition, R^{L1} is preferably a group in which the carbon in bond with acetal carbon is secondary, when R^{L2} is a relatively large alkyl group substituted at the end and the acid labile group is designed to undergo a substantial change of solubility by decomposition. Examples of R^{L1} bonded to acetal carbon via secondary carbon include isopropyl, sec-butyl, cyclopentyl, and cyclohexyl, but are not limited thereto.

In formula (B2-1-1), R^{L2} is a C₁-C₃₀ hydrocarbyl group. The hydrocarbyl group may be saturated or unsaturated and straight, branched or cyclic. Some constituent -CH₂-in the hydrocarbyl group may be replaced by a heteroatom such as oxygen or sulfur so that the group may contain an ether bond or sulfide bond. Illustrative are C₁-C₃₀ saturated hydrocarbyl groups and C₆-C₃₀ aryl groups. R^{L2} is preferably a C₁-C₆ hydrocarbyl group for acquiring a higher resolution in forming small-size patterns. When R^{L2} is a C₁-C₆ hydrocarbyl group, the alcohol created after a progress of acid-aided deprotection reaction is water soluble. Then, when a positive pattern is formed using an alkaline developer, the alcohol is dissolved in the developer so that defects remaining in the exposed region are minimized.

Preferred examples of the group having formula (B2-1-1) are given below, but not limited thereto. Herein R^{L1} is as defined above.

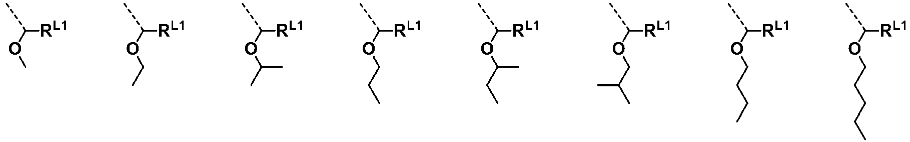

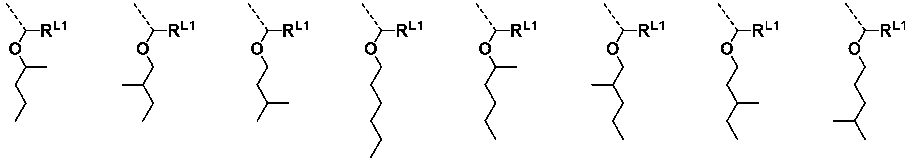

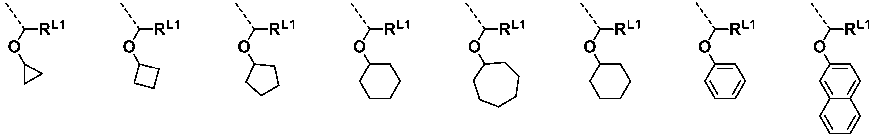

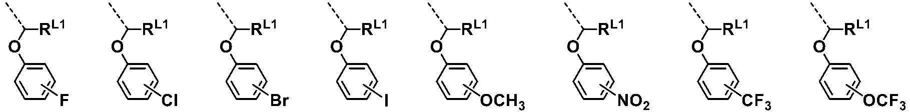

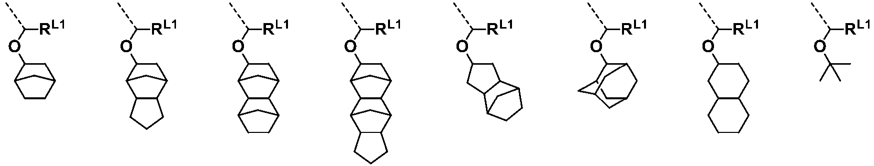

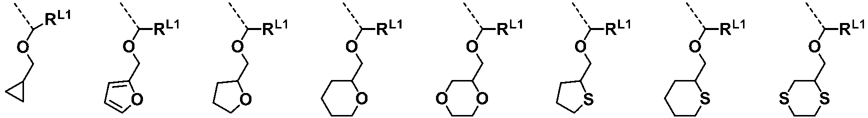

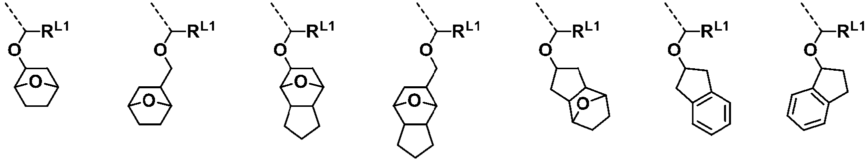

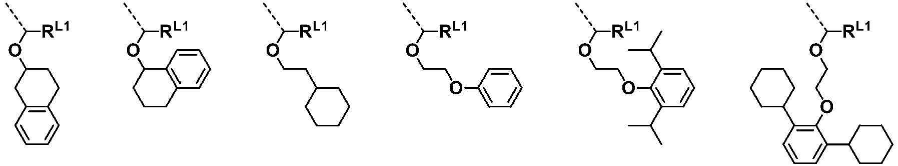

Another acid labile group which can be used herein is a phenolic hydroxy group whose hydrogen is substituted by -CH₂COO-(tertiary saturated hydrocarbyl group). The tertiary saturated hydrocarbyl group may be the same as the foregoing tertiary saturated hydrocarbyl group used for the protection of a phenolic hydroxy group.

Another example of repeat unit B2 is a repeat unit having the following formula (B2-2), referred to as repeat unit B2-2. The repeat unit B2-2 which enables to increase the dissolution rate in the exposed region is a useful choice of the acid labile group-containing unit which affords satisfactory performance against line width variations during develop loading.

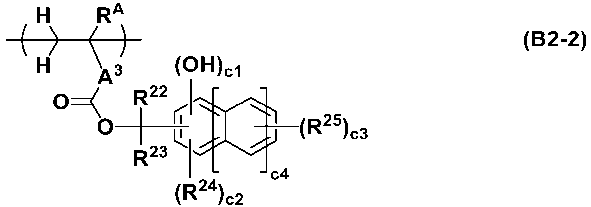

In formula (B2-2), c1 is 0, 1 or 2, c2 is 0, 1 or 2, c3 is 0, 1, 2, 3, 4 or 5, and c4 is 0, 1 or 2.

In formula (B2-2), R^{A} is hydrogen, fluorine, methyl or trifluoromethyl.

In formula (B2-2), R²² and R²³ are each independently a C₁-C₁₀ hydrocarbyl group which may contain a heteroatom. R²² and R²³ may bond together to form a ring with the carbon atom to which they are attached.

In formula (B2-2), R²⁴ is each independently fluorine, C₁-C₅ fluorinated alkyl group or C₁-C₅ fluorinated alkoxy group. A plurality of R²⁴ may be identical or different when c2=2.

In formula (B2-2), R²⁵ is each independently a C₁-C₁₀ hydrocarbyl group which may contain a heteroatom. A plurality of R²⁵ may be identical or different when c3 is 2, 3, 4 or 5.

In formula (B2-2), A³ is a single bond, phenylene group, naphthylene group, or *-C(=O)-O-A³¹-. A³¹ is a C₁-C₂₀ aliphatic hydrocarbylene group which may contain hydroxy, ether bond, ester bond or lactone ring, or phenylene or naphthylene group, and * is a point of attachment to the carbon atom in the backbone.

Preferred examples of the repeat unit B2-2 are shown below, but not limited thereto. Herein R^{A} is as defined above.

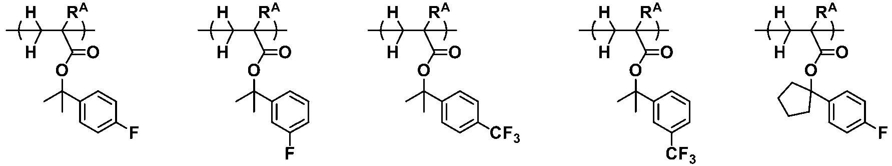

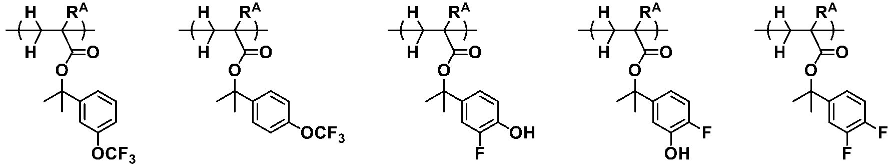

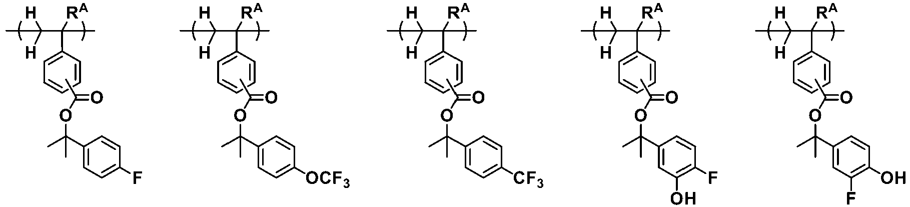

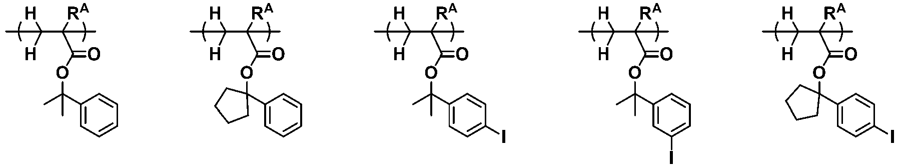

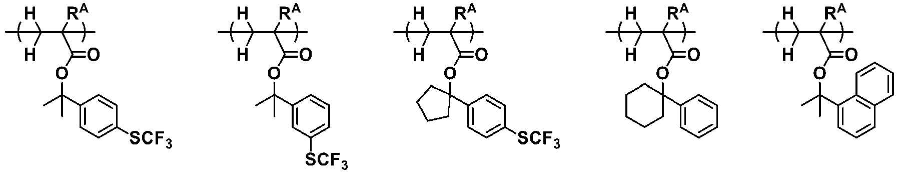

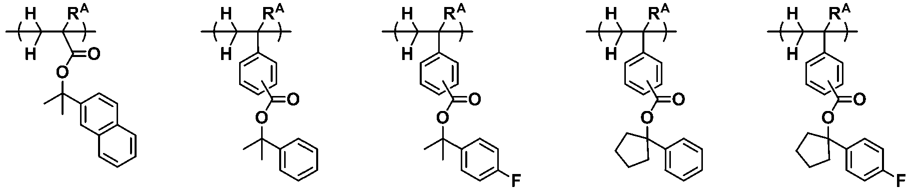

The content of repeat units B2 is preferably 5 to 95 mol%, more preferably 20 to 80 mol% based on the overall repeat units of the polymer. Each of repeat units B2 may be of one type or a mixture of two or more types.

In a preferred embodiment, the polymer further comprises repeat units of at least one type selected from units having the formulae (B3), (B4) and (B5). These repeat units are simply referred to as repeat units B3, B4 and B5, respectively.

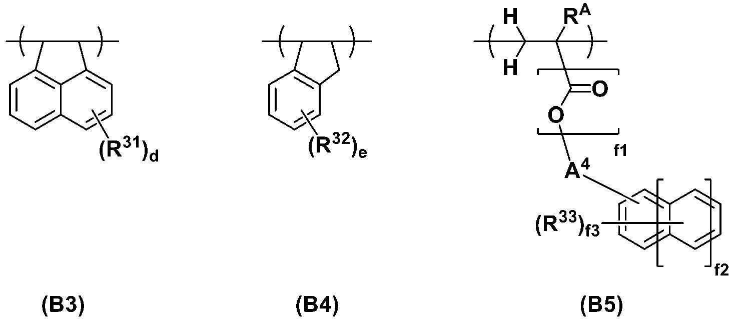

In formulae (B3) and (B4), d is 0, 1, 2, 3, 4, 5 or 6 and e is 0, 1, 2, 3 or 4.

In formulae (B3) and (B4), R³¹ and R³² are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group. The saturated hydrocarbyl group, saturated hydrocarbyloxy group and saturated hydrocarbylcarbonyloxy group may be straight, branched or cyclic. When d is 2, 3, 4, 5 or 6, a plurality of R³¹ may be identical or different. When e is 2, 3 or 4, a plurality of R³² may be identical or different.

In formula (B5), f1 is 0 or 1. The subscript f2 is 0, 1 or 2. The relevant structure represents a benzene skeleton when f2=0, a naphthalene skeleton when f2=1, and an anthracene skeleton when f2=2. The subscript f3 is 0, 1, 2, 3, 4 or 5. When f2=0, preferably f3 is 0, 1, 2 or 3. When f2=1 or 2, preferably f3 is 0, 1, 2, 3 or 4.

In formula (B5), R^{A} is hydrogen, fluorine, methyl or trifluoromethyl.

In formula (B5), R³³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen atom, nitro group, or cyano group. When f2 is 1 or 2, R³³ may also be hydroxy. The saturated hydrocarbyl group, saturated hydrocarbyloxy group, saturated hydrocarbylcarbonyloxy group, saturated hydrocarbyloxyhydrocarbyl group, and saturated hydrocarbylthiohydrocarbyl group may be straight, branched or cyclic. When f3 is 2, 3, 4 or 5, a plurality of R³³ may be identical or different.

In formula (B5), A⁴ is a single bond or a C₁-C₁₀ saturated hydrocarbylene group in which some constituent -CH₂- may be replaced by -O-. The saturated hydrocarbylene group may be straight, branched or cyclic. Examples thereof are as exemplified above for A¹ in formula (B1).

When repeat units of at least one type selected from repeat units B3 to B5 are incorporated, better performance is obtained because not only the aromatic ring possesses etch resistance, but the cyclic structure incorporated into the backbone also exerts the effect of improving etch resistance and resistance to EB irradiation during pattern inspection step.

The content of repeat units B3 to B5 is preferably at least 5 mol% based on the overall repeat units of the polymer for obtaining the effect of improving etch resistance. Also, the content of repeat units B3 to B5 is preferably up to 25 mol%, more preferably up to 20 mol% based on the overall repeat units of the polymer. When the relevant units are free of functional groups or have a functional group other than hydroxy, their content of up to 25 mol% is preferred because the risk of forming development defects is eliminated. Each of the repeat units B3 to B5 may be of one type or a combination of plural types.

It is preferred that the polymer comprise repeat units B1, repeat units B2, and repeat units of at least one type selected from repeat units B3 to B5, because both etch resistance and high resolution are achievable. The total content of these repeat units is preferably at least 60 mol%, more preferably at least 70 mol%, even more preferably at least 80 mol%, most preferably at least 90 mol% based on the overall repeat units of the polymer.

In another preferred embodiment, the polymer further comprises repeat units of at least one type selected from repeat units having the formula (B6), repeat units having the formula (B7), repeat units having the formula (B8), repeat units having the formula (B9), and repeat units having the formula (B10), shown below. Notably these repeat units are also referred to as repeat units B6 to B10.

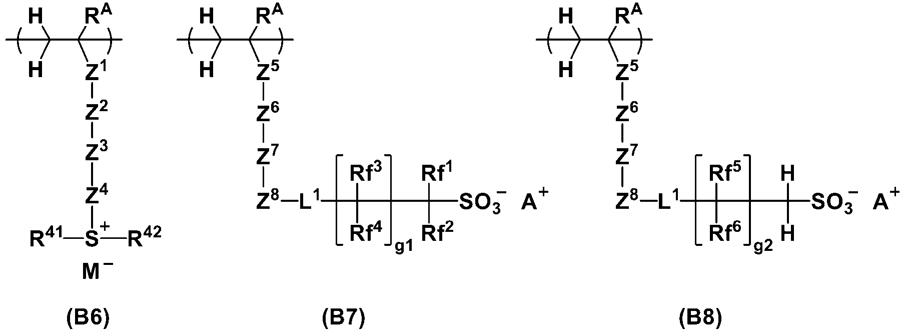

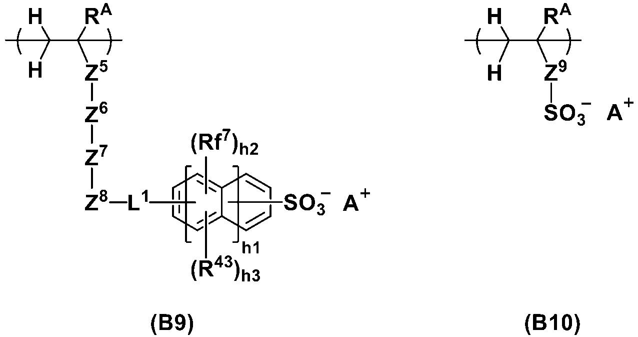

In formulae (B6) to (B10), R^{A} is each independently hydrogen, fluorine, methyl or trifluoromethyl. Z¹ is a single bond or an optionally substituted phenylene group. Z² is a single bond, **-C(=O)-O-Z²¹-, **-C(=O)-NH-Z²¹-, or **-O-Z²¹-, wherein Z²¹ is a C₁-C₆ aliphatic hydrocarbylene group, phenylene group or a divalent group obtained by combining the foregoing, which may contain halogen, carbonyl moiety, ester bond, ether bond or hydroxy moiety. Z³ is a single bond, ether bond, ester bond, amide bond, sulfonate ester bond, sulfonamide bond, carbonate bond or carbamate bond. Z⁴ is a single bond, or a C₁-C₆ aliphatic hydrocarbylene group, phenylene group or a divalent group obtained by combining the foregoing, which may contain halogen, carbonyl moiety, ester bond, ether bond or hydroxy moiety. Z⁵ is each independently a single bond, an optionally substituted phenylene, naphthylene, or *-C(=O)-O-Z⁵¹-, wherein Z⁵¹ is a C₁-C₁₀ aliphatic hydrocarbylene group which may contain halogen, hydroxy moiety, ether bond, ester bond or lactone ring, or phenylene or naphthylene group. Z⁶ is a single bond, ether bond, ester bond, amide bond, sulfonate ester bond, sulfonamide bond, carbonate bond or carbamate bond. Z⁷ is each independently a single bond, ***-Z⁷¹-C(=O)-O-, ***-C(=O)-NH-Z⁷¹-, or ***-O-Z⁷¹-, wherein Z⁷¹ is a C₁-C₂₀ hydrocarbylene group which may contain a heteroatom. Z⁸ is each independently a single bond, ****-Z⁸¹-C(=O)-O-, ****-C(=O)-NH-Z⁸¹-, or ****-O-Z⁸¹-, wherein Z⁸¹ is a C₁-C₂₀ hydrocarbylene group which may contain a heteroatom. Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, wherein Z⁹¹ is a C₁-C₆ aliphatic hydrocarbylene group, phenylene group, fluorinated phenylene group or trifluoromethyl-substituted phenylene group, which may contain a carbonyl moiety, ester bond, ether bond or hydroxy moiety. The asterisk * designates a point of attachment to the carbon atom in the backbone, ** is a point of attachment to Z¹, *** is a point of attachment to Z⁶, and **** is a point of attachment to Z⁷.

The aliphatic hydrocarbylene group represented by Z²¹, Z⁵¹ and Z⁹¹ may be straight, branched or cyclic. Examples thereof include alkanediyl groups such as methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, 1,1-dimethylethane-1,2-diyl, pentane-1,5-diyl, 2-methylbutane-1,2-diyl, and hexane-1,6-diyl; cycloalkanediyl groups such as cyclopropanediyl, cyclobutanediyl, cyclopentanediyl, and cyclohexanediyl; and combinations thereof.

The hydrocarbylene group represented by Z⁷¹ and Z⁸¹ may be saturated or unsaturated and straight, branched or cyclic. Examples thereof are shown below, but not limited thereto.

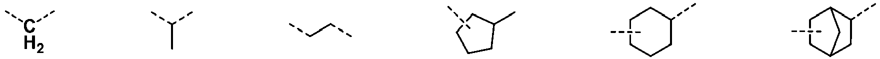

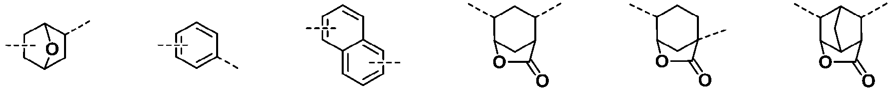

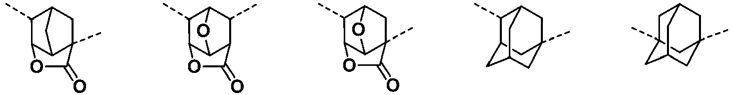

In formula (B6), R⁴¹ and R⁴² are each independently a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, and tert-butyl; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl, and adamantyl; C₂-C₂₀ alkenyl groups such as vinyl, 1-propenyl, 2-propenyl, butenyl, and hexenyl; C₃-C₂₀ cyclic unsaturated hydrocarbyl groups such as cyclohexenyl; C₆-C₂₀ aryl groups such as phenyl, naphthyl and thienyl; C₇-C₂₀ aralkyl groups such as benzyl, 1-phenylethyl and 2-phenylethyl, and combinations thereof. Inter alia, aryl groups are preferred. In the hydrocarbyl group, some or all of the hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-), or haloalkyl moiety.

R⁴¹ and R⁴² may bond together to form a ring with the sulfur atom to which they are attached. Examples of the ring are shown below.

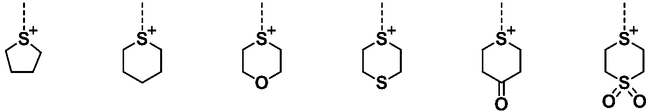

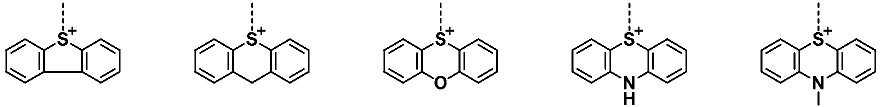

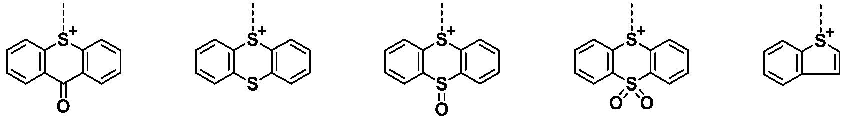

Herein the broken line designates a point of attachment to Z⁴.

Examples of the cation in repeat units B6 are shown below, but not limited thereto. Herein R^{A} is as defined above.

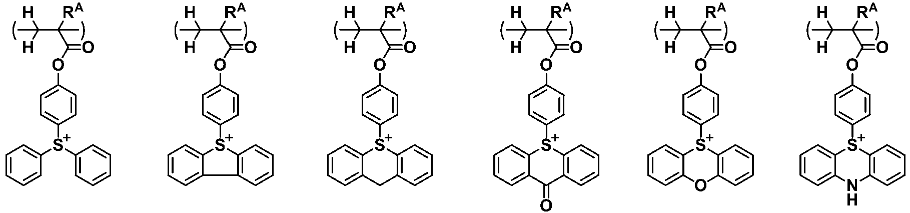

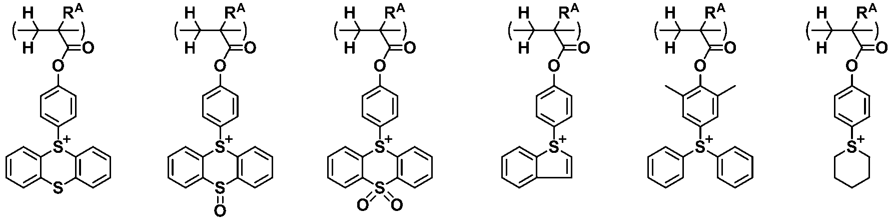

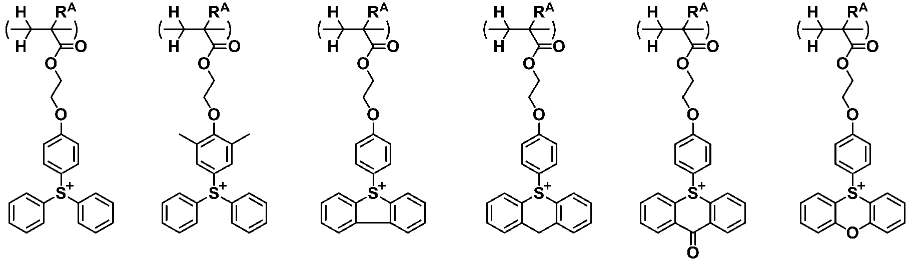

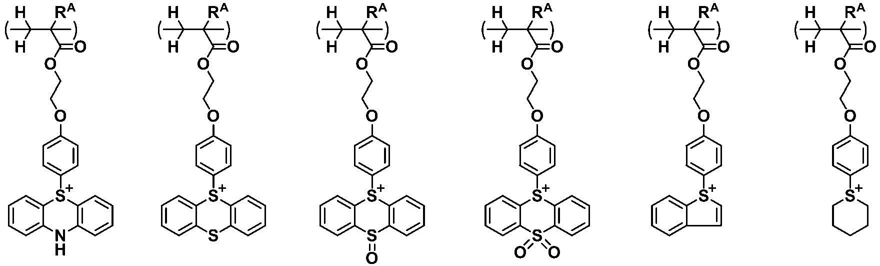

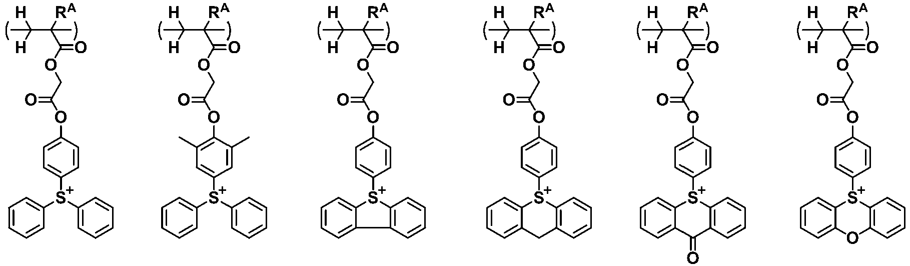

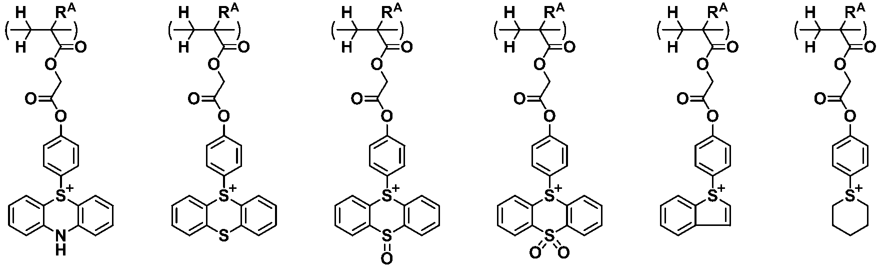

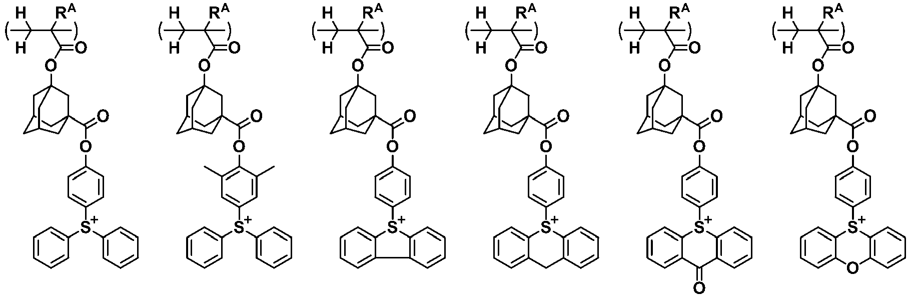

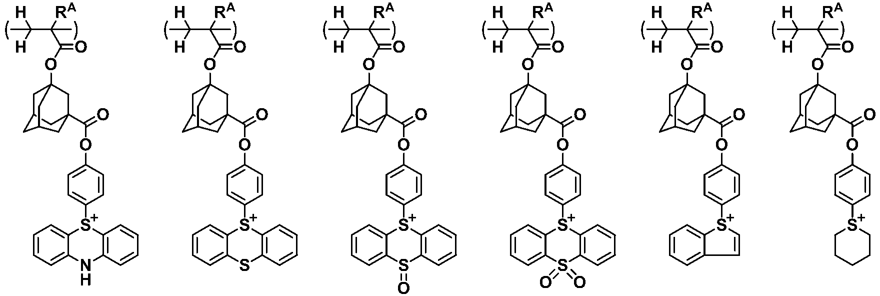

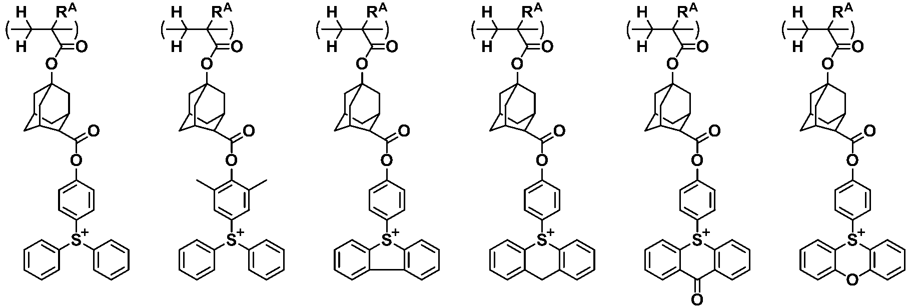

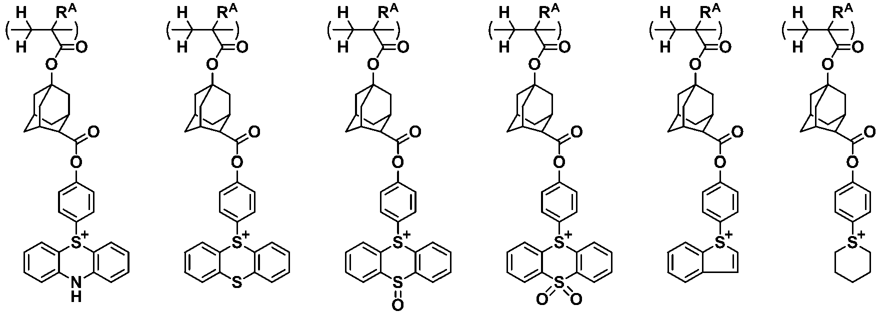

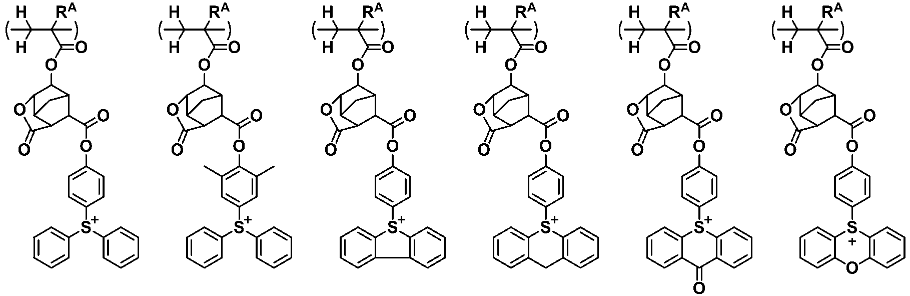

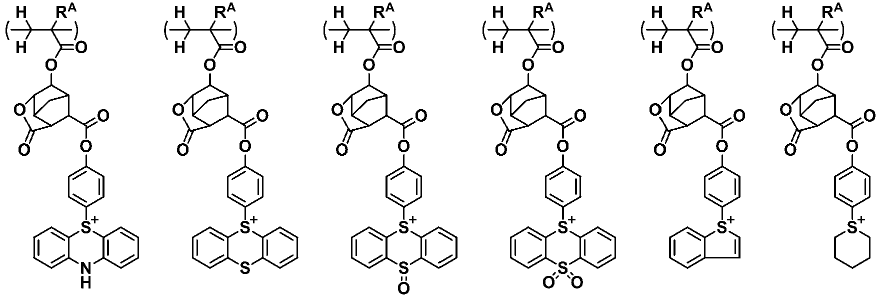

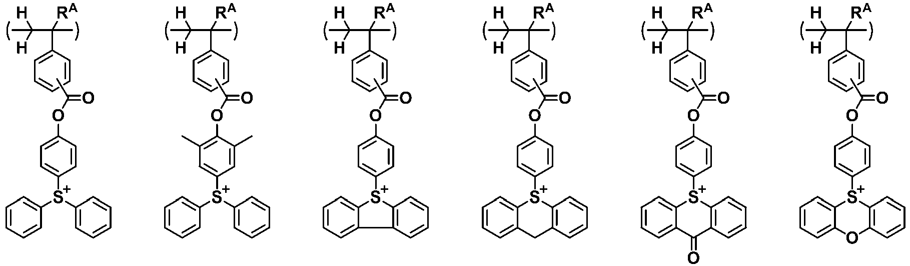

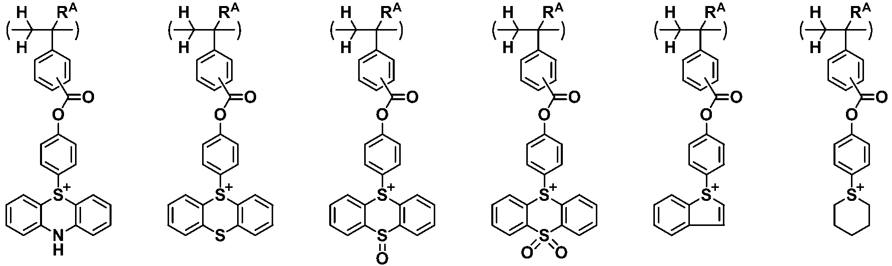

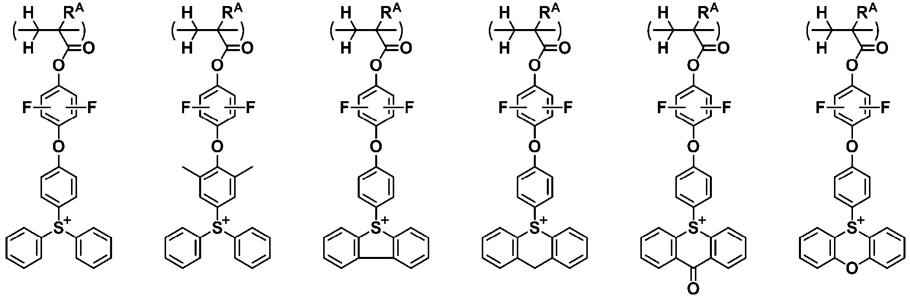

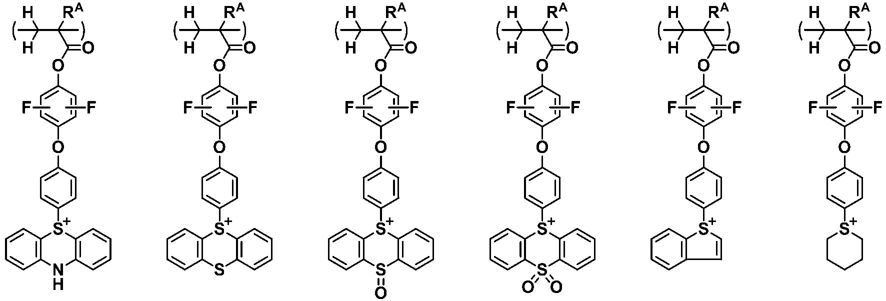

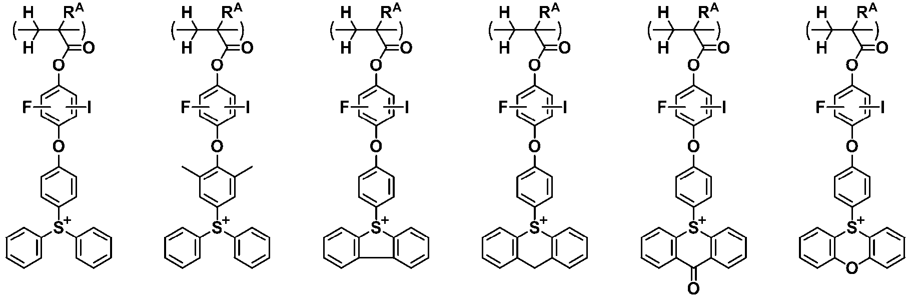

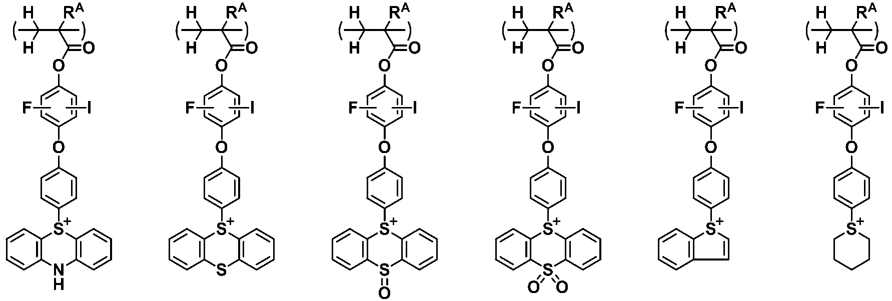

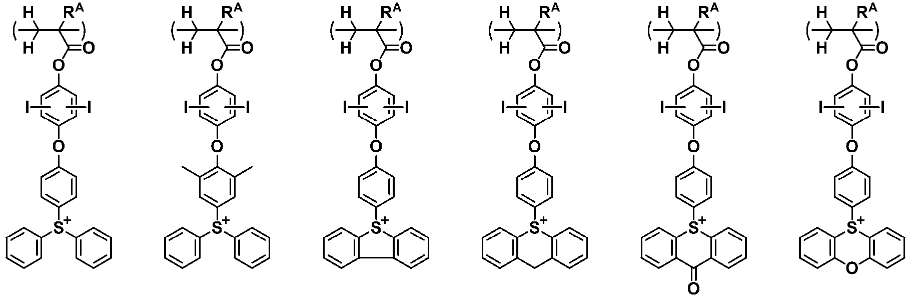

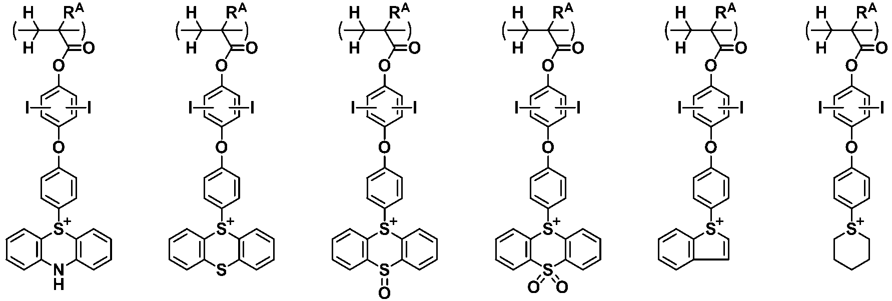

In formula (B6), M⁻ is a non-nucleophilic counter ion. Halide ions, sulfonate anions, imide anions, and methide anions are preferred. Examples of the non-nucleophilic counter ion include halide ions such as chloride and bromide ions; sulfonate anions, specifically fluoroalkylsulfonate ions such as triflate, 1,1,1-trifluoroethanesulfonate, and nonafluorobutanesulfonate, arylsulfonate ions such as tosylate, benzenesulfonate, 4-fluorobenzenesulfonate, and 1,2,3,4,5-pentafluorobenzenesulfonate, alkylsulfonate ions such as mesylate and butanesulfonate; imide ions such as bis(trifluoromethylsulfonyl)imide, bis(perfluoroethylsulfonyl)imide and bis(perfluorobutylsulfonyl)imide; and methide ions such as tris(trifluoromethylsulfonyl)methide and tris(perfluoroethylsulfonyl)methide.

Anions having the following formulae (B6-1) to (B6-4) are also useful as the non-nucleophilic counter ion.

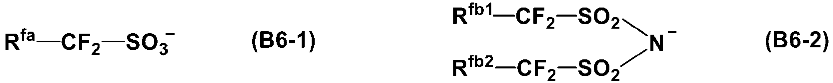

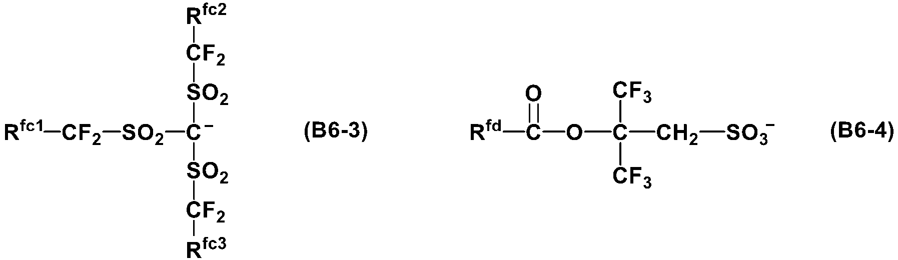

In formula (B6-1), R^{fa} is fluorine or a C₁-C₄₀ hydrocarbyl group which may contain a heteroatom. The hydrocarbyl group may be saturated or unsaturated and straight, branched or cyclic. Examples thereof are as will be exemplified below for the hydrocarbyl group R^{fa1} in formula (B6-1-1).

Of the anions of formula (B6-1), an anion having the formula (B6-1-1) is preferred.

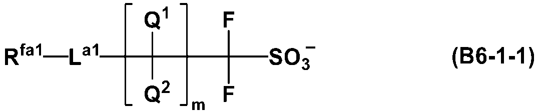

In formula (B6-1-1), Q¹ and Q² are each independently hydrogen, fluorine or a C₁-C₆ fluorinated saturated hydrocarbyl group. It is preferred for solvent solubility that at least one of Q¹ and Q² be trifluoromethyl. The subscript m is 0, 1, 2, 3 or 4, preferably 1.

R^{fa1} is a C₁-C₃₅ hydrocarbyl group which may contain a heteroatom. As the heteroatom, oxygen, nitrogen, sulfur and halogen atoms are preferred, with oxygen being most preferred. Of the hydrocarbyl groups, those groups of 6 to 30 carbon atoms are preferred from the aspect of achieving a high resolution in forming patterns of small feature size. The hydrocarbyl group may be saturated or unsaturated and straight, branched or cyclic. Examples thereof include C₁-C₃₅ alkyl groups such as methyl, ethyl, n-propyl, isopropyl, n-butyl, isobutyl, sec-butyl, tert-butyl, pentyl, neopentyl, hexyl, heptyl, 2-ethylhexyl, nonyl, undecyl, tridecyl, pentadecyl, heptadecyl, and icosyl; C₃-C₃₅ cyclic saturated hydrocarbyl groups such as cyclopentyl, cyclohexyl, 1-adamantyl, 2-adamantyl, 1-adamantylmethyl, norbornyl, norbornylmethyl, tricyclodecyl, tetracyclododecyl, tetracyclododecylmethyl, and dicyclohexylmethyl; C₂-C₃₅ unsaturated aliphatic hydrocarbyl groups such as 2-propenyl and 3-cyclohexenyl; C₆-C₃₅ aryl groups such as phenyl, 1-naphthyl, 2-naphthyl and 9-fluorenyl; and C₇-C₃₅ aralkyl groups such as benzyl and diphenylmethyl, and combinations thereof.

In the foregoing hydrocarbyl groups, some or all hydrogen may be substituted by a moiety containing a heteroatom such as oxygen, sulfur, nitrogen or halogen, or some constituent -CH₂- may be replaced by a moiety containing a heteroatom such as oxygen, sulfur or nitrogen, so that the group may contain a hydroxy, fluorine, chlorine, bromine, iodine, cyano, nitro, carbonyl, ether bond, ester bond, sulfonate ester bond, carbonate bond, lactone ring, sultone ring, carboxylic anhydride (-C(=O)-O-C(=O)-) or haloalkyl moiety. Examples of the heteroatom-containing hydrocarbyl group include tetrahydrofuryl, methoxymethyl, ethoxymethyl, methylthiomethyl, acetamidomethyl, trifluoroethyl, (2-methoxyethoxy)methyl, acetoxymethyl, 2-carboxy-1-cyclohexyl, 2-oxopropyl, 4-oxo-1-adamantyl, and 3-oxocyclohexyl.

In formula (B6-1-1), L^{a1} is a single bond, ether bond, ester bond, sulfonate ester bond, carbonate bond or carbamate bond. From the aspect of synthesis, an ether bond or ester bond is preferred, with the ester bond being more preferred.

Examples of the anion having formula (B6-1) are shown below, but not limited thereto. Herein Q¹ is as defined above.

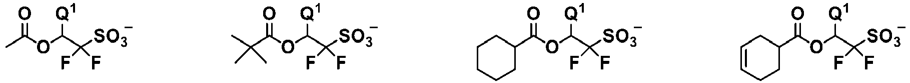

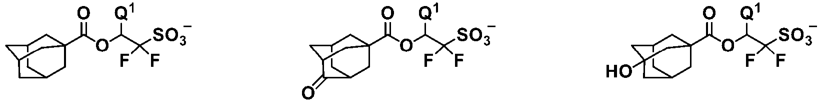

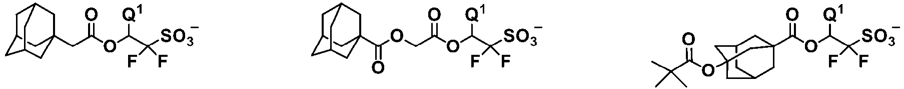

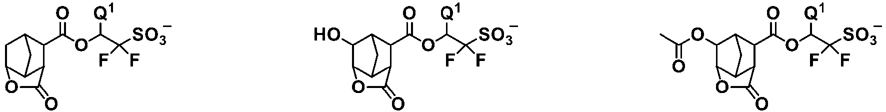

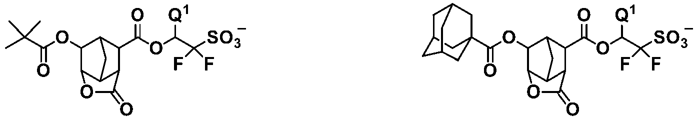

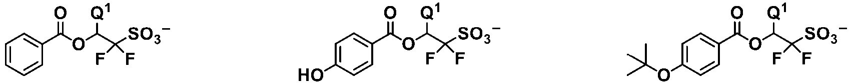

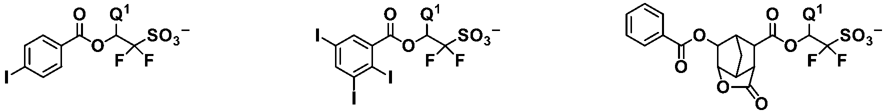

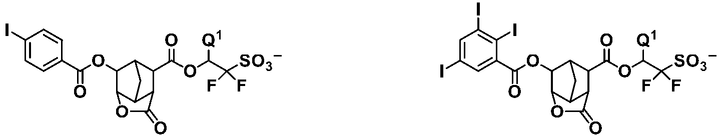

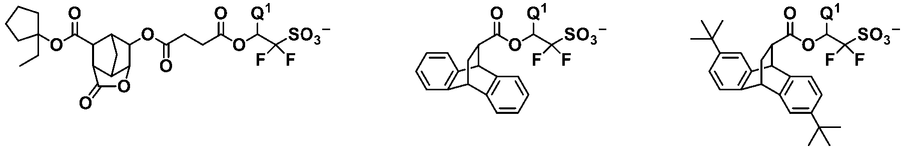

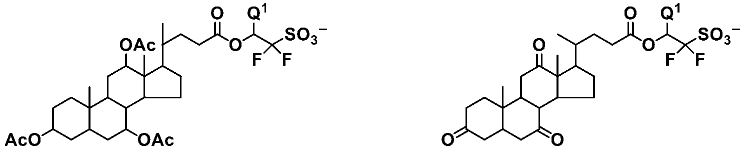

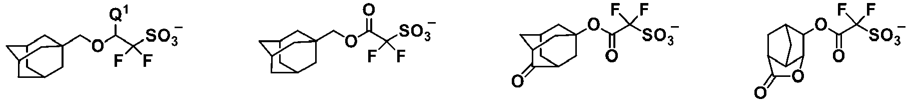

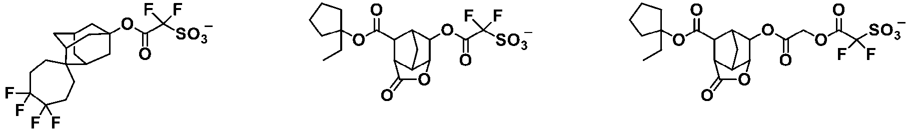

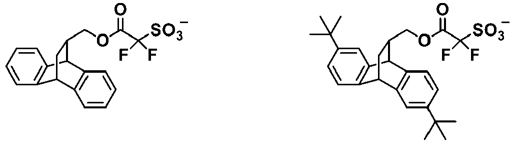

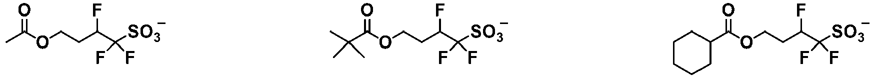

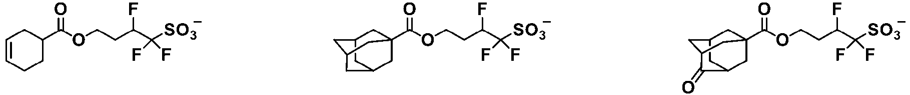

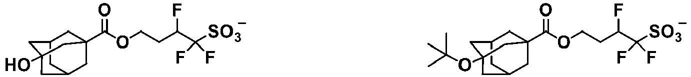

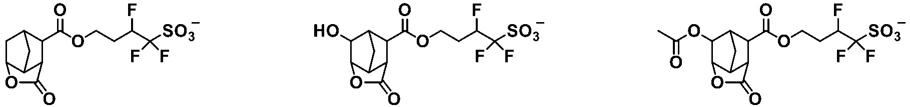

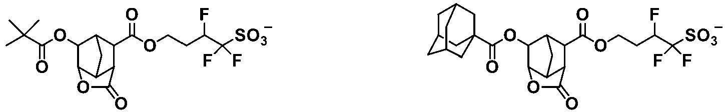

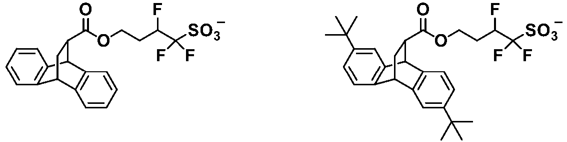

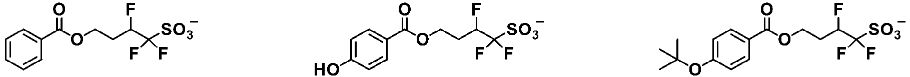

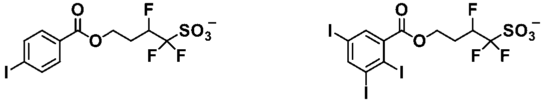

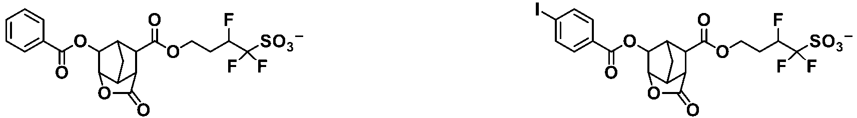

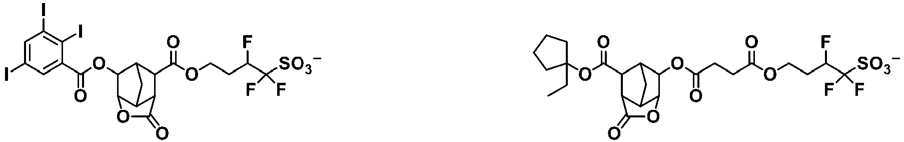

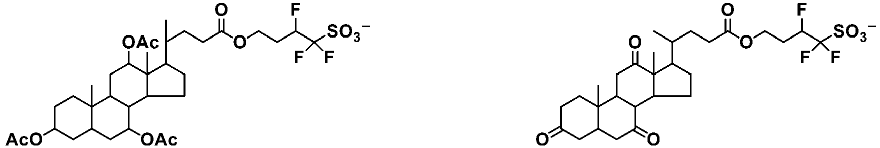

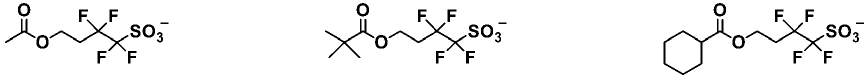

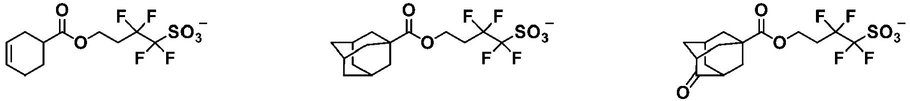

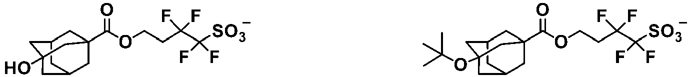

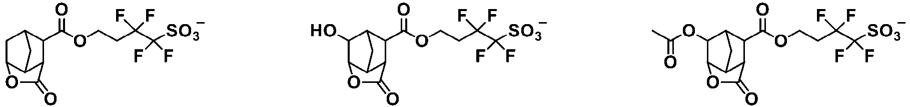

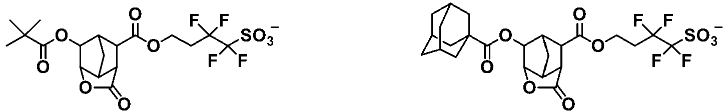

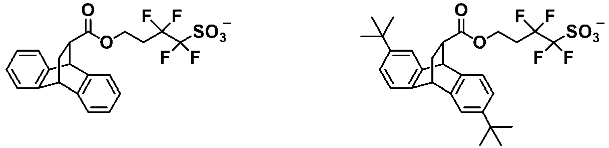

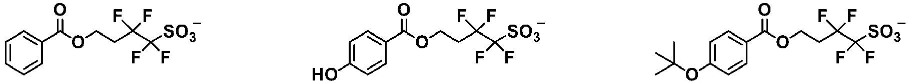

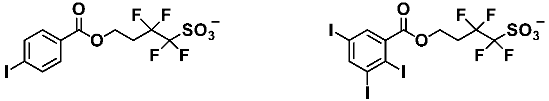

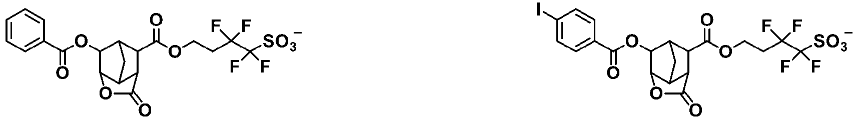

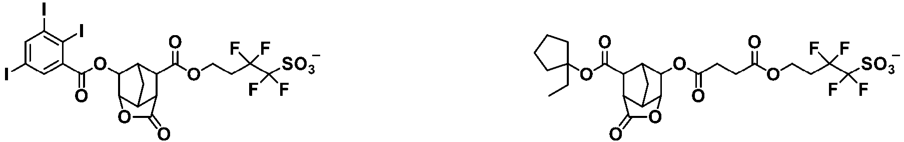

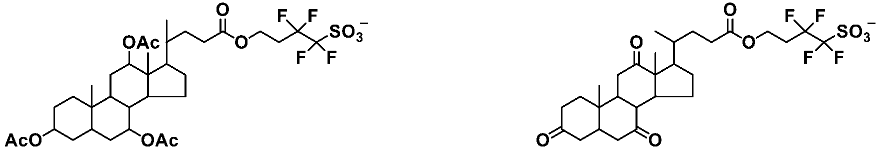

In formula (B6-2), R^{fb1} and R^{fb2} are each independently fluorine or a C₁-C₄₀ hydrocarbyl group which may contain a heteroatom. The hydrocarbyl group may be saturated or unsaturated and straight, branched or cyclic. Examples thereof are as exemplified above for R^{fa1} in formula (B6-1-1). Preferably R^{fb1} and R^{fb2} are fluorine or C₁-C₄ straight fluorinated alkyl groups. Also, R^{fb1} and R^{fb2} may bond together to form a ring with the linkage: -CF₂-SO₂-N⁻-SO₂-CF₂- to which they are attached. It is preferred that a combination of R^{fb1} and R^{fb2} be a fluorinated ethylene or fluorinated propylene group.

In formula (B6-3), R^{fc1}, R^{fc2} and R^{fc3} are each independently fluorine or a C₁-C₄₀ hydrocarbyl group which may contain a heteroatom. The hydrocarbyl group may be saturated or unsaturated and straight, branched or cyclic. Examples thereof are as exemplified for R^{fa1} in formula (B6-1-1). Preferably R^{fc1}, R^{fc2} and R^{fc3} are fluorine or C₁-C₄ straight fluorinated alkyl groups. Also, R^{fc1} and R^{fc2} may bond together to form a ring with the linkage: -CF₂-SO₂-C⁻-SO₂-CF₂- to which they are attached. It is preferred that a combination of R^{fc1} and R^{fc2} be a fluorinated ethylene or fluorinated propylene group.

In formula (B6-4), R^{fd} is a C₁-C₄₀ hydrocarbyl group which may contain a heteroatom. The hydrocarbyl group may be saturated or unsaturated and straight, branched or cyclic. Examples thereof are as exemplified above for R^{fa1} in formula (B6-1-1).

Examples of the anion having formula (B6-4) are shown below, but not limited thereto.

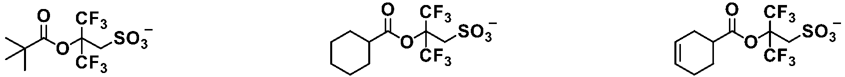

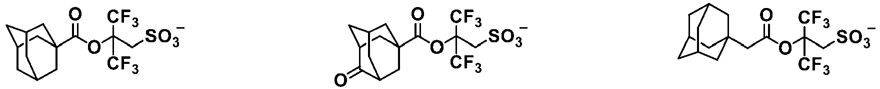

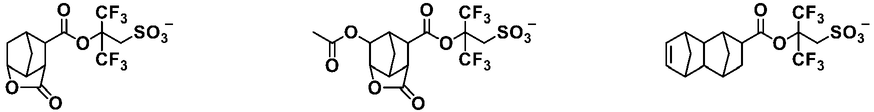

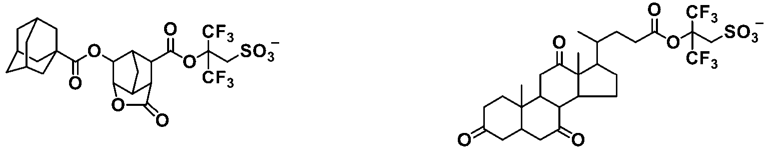

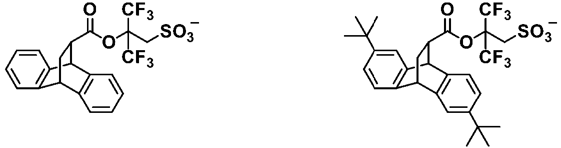

Anions having an iodized or brominated aromatic ring are also useful as the non-nucleophilic counter ion. These anions have the formula (B6-5).

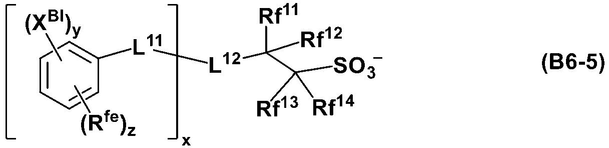

In formula (B6-5), x is 1, 2 or 3, y is 1, 2, 3, 4 or 5, z is 0, 1, 2 or 3, and y+z is from 1 to 5. Preferably, y is 1, 2 or 3, more preferably 2 or 3, and z is 0, 1 or 2.

In formula (B6-5), X^{BI} is iodine or bromine. A plurality of X^{BI} may be identical or different when x and/or y is 2 or more.

In formula (B6-5), L¹¹ is a single bond, ether bond, ester bond, or a C₁-C₆ saturated hydrocarbylene group which may contain an ether bond or ester bond. The saturated hydrocarbylene group may be straight, branched or cyclic.

In formula (B6-5), L¹² is a single bond or a C₁-C₂₀ divalent linking group when x=1. L¹² is a C₁-C₂₀ (x+1)-valent linking group when x=2 or 3. The linking group may contain an oxygen, sulfur or nitrogen atom.

In formula (B6-5), R^{fe} is hydroxy, carboxy, fluorine, chlorine, bromine, amino group, or a C₁-C₂₀ hydrocarbyl, C₁-C₂₀ hydrocarbyloxy, C₂-C₂₀ hydrocarbylcarbonyl, C₂-C₂₀ hydrocarbyloxycarbonyl, C₂-C₂₀ hydrocarbylcarbonyloxy, or C₁-C₂₀ hydrocarbylsulfonyloxy group, which may contain fluorine, chlorine, bromine, hydroxy, amino or ether bond, or -N(R^{feA})(R^{feB}), -N(R^{feC})-C(=O)-R^{feD} or -N(R^{feC})-C(=O)-O-R^{feD}. R^{feA} and R^{feB} are each independently hydrogen or a C₁-C₆ saturated hydrocarbyl group. R^{feC} is hydrogen, or a C₁-C₆ saturated hydrocarbyl group which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. R^{feD} is a C₁-C₁₆ aliphatic hydrocarbyl group, C₆-C₁₂ aryl group or C₇-C₁₅ aralkyl group, which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. The aliphatic hydrocarbyl group may be saturated or unsaturated and straight, branched or cyclic. The hydrocarbyl, hydrocarbyloxy, hydrocarbylcarbonyl, hydrocarbyloxycarbonyl, hydrocarbylcarbonyloxy, and hydrocarbylsulfonyloxy groups may be straight, branched or cyclic. A plurality of R^{fe} may be identical or different when x and/or z is 2 or more.

Of these, R^{fe} is preferably hydroxy, -N(R^{feC})-C(=O)-R^{feD}, -N(R^{feC})-C(=O)-O-R^{feD}, fluorine, chlorine, bromine, methyl or methoxy.

In formula (B6-5), Rf¹¹ to Rf¹⁴ are each independently hydrogen, fluorine or trifluoromethyl, at least one of RP¹¹ to Rf¹⁴ is fluorine or trifluoromethyl. Rf¹¹ and Rf¹², taken together, may form a carbonyl group. More preferably, both Rf¹³ and Rf¹⁴ are fluorine.

Examples of the anion having formula (B6-5) are shown below, but not limited thereto. X^{B1} is as defined above.

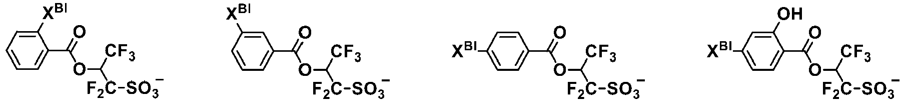

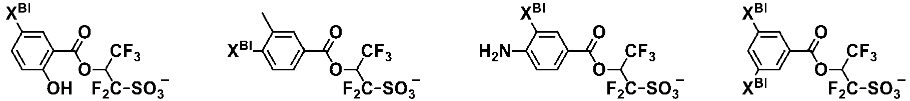

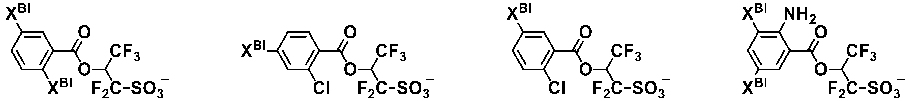

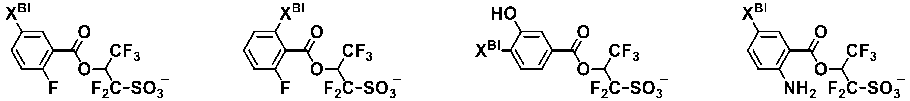

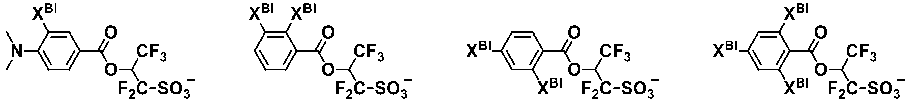

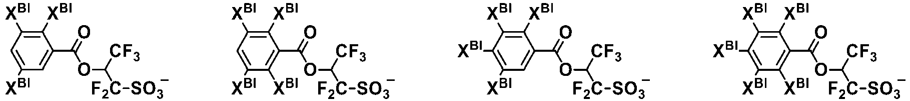

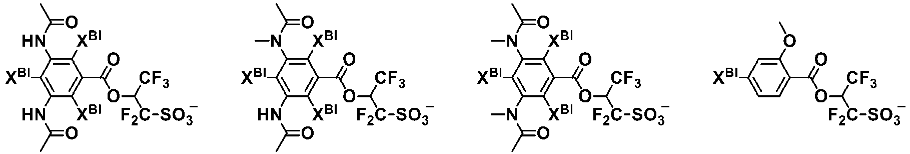

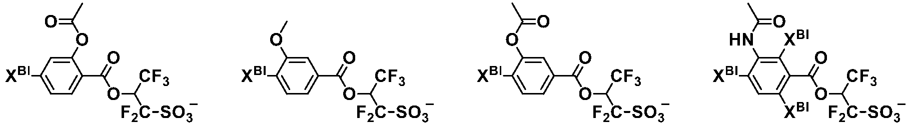

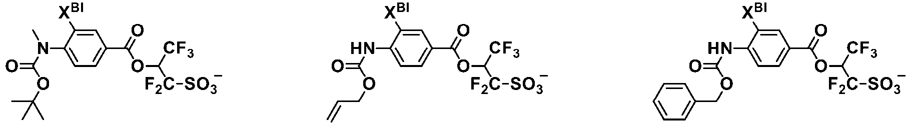

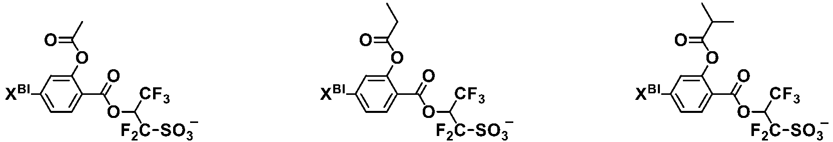

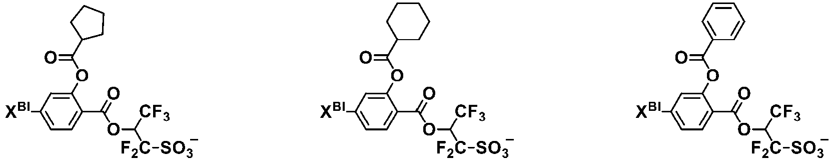

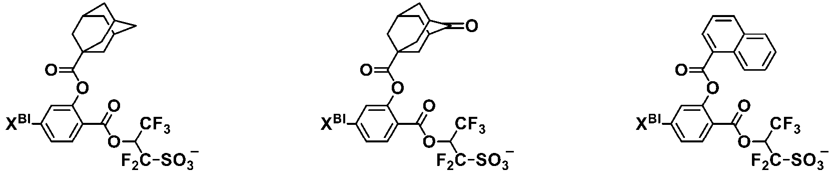

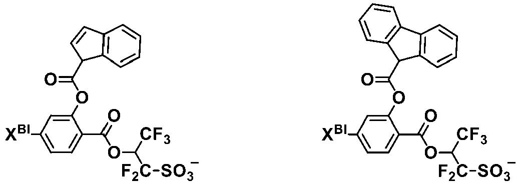

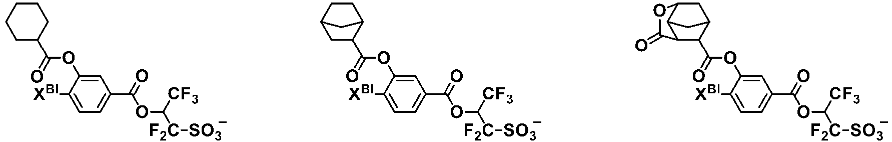

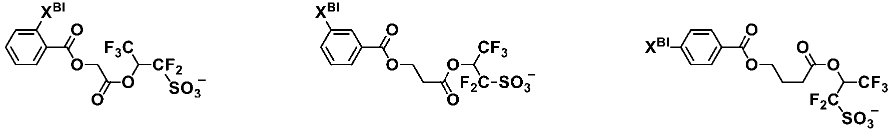

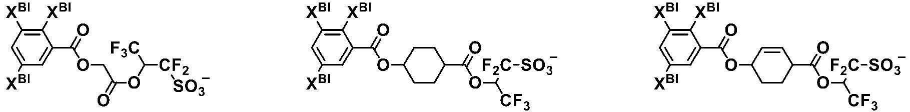

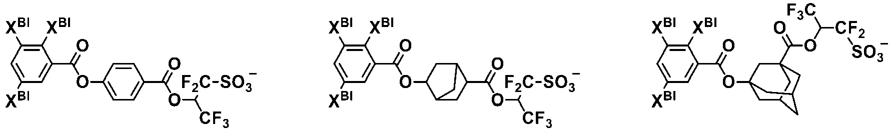

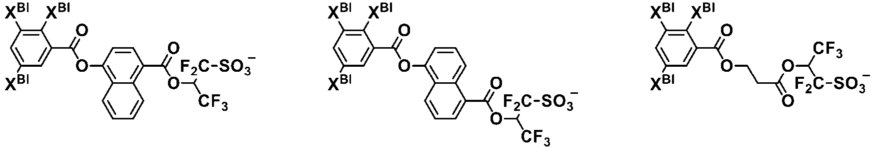

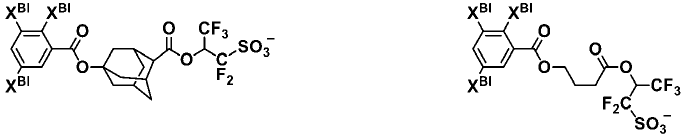

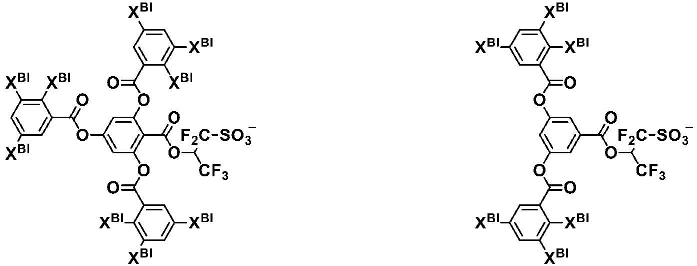

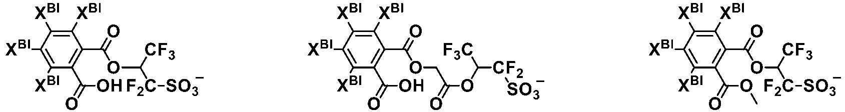

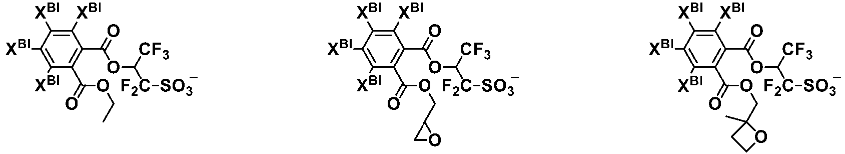

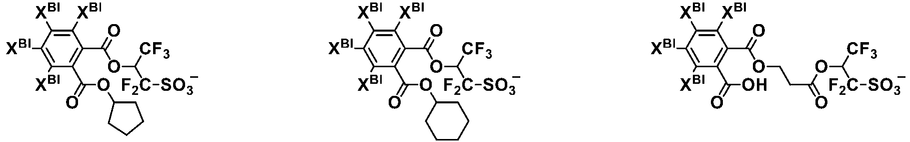

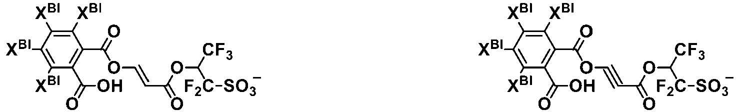

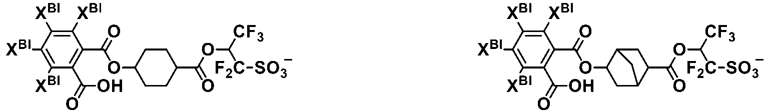

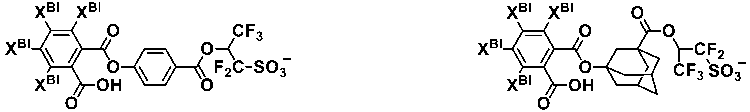

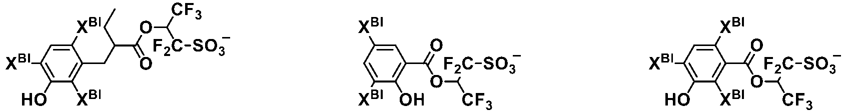

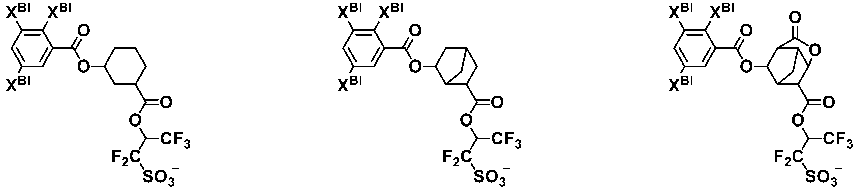

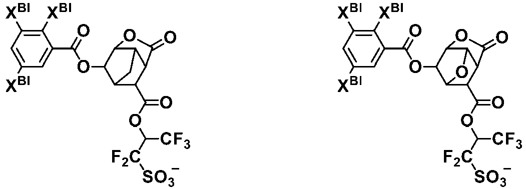

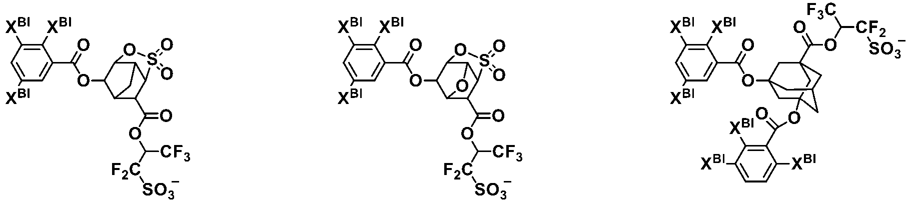

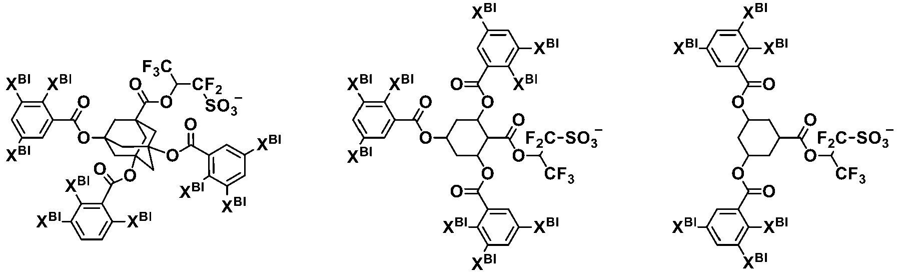

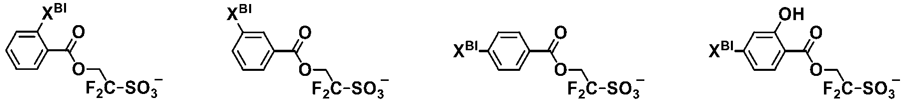

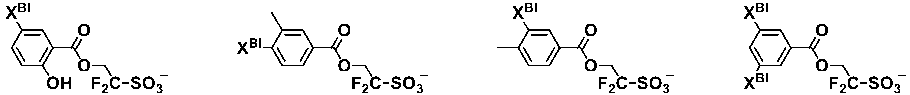

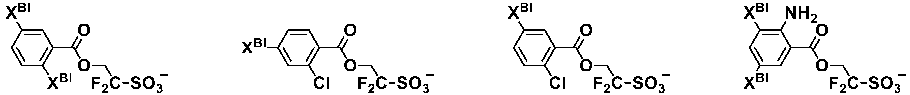

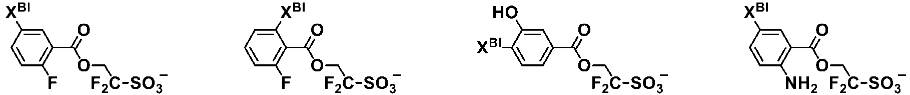

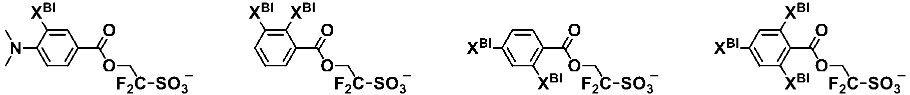

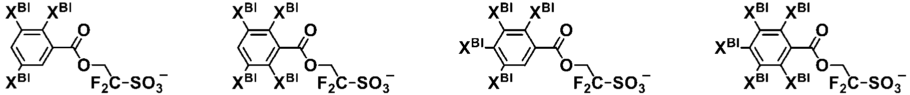

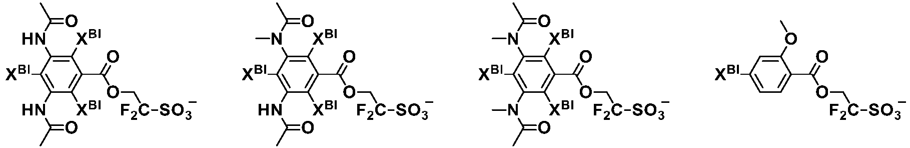

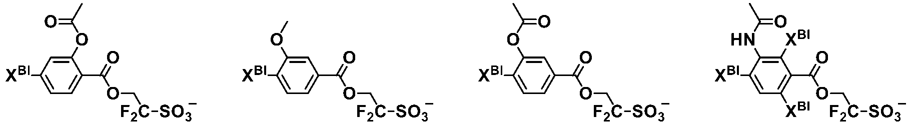

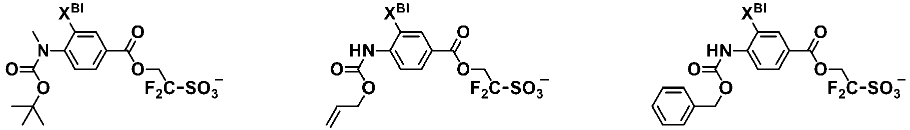

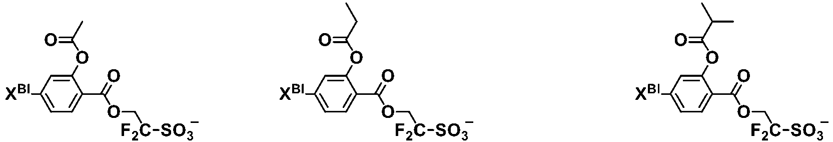

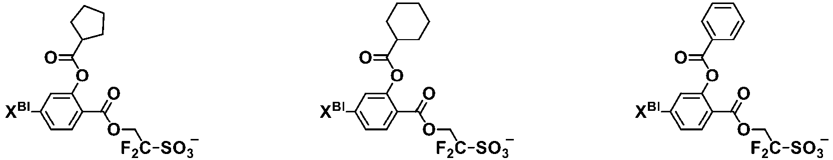

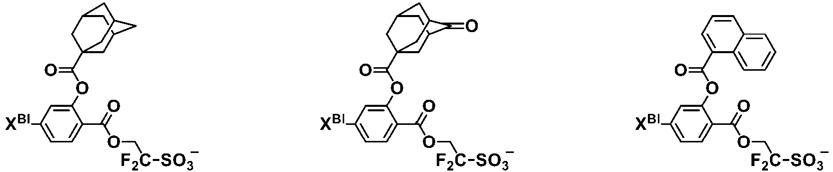

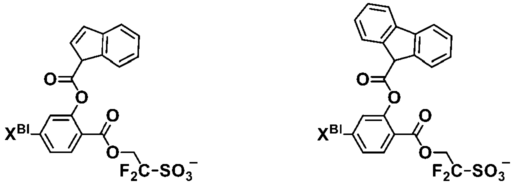

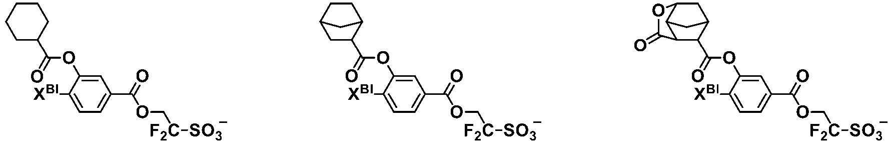

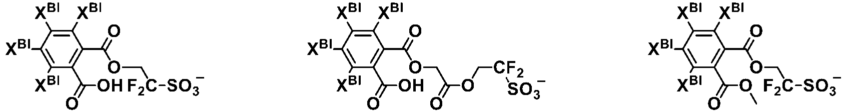

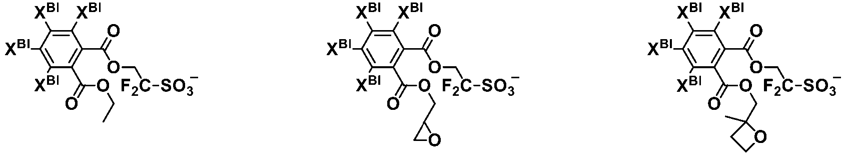

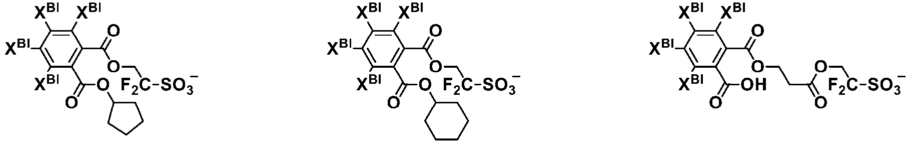

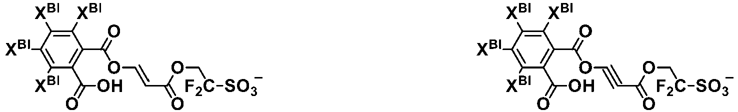

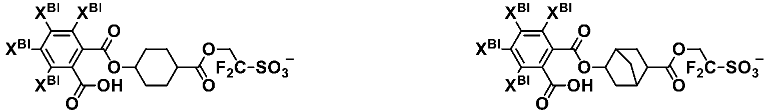

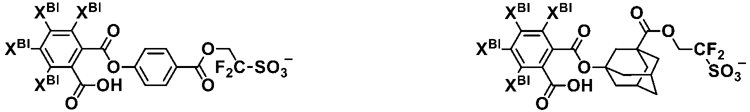

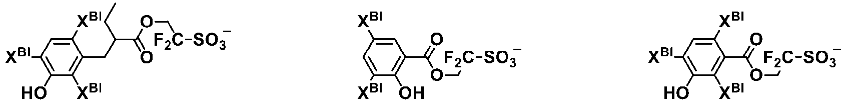

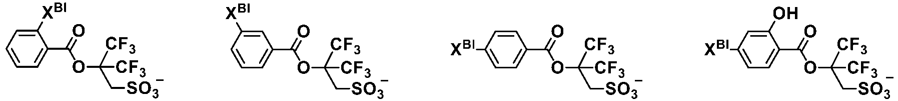

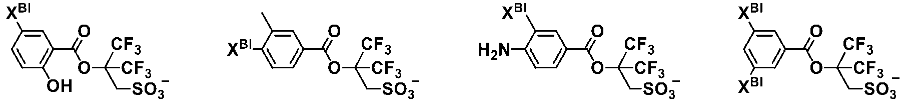

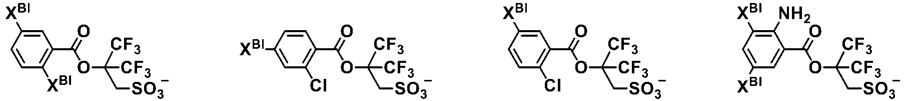

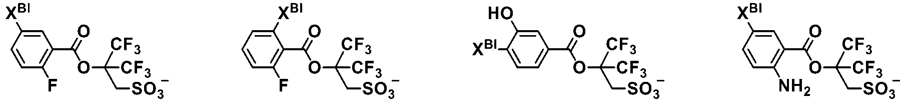

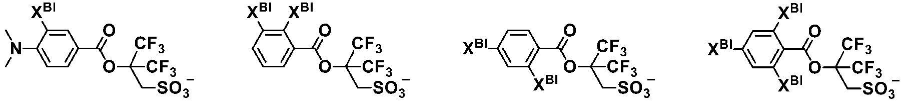

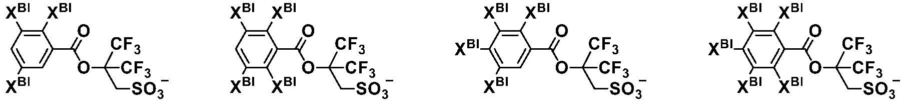

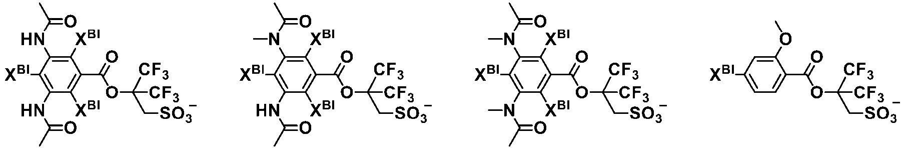

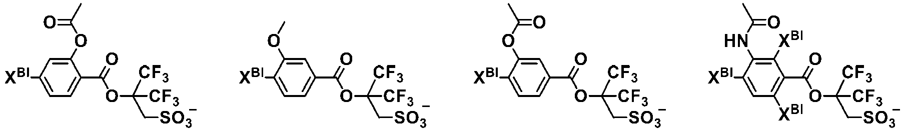

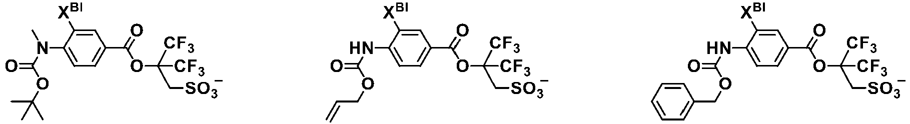

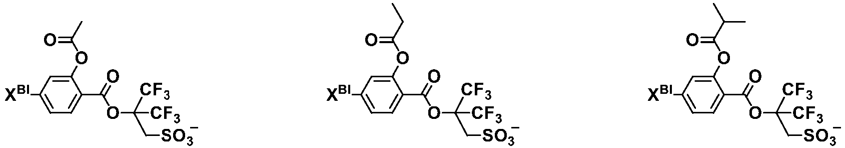

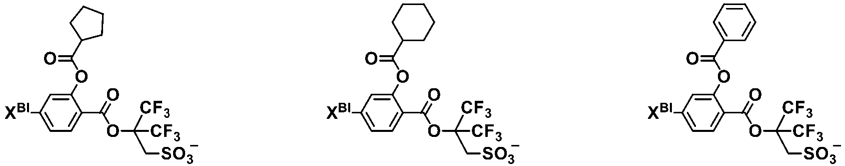

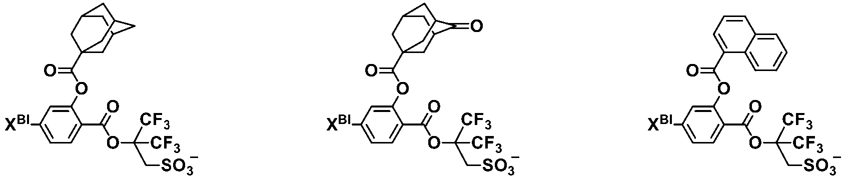

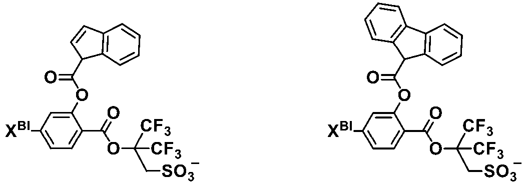

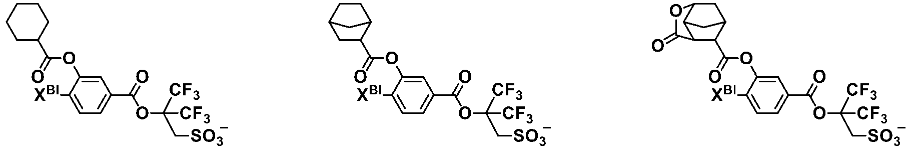

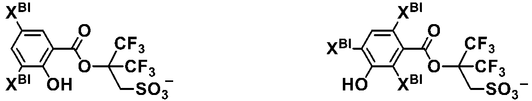

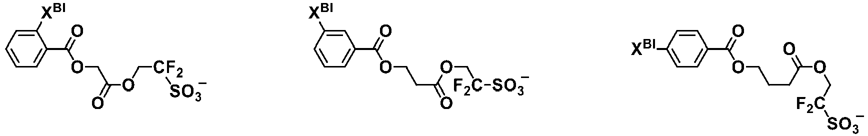

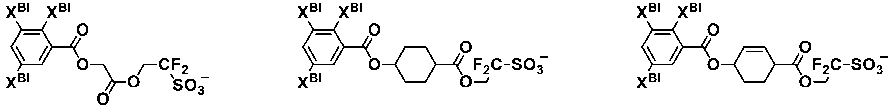

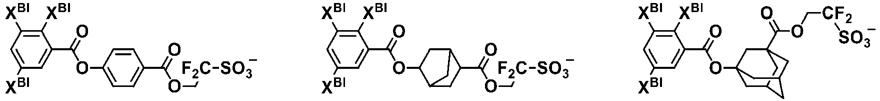

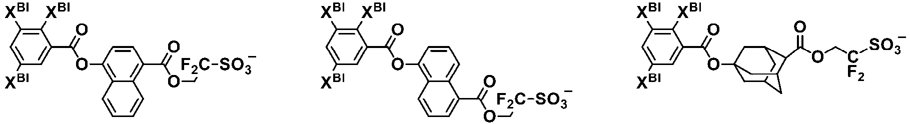

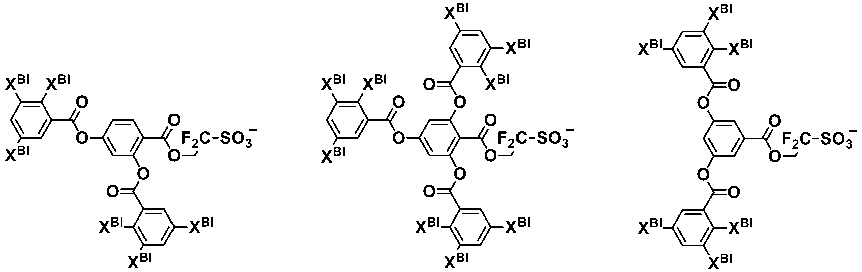

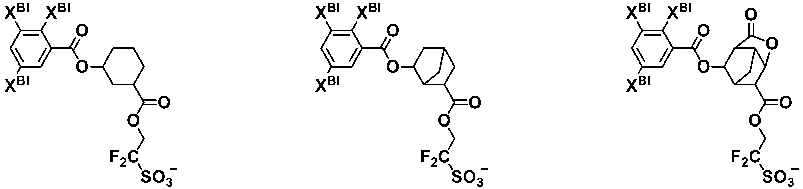

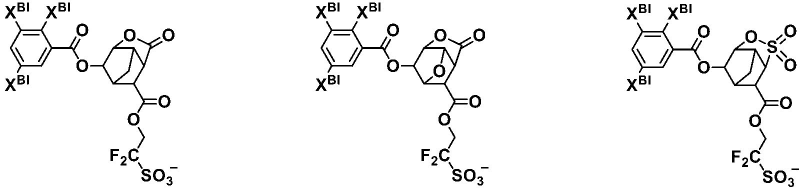

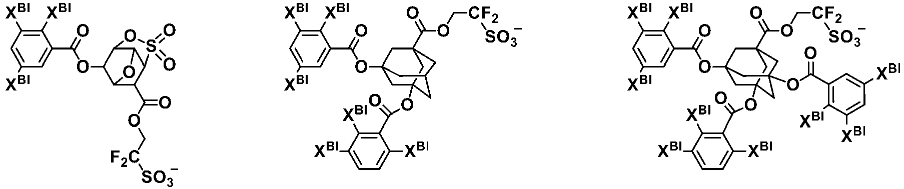

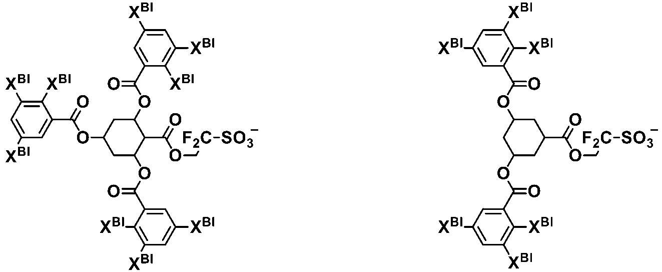

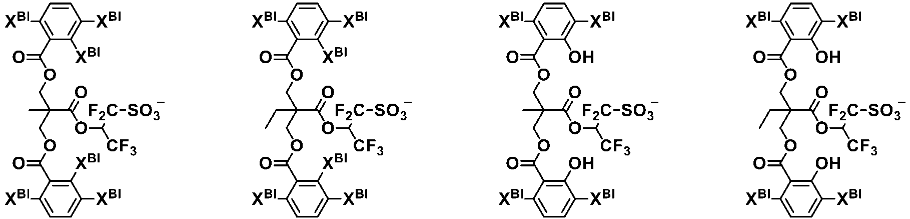

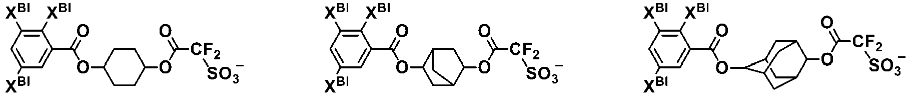

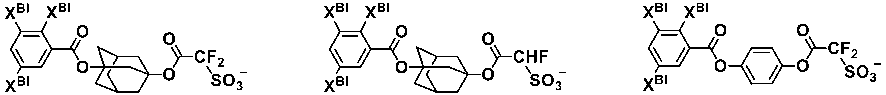

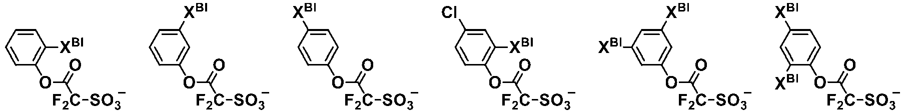

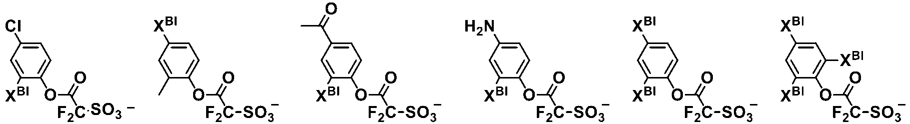

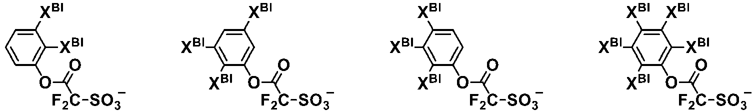

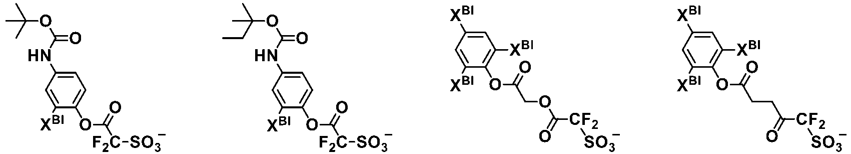

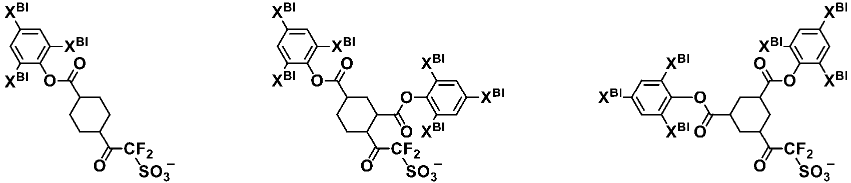

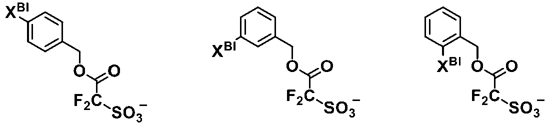

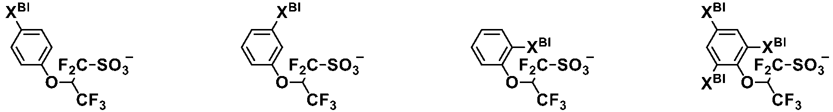

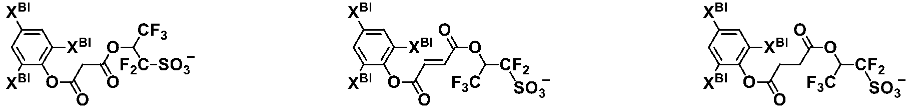

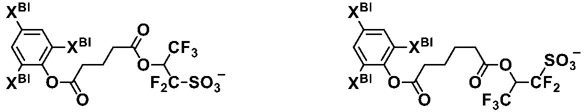

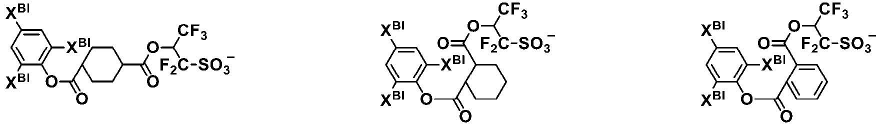

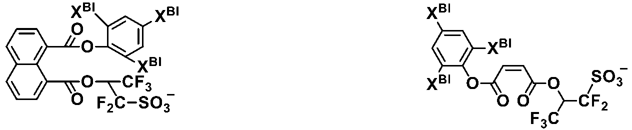

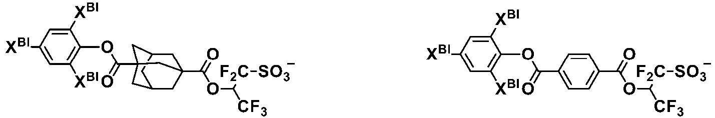

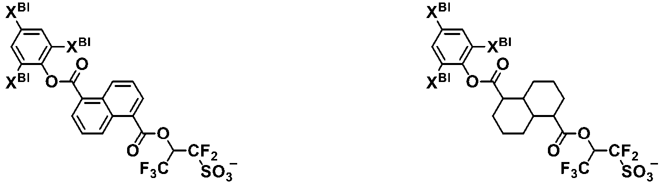

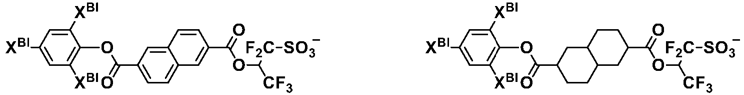

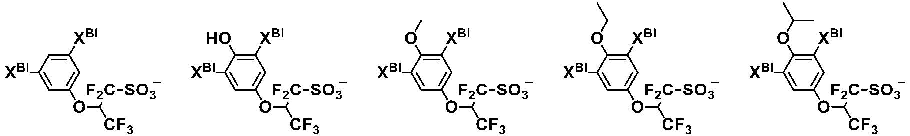

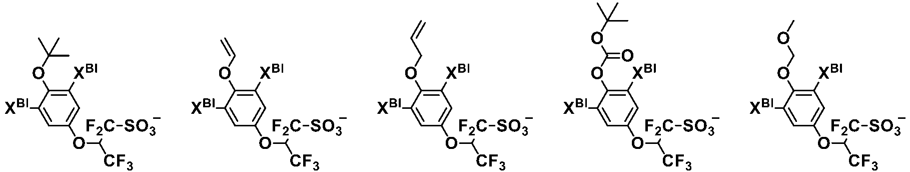

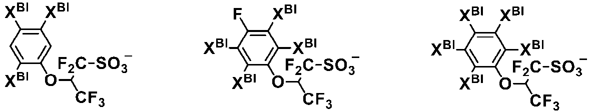

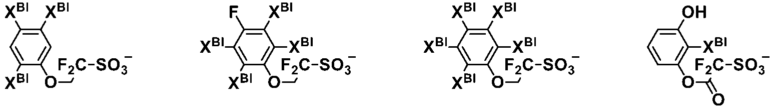

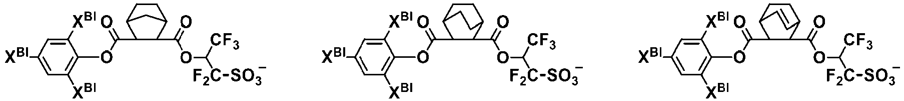

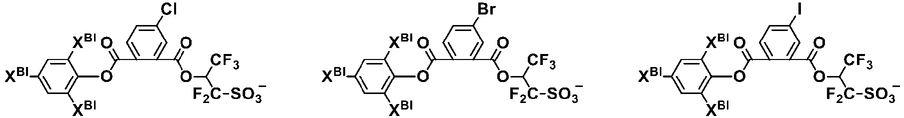

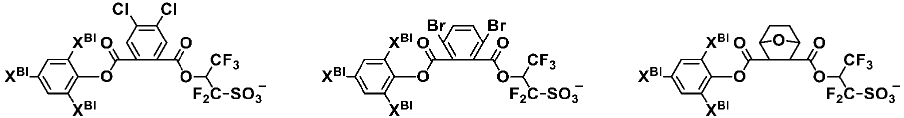

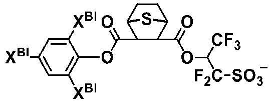

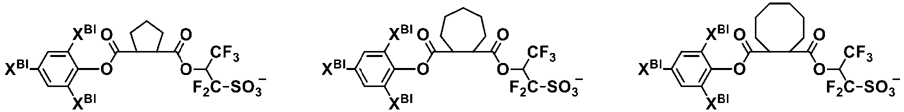

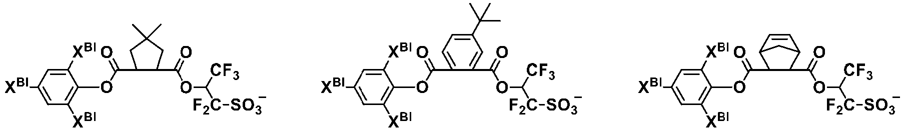

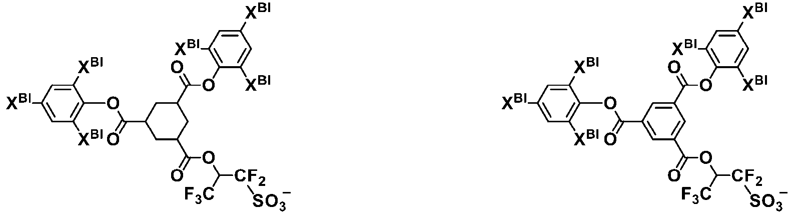

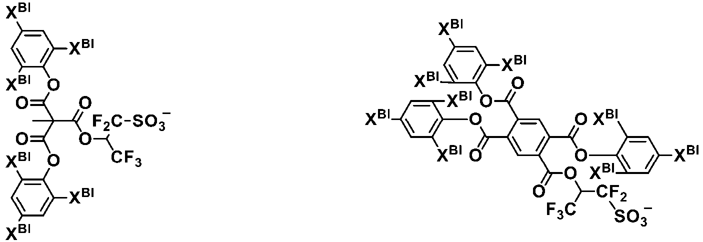

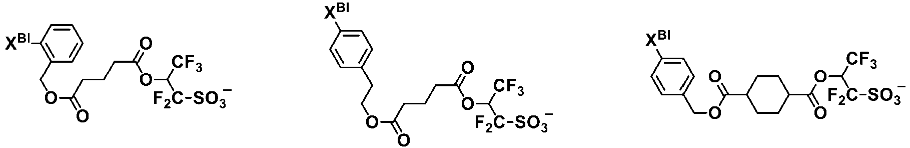

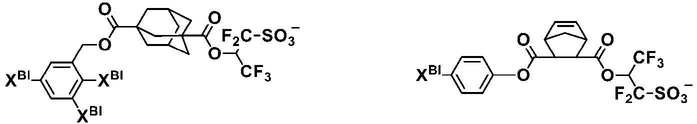

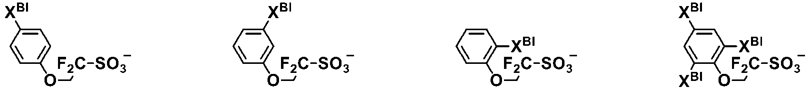

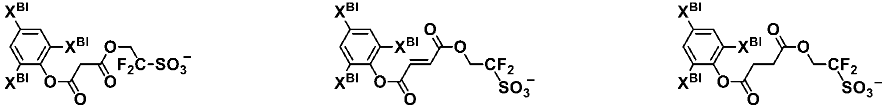

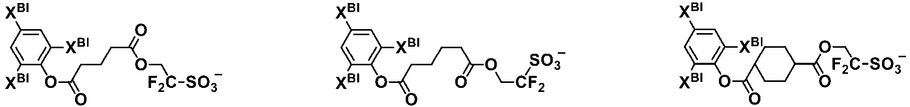

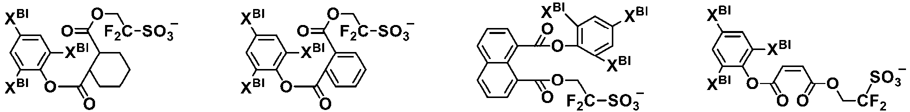

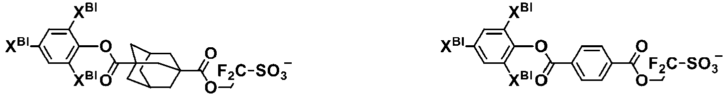

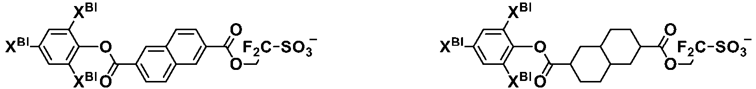

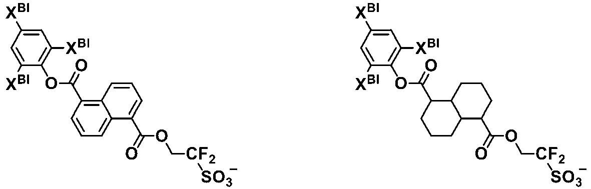

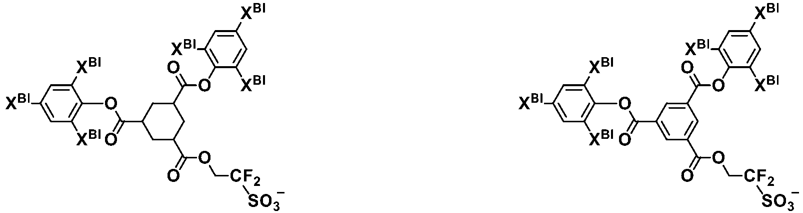

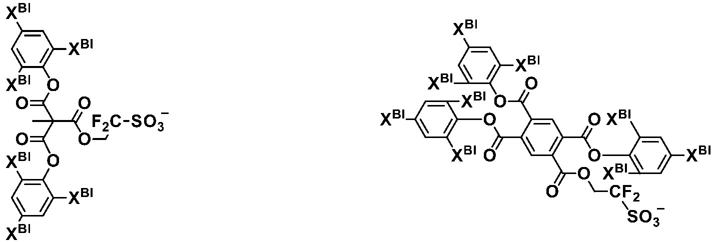

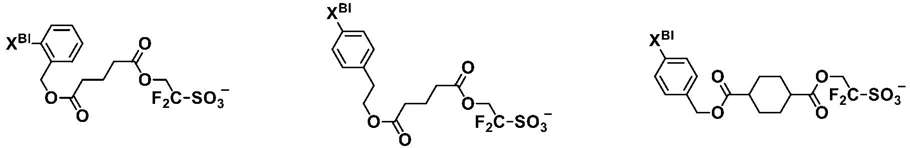

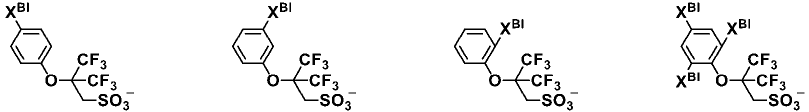

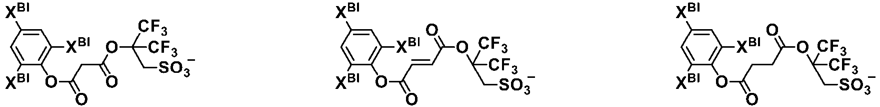

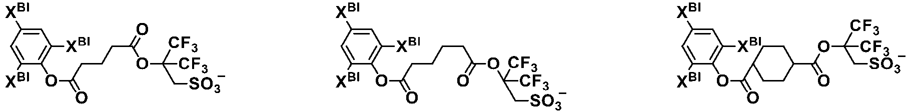

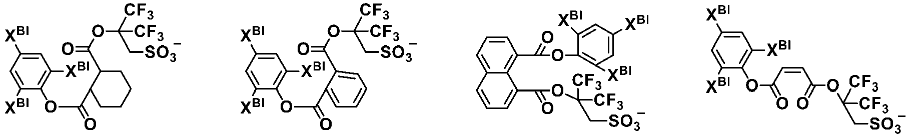

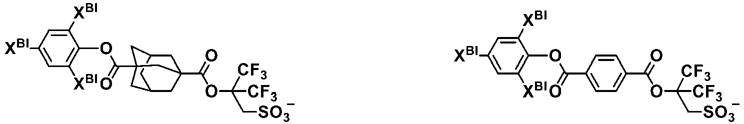

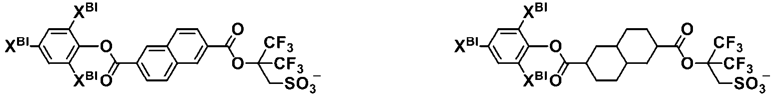

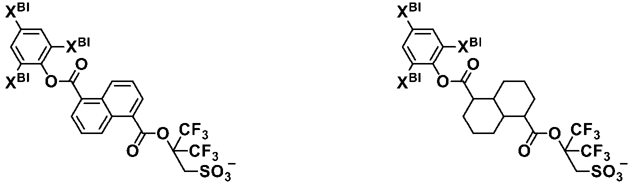

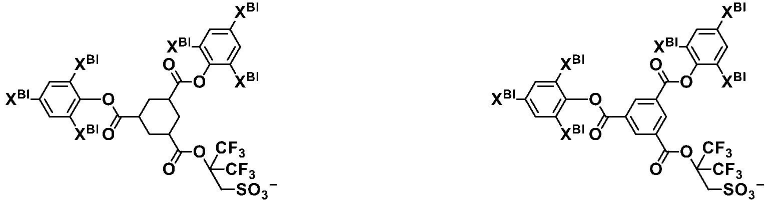

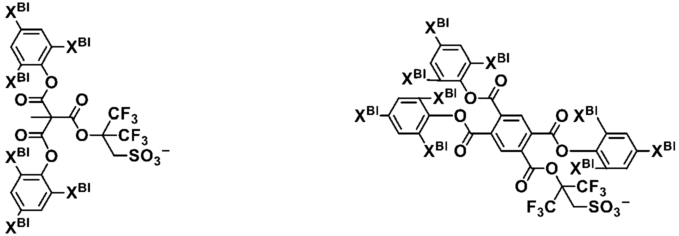

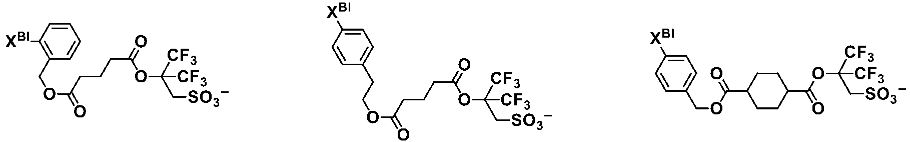

Other useful examples of the non-nucleophilic counter ion include fluorobenzenesulfonic acid anions having an iodized aromatic ring bonded thereto as described in JP 6648726, anions having an acid-catalyzed decomposition mechanism as described in WO 2021/200056 and JP-A 2021-070692, anions having a cyclic ether group as described in JP-A 2018-180525 and JP-A 2021-035935, and anions as described in JP-A 2018-092159.

Further useful examples of the non-nucleophilic counter ion include fluorine-free bulky benzenesulfonic acid anions as described in JP-A 2006-276759, JP-A 2015-117200, JP-A 2016-065016, and JP-A 2019-202974; fluorine-free benzenesulfonic acid or alkylsulfonic acid anions having an iodized aromatic group bonded thereto as described in JP 6645464.

Also useful are bissulfonic acid anions as described in JP-A 2015-206932, sulfonamide or sulfonimide anions having sulfonic acid side and different side as described in WO 2020/158366, and anions having a sulfonic acid side and a carboxylic acid side as described in JP-A 2015-024989.

In formulae (B7) and (B8), g1 and g2 are each independently 0, 1, 2 or 3, preferably 1.

In formula (B9), h1 is 0 or 1, h2 is 0, 1, 2, 3 or 4, and h3 is 0, 1, 2, 3 or 4, meeting the range: 0 ≤ h2+h3 ≤ 4 when h1=0, and 0 ≤ h2+h3 ≤ 6 when h1=1.

In formulae (B7), (B8) and (B9), L¹ is a single bond, ether bond, ester bond, carbonyl, sulfonate ester bond, sulfonamide bond, carbonate bond or carbamate bond. From the aspect of synthesis, an ether bond, ester bond or carbonyl is preferred, with the ester bond or carbonyl being more preferred.

In formula (B7), Rf¹ and Rf² are each independently fluorine or a C₁-C₆ fluorinated saturated hydrocarbyl group. It is preferred that both Rf¹ and Rf² be fluorine because the generated acid has a higher acid strength. Rf³ and Rf⁴ are each independently hydrogen, fluorine or a C₁-C₆ fluorinated saturated hydrocarbyl group. It is preferred for solvent solubility that at least one of Rf³ and Rf⁴ be trifluoromethyl.

In formula (B8), Rf⁵ and Rf⁶ are each independently hydrogen, fluorine or a C₁-C₆ fluorinated saturated hydrocarbyl group. It is excluded that all Rf⁵ and Rf⁶ are hydrogen at the same time. It is preferred for solvent solubility that at least one of Rf⁵ and Rf⁶ be trifluoromethyl.

In formula (B9), Rf⁷ is fluorine, a C₁-C₆ fluorinated alkyl group, C₁-C₆ fluorinated alkoxy group, or C₁-C₆ fluorinated alkylthio group. Rf⁷ is preferably fluorine, trifluoromethyl, difluoromethyl, trifluoromethoxy, difluoromethoxy, trifluoromethylthio or difluoromethylthio, more preferably fluorine, trifluoromethyl or trifluoromethoxy. When h2 is 2, 3 or 4, a plurality of Rf⁷ may be identical or different.

In formula (B9), R⁴³ is halogen exclusive of fluorine or a C₁-C₂₀ hydrocarbyl group which may contain a heteroatom. The hydrocarbyl group may be saturated or unsaturated and straight, branched or cyclic. Examples thereof are as exemplified above for the hydrocarbyl group R² in formula (A), but not limited thereto. When h3 is 2, 3 or 4, a plurality of R⁴³ may be identical or different.

When h3 is 2, 3 or 4, a plurality of R⁴³ may bond together to form a ring with the carbon atom to which they are attached. Examples of the ring thus formed include cyclopropane, cyclobutane, cyclopentane, cyclohexane, norbornane, and adamantane rings. In the ring, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the ring may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-) or haloalkyl moiety.

Examples of the anion in repeat unit B7 are shown below, but not limited thereto. Herein R^{A} is as defined above.

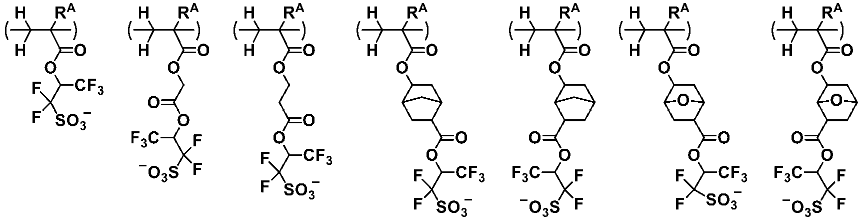

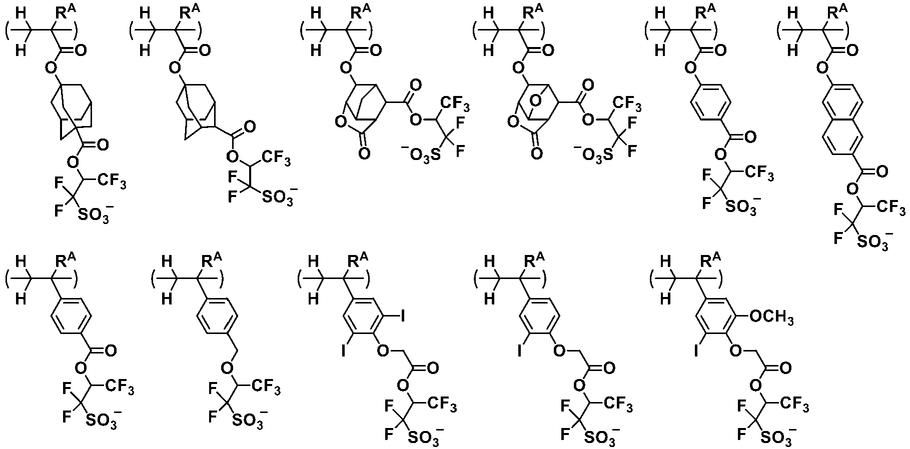

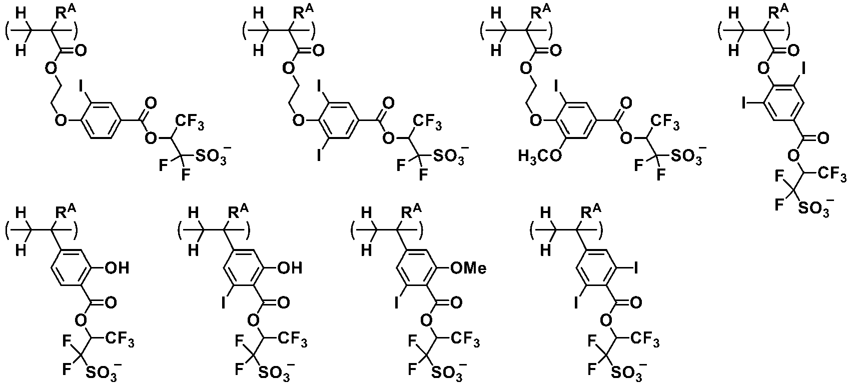

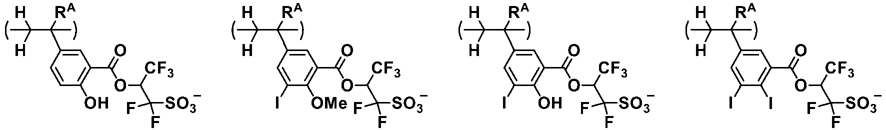

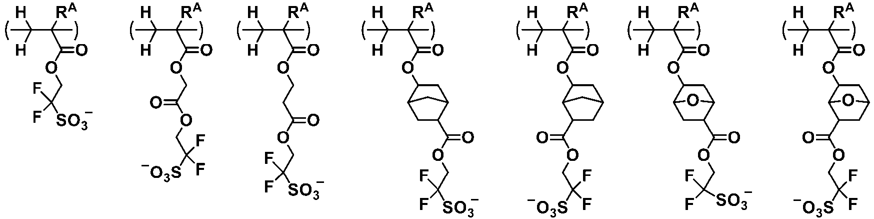

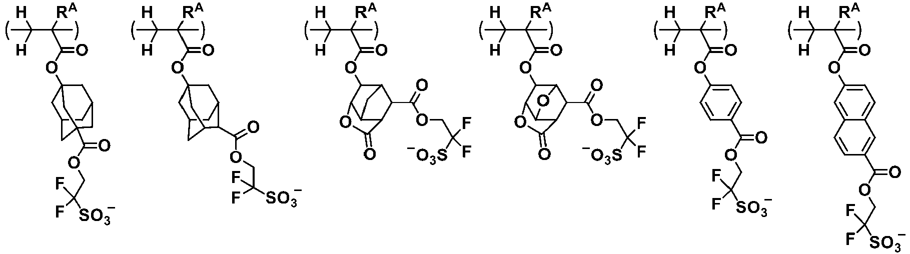

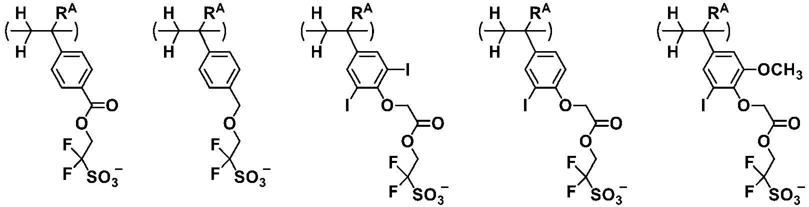

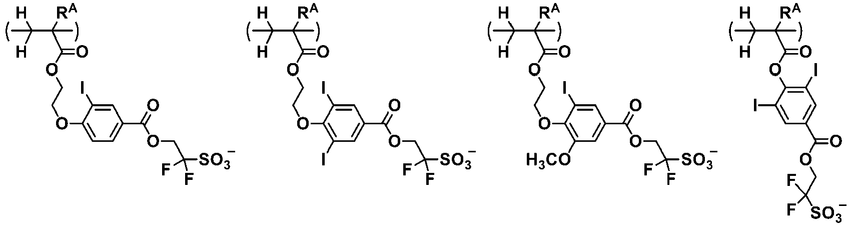

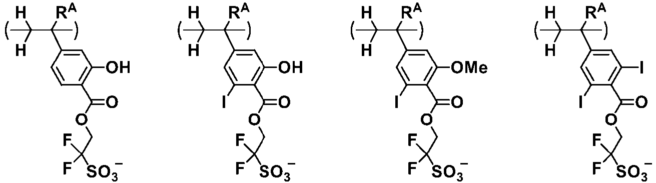

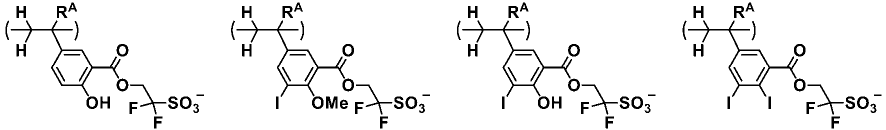

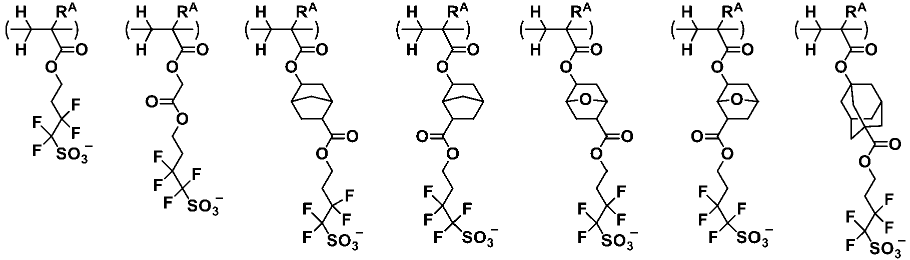

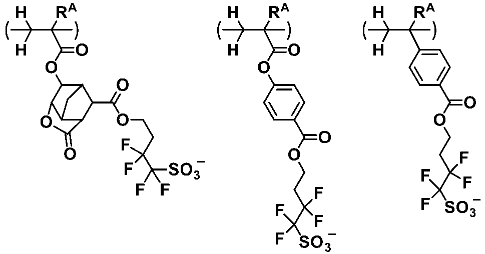

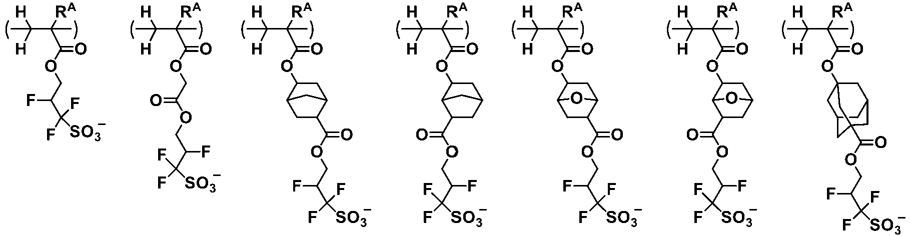

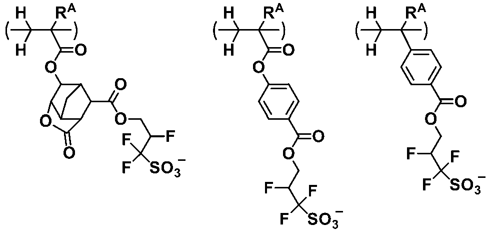

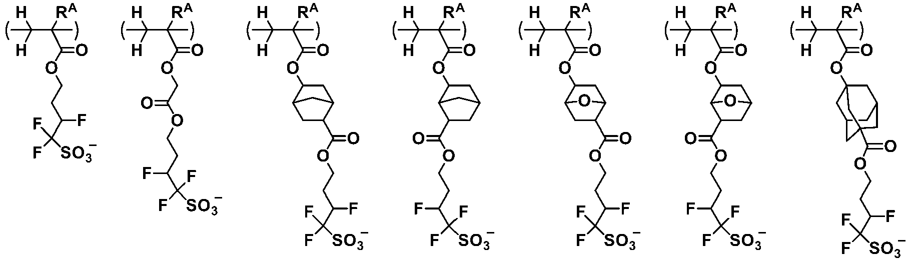

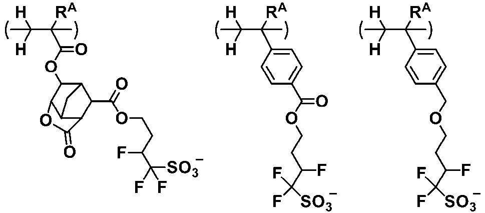

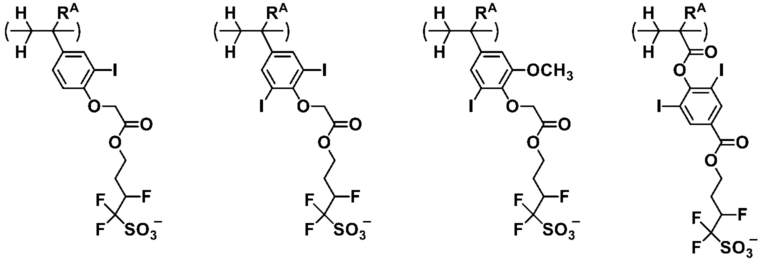

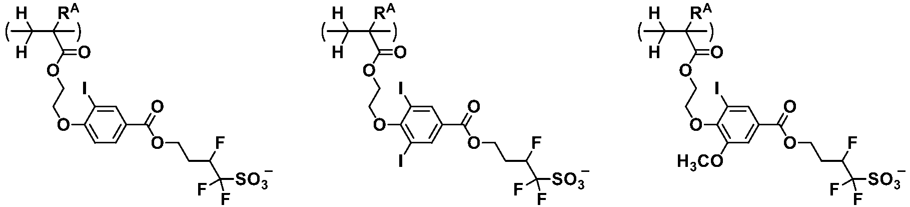

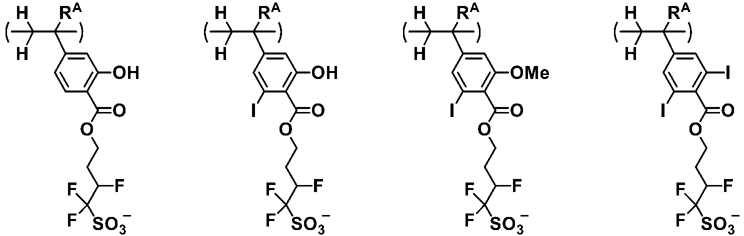

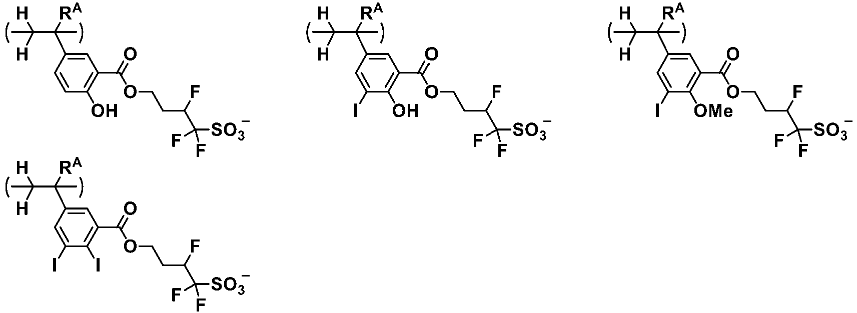

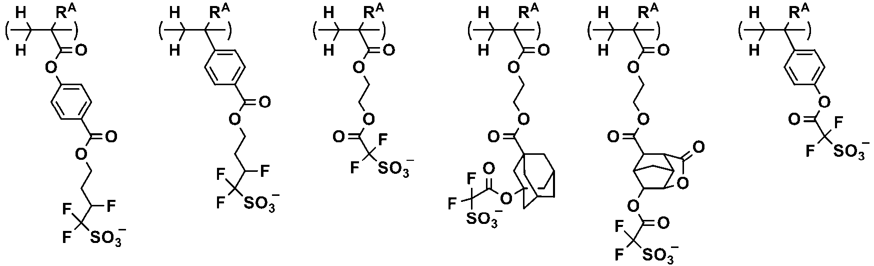

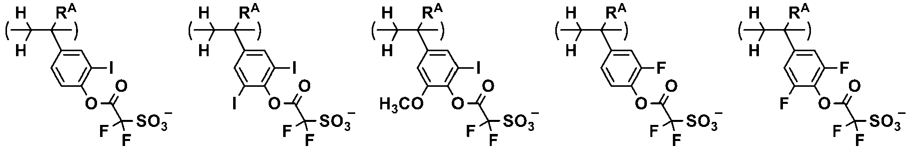

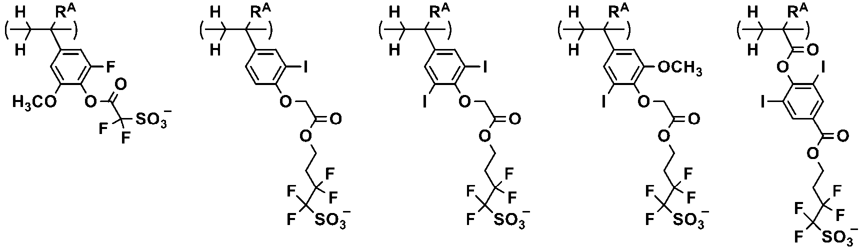

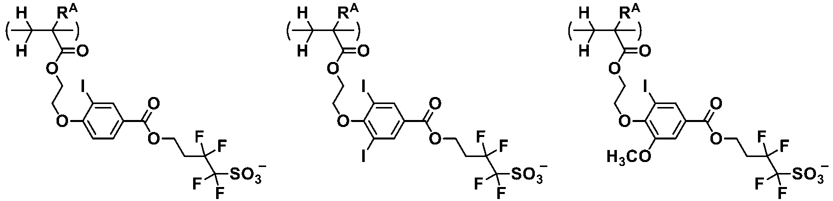

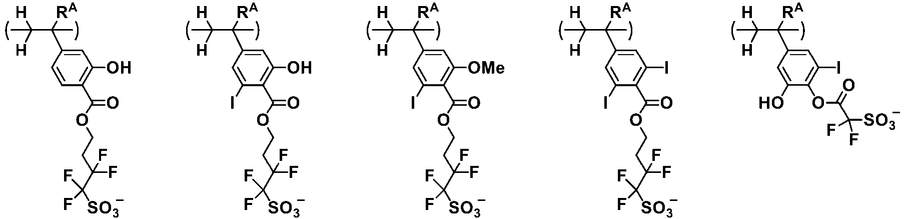

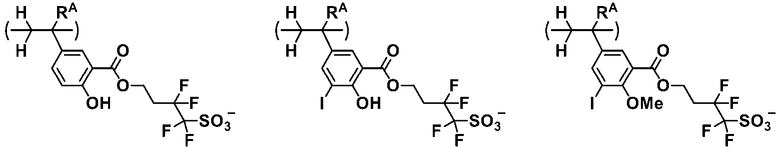

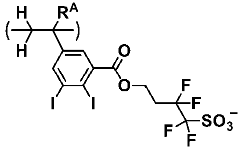

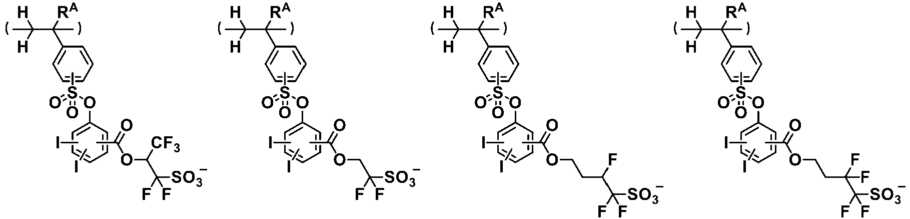

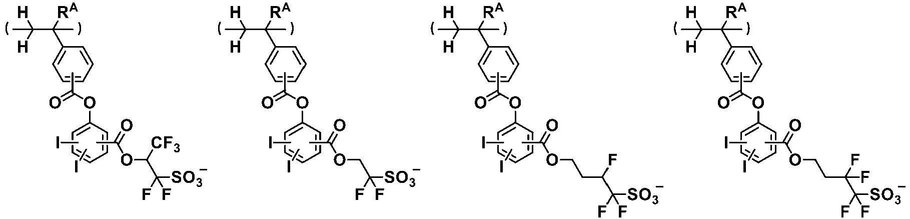

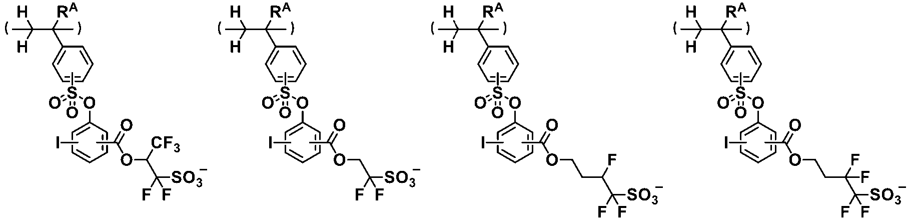

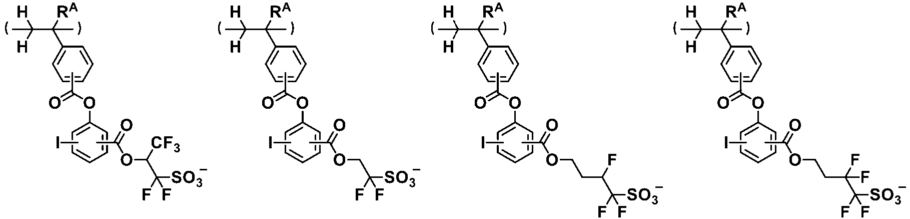

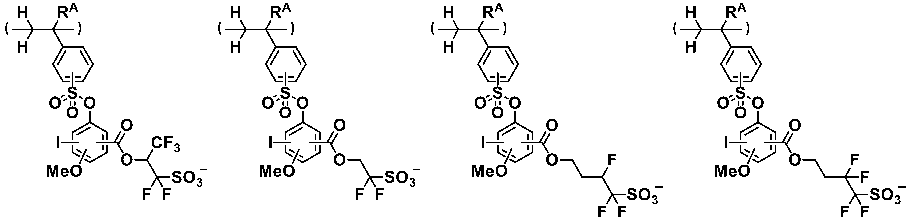

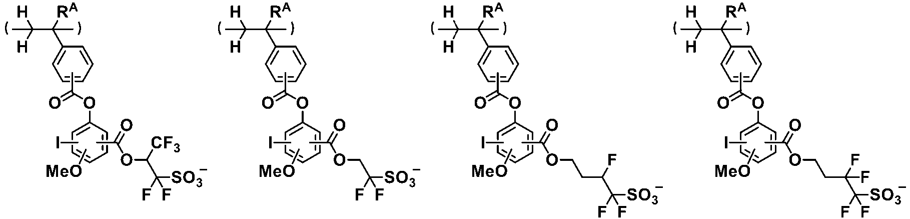

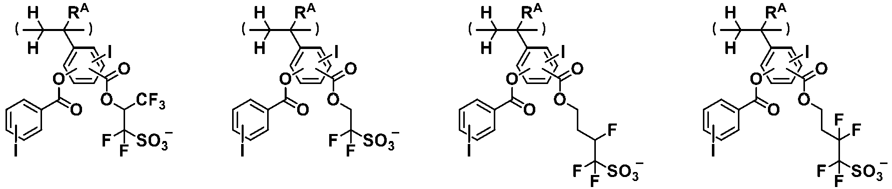

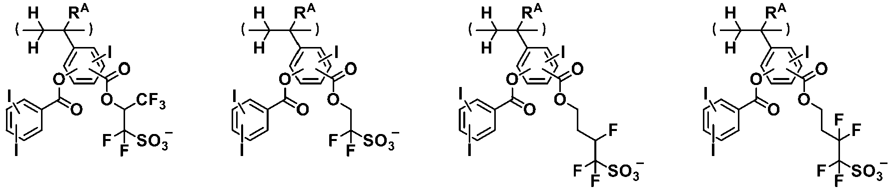

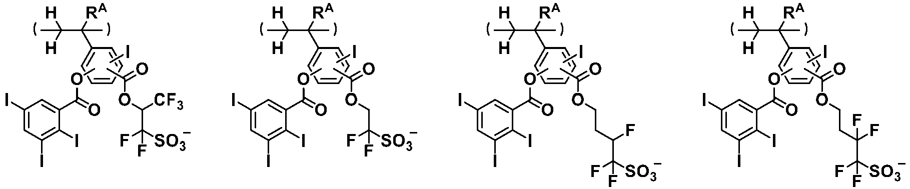

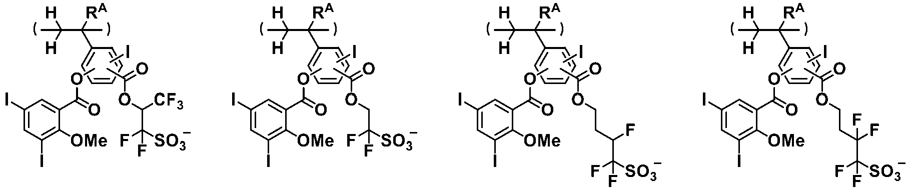

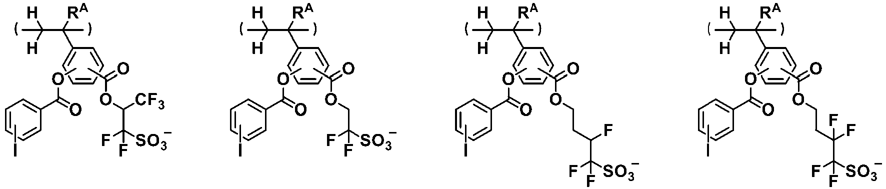

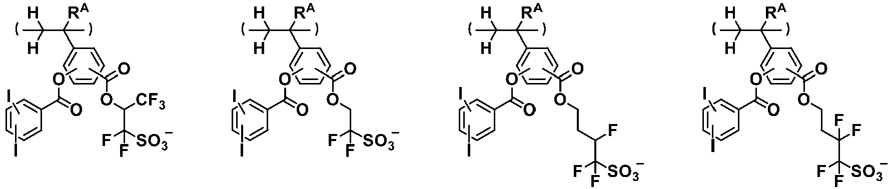

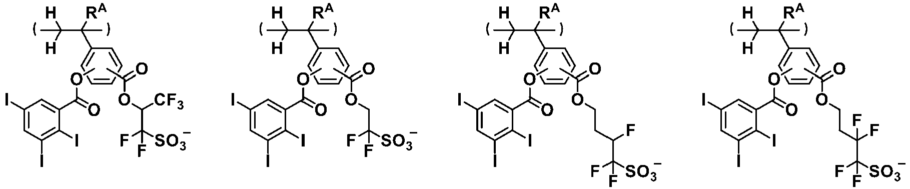

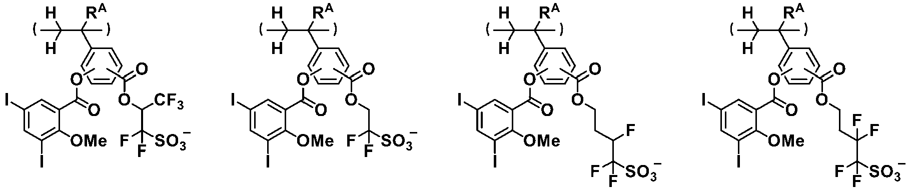

Examples of the anion in repeat unit B8 are shown below, but not limited thereto. Herein R^{A} is as defined above.

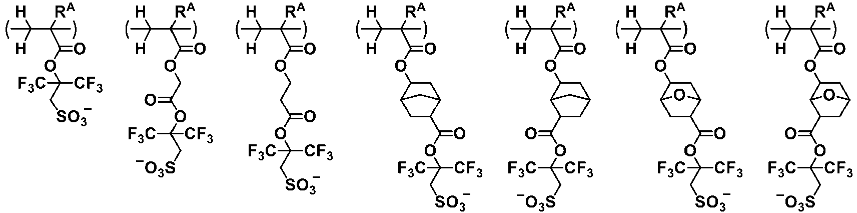

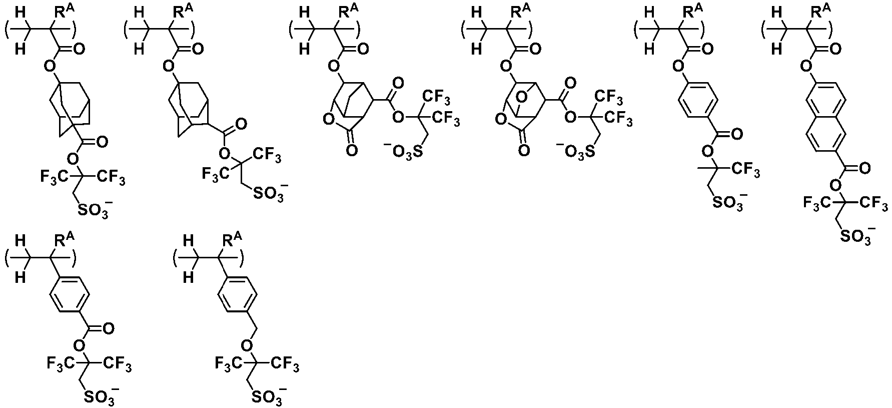

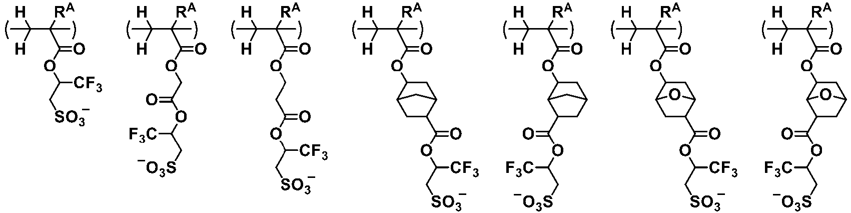

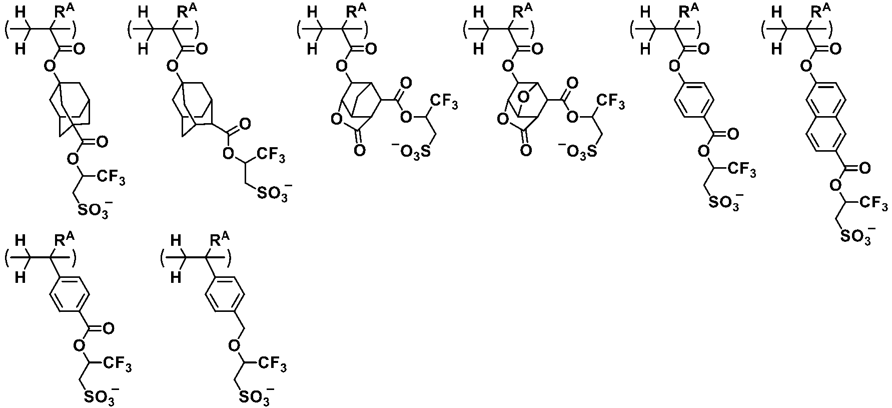

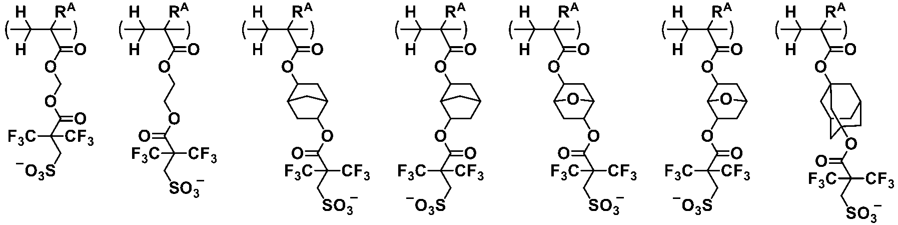

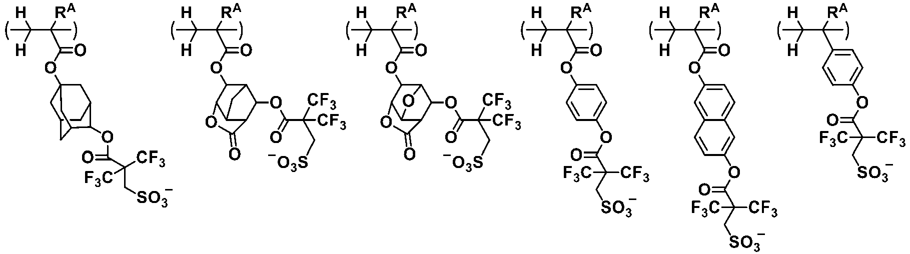

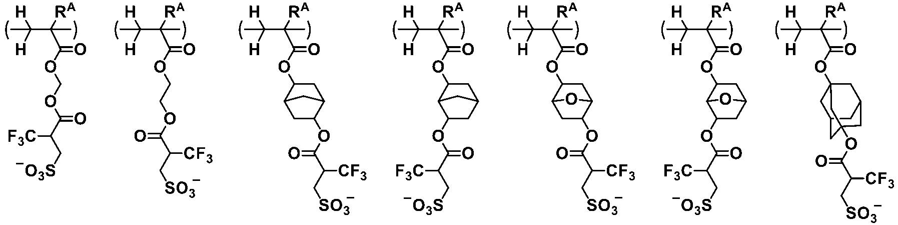

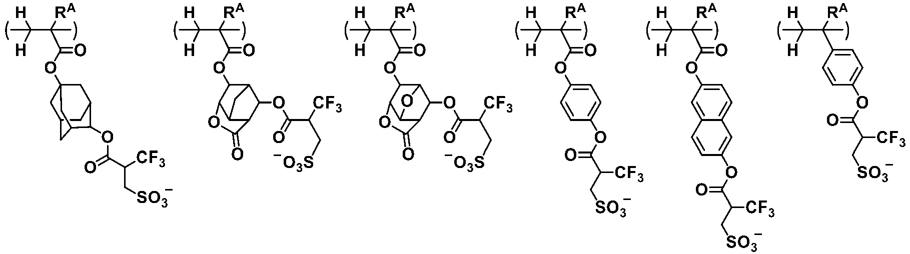

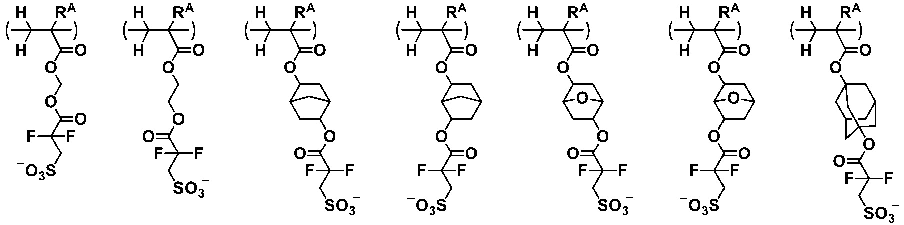

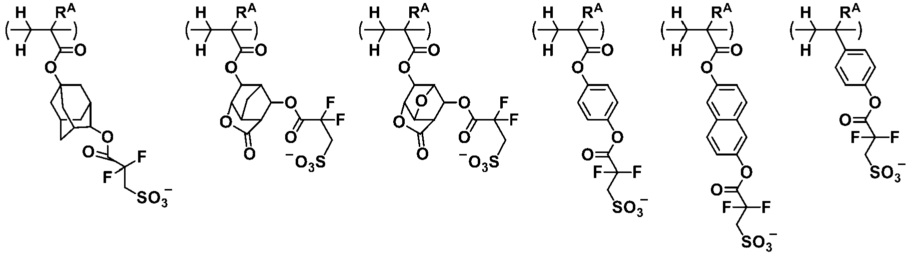

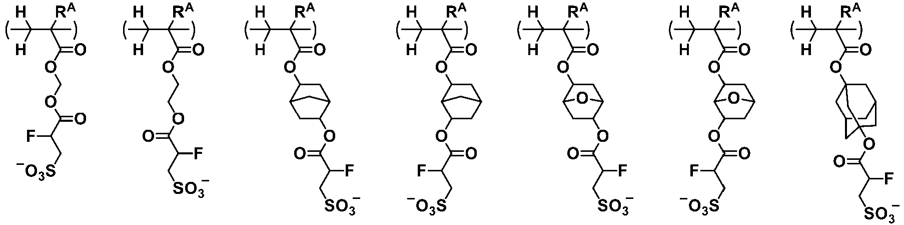

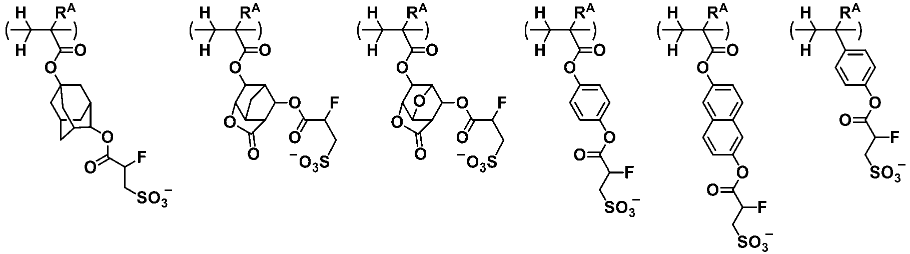

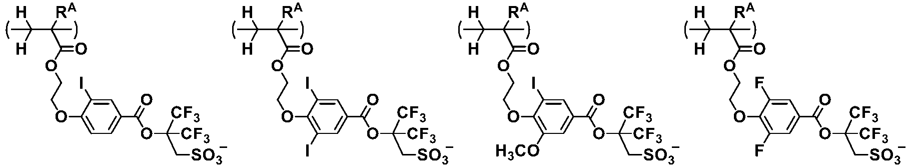

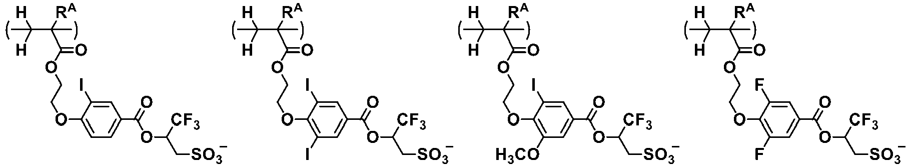

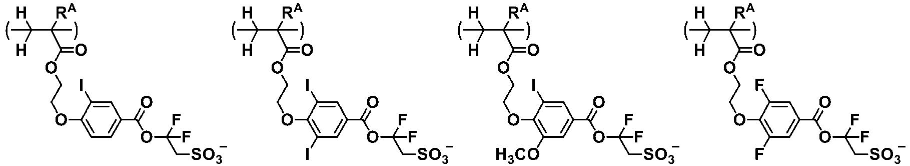

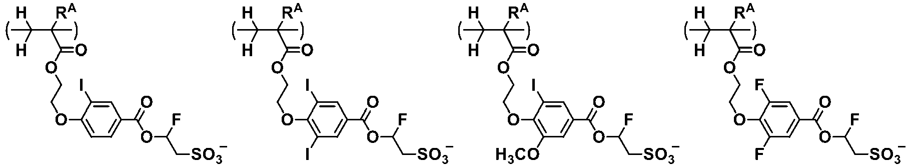

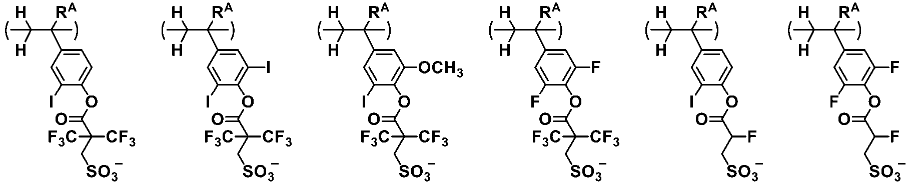

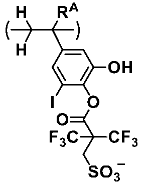

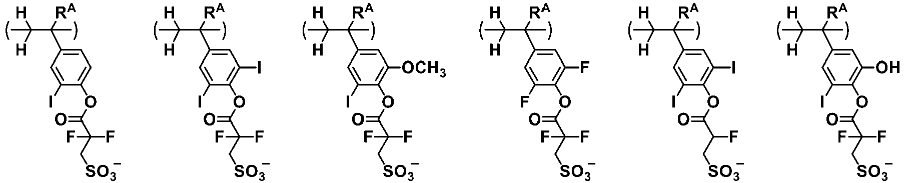

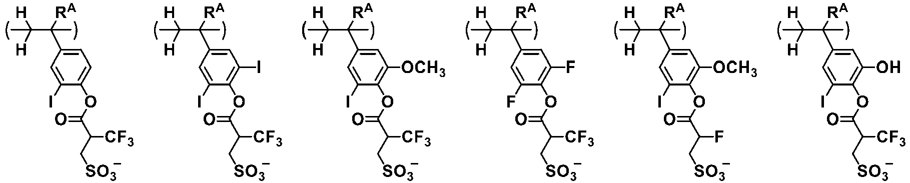

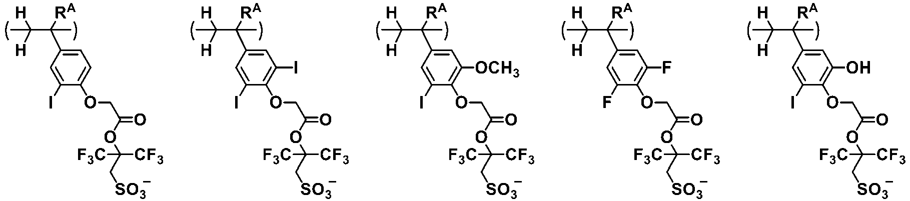

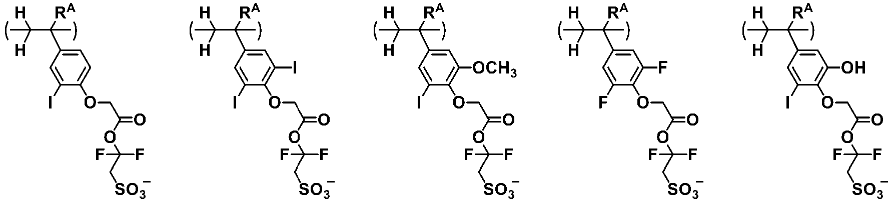

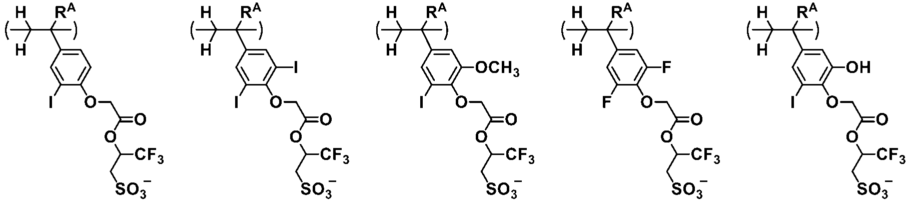

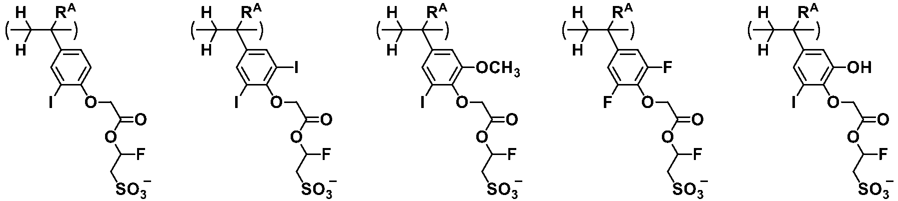

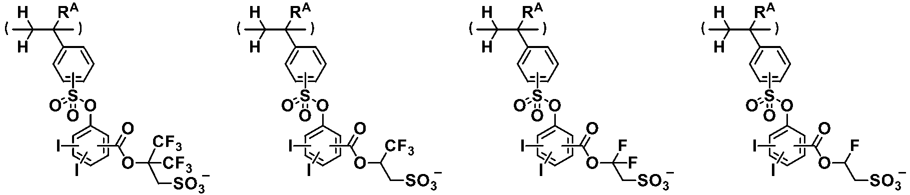

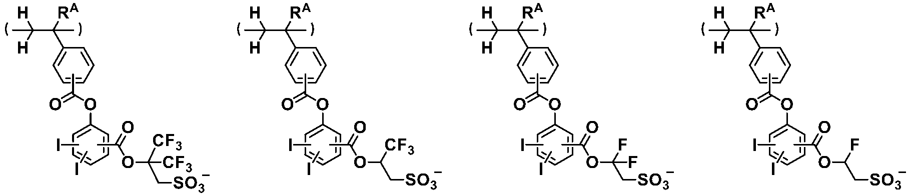

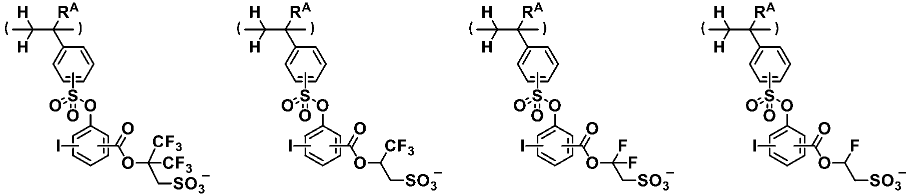

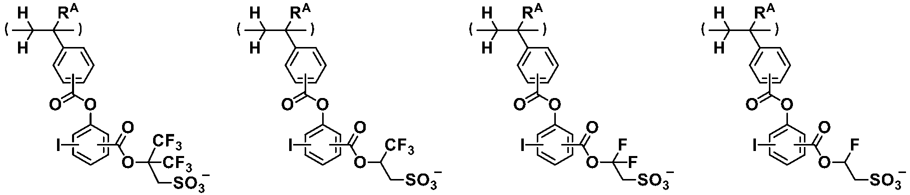

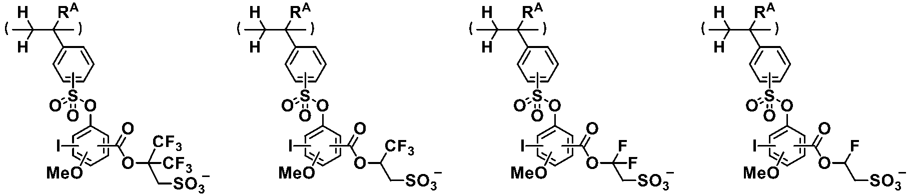

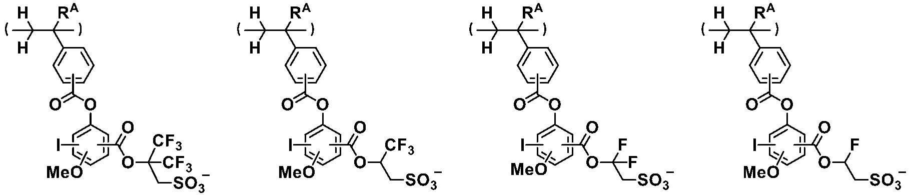

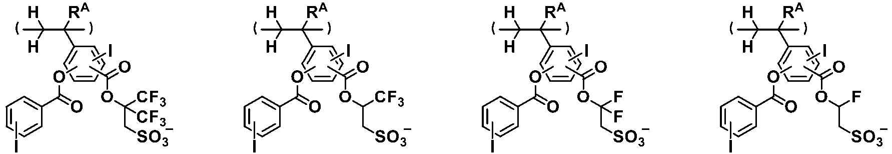

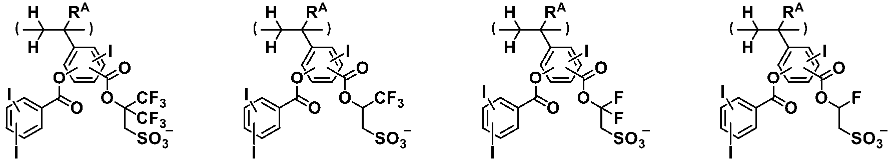

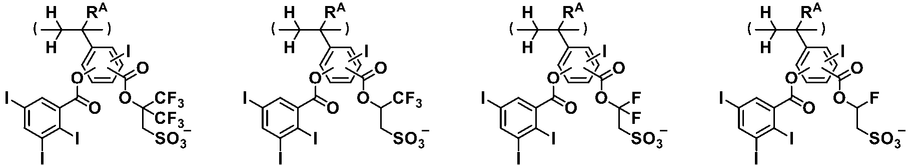

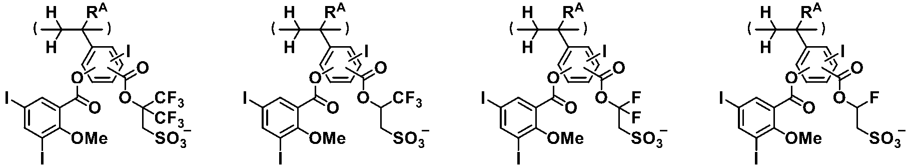

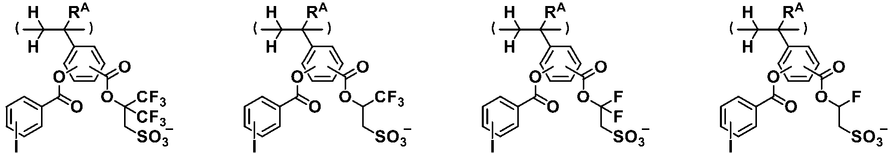

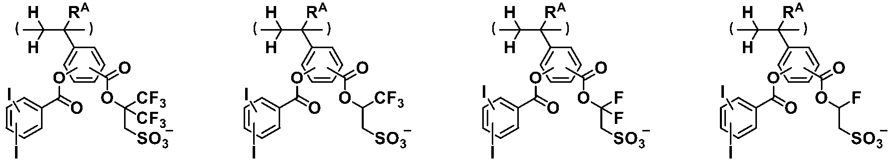

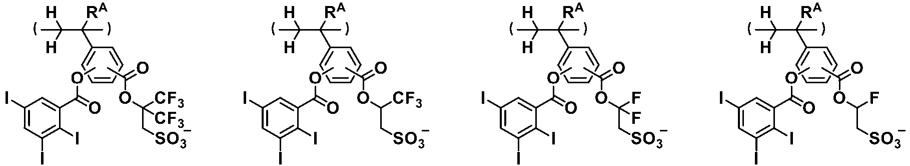

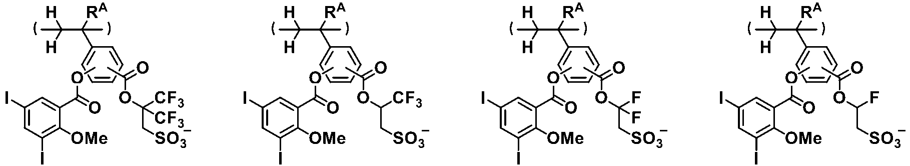

Examples of the anion in repeat unit B9 are shown below, but not limited thereto. Herein R^{A} is as defined above.

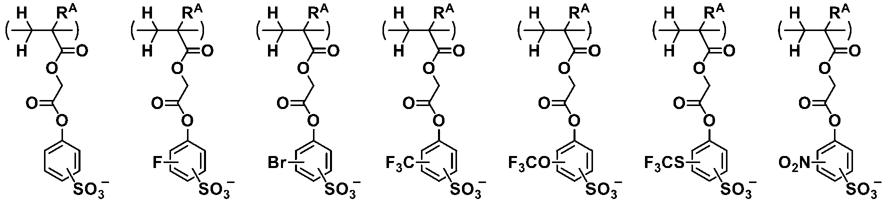

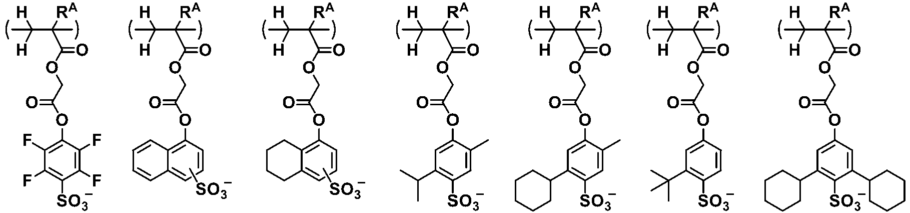

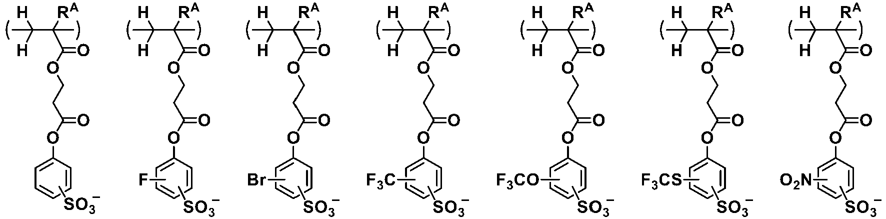

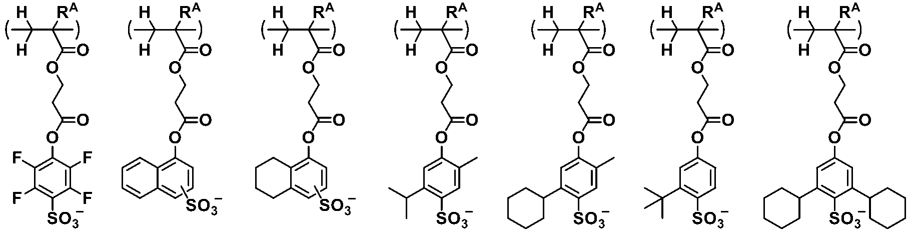

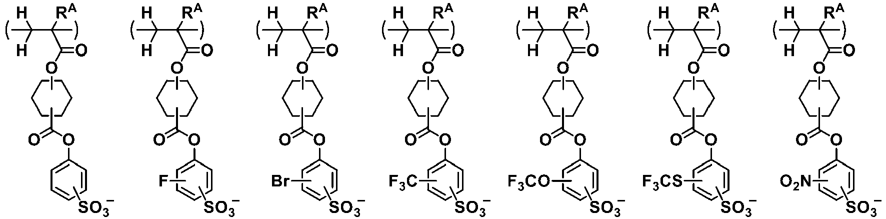

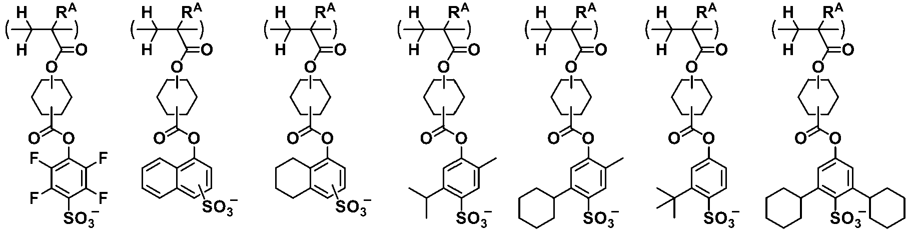

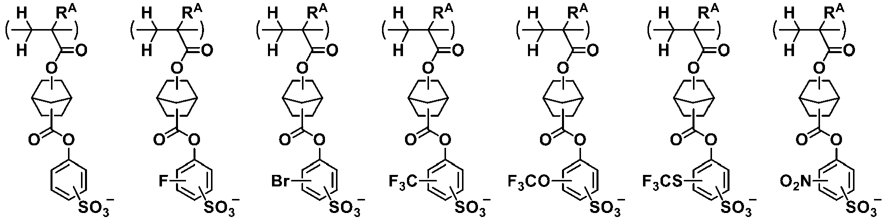

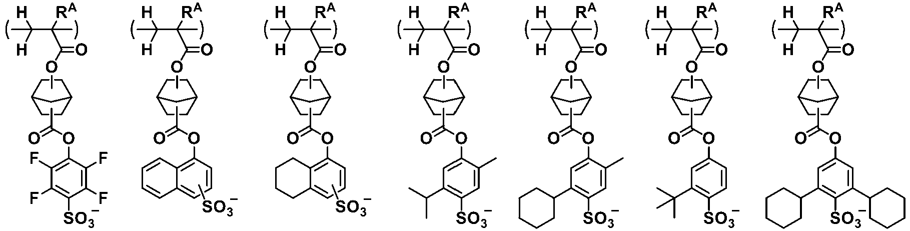

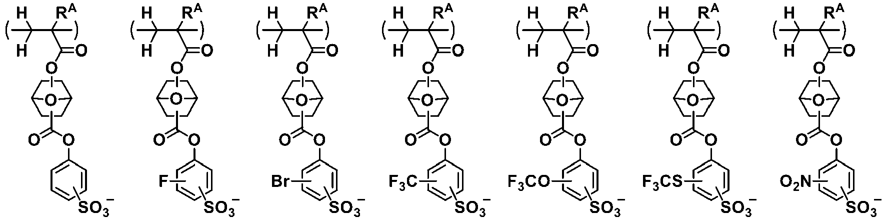

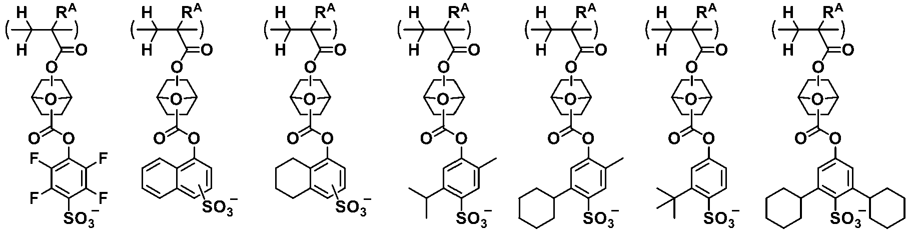

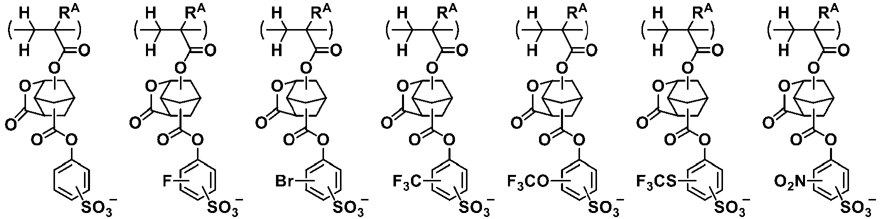

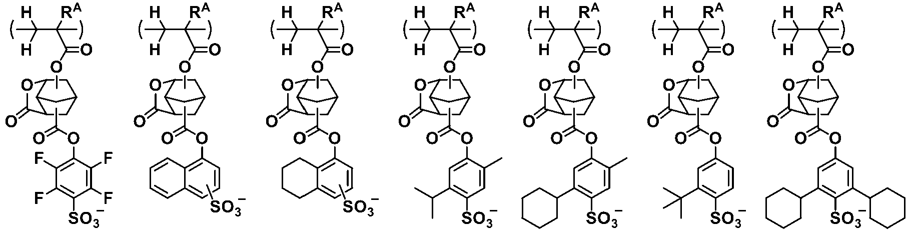

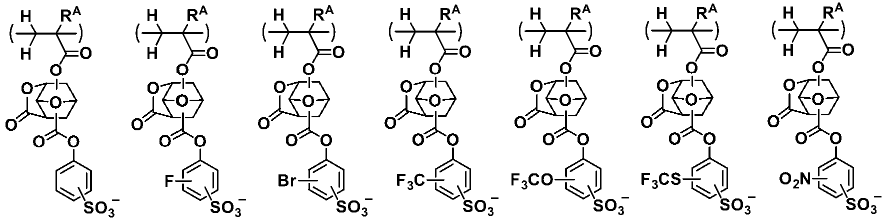

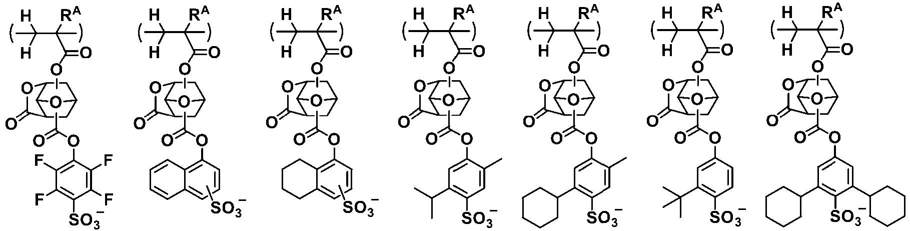

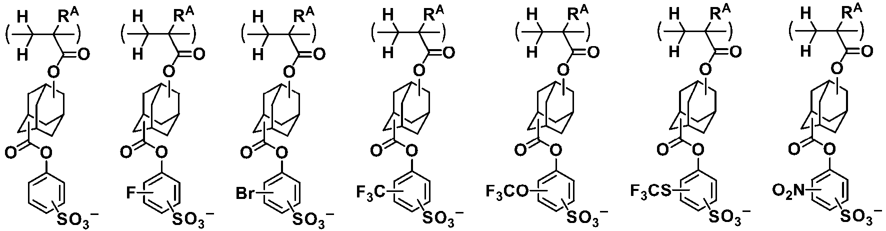

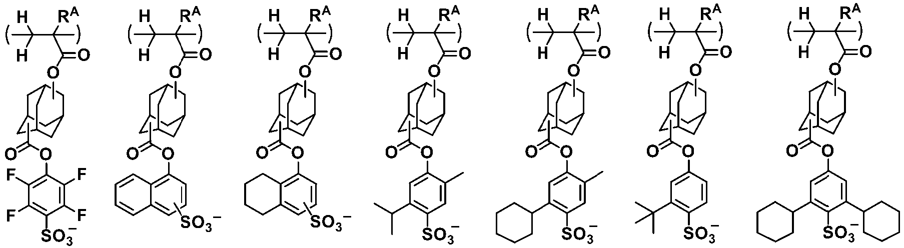

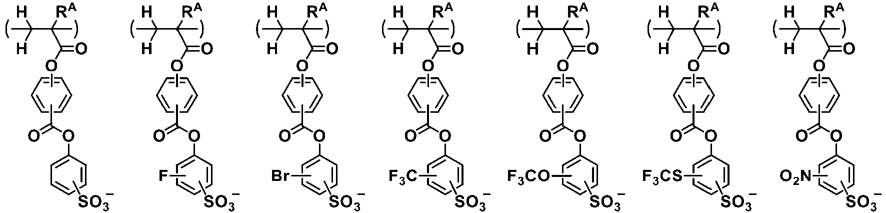

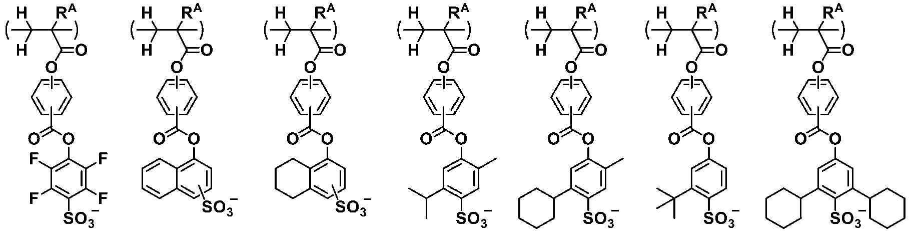

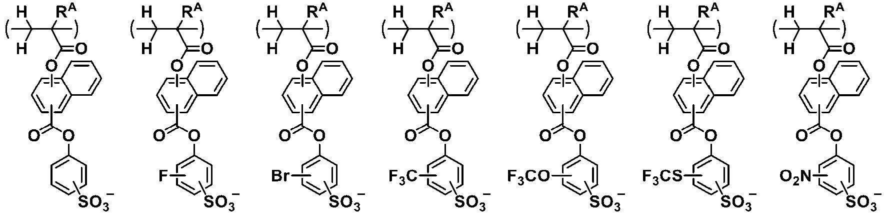

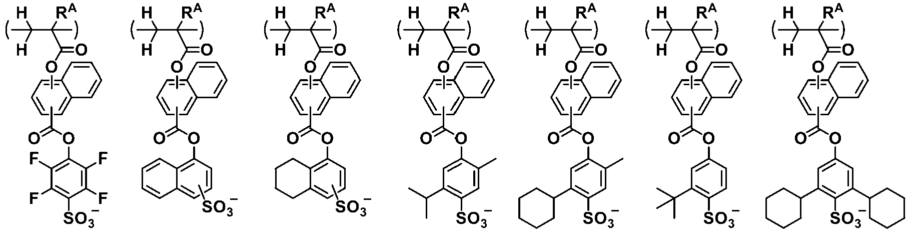

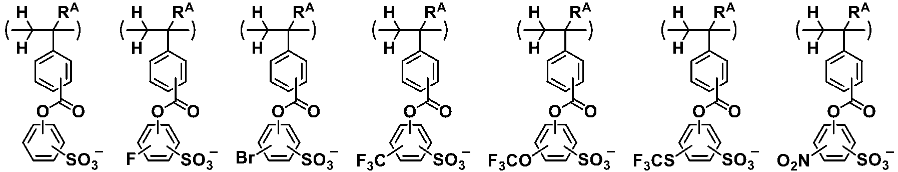

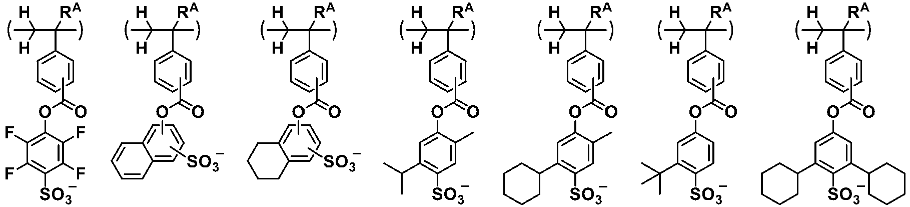

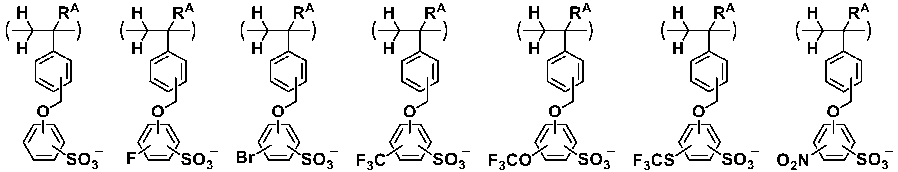

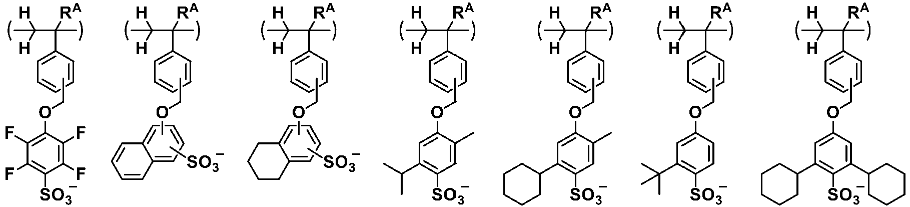

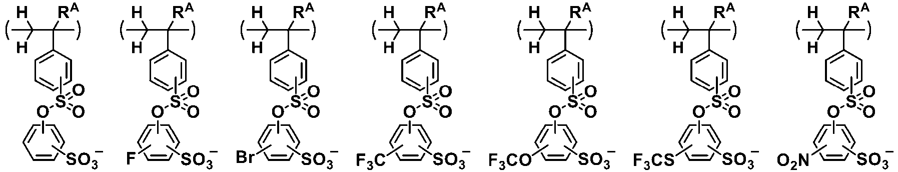

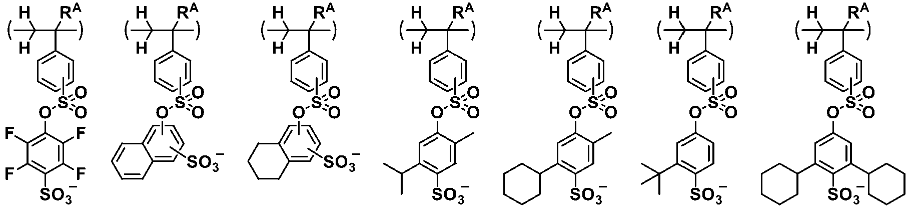

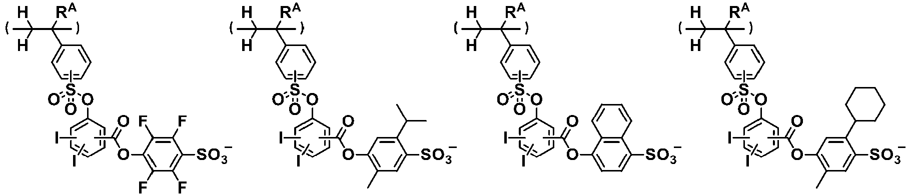

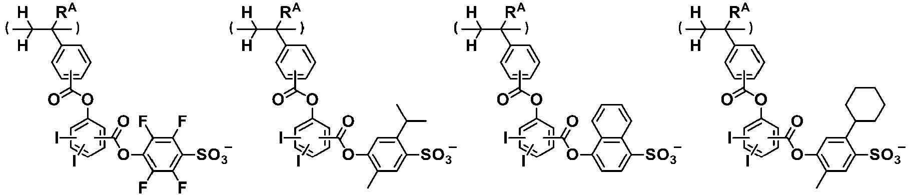

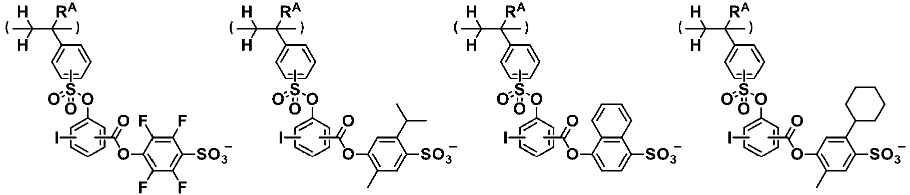

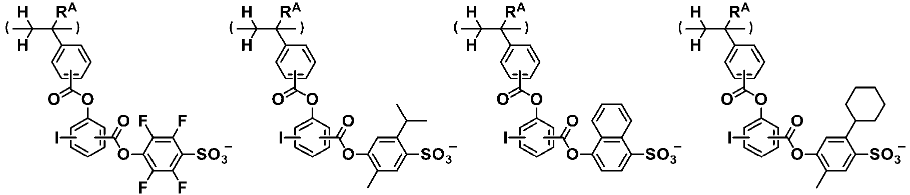

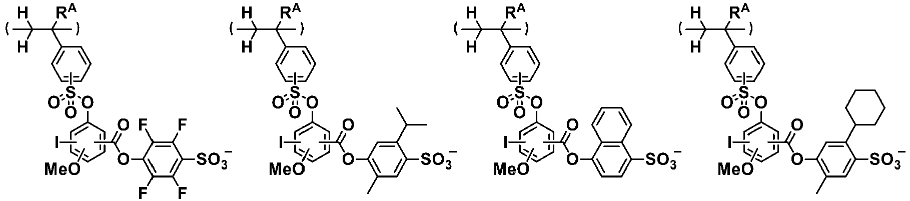

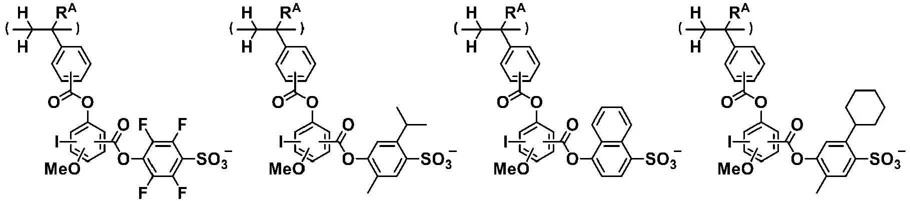

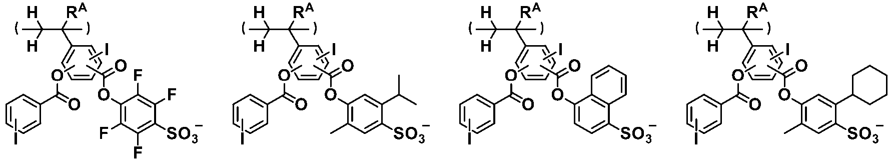

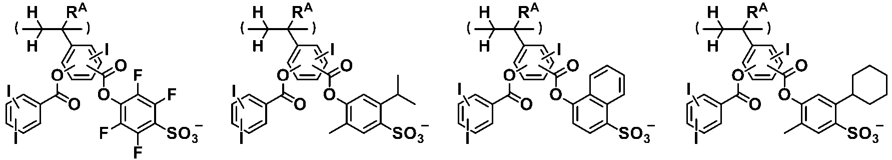

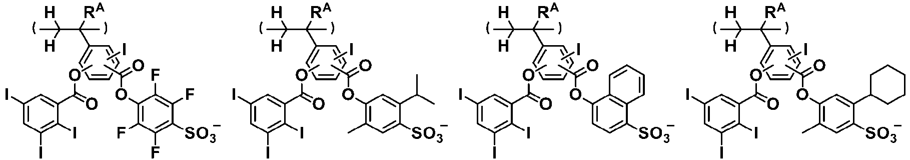

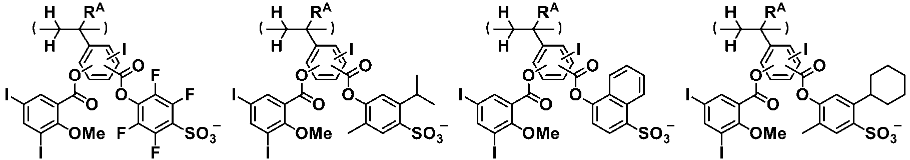

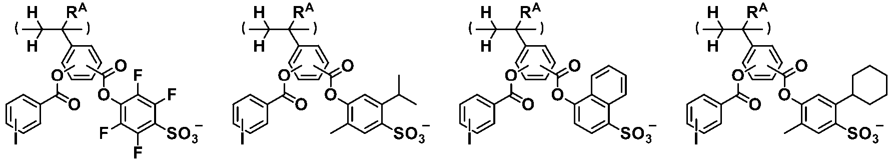

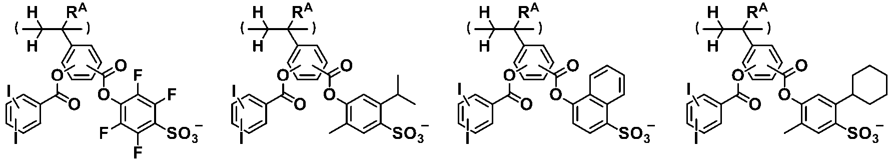

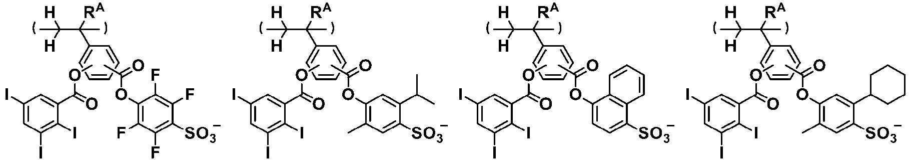

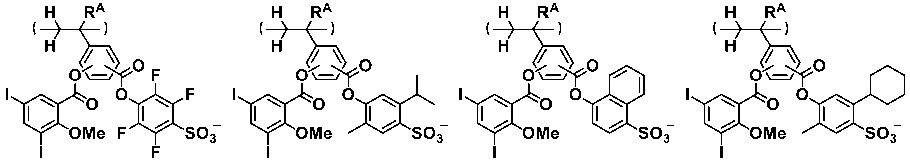

Examples of the anion in repeat unit B10 are shown below, but not limited thereto. Herein R^{A} is as defined above.

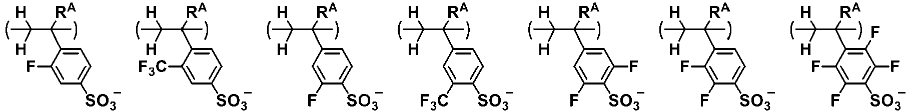

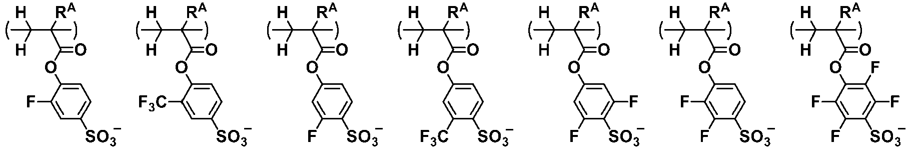

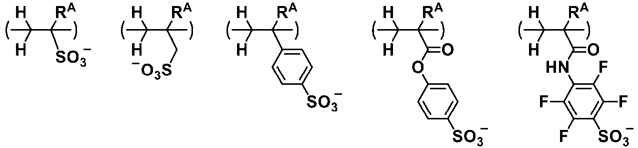

In formulae (B7) to (B10), A⁺ is an onium cation. The preferred onium cations are sulfonium and iodonium cations. Examples of the sulfonium cation include those exemplified above for the sulfonium cation having formula (Z-1) and the sulfonium cation having formula (Z-3), but are not limited thereto. Examples of the iodonium cation include those exemplified above for the iodonium cation having formula (Z-2), but are not limited thereto.

Illustrative structures of repeat units B6 to B10 include arbitrary combinations of anions with cations, both as mentioned above.

The repeat units B6 to B10 are capable of generating an acid upon exposure to high-energy radiation. It is believed that binding of the relevant units to a polymer enables to appropriately control acid diffusion and to form a pattern with reduced LER. Since the acid-generating unit is bound to a polymer, the phenomenon that acid volatilizes from the exposed region and re-deposits on the unexposed region during bake in vacuum is suppressed. This is effective for reducing LER and for suppressing profile degradation due to unwanted film thickness loss in the unexposed region.

Of repeat units B6 to B10, repeat units B7 to B10 are preferred for the processing of photomask blanks because an optimum acid strength is available for the suppression of acid diffusion and the design of an acid labile group on the polymer. The repeat units B8, B9 and B10 are more preferred

When repeat units B6 to B10 are included, their content is preferably 0.1 to 30 mol%, more preferably 0.5 to 20 mol% based on the overall repeat units of the polymer. Each of repeat units B6 to B10 may be of one type or a combination of plural types.

The content of repeat units having an aromatic ring structure is preferably at least 65 mol%, more preferably at least 75 mol%, even more preferably at least 85 mol% based on the overall repeat units of the polymer. When the polymer does not contain repeat units B6 to B10, it is preferred that all units have an aromatic ring structure.

The polymer may further comprise (meth)acrylate units protected with an acid labile group or (meth)acrylate units having an adhesive group such as lactone structure or hydroxy group other than phenolic hydroxy as commonly used in the art. These repeat units are effective for fine adjustment of properties of a resist film, but not essential.

Examples of the (meth)acrylate unit having an adhesive group include repeat units having the following formulae (B11) to (B13), which are also referred to as repeat units B11 to B13. While these units do not exhibit acidity, they may be used as auxiliary units for providing adhesion to substrates or adjusting solubility.

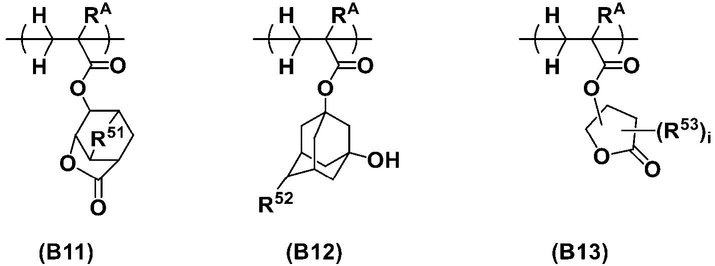

In formulae (B11) to (B13), R^{A} is each independently hydrogen, fluorine, methyl or trifluoromethyl. R⁵¹ is -O- or methylene. R⁵² is hydrogen or hydroxy. R⁵³ is a C₁-C₄ saturated hydrocarbyl group, and i is 0, 1, 2 or 3.

When repeat units B11 to B13 are included, their content is preferably 0 to 20 mol%, more preferably 0 to 10 mol% based on the overall repeat units of the polymer. Each of repeat units B11 to B13 may be of one type or a combination of plural types.

The polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630, for example.

The polymer should preferably have a Mw of 1,000 to 50,000, and more preferably 2,000 to 20,000. A Mw of at least 1,000 eliminates the risk that pattern features are rounded at their top to invite degradations of resolution and LER. A Mw of up to 50,000 eliminates the risk that LER is degraded when a pattern with a line width of up to 100 nm is formed. As used herein, Mw is measured by GPC versus polystyrene standards using tetrahydrofuran (THF) or dimethylformamide (DMF) solvent.

The polymer preferably has a narrow molecular weight distribution or dispersity (Mw/Mn) of 1.0 to 2.0, more preferably 1.0 to 1.9, even more preferably 1.0 to 1.8. A polymer with such a narrow dispersity eliminates the risk that foreign particles are left on the pattern after development and the pattern profile is aggravated.

The base polymer is designed such that the dissolution rate in alkaline developer is preferably up to 10 nm/min, more preferably up to 7 nm/min, even more preferably up to 5 nm/min. In the advanced generation of lithography wherein the coating film on the substrate is in a thin film range of up to 100 nm, the influence of pattern film thickness loss during alkaline development becomes strong. When the polymer has an alkaline dissolution rate of greater than 10 nm/min, pattern collapse occurs, i.e., a small-size pattern cannot be formed. The problem becomes outstanding in the fabrication of photomasks requiring to be defectless and having a tendency of strong development process. It is noted that the dissolution rate of a base polymer in alkaline developer is computed by spin coating a 16.7 wt% solution of a polymer in propylene glycol monomethyl ether acetate (PGMEA) solvent onto a 8-inch silicon wafer, baking at 100°C for 90 seconds to form a film of 1,000 nm thick, developing the film in a 2.38 wt% aqueous solution of tetramethylammonium hydroxide (TMAH) at 23°C for 100 seconds, and measuring a loss of film thickness.

In addition to the polymer defined above, the base polymer (B) may contain another polymer. The other polymer may be any of prior art well-known base polymers used in resist compositions. The content of the other polymer is not particularly limited as long as the benefits of the invention are not impaired.

### (C) Organic solvent

The chemically amplified positive resist composition may comprise an organic solvent as component (C). The organic solvent used herein is not particularly limited as long as the components are soluble therein. Examples of the organic solvent are described in JP-A 2008-111103, paragraphs [0144] to [0145] (USP 7,537,880). Specifically, exemplary solvents include ketones such as cyclohexanone, cyclopentanone, methyl-2-n-pentyl ketone, and 2-heptanone; alcohols such as 3-methoxybutanol, 3-methyl-3-methoxybutanol, 1-methoxy-2-propanol, 1-ethoxy-2-propanol, and diacetone alcohol (DAA); ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate (EL), ethyl pyruvate, butyl acetate, methyl 3-methoxypropionate, ethyl 3-ethoxypropionate, tert-butyl acetate, tert-butyl propionate, and propylene glycol mono-tert-butyl ether acetate; and lactones such as γ-butyrolactone (GBL), and mixtures thereof. Where an acid labile group of acetal form is used, a high boiling alcohol solvent such as diethylene glycol, propylene glycol, glycerol, 1,4-butanediol or 1,3-butanediol may be added to accelerate deprotection reaction of acetal.

Of the above organic solvents, it is recommended to use 1-ethoxy-2-propanol, PGMEA, PGME, cyclohexanone, EL, GBL, and mixtures thereof.

In the resist composition, the organic solvent (C) is preferably used in an amount of 200 to 10,000 parts, more preferably 400 to 5,000 parts by weight per 80 parts by weight of the base polymer (B). The organic solvent may be used alone or in admixture.

### (D) Quencher

The chemically amplified positive resist composition optionally comprises a quencher as component (D). As used herein, the quencher refers to a compound capable of trapping an acid generated from the acid generator upon exposure. The quencher is effective for holding down the rate of diffusion of the acid (generated by the acid generator) in the resist film. Even when a substrate whose outermost surface is made of a chromium-containing material is used, the quencher is effective for suppressing the influence of the acid (generated in the resist film) on the chromium-containing material.

The quencher is typically selected from conventional basic compounds. Conventional basic compounds include primary, secondary, and tertiary aliphatic amines, mixed amines, aromatic amines, heterocyclic amines, nitrogen-containing compounds with carboxy group, nitrogen-containing compounds with sulfonyl group, nitrogen-containing compounds with hydroxy group, nitrogen-containing compounds with hydroxyphenyl group, alcoholic nitrogen-containing compounds, amide derivatives, imide derivatives, and carbamate derivatives. Also included are primary, secondary, and tertiary amine compounds, specifically amine compounds having a hydroxy, ether bond, ester bond, lactone ring, cyano, or sulfonate ester group as described in JP-A 2008-111103, paragraphs [0146]-[0164], and compounds having a carbamate group as described in JP 3790649. Inter alia, tris[2-(methoxymethoxy)ethyl]amine, tris[2-(methoxymethoxy)ethyl]amine-N-oxide, dibutylaminobenzoic acid, morpholine derivatives, and imidazole derivatives are preferred. Addition of a basic compound may be effective for further suppressing the diffusion rate of acid in the resist film or correcting the pattern profile.

Onium salts such as sulfonium, iodonium and ammonium salts of carboxylic acids which are not fluorinated at α-position as described in USP 8,795,942 (JP-A 2008-158339) may also be used as the quencher. While an α-fluorinated sulfonic acid, imide acid, and methide acid are necessary to deprotect the acid labile group, an α-non-fluorinated carboxylic acid is released by salt exchange with an α-non-fluorinated onium salt. The α-non-fluorinated carboxylic acid functions as a quencher because it does not induce substantial deprotection reaction.

Examples of the onium salt of α-non-fluorinated carboxylic acid include compounds having the formula (D1).

**R¹⁰¹-CO₂⁻ Mq_{A}⁺** **(D1)**

In formula (D1), R¹⁰¹ is hydrogen or a C₁-C₄₀ hydrocarbyl group which may contain a heteroatom, exclusive of the hydrocarbyl group in which the hydrogen bonded to the carbon atom at α-position of the carboxy group is substituted by fluorine or fluoroalkyl.

The hydrocarbyl group R¹⁰¹ may be saturated or unsaturated and straight, branched or cyclic. Examples thereof include C₁-C₄₀ alkyl groups such as methyl, ethyl, n-propyl, isopropyl, n-butyl, sec-butyl, tert-butyl, n-pentyl, tert-pentyl, n-hexyl, n-octyl, 2-ethylhexyl, n-nonyl, n-decyl; C₃-C₄₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; C₂-C₄₀ alkenyl groups such as vinyl, allyl, propenyl, butenyl and hexenyl; C₃-C₄₀ cyclic unsaturated aliphatic hydrocarbyl groups such as cyclohexenyl; C₆-C₄₀ aryl groups such as phenyl, naphthyl, alkylphenyl groups (e.g., 2-methylphenyl, 3-methylphenyl, 4-methylphenyl, 4-ethylphenyl, 4-tert-butylphenyl, 4-n-butylphenyl), di- or trialkylphenyl groups (e.g., 2,4-dimethylphenyl and 2,4,6-triisopropylphenyl), alkylnaphthyl groups (e.g., methylnaphthyl and ethylnaphthyl), dialkylnaphthyl groups (e.g., dimethylnaphthyl and diethylnaphthyl); and C₇-C₄₀ aralkyl groups such as benzyl, 1-phenylethyl and 2-phenylethyl.

In the hydrocarbyl groups, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy moiety, cyano moiety, carbonyl moiety, ether bond, thioether bond, ester bond, sulfonate ester bond, carbonate bond, lactone ring, sultone ring, carboxylic anhydride (-C(=O)-O-C(=O)-), or haloalkyl moiety. Suitable heteroatom-containing hydrocarbyl groups include heteroaryl groups such as thienyl; alkoxyphenyl groups such as 4-hydroxyphenyl, 4-methoxyphenyl, 3-methoxyphenyl, 2-methoxyphenyl, 4-ethoxyphenyl, 4-tert-butoxyphenyl, 3-tert-butoxyphenyl; alkoxynaphthyl groups such as methoxynaphthyl, ethoxynaphthyl, n-propoxynaphthyl and n-butoxynaphthyl; dialkoxynaphthyl groups such as dimethoxynaphthyl and diethoxynaphthyl; and aryloxoalkyl groups, typically 2-aryl-2-oxoethyl groups such as 2-phenyl-2-oxoethyl, 2-(1-naphthyl)-2-oxoethyl and 2-(2-naphthyl)-2-oxoethyl.

In formula (D1), Mq_{A}⁺ is an onium cation. The onium cation is preferably selected from sulfonium, iodonium and ammonium cations, more preferably sulfonium and iodonium cations. Exemplary sulfonium cations include those exemplified above for the sulfonium cation having formula (Z-1) and the sulfonium cation having formula (Z-3). Exemplary iodonium cations include those exemplified above for the iodonium cation having formula (Z-2).

Examples of the anion in the onium salt having formula (D1) are shown below, but not limited thereto.

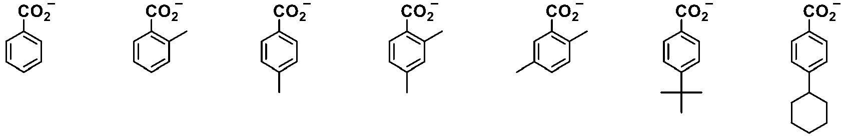

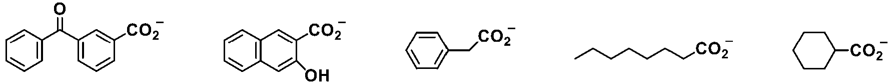

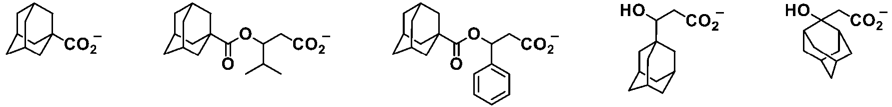

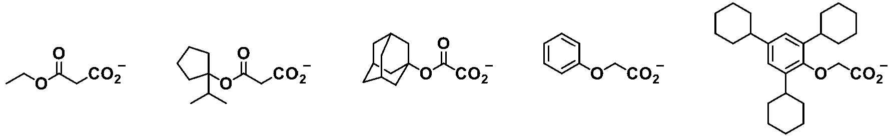

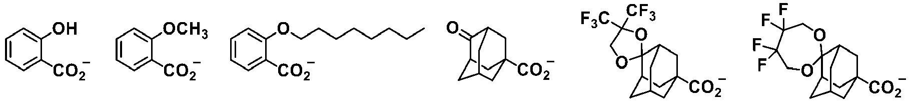

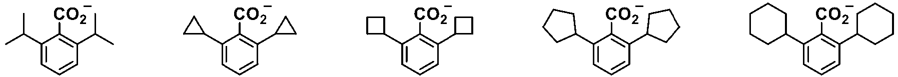

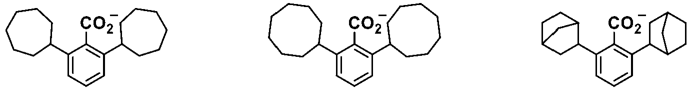

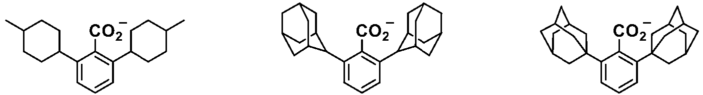

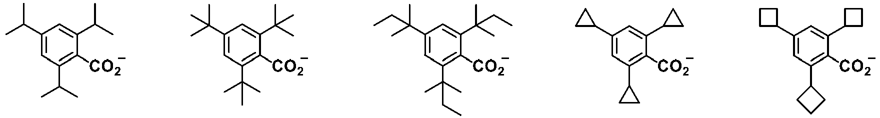

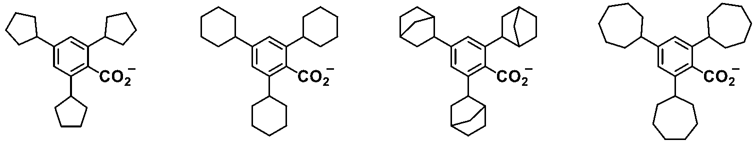

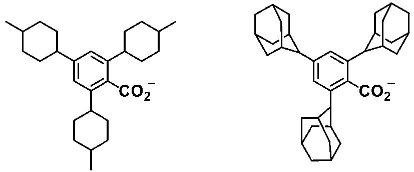

A sulfonium salt of iodized benzene ring-containing carboxylic acid having the formula (D2) is also useful as the quencher.

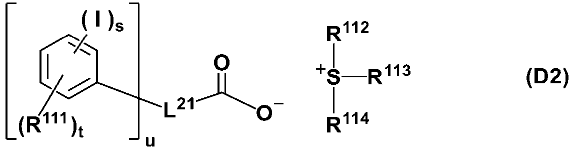

In formula (D2), s is 1, 2, 3, 4 or 5, t is 0, 1, 2 or 3, s+t is from 1 to 5, and u is 1, 2 or 3.

In formula (D2), R¹¹¹ is hydroxy, fluorine, chlorine, bromine, amino, nitro, cyano, or a C₁-C₆ saturated hydrocarbyl, C₁-C₆ saturated hydrocarbyloxy, C₂-C₆ saturated hydrocarbylcarbonyloxy or C₁-C₄ saturated hydrocarbylsulfonyloxy group, in which some or all hydrogen may be substituted by halogen, or -N(R^{111A})-C(-O)-R^{111B}, or -N(R^{111A})-C(=O)-O-R^{111B}. R^{111A} is hydrogen or a C₁-C₆ saturated hydrocarbyl group. R^{111B} is a C₁-C₆ saturated hydrocarbyl or C₂-C₈ unsaturated aliphatic hydrocarbyl group. A plurality of R¹¹¹ may be identical or different when t and/or u is 2 or 3.

In formula (D2), L²¹ is a single bond or a C₁-C₂₀ (u+1)-valent linking group which may contain at least one moiety selected from ether bond, carbonyl moiety, ester bond, amide bond, sultone ring, lactam ring, carbonate bond, halogen, hydroxy moiety, and carboxy moiety. The saturated hydrocarbyl, saturated hydrocarbyloxy, saturated hydrocarbylcarbonyloxy, and saturated hydrocarbylsulfonyloxy groups may be straight, branched or cyclic.

In formula (D2), R¹¹², R¹¹³ and R¹¹⁴ are each independently halogen, or a C₁-C₂₀ hydrocarbyl group which may contain a heteroatom. The hydrocarbyl group may be saturated or unsaturated and straight, branched or cyclic. Examples thereof include C₁-C₂₀ alkyl, C₂-C₂₀ alkenyl, C₆-C₂₀ aryl, and C₇-C₂₀ aralkyl groups. In these groups, some or all hydrogen may be substituted by hydroxy, carboxy, halogen, oxo, cyano, nitro, sultone ring, sulfo, or sulfonium salt-containing moiety, or some -CH₂- may be replaced by an ether bond, ester bond, carbonyl moiety, amide bond, carbonate bond or sulfonate ester bond. Also, R¹¹² and R¹¹³ may bond together to form a ring with the sulfur atom to which they are attached.

Examples of the compound having formula (D2) include those described in USP 10,295,904 (JP-A 2017-219836). These compounds exert a sensitizing effect due to remarkable absorption and an acid diffusion-controlling effect.

A nitrogen-containing carboxylic acid salt compound having the formula (D3) is also useful as the quencher.

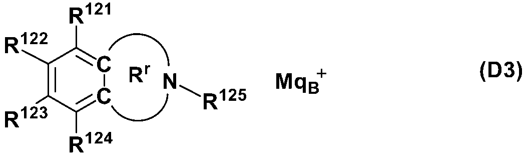

In formula (D3), R¹²¹ to R¹²⁴ are each independently hydrogen, -L²²-CO₂⁻, or a C₁-C₂₀ hydrocarbyl group which may contain a heteroatom. R¹²¹ and R¹²², R¹²² and R¹²³, or R¹²³ and R¹²⁴ may bond together to form a ring with the carbon atom to which they are attached. L²² is a single bond or a C₁-C₂₀ hydrocarbylene group which may contain a heteroatom. R¹²⁵ is hydrogen or a C₁-C₂₀ hydrocarbyl group which may contain a heteroatom.

In formula (D3), the ring R^{r} is a C₂-C₆ ring containing the carbon and nitrogen atoms in the formula, in which some or all of the carbon-bonded hydrogen atoms may be substituted by a C₁-C₂₀ hydrocarbyl group or -L²²-CO₂⁻ and in which some -CH₂- may be replaced by sulfur, oxygen or nitrogen. The ring may be alicyclic or aromatic and is preferably a 5- or 6-membered ring. Suitable rings include pyridine, pyrrole, pyrrolidine, piperidine, pyrazole, imidazoline, pyridazine, pyrimidine, pyrazine, imidazoline, oxazole, thiazole, morpholine, thiazine, and triazole rings.

The carboxylic onium salt having formula (D3) has at least one -L²²-CO₂⁻. That is, at least one of R¹²¹ to R¹²⁴ is -L²²-CO₂⁻, and/or at least one of hydrogen atoms bonded to carbon atoms in the ring R^{r} is substituted by -L²²-CO₂⁻.

In formula (D3), Mq_{B}⁺ is a sulfonium, iodonium or ammonium cation, with the sulfonium cation being preferred. Examples of the sulfonium cation include those exemplified above for the sulfonium cation having formula (Z-1) and the sulfonium cation having formula (Z-3).

Examples of the anion in the compound having formula (D3) are shown below, but not limited thereto.

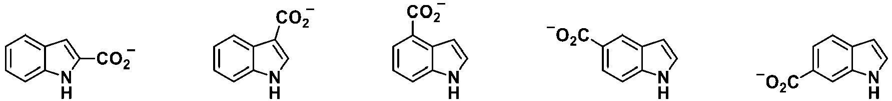

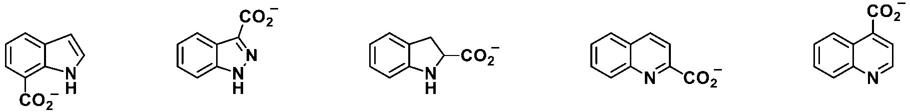

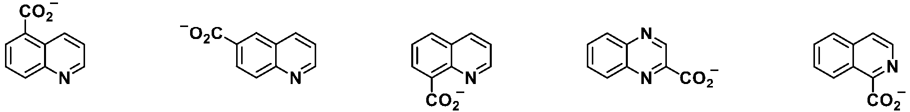

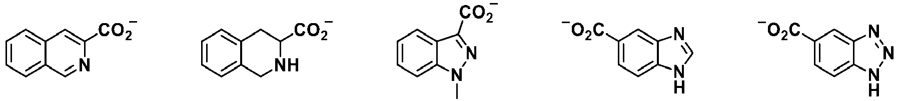

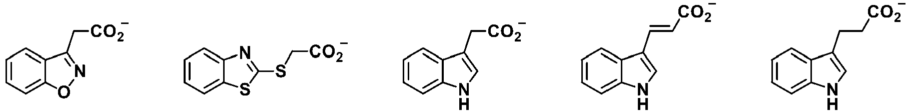

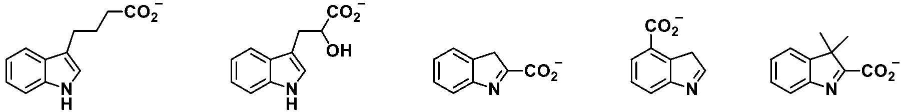

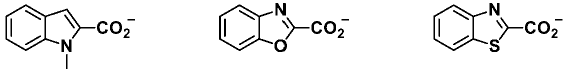

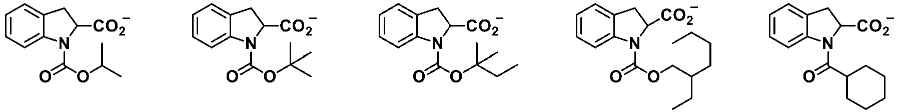

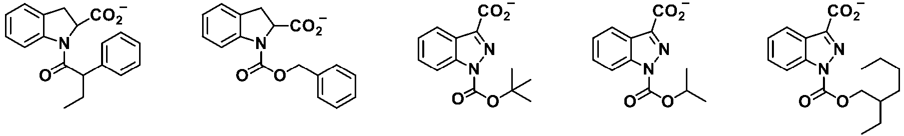

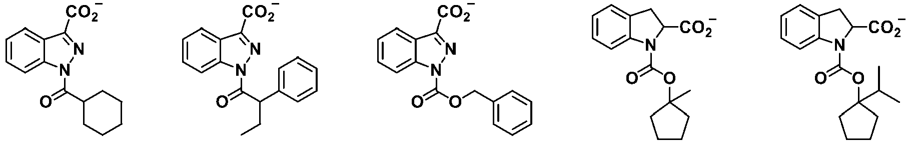

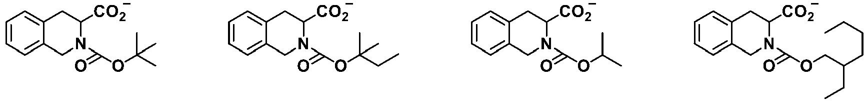

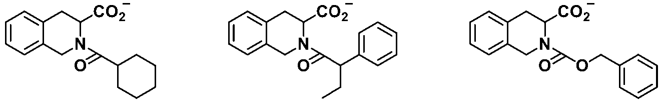

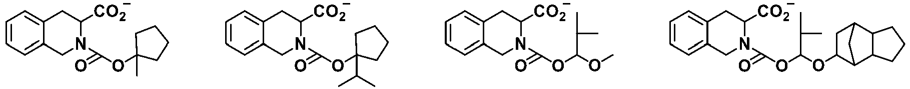

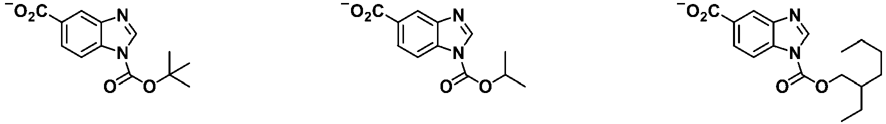

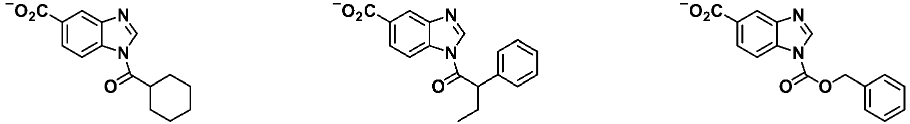

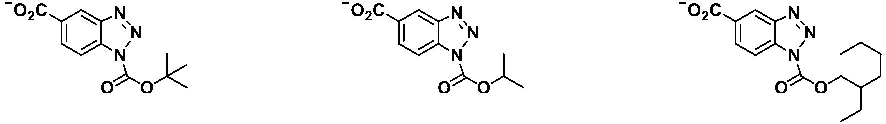

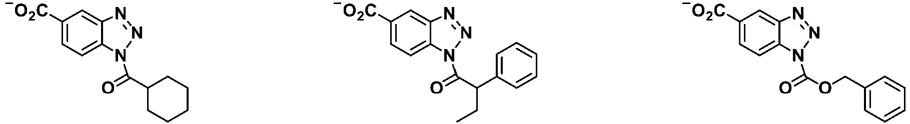

Weak acid betaine compounds are also useful as the quencher. Non-limiting examples thereof are shown below.

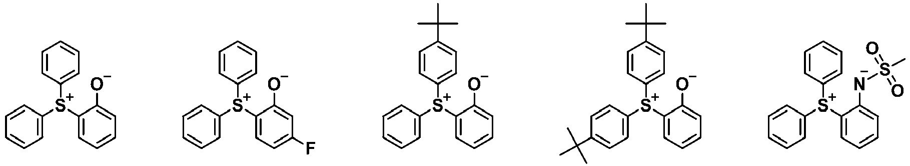

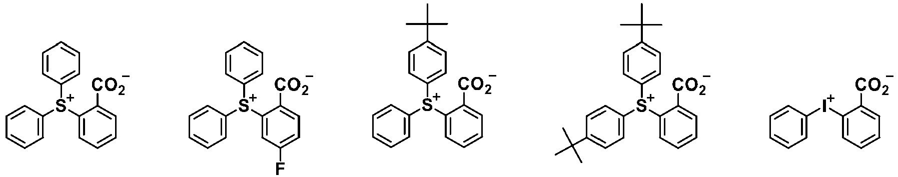

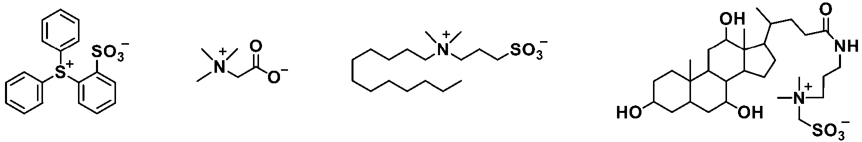

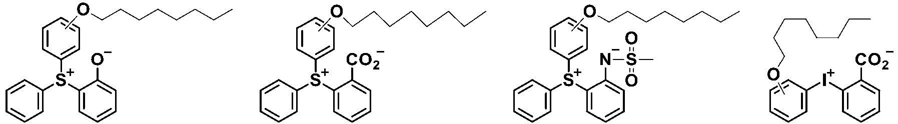

Also useful are quenchers of polymer type as described in USP 7,598,016 (JP-A 2008-239918). The polymeric quencher segregates at the resist surface after coating and thus enhances the rectangularity of resist pattern. When a protective film is applied as is often the case in the immersion lithography, the polymeric quencher is also effective for preventing a film thickness loss of resist pattern or rounding of pattern top.

When used, the quencher (D) is preferably added in an amount of 0 to 50 parts, more preferably 0.1 to 40 parts by weight per 80 parts by weight of the base polymer (B). The quencher may be used alone or in admixture.

### (E) Fluorinated polymer

The chemically amplified positive resist composition may further comprise a fluorinated polymer for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. The fluorinated polymer contains repeat units of at least one type selected from repeat units having the formula (E1), repeat units having the formula (E2), repeat units having the formula (E3), and repeat units having the formula (E4), and may contain repeat units of at least one type selected from repeat units having the formula (E5) and repeat units having the formula (E6). It is noted that repeat units having formulae (E1), (E2), (E3), (E4), (E5), and (E6) are also referred to as repeat units E1, E2, E3, E4, E5, and E6, respectively, hereinafter. Since the fluorinated polymer also has a surface-active function, it can prevent insoluble residues from re-depositing onto the substrate during the development step and is thus effective for preventing development defects.

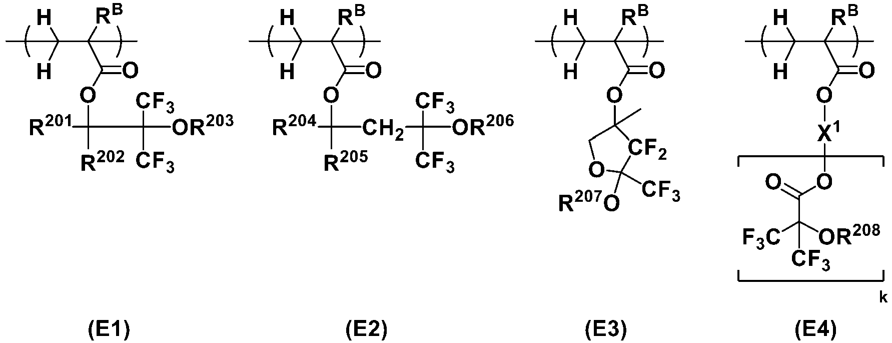

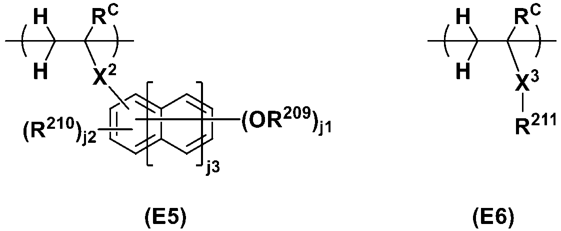

In formulae (E1) to (E6), j1 is 1, 2 or 3, j2 is an integer meeting: 0 ≤ j2 ≤ 5+2(j3)-j1, j3 is 0 or 1, and k is 1, 2 or 3. R^{B} is each independently hydrogen, fluorine, methyl or trifluoromethyl. R^{C} is each independently hydrogen or methyl. R²⁰¹, R²⁰², R²⁰⁴ and R²⁰⁵ are each independently hydrogen or a C₁-C₁₀ saturated hydrocarbyl group. R²⁰³, R²⁰⁶, R²⁰⁷ and R²⁰⁸ are each independently hydrogen, a C₁-C₁₅ hydrocarbyl group or fluorinated hydrocarbyl group, or an acid labile group. An ether bond or carbonyl moiety may intervene in a carbon-carbon bond in the hydrocarbyl groups or fluorinated hydrocarbyl groups represented by R²⁰³, R²⁰⁶, R²⁰⁷ and R²⁰⁸. R²⁰⁹ is hydrogen or a C₁-C₅ straight or branched hydrocarbyl group in which a heteroatom-containing moiety may intervene in a carbon-carbon bond. A plurality of R²⁰⁹ may be identical or different when j1 is 2 or 3. R²¹⁰ is a C₁*-*C₅ straight or branched hydrocarbyl group in which a heteroatom-containing moiety may intervene in a carbon-carbon bond. A plurality of R²¹⁰ may be identical or different when j2 is 2 or more. R²¹¹ is a C₁-C₂₀ saturated hydrocarbyl group in which at least one hydrogen is substituted by fluorine and some constituent -CH₂- may be replaced by an ester bond or ether bond. X¹ is a C₁-C₂₀ (k+1)-valent hydrocarbon group or C₁-C₂₀ (k+1)-valent fluorinated hydrocarbon group. X² is a single bond, *-C(=O)-O- or *-C(=O)-NH- wherein * designates a point of attachment to the carbon atom in the backbone. X³ is a single bond, -O-, *-C(=O)-O-X³¹-X³²- or *-C(=O)-NH-X³¹-X³²-, wherein X³¹ is a single bond or a C₁-C₁₀ saturated hydrocarbylene group, X³² is a single bond, ester bond, ether bond or sulfonamide bond, and * designates a point of attachment to the carbon atom in the backbone.

In formulae (E1) and (E2), the C₁-C₁₀ saturated hydrocarbyl group represented by R²⁰¹, R²⁰², R²⁰⁴ and R²⁰⁵ may be straight, branched or cyclic and examples thereof include C₁-C₁₀ alkyl groups such as methyl, ethyl, n-propyl, isopropyl, n-butyl, isobutyl, sec-butyl, tert-butyl, n-pentyl, n-hexyl, n-heptyl, n-octyl, n-nonyl, and n-decyl, and C₃-C₁₀ cyclic saturated hydrocarbyl groups such as cyclopropyl, cyclobutyl, cyclopentyl, cyclohexyl, adamantyl, and norbornyl. Inter alia, C₁-C₆ saturated hydrocarbyl groups are preferred.

In formulae (E1) to (E4), the C₁*-*C₁₅ hydrocarbyl group represented by R²⁰³, R²⁰⁶, R²⁰⁷ and R²⁰⁸ may be straight, branched or cyclic and examples thereof include C₁-C₁₅ alkyl, C₂-C₁₅ alkenyl and C₂-C₁₅ alkynyl groups, with the alkyl groups being preferred. Suitable alkyl groups include methyl, ethyl, n-propyl, isopropyl, n-butyl, isobutyl, sec-butyl, n-pentyl, n-hexyl, n-heptyl, n-octyl, n-nonyl, n-decyl, n-undecyl, n-dodecyl, n-tridecyl, n-tetradecyl and n-pentadecyl. The fluorinated hydrocarbyl groups correspond to the foregoing hydrocarbyl groups in which some or all carbon-bonded hydrogen atoms are substituted by fluorine atoms.

In formula (E4), examples of the C₁-C₂₀ (k+1)-valent hydrocarbon group X¹ include the foregoing C₁-C₂₀ alkyl groups and C₃-C₂₀ cyclic saturated hydrocarbyl groups, with "k" number of hydrogen atoms being eliminated. Examples of the C₁-C₂₀ (k+1)-valent fluorinated hydrocarbon group X¹ include the foregoing (k+1)-valent hydrocarbon groups in which at least one hydrogen atom is substituted by fluorine.

Examples of the repeat units E1 to E4 are given below, but not limited thereto. Herein R^{B} is as defined above.

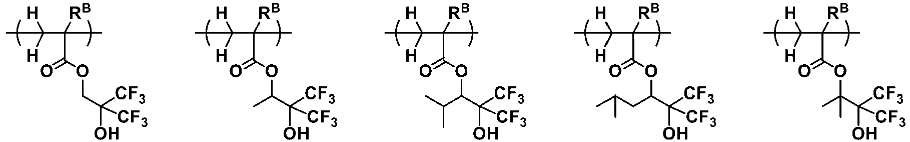

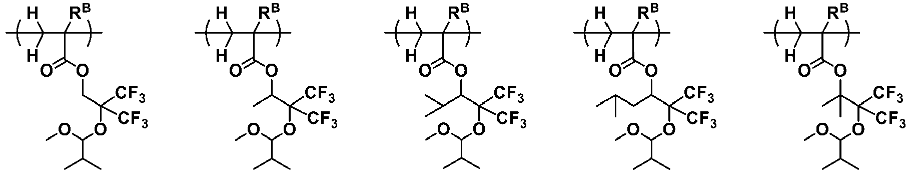

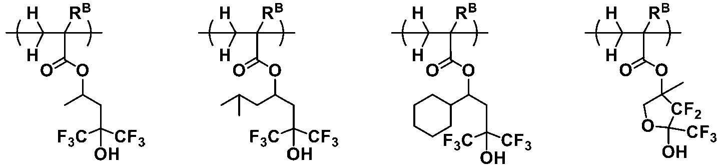

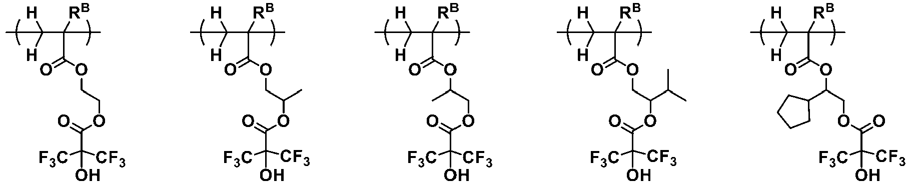

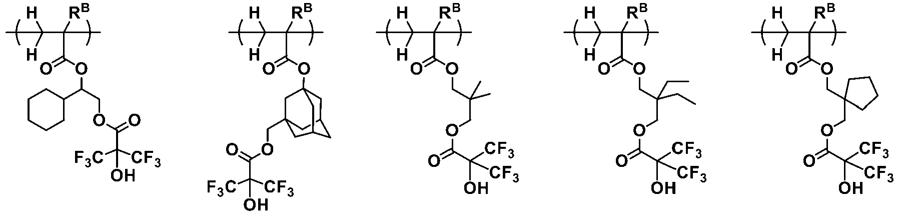

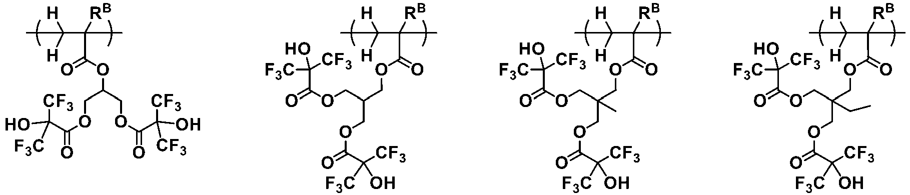

In formula (E5), examples of the C₁-C₅ hydrocarbyl groups R¹⁰⁹ and R¹¹⁰ include alkyl, alkenyl and alkynyl groups, with the alkyl groups being preferred. Suitable alkyl groups include methyl, ethyl, n-propyl, isopropyl, n-butyl, isobutyl, sec-butyl, and n-pentyl. In the hydrocarbyl group, a moiety containing a heteroatom such as oxygen, sulfur or nitrogen may intervene in a carbon-carbon bond.

In formula (E5), -OR¹⁰⁹ is preferably a hydrophilic group. In this case, R¹⁰⁹ is preferably hydrogen or a C₁-C₅ alkyl group in which oxygen intervenes in a carbon-carbon bond.

In formula (E5), X² is preferably *-C(=O)-O- or *-C(=O)-NH-. Also preferably R^{C} is methyl. The inclusion of carbonyl in X² enhances the ability to trap the acid originating from the anti-charging film. A polymer wherein R^{C} is methyl is a robust polymer having a high glass transition temperature (Tg) which is effective for suppressing acid diffusion. As a result, the resist film is improved in stability with time, and neither resolution nor pattern profile is degraded.

Examples of the repeat unit E5 are given below, but not limited thereto. Herein R^{C} is as defined above.

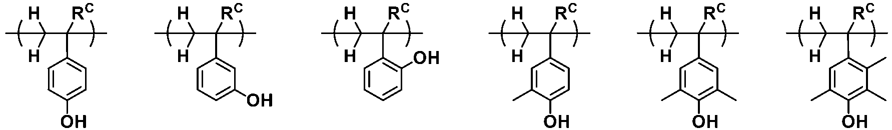

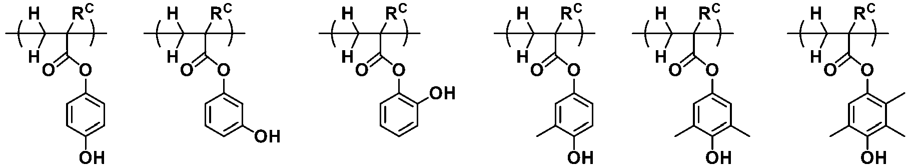

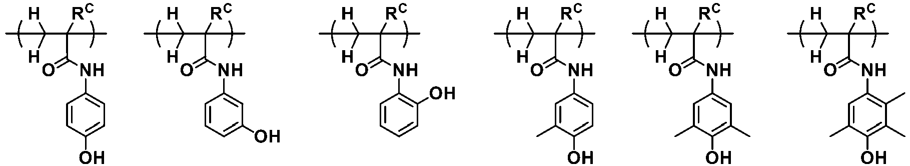

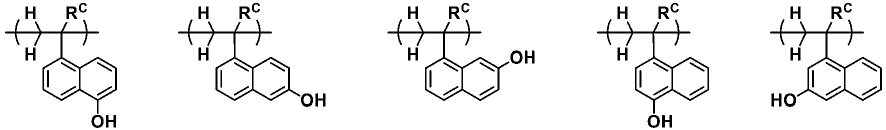

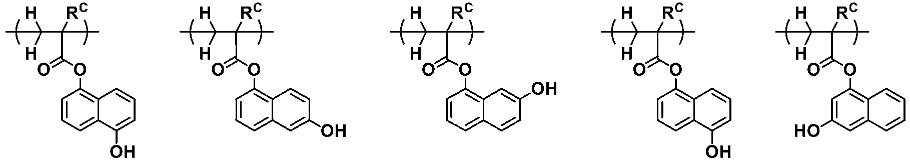

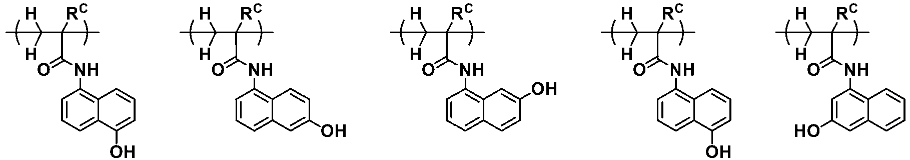

In formula (E6), the C₁-C₁₀ saturated hydrocarbylene group X³ may be straight, branched or cyclic and examples thereof include methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, and 1,1-dimethylethane-1,2-diyl.

The C₁-C₂₀ saturated hydrocarbyl group having at least one hydrogen substituted by fluorine, represented by R²¹¹, may be straight, branched or cyclic and examples thereof include C₁-C₂₀ alkyl groups and C₃-C₂₀ cyclic saturated hydrocarbyl groups in which at least one hydrogen is substituted by fluorine.

Examples of the repeat unit E6 are given below, but not limited thereto. Herein R^{C} is as defined above.

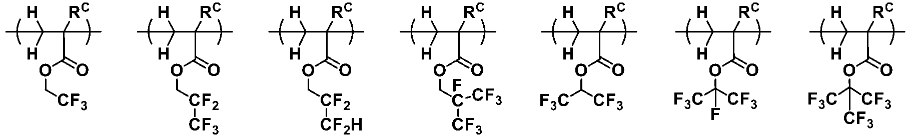

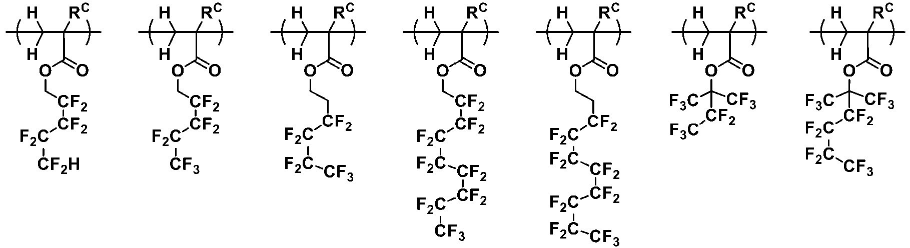

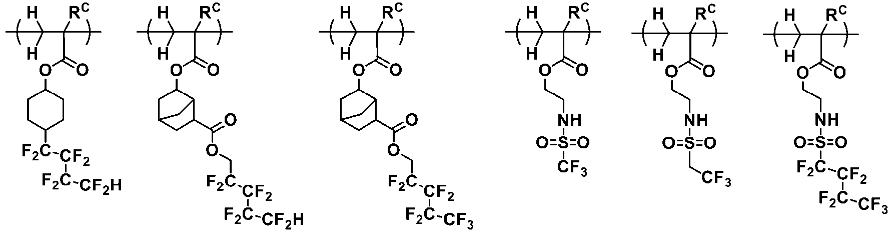

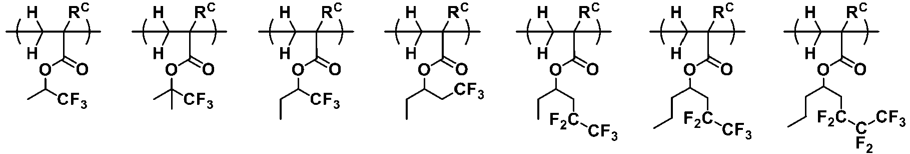

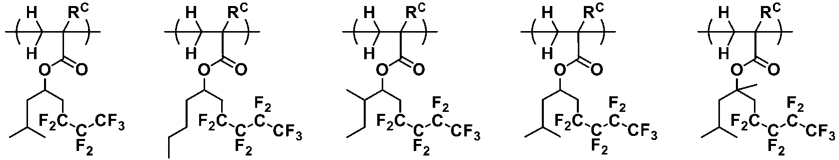

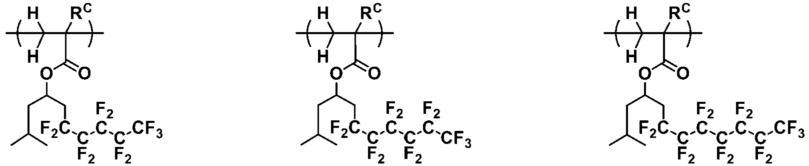

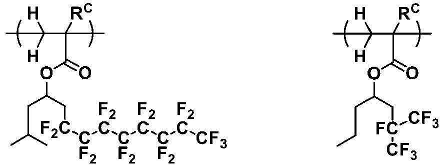

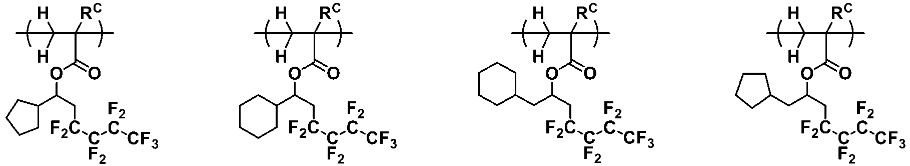

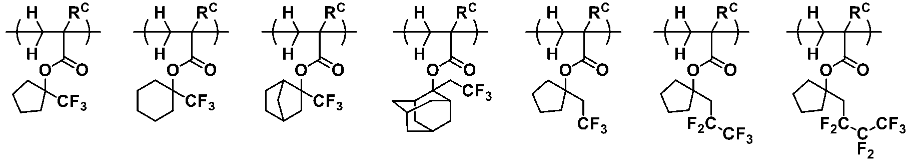

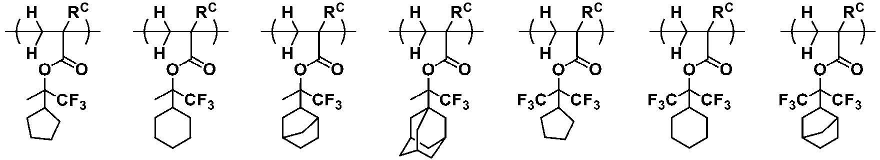

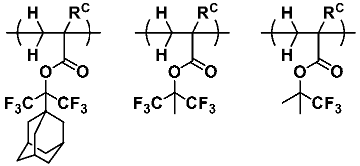

The content of repeat units E1 to E4 is preferably 15 to 95 mol%, more preferably 20 to 85 mol% based on the overall repeat units of the fluorinated polymer. The content of repeat unit E5 and/or E6 is preferably 5 to 85 mol%, more preferably 15 to 80 mol% based on the overall repeat units of the fluorinated polymer. Each of repeat units E1 to E6 may be used alone or in admixture.

The fluorinated polymer may comprise additional repeat units as well as the repeat units E1 to E6. Suitable additional repeat units include those described in USP 9,091,918 (JP-A 2014-177407, paragraphs [0046]-[0078]). When the fluorinated polymer comprises additional repeat units, their content is preferably up to 50 mol% based on the overall repeat units.

The fluorinated polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630.

The fluorinated polymer should preferably have a Mw of 2,000 to 50,000, and more preferably 3,000 to 20,000. A fluorinated polymer with a Mw of less than 2,000 helps acid diffusion, degrading resolution and detracting from age stability. A polymer with too high Mw has a reduced solubility in solvent, with a risk of leaving coating defects. The fluorinated polymer preferably has a dispersity (Mw/Mn) of 1.0 to 2.2, more preferably 1.0 to 1.7.

In the resist composition, the fluorinated polymer (E) is preferably used in an amount of 0.01 to 30 parts by weight, more preferably 0.1 to 20 parts by weight per 80 parts by weight of the base polymer (B). The fluorinated polymer may be used alone or in admixture.

### (F) Other photoacid generator

In addition to the PAG in the form of the onium salt having formula (A), the chemically amplified positive resist composition may further comprise another photoacid generator (PAG) as component (F). The other PAG used herein may be any compound capable of generating an acid upon exposure to high-energy radiation. Suitable PAGs include sulfonium salts, iodonium salts, sulfonyldiazomethane, N-sulfonyloxyimide, and oxime-O-sulfonate acid generators.

Suitable other PAGs include nonafluorobutane sulfonate, partially fluorinated sulfonates described in JP-A 2012-189977, paragraphs [0247]-[0251], partially fluorinated sulfonates described in JP-A 2013-101271, paragraphs [0261]-[0265], and those described in JP-A 2008-111103, paragraphs [0122]-[0142] and JP-A 2010-215608, paragraphs [0080]-[0081]. Among others, arenesulfonate and alkanesulfonate type PAGs are preferred because they generate acids having an appropriate strength to deprotect the acid labile group in repeat unit B2.

The preferred PAGs are salt compounds having an anion of the structure shown below.

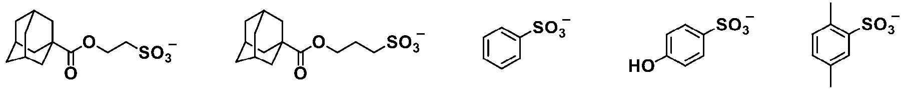

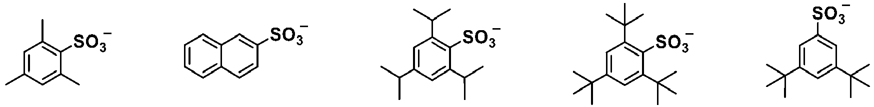

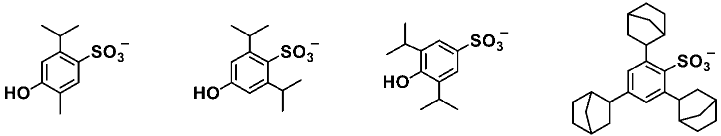

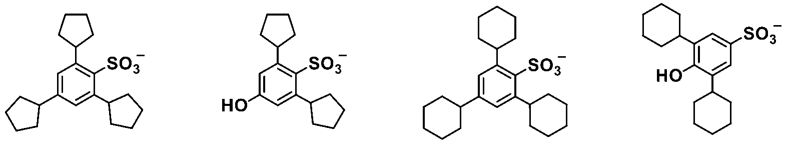

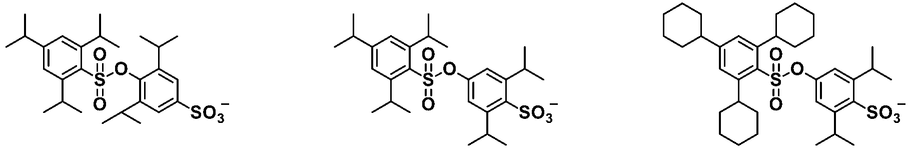

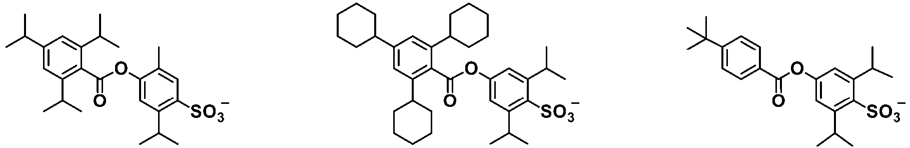

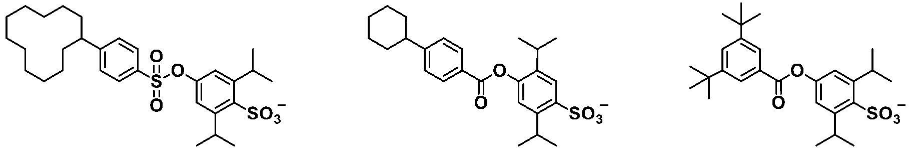

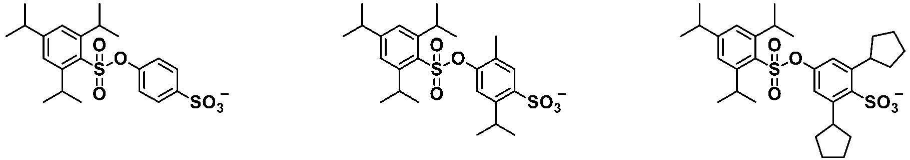

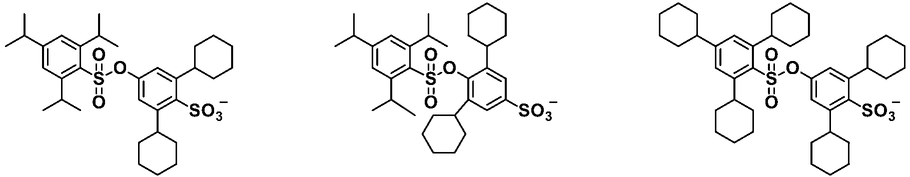

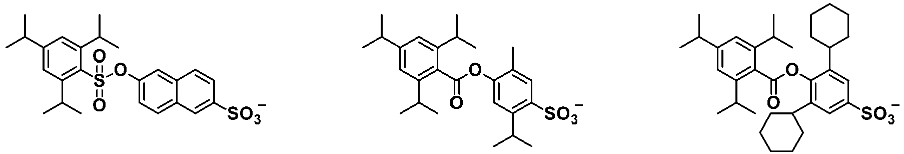

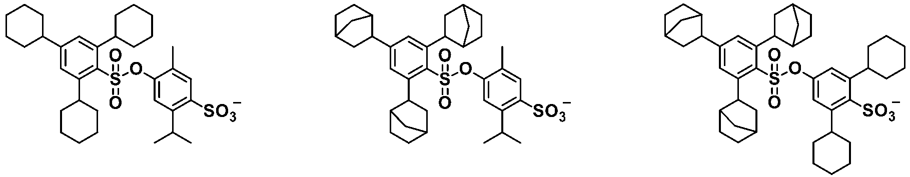

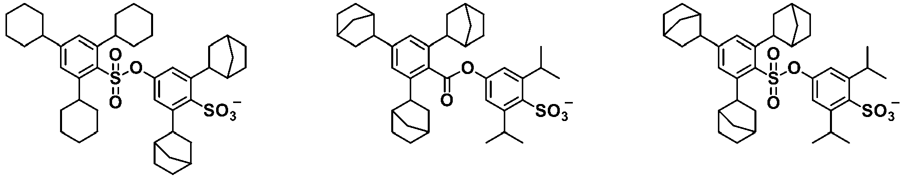

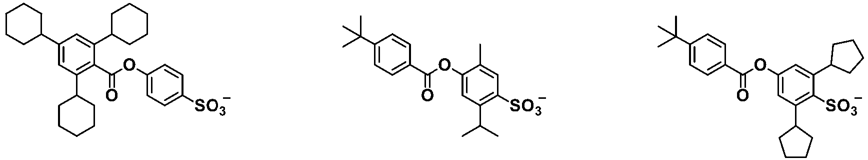

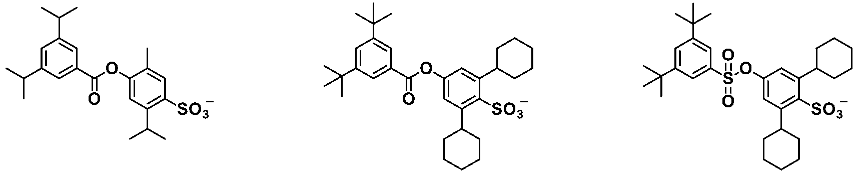

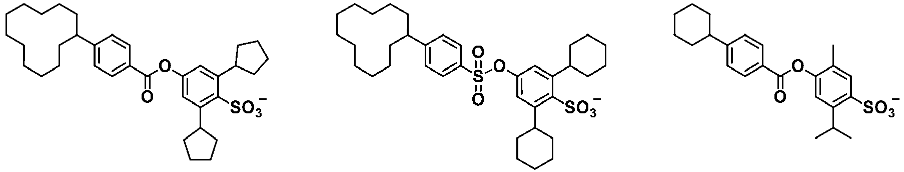

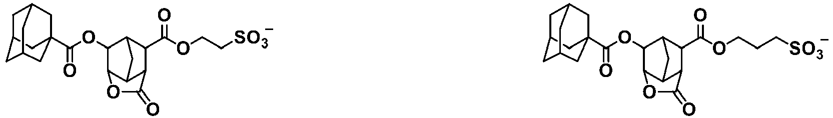

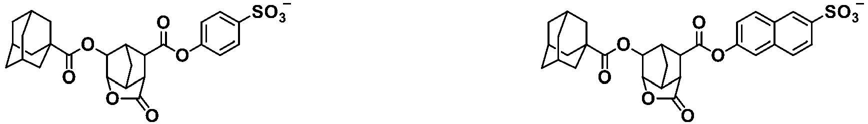

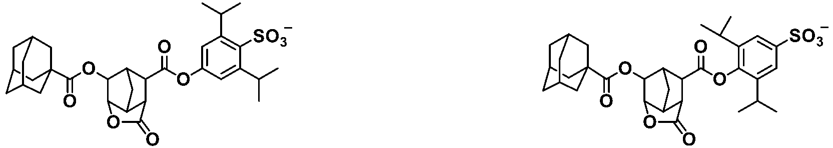

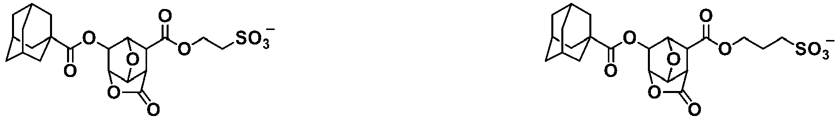

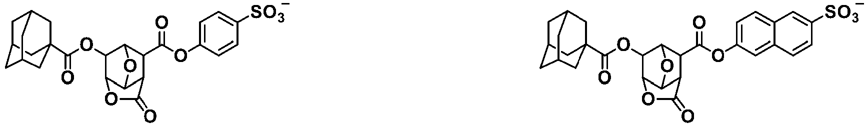

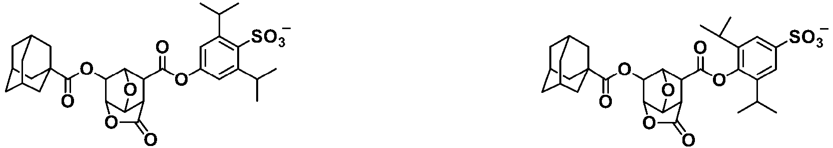

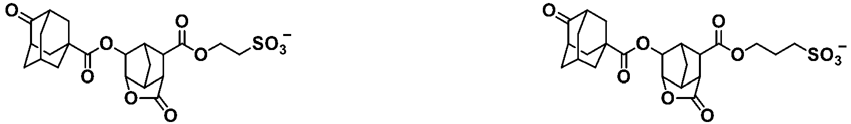

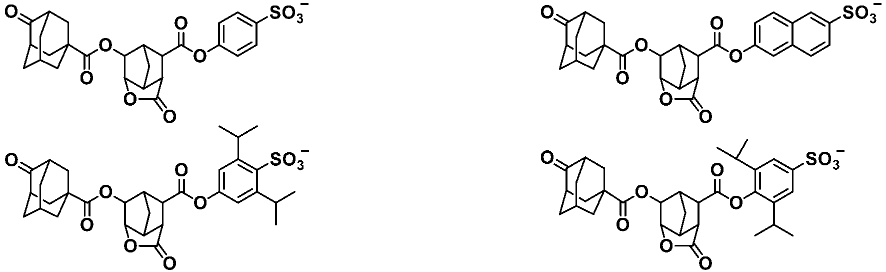

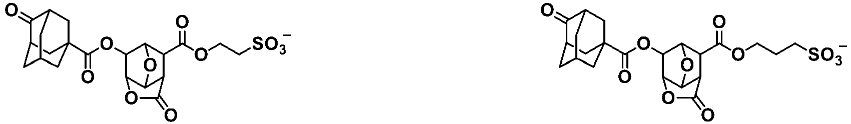

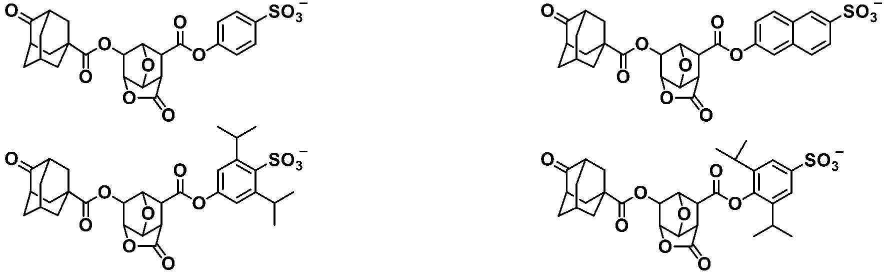

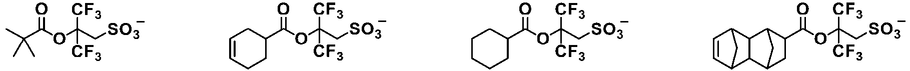

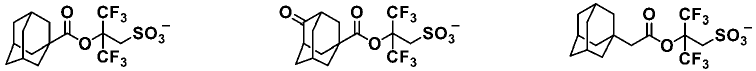

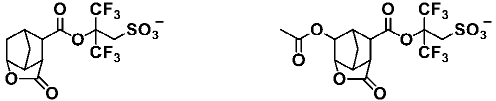

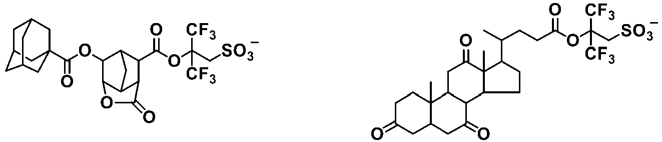

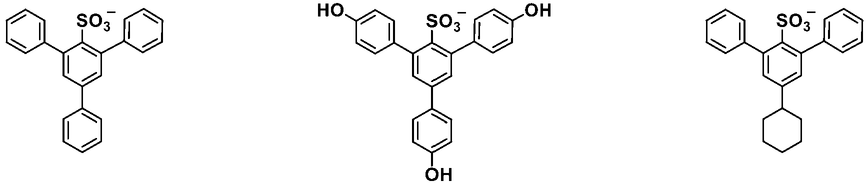

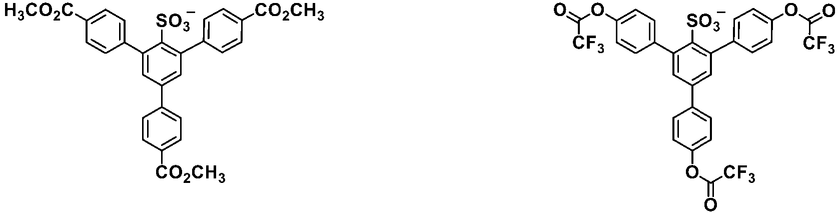

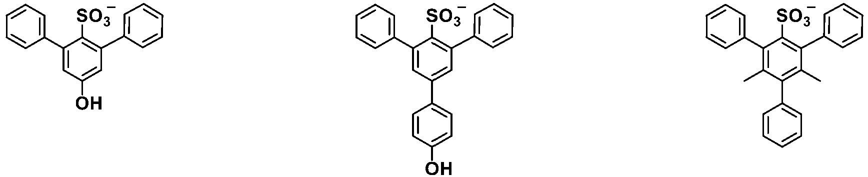

Also preferred as the PAG is a salt compound containing an anion having the formula (F1).

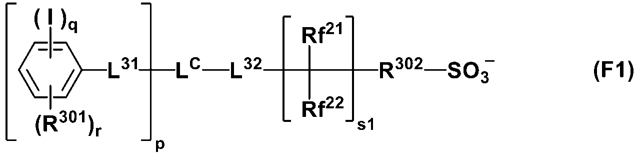

In formula (F1), p is 1, 2 or 3, q is 1, 2, 3, 4 or 5, r is 0, 1, 2 or 3, and s1 is 0 or 1.

In formula (F1), L³¹ is a single bond, ether bond, ester bond, sulfonate ester bond, carbonate bond or carbamate bond.

In formula (F1), L³² is an ether bond, ester bond, sulfonate ester bond, carbonate bond or carbamate bond.

In formula (F1), L^{C} is a single bond or a C₁-C₂₀ hydrocarbylene group when p=1. L^{C} is a C₁-C₂₀ (p+1)-valent hydrocarbon group when p=2 or 3. The hydrocarbylene group and (p+1)-valent hydrocarbon group may contain at least one moiety selected from ether bond, carbonyl moiety, ester bond, amide bond, sultone ring, lactam ring, carbonate bond, halogen, hydroxy moiety and carboxy moiety.

The C₁-C₂₀ hydrocarbylene group L^{C} may be saturated or unsaturated and straight, branched or cyclic. Examples thereof include C₁-C₂₀ alkanediyl groups such as methanediyl, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl, dodecane-1,12-diyl; C₃-C₂₀ cyclic saturated hydrocarbylene groups such as cyclopentanediyl, cyclohexanediyl, norbornanediyl and adamantanediyl; C₂-C₂₀ unsaturated aliphatic hydrocarbylene groups such as vinylene and propene-1,3-diyl; C₆-C₂₀ arylene groups such as phenylene and naphthylene; and combinations thereof. The C₁-C₂₀ (p+1)-valent hydrocarbon group L^{C} may be saturated or unsaturated and straight, branched or cyclic. Examples thereof include those exemplified above for the C₁-C₂₀ hydrocarbylene group, with one or two hydrogen atoms being eliminated.

In formula (F1), Rf²¹ and Rf²² are each independently hydrogen, fluorine or trifluoromethyl, at least one being fluorine or trifluoromethyl.

In formula (F1), R³⁰¹ is hydroxy, carboxy, a C₁-C₆ saturated hydrocarbyl group, C₁-C₆ saturated hydrocarbyloxy group, C₂-C₆ saturated hydrocarbylcarbonyloxy group, fluorine, chlorine, bromine, -N(R^{301A})(R^{301B}), -N(R^{301C})-C(=O)-R^{301D} or -N(R^{301C})-C(=O)-O-R^{301D}. R^{301A} and R^{301B} are each independently hydrogen or a C₁-C₆ saturated hydrocarbyl group. R^{301C} is hydrogen or a C₁-C₆ saturated hydrocarbyl group. R^{301D} is a C₁-C₆ saturated hydrocarbyl group or C₂-C₈ unsaturated aliphatic hydrocarbyl group.

The C₁-C₆ saturated hydrocarbyl group represented by R³⁰¹, R^{301A}, R^{301B} and R^{301C} may be straight, branched or cyclic. Examples thereof include C₁-C₆ alkyl groups such as methyl, ethyl, n-propyl, isopropyl, n-butyl, isobutyl, sec-butyl, tert-butyl, n-pentyl, and n-hexyl; and C₃-C₆ cyclic saturated hydrocarbyl groups such as cyclopropyl, cyclobutyl, cyclopentyl, and cyclohexyl. Examples of the saturated hydrocarbyl moiety in the C₁-C₆ saturated hydrocarbyloxy group represented by R³⁰¹ are as exemplified above for the saturated hydrocarbyl group. Examples of the saturated hydrocarbyl moiety in the C₂-C₆ saturated hydrocarbylcarbonyloxy group represented by R³⁰¹ are as exemplified above for the C₁-C₆ saturated hydrocarbyl group, but of 1 to 5 carbon atoms.

The C₂-C₈ unsaturated aliphatic hydrocarbyl group represented by R^{301D} may be straight, branched or cyclic and examples thereof include C₂-C₈ alkenyl groups such as vinyl, propenyl, butenyl, and hexenyl; C₂-C₈ alkynyl groups such as ethynyl, propynyl, and butynyl; and C₃-C₈ cyclic unsaturated aliphatic hydrocarbyl groups such as cyclohexenyl and norbornenyl.

In formula (F1), R³⁰² is a C₁-C₂₀ saturated hydrocarbylene group or C₆-C₁₄ arylene group. Some or all of the hydrogen atoms in the saturated hydrocarbylene group may be substituted by halogen other than fluorine. Some or all of the hydrogen atoms in the arylene group may be substituted by a substituent selected from C₁-C₂₀ saturated hydrocarbyl groups, C₁-C₂₀ saturated hydrocarbyloxy groups, C₆-C₁₄ aryl groups, halogen, and hydroxy.

The C₁-C₂₀ saturated hydrocarbylene group represented by R³⁰² may be straight, branched or cyclic. Examples thereof C₁-C₂₀ alkanediyl groups such as methanediyl, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl undecane-1,11-diyl and dodecane-1,12-diyl; and C₃-C₂₀ cyclic saturated hydrocarbylene groups such as cyclopentanediyl, cyclohexanediyl, norbornanediyl and adamantanediyl.

Examples of the C₆-C₂₀ arylene group represented by R³⁰² include phenylene, naphthylene, phenanthrenediyl, and anthracenediyl. The C₁-C₂₀ saturated hydrocarbyl moiety and hydrocarbyl moiety in the C₁-C₂₀ hydrocarbyloxy moiety, which are substituents on the arylene group, may be straight, branched or cyclic and examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl, and icosyl; and C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl. Examples of the C₆-C₁₄ arylene moiety which is a substituent on the arylene group include phenylene, naphthylene, phenanthrenediyl and anthracenediyl.

More preferably, the anion has the formula (F2).

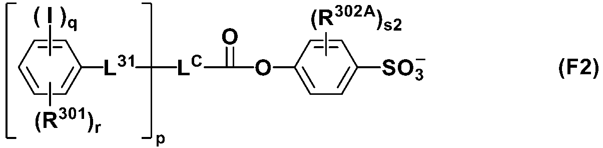

In formula (F2), p, q, r, L³¹, L^{C}, and R³⁰¹ are as defined above. The subscript s2 is 1, 2, 3 or 4. R^{302A} is a C₁-C₁₄ saturated hydrocarbyl group, C₁-C₁₄ saturated hydrocarbyloxy group, C₆-C₁₄ aryl group, halogen or hydroxy group. When s2 is 2, 3 or 4, a plurality of R^{302A} may be identical or different.

Examples of the anion having formula (F1) are shown below, but not limited thereto.

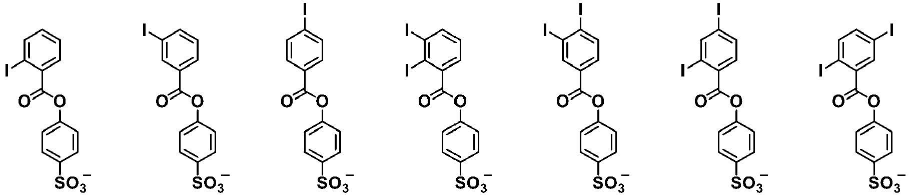

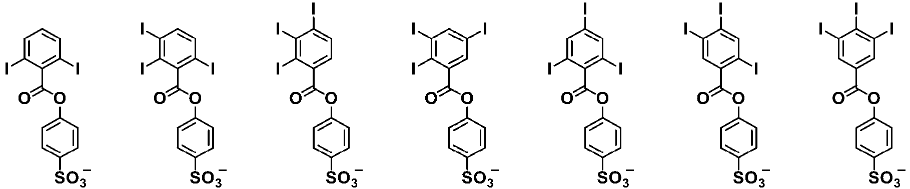

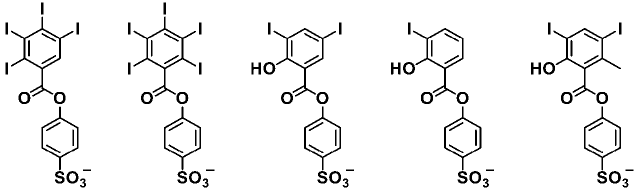

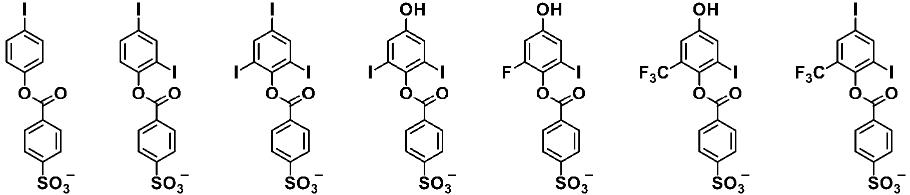

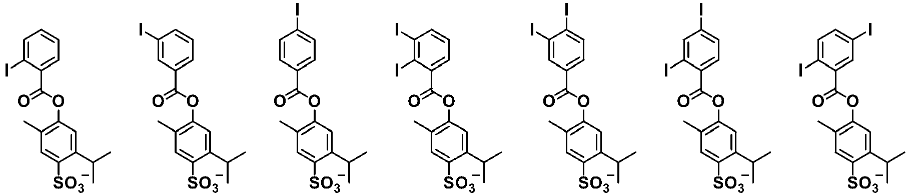

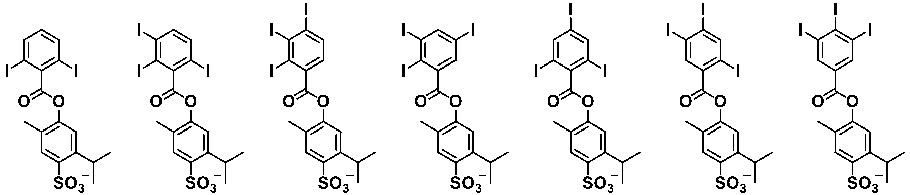

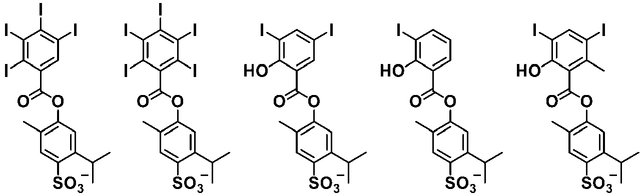

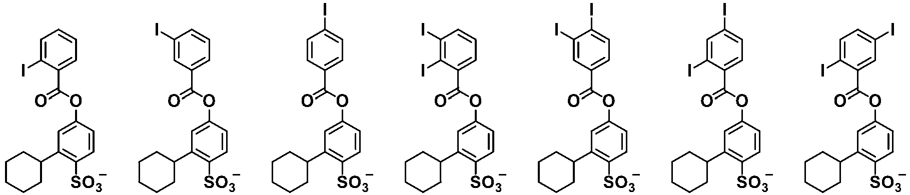

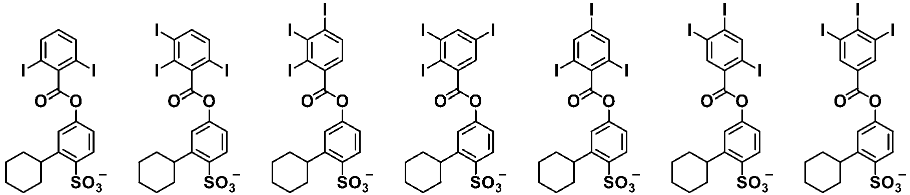

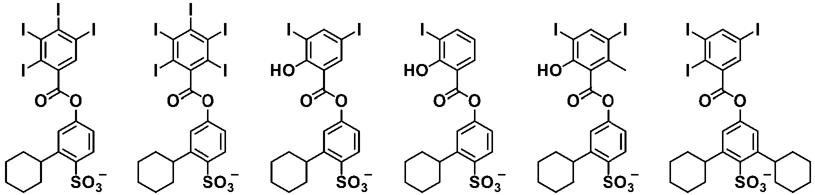

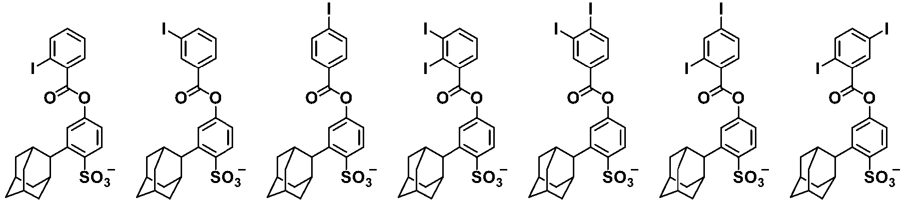

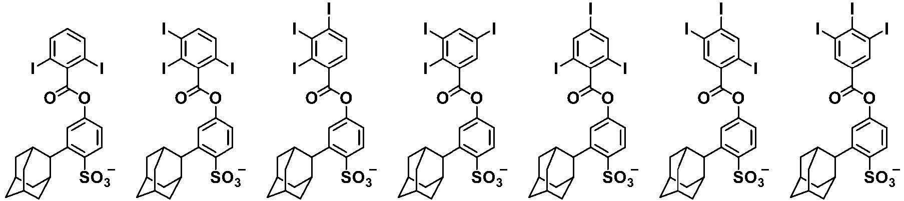

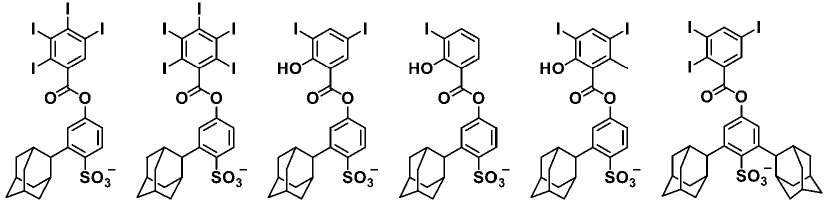

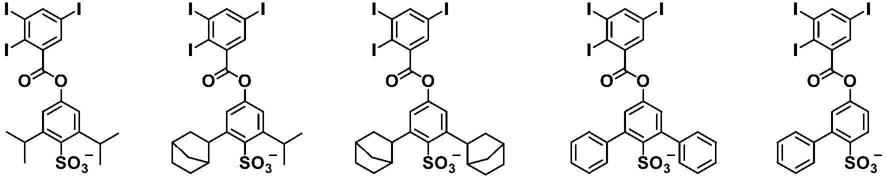

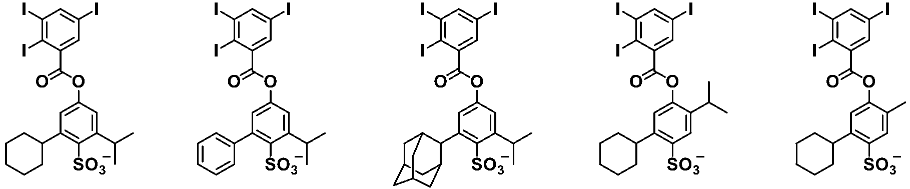

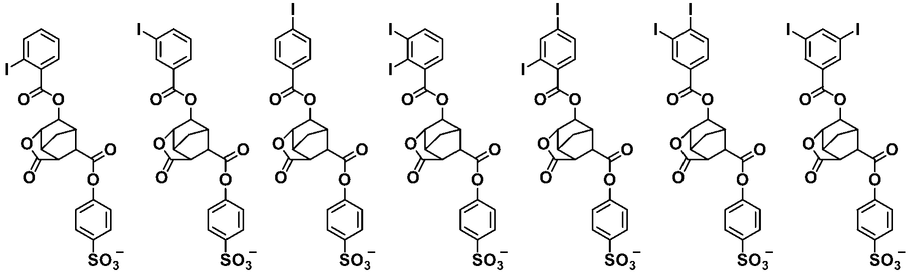

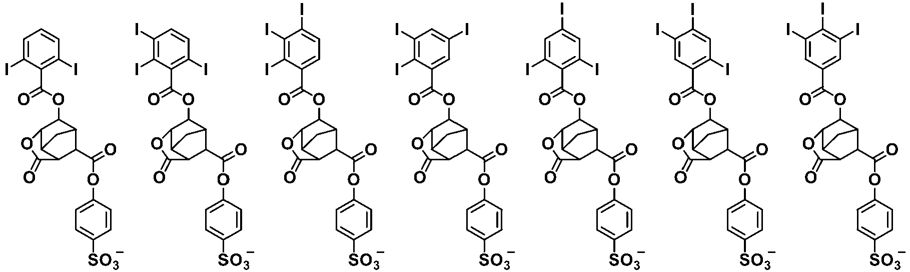

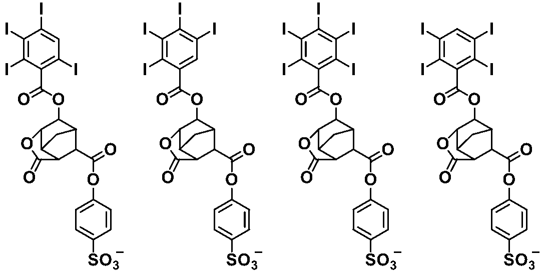

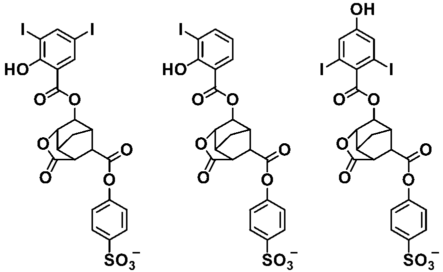

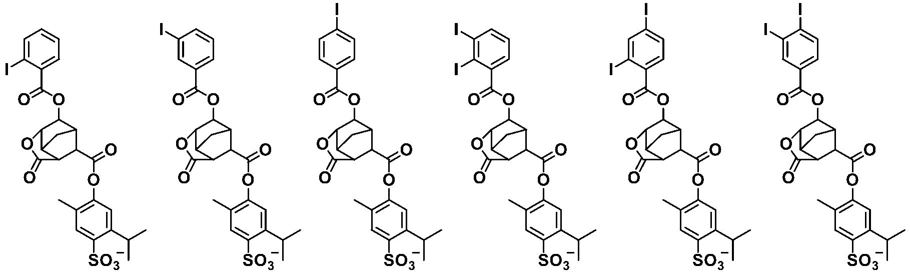

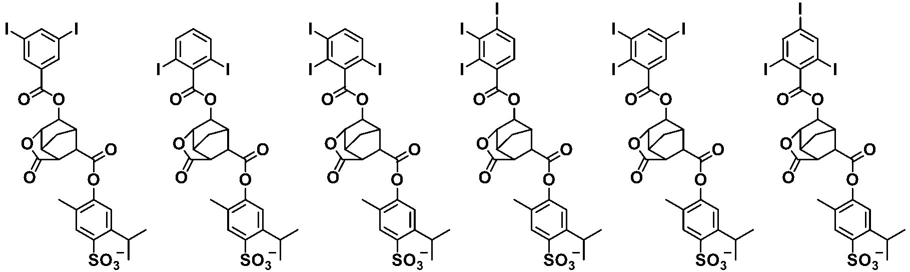

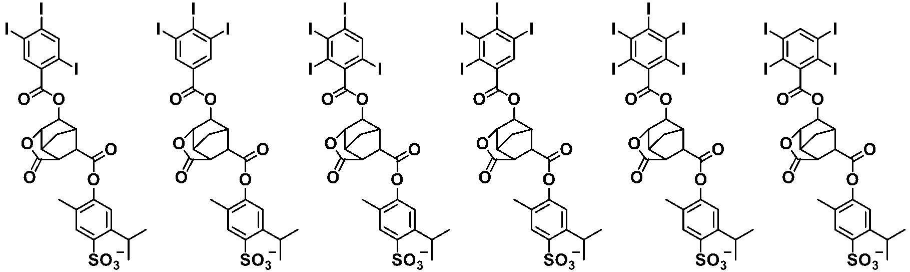

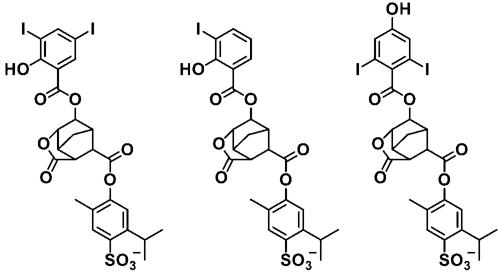

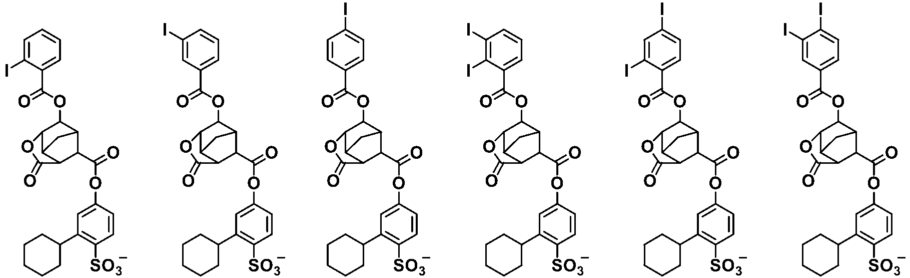

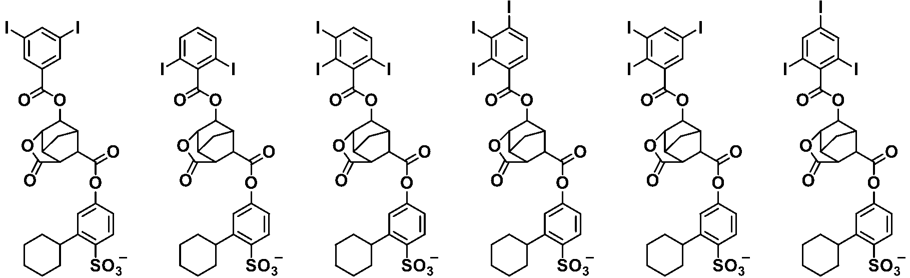

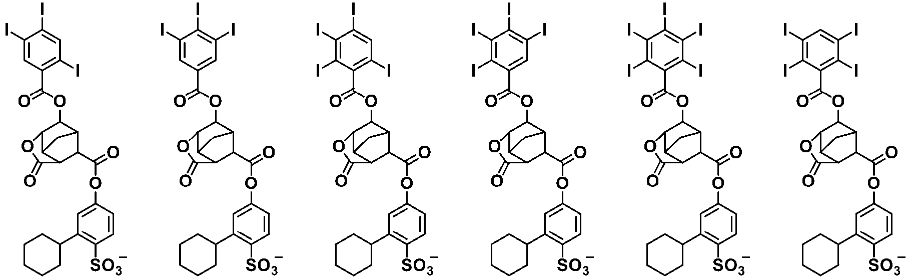

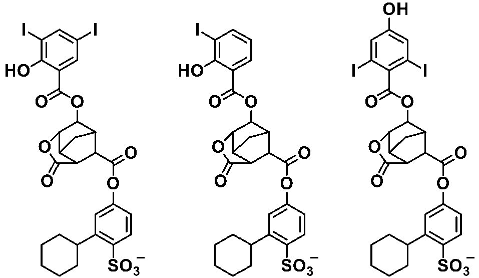

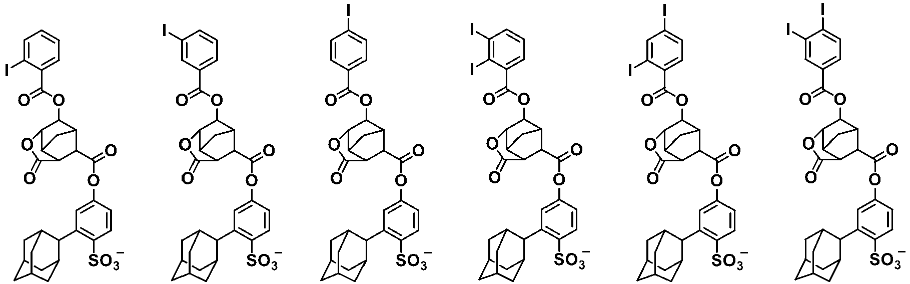

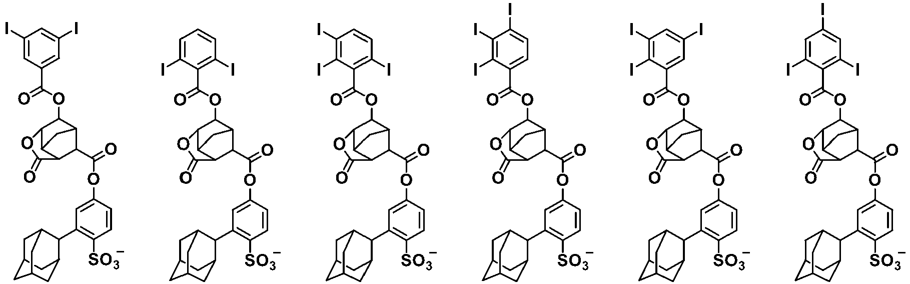

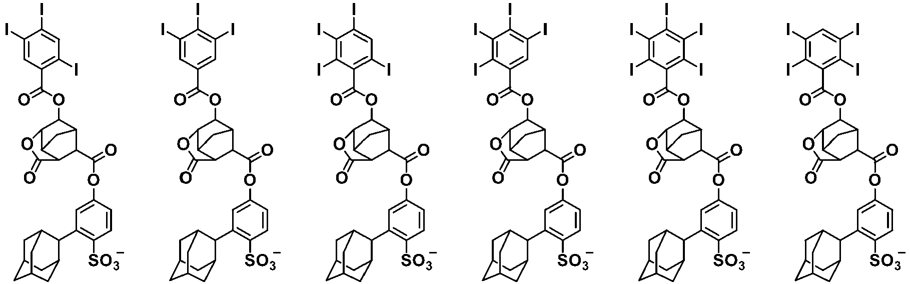

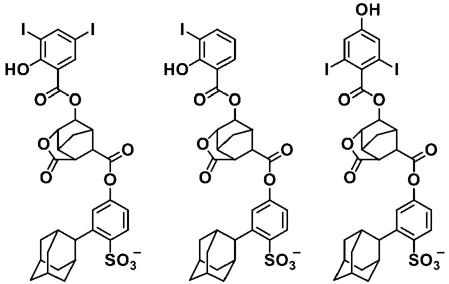

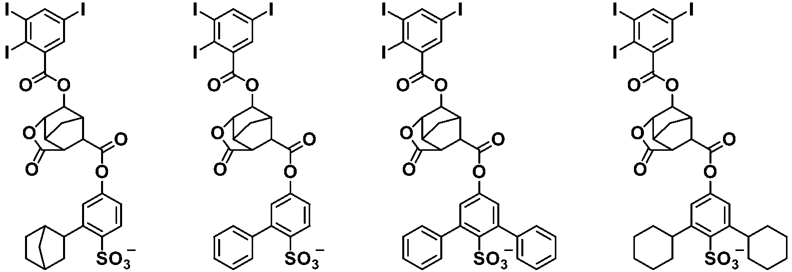

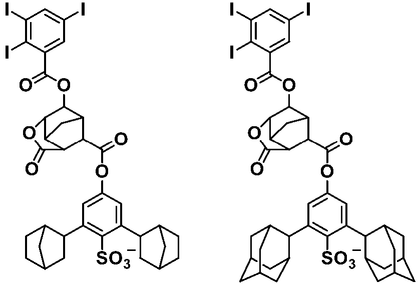

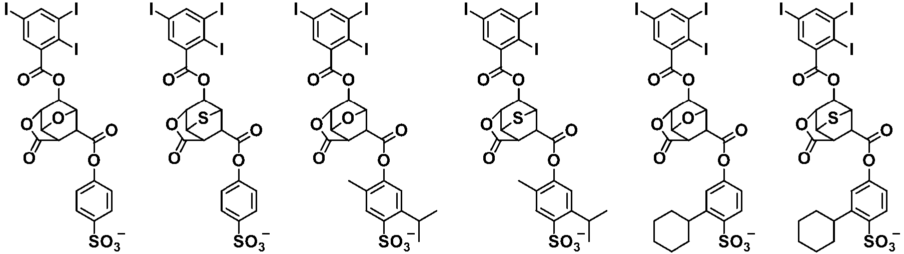

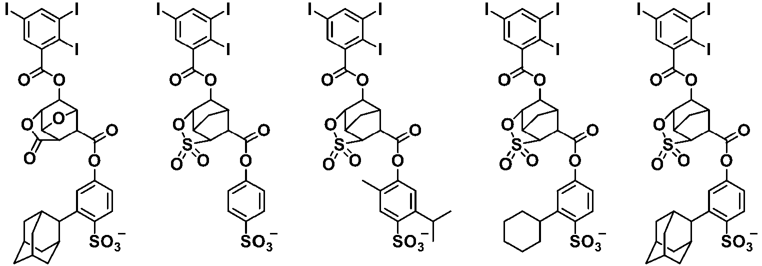

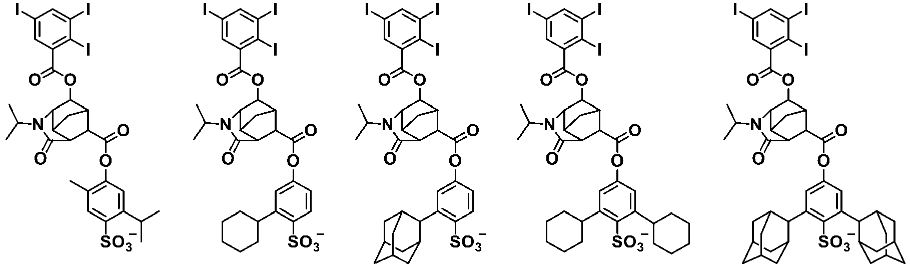

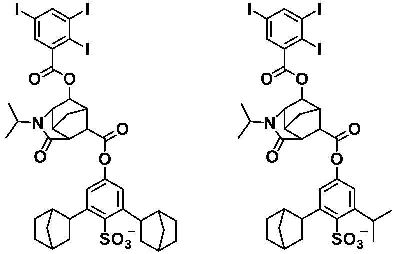

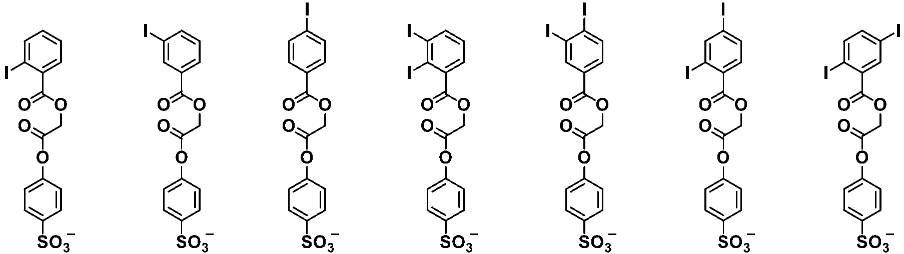

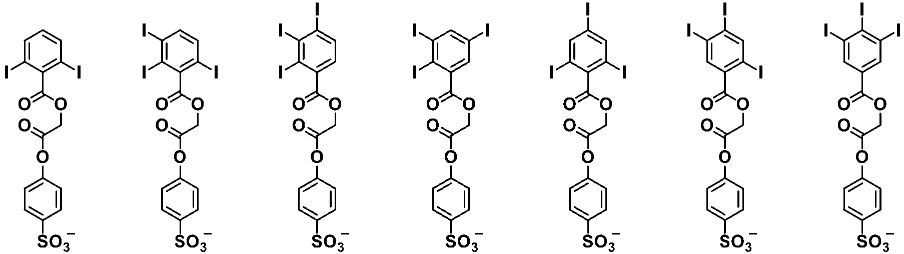

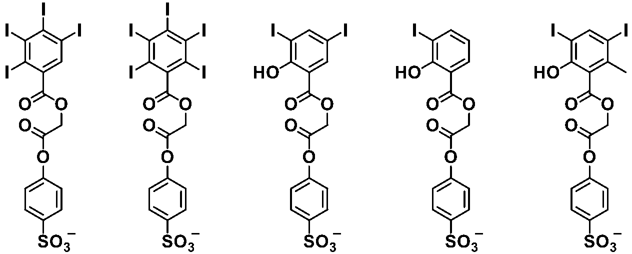

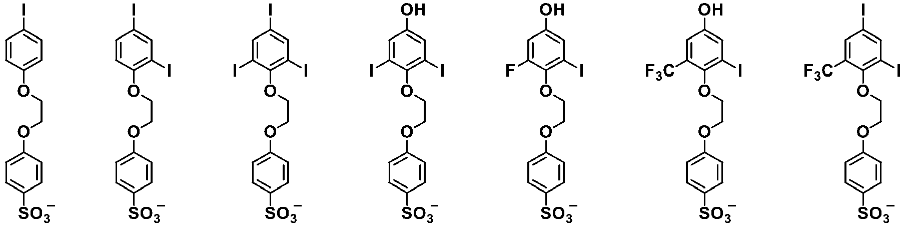

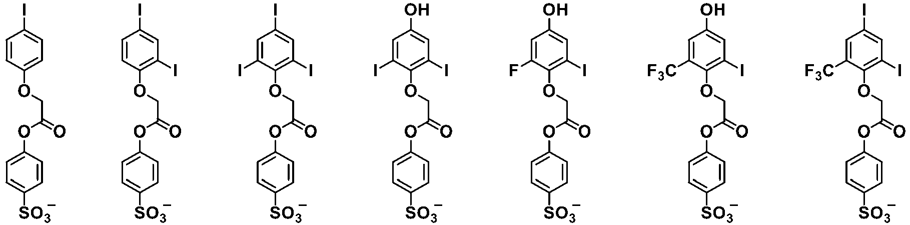

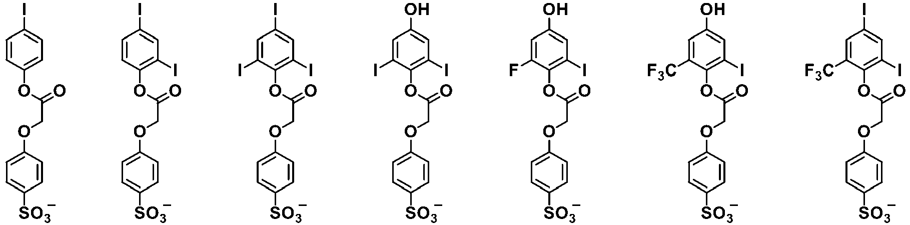

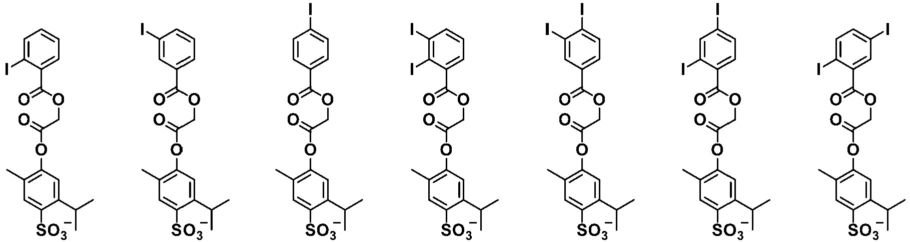

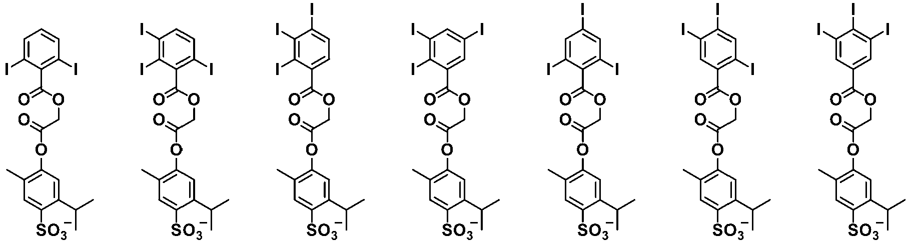

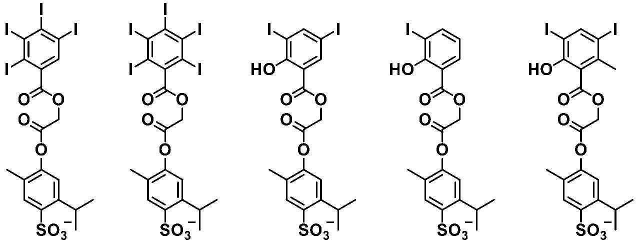

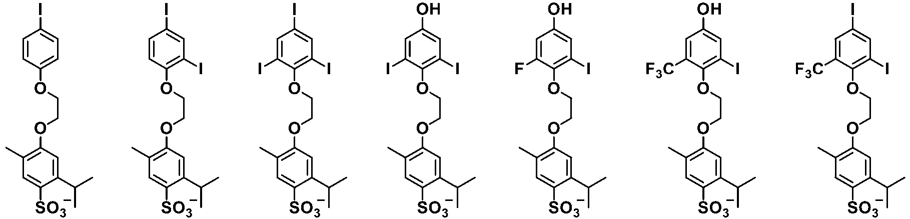

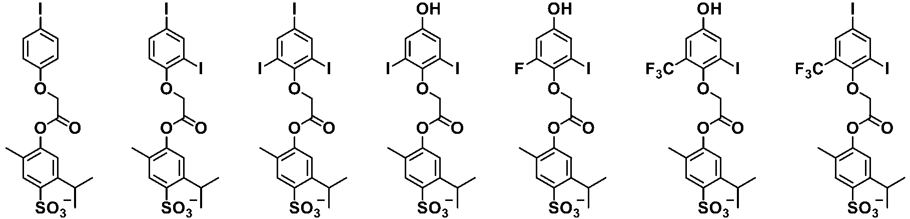

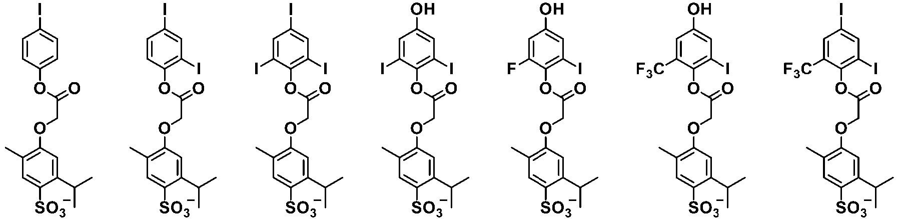

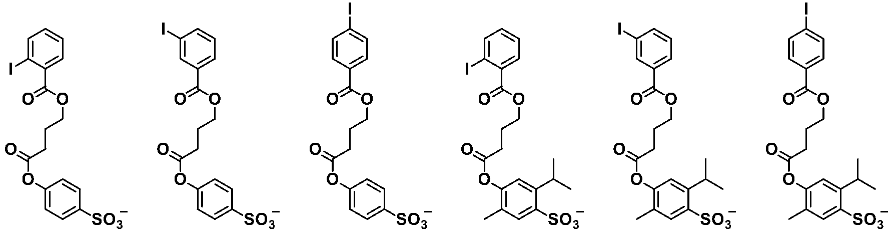

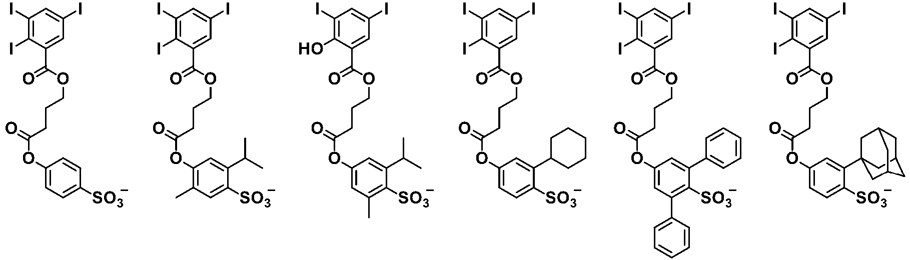

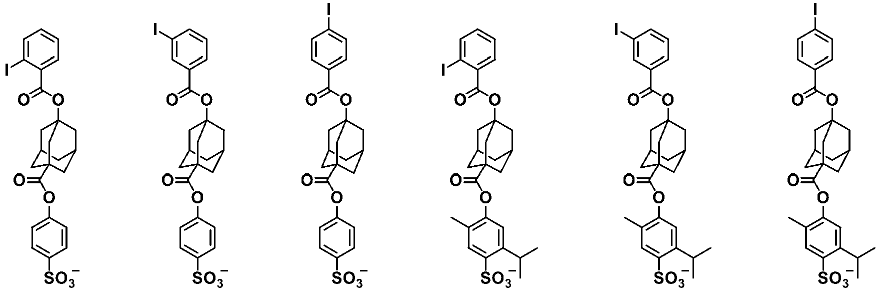

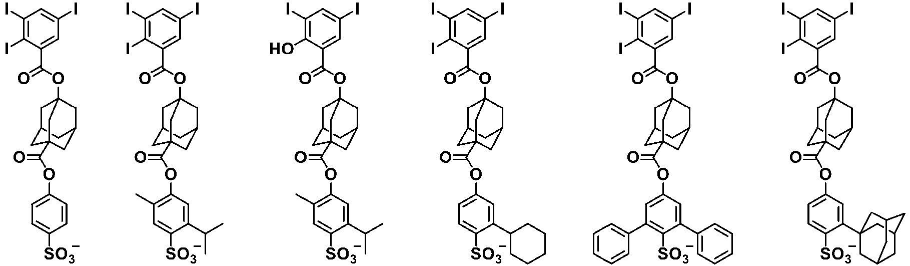

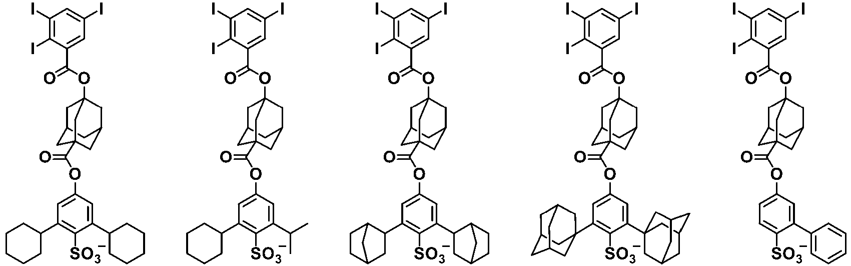

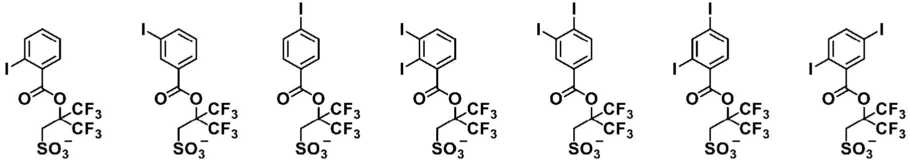

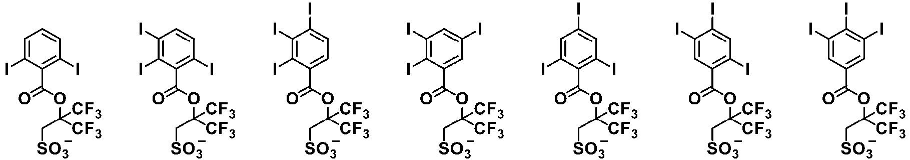

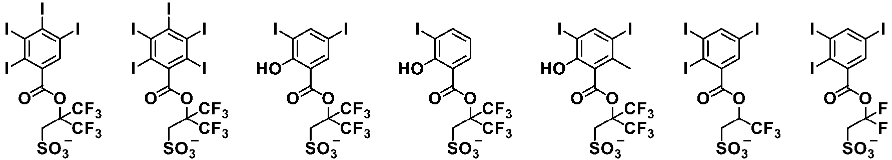

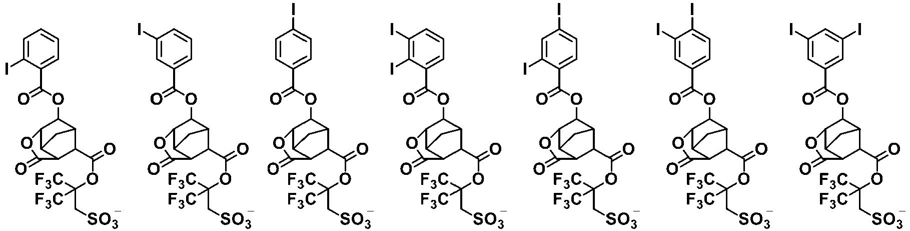

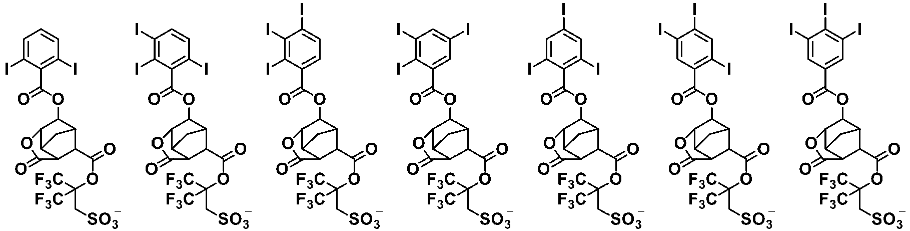

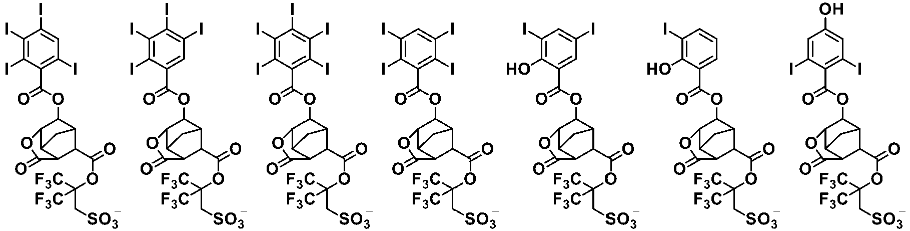

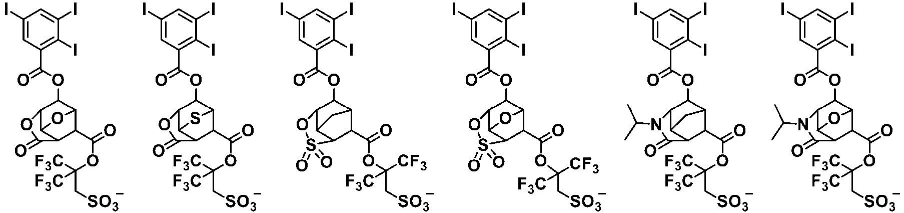

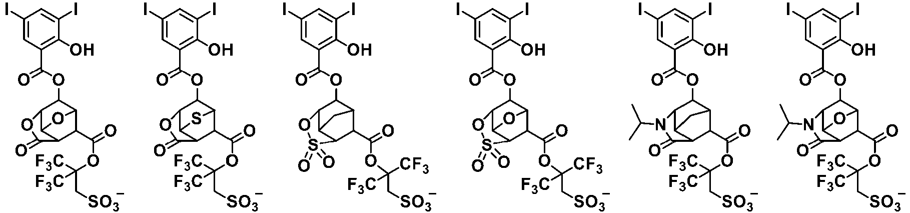

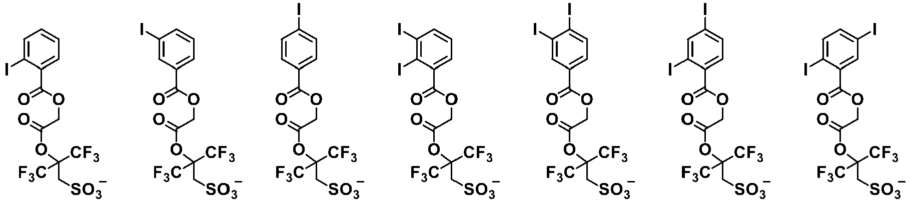

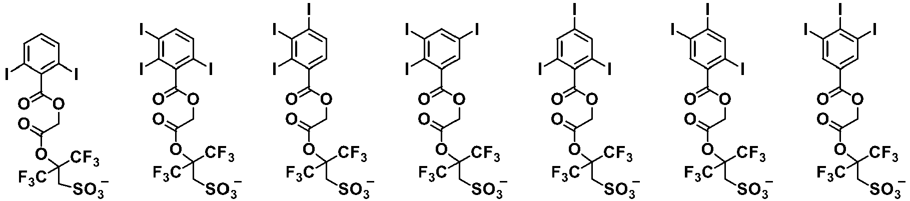

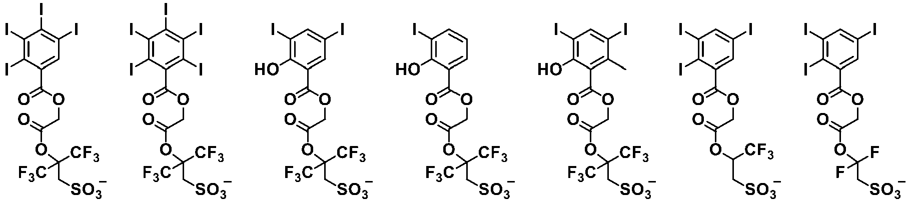

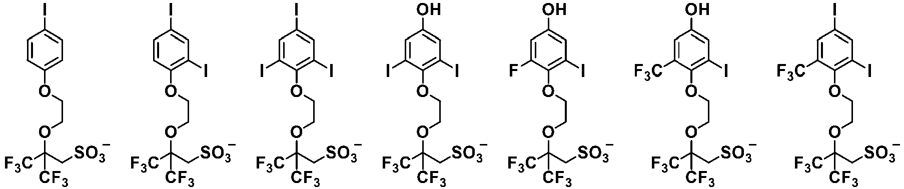

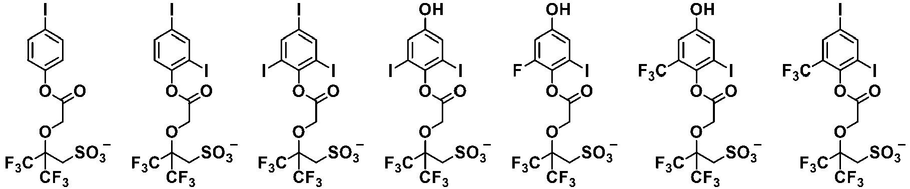

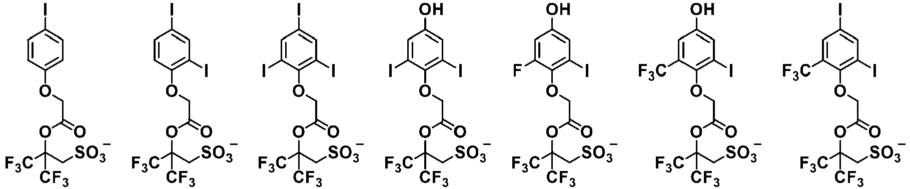

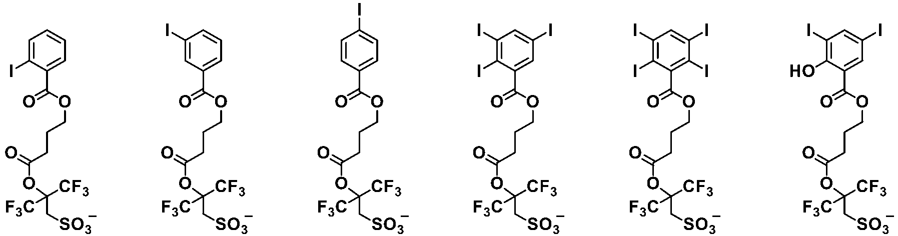

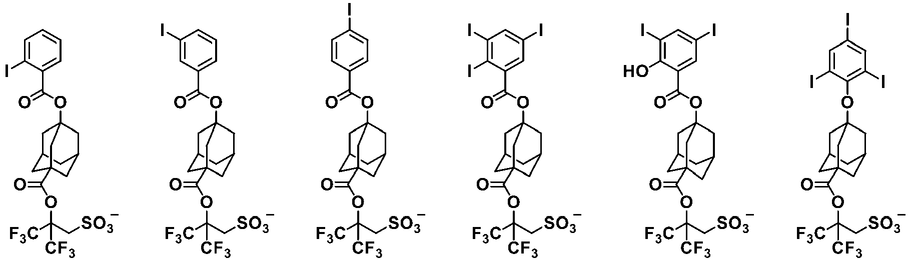

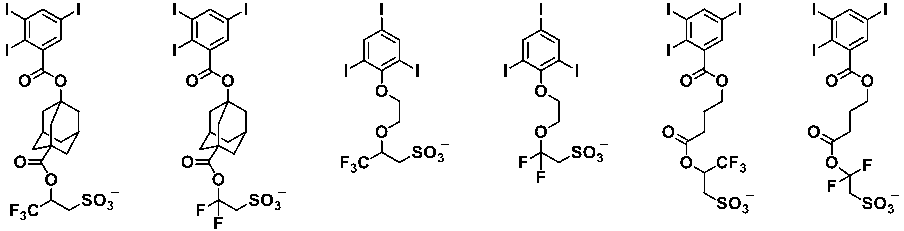

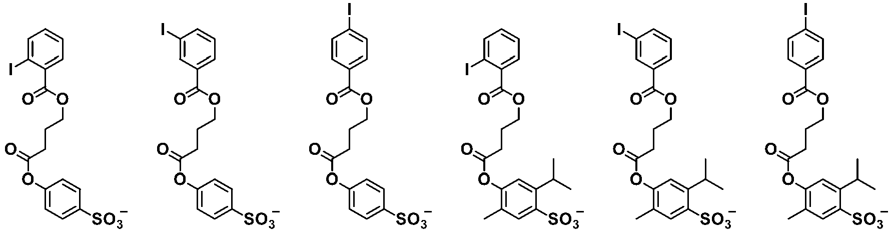

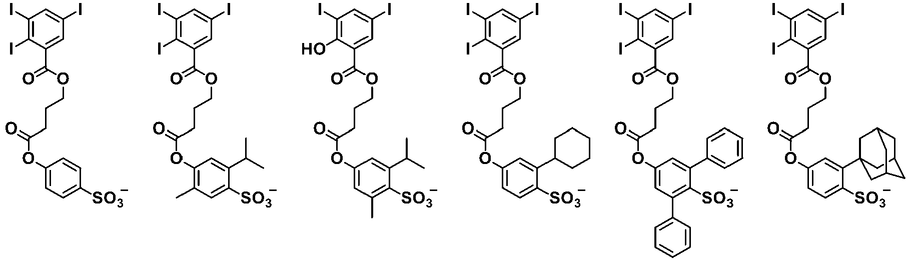

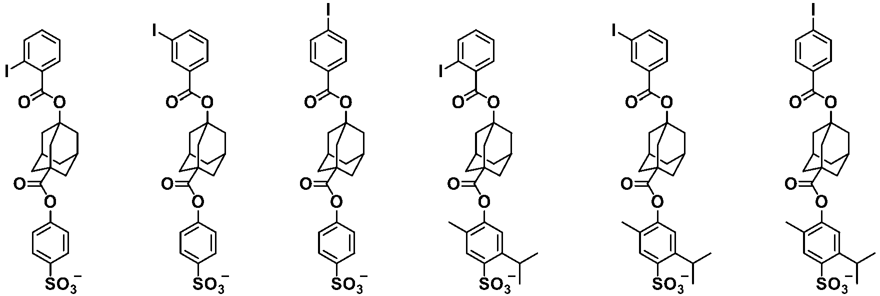

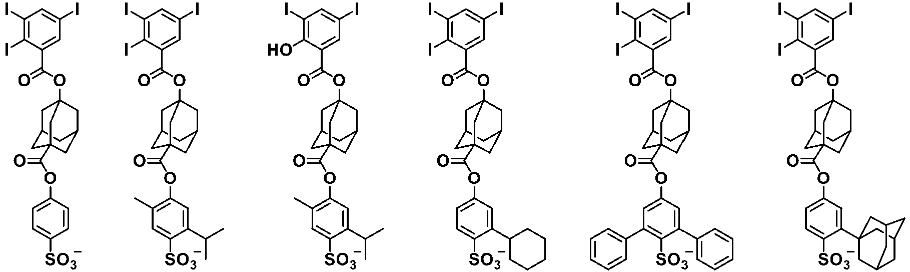

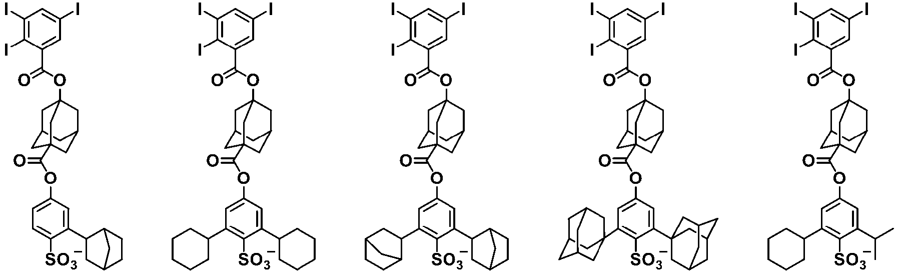

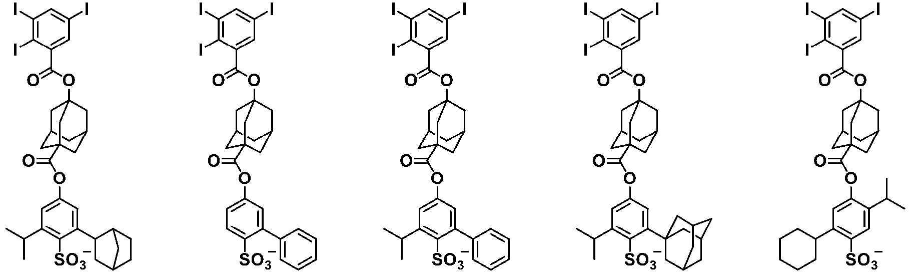

Preferred examples of the cation that pairs with the anion include sulfonium and iodonium cations. Examples of the sulfonium cation are as exemplified above for the sulfonium cation having formula (Z-1) and the sulfonium cation having formula (Z-3), but not limited thereto. Examples of the iodonium cation are as exemplified above for the iodonium cation having formula (Z-2), but not limited thereto.

The other PAG generates an acid having a pKa value of preferably -2.0 or larger, more preferably -1.0 or larger. The upper limit of pKa is preferably 2.0. Notably, the pKa value is computed using pKa DB in software ACD/Chemsketch ver: 9.04 of Advanced Chemistry Development Inc.

When the resist composition contains the other PAG (F), the amount of the PAG (F) used is preferably 1 to 10 parts, more preferably 1 to 5 parts by weight per 80 parts by weight of the base polymer (B). The inclusion of the other PAG provides for appropriate adjustment of the amount of acid generated in the exposed region and the degree of dissolution inhibition in the unexposed region. The other PAG may be used alone or in admixture.

### (G) Surfactant

The resist composition may contain any conventional surfactants for facilitating to coat the composition to the substrate. A number of surfactants are known in the art as described in WO 2006/121096, JP-A 2008-102383, JP-A 2008-304590, JP-A 2004-115630, and JP-A 2005-008766, and any suitable one may be chosen therefrom.

When the resist composition contains the surfactant (G), the amount of the surfactant (G) added is preferably up to 2 parts by weight, more preferably up to 1 part by weight and preferably at least 0.01 part by weight per 80 parts by weight of the base polymer (B). The surfactant may be used alone or in admixture.

### Process

A further embodiment of the invention is a pattern forming process comprising the steps of applying the chemically amplified positive resist composition defined above onto a substrate to form a resist film thereon, exposing the resist film to a pattern of high-energy radiation, and developing the exposed resist film in an alkaline developer.

The substrate used herein may be selected from, for example, substrates for IC fabrication, e.g., Si, SiO, SiO₂, SiN, SiON, TiN, WSi, BPSG, SOG, and organic antireflective coating, and substrates for mask circuit fabrication, e.g., Cr, CrO, CrON, MoSi₂, Si, SiO, and SiO₂.

The resist composition is first applied onto a substrate by a suitable coating technique such as spin coating. The coating is prebaked on a hotplate preferably at a temperature of 60 to 150°C for 1 to 20 minutes, more preferably at 80 to 140°C for 1 to 10 minutes to form a resist film of 0.03 to 2 µm thick.

Then the resist film is exposed patternwise to high-energy radiation. Examples of the high-energy radiation include UV, deep UV, excimer laser radiation (typically, KrF and ArF), EB, EUV, X-ray, γ-ray, and synchrotron radiation.

On use of UV, deep UV, excimer laser radiation, EUV, X-ray, γ-ray, and synchrotron radiation, the resist film is exposed through a mask having the desired pattern, preferably in a dose of 1 to 300 mJ/cm², more preferably 10 to 200 mJ/cm². On use of EB, a pattern may be directly written preferably in a dose of 1 to 300 µC/cm², more preferably 10 to 200 µC/cm². The resist composition of the invention is particularly useful in the EUV and EB lithography processes.

The exposure may be performed by conventional lithography whereas the immersion lithography of holding a liquid, typically water between the resist film and the mask may be employed if desired. In the case of immersion lithography, a protective film which is insoluble in water may be formed on the resist film.

After the exposure, the resist film may be baked (PEB), for example, on a hotplate preferably at 60 to 150°C for 1 to 20 minutes, and more preferably at 80 to 140°C for 1 to 10 minutes.

Finally, development is carried out using as the developer an aqueous alkaline solution, such as a 0.1 to 5 wt%, preferably 2 to 3 wt%, aqueous solution of tetramethylammonium hydroxide (TMAH), this being done by a conventional method such as dip, puddle, or spray development for a period of 0.1 to 3 minutes, and preferably 0.5 to 2 minutes. In this way the exposed region of resist film is dissolved away, forming the desired pattern on the substrate.

The resist composition of the invention is advantageous particularly on use under the situation that requires high etching resistance, and a minimal change of pattern line width and minimal LER even when the time duration from exposure to PEB is prolonged. It is also advantageous for pattern formation on a substrate having a surface layer of material to which the resist pattern is less adherent with a likelihood of pattern stripping or pattern collapse, specifically a substrate having sputter deposited thereon a layer of metallic chromium or a chromium compound containing one or more light elements such as oxygen, nitrogen and carbon. The resist composition is particularly useful in forming a pattern on a photomask blank as the substrate.

### EXAMPLES

Examples of the invention are given below by way of illustration and not by way of limitation. The abbreviation "pbw" is parts by weight. Analysis is made by time-of-flight mass spectrometry (TOF-MS) using MALDI TOF-MS: S3000 by JEOL Ltd.

### [1] Synthesis of onium salts

### Example 1-1

### Synthesis of onium salt PAG-1

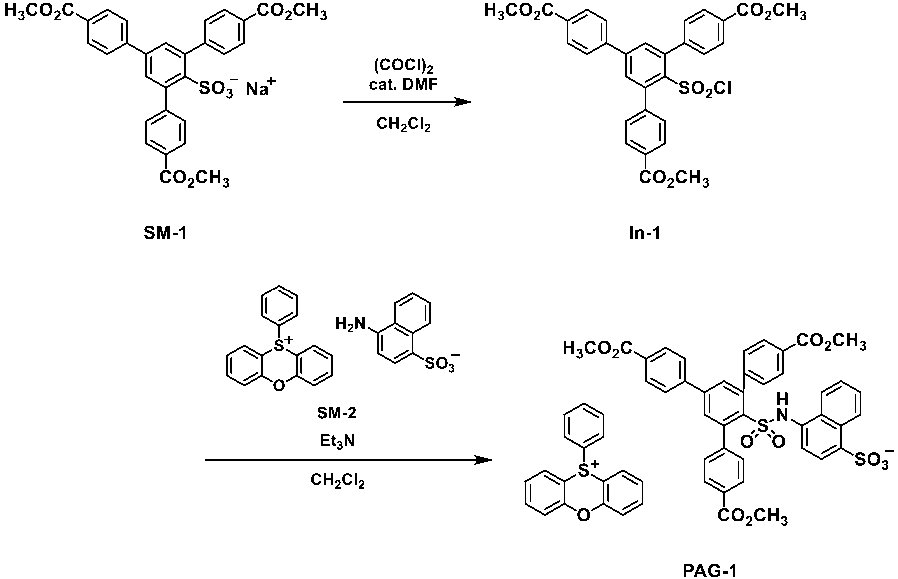

### (1) Synthesis of Intermediate In-1

In a reactor under nitrogen atmosphere, 14.5 g of reactant SM-1 and 0.2 g of N,N-dimethylformamide were dissolved in 100 g of methylene chloride. After the reactor was heated at a temperature of 40°C, 9.5 g of oxalyl chloride was added dropwise to the solution, which was aged in the reactor at a temperature of 40°C for 2 hours. After aging, the reaction solution was cooled down and 50 g of water was added to quench the reaction. The desired compound was extracted with 50 g of methylene chloride. This was followed by ordinary aqueous workup, solvent distillation, and recrystallization from hexane, obtaining 13.7 g of Intermediate In-1 as white crystals (yield 95%).

### (2) Synthesis of onium salt PAG-1

In a reactor under nitrogen atmosphere, 5.8 g of Intermediate In-1 and 5.0 g of reactant SM-2 were suspended in 70 g of methylene chloride. 1.4 g of triethylamine was added dropwise to the suspension, which was aged at room temperature for 10 hours. At the end of aging, 30 g of water was added to quench the reaction. The target compound was extracted with 30 g of methylene chloride. This was followed by ordinary aqueous workup, solvent distillation, and purification by silica gel chromatography, obtaining 8.6 g of onium salt PAG-1 as white crystals (yield 83%).

PAG-1 was analyzed by TOF-MS, with the results shown below.

### MALDI TOF-MS:

positive M⁺ 277 (corresponding to C₁₈H₁₃OS⁺)
negative M⁻ 764 (corresponding to C₄₀H₃₀NO₁₁S₂⁻)

### Examples 1-2 to 1-10

### Synthesis of PAG-2 to PAG-10

Onium salts PAG-2 to PAG-10 shown below were synthesized by well-known organic synthesis reaction using corresponding reactants.

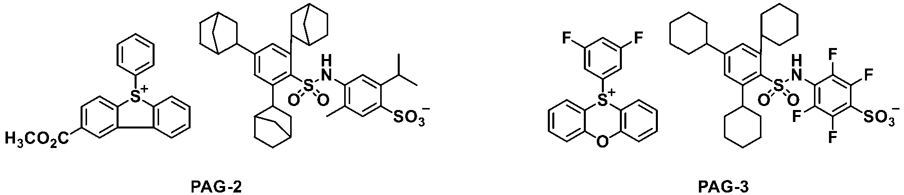

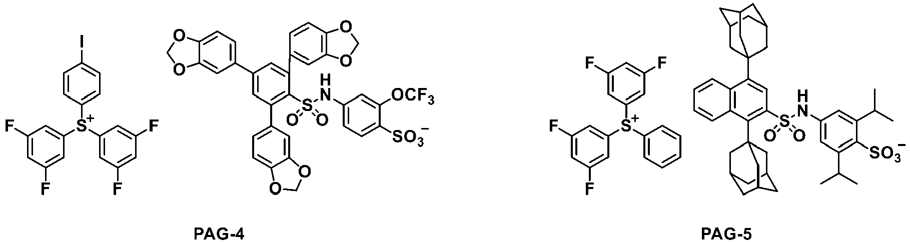

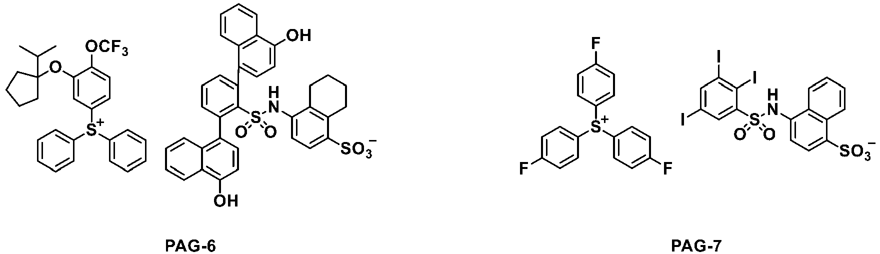

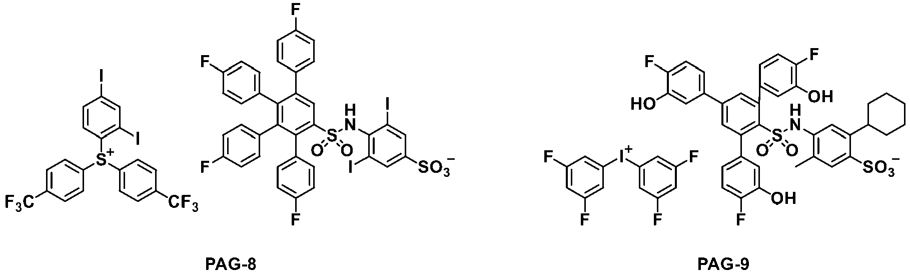

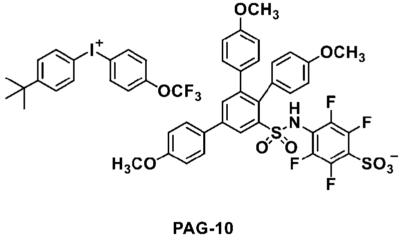

### [2] Synthesis of base polymers

### Synthesis Example

### Synthesis of Base Polymers P-1 to P-6

Base polymers P-1 to P-6 were synthesized by combining monomers, performing copolymerization reaction in a solvent, pouring the reaction solution to hexane for precipitation, washing the solid precipitate with hexane, isolation and drying. The polymer was analyzed for composition by ¹H-NMR and ¹³C-NMR spectroscopy and for Mw and Mw/Mn by GPC versus polystyrene standards using THF solvent.

| | |
|---|---|
| **P-1 (a=0.66, b=0.14, c=0.20, Mw=4400, Mw/Mn=1.10)** | **P-2 (a=0.70, b=0.30, Mw=6200, Mw/Mn=1.34)** |
| 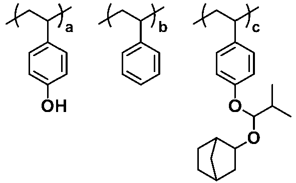 | 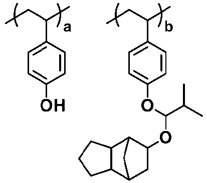 |
| **P-3 (a=0.66, b=0.11, c=0.23, Mw=5800, Mw/Mn=1.32)** | **P-4 (a=0.56, b=0.08, c=0.26, d=0.10, Mw=5800, Mw/Mn=1.40)** |
| 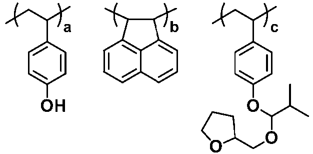 | 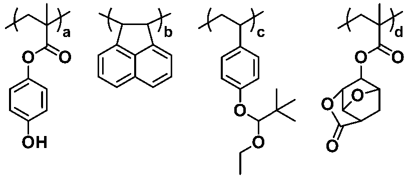 |
| **P-5 (a=0.70, b=0.15, c=0.15, Mw=4700, Mw/Mn=1.52)** | **P-6 (a=0.70, b=0.25, c=0.05, Mw=10200, Mw/Mn=1.65)** |
| 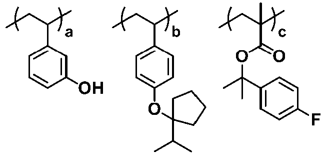 | 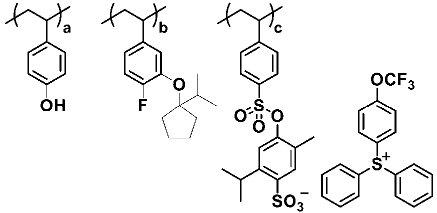 |

### [3] Preparation of chemically amplified positive resist compositions

### Examples 2-1 to 2-50 and Comparative Examples 1-1 to 1-42

A chemically amplified positive resist composition was prepared by dissolving selected components in an organic solvent in accordance with the formulation shown in Tables 1 to 3, and filtering the solution through a nylon filter with a pore size of 5 nm and a UPE filter with a pore size of 1 nm. The organic solvent was a mixture of 940 pbw of PGMEA, 1,870 pbw of EL and 1,870 pbw of PGME.

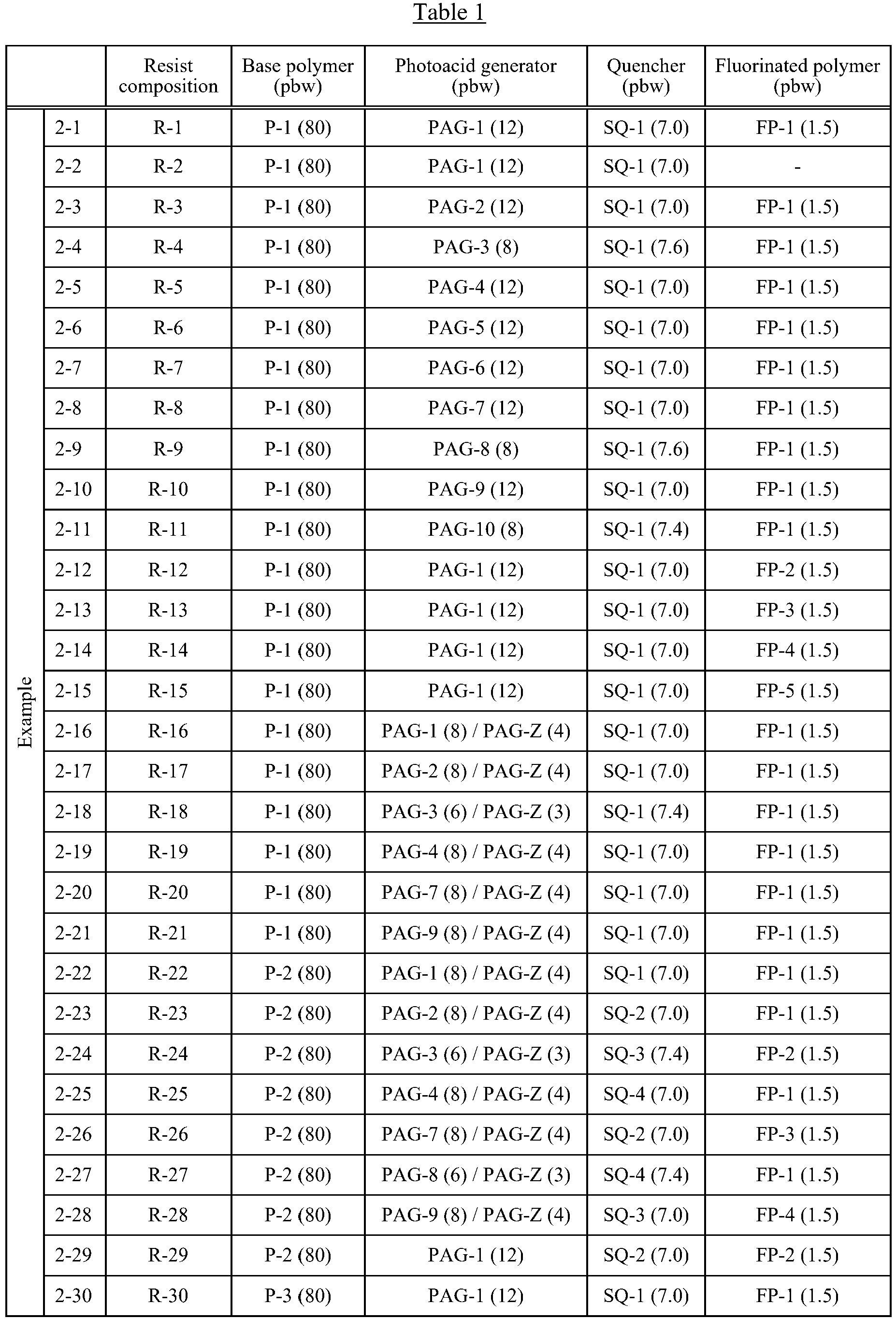

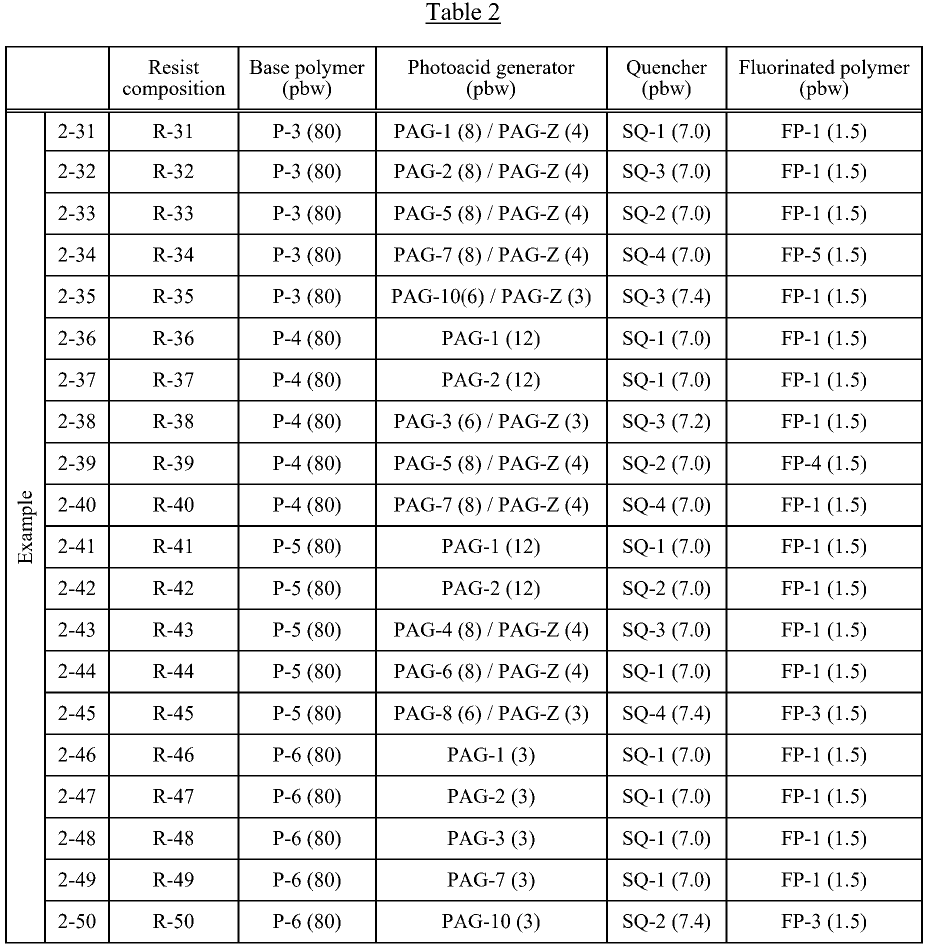

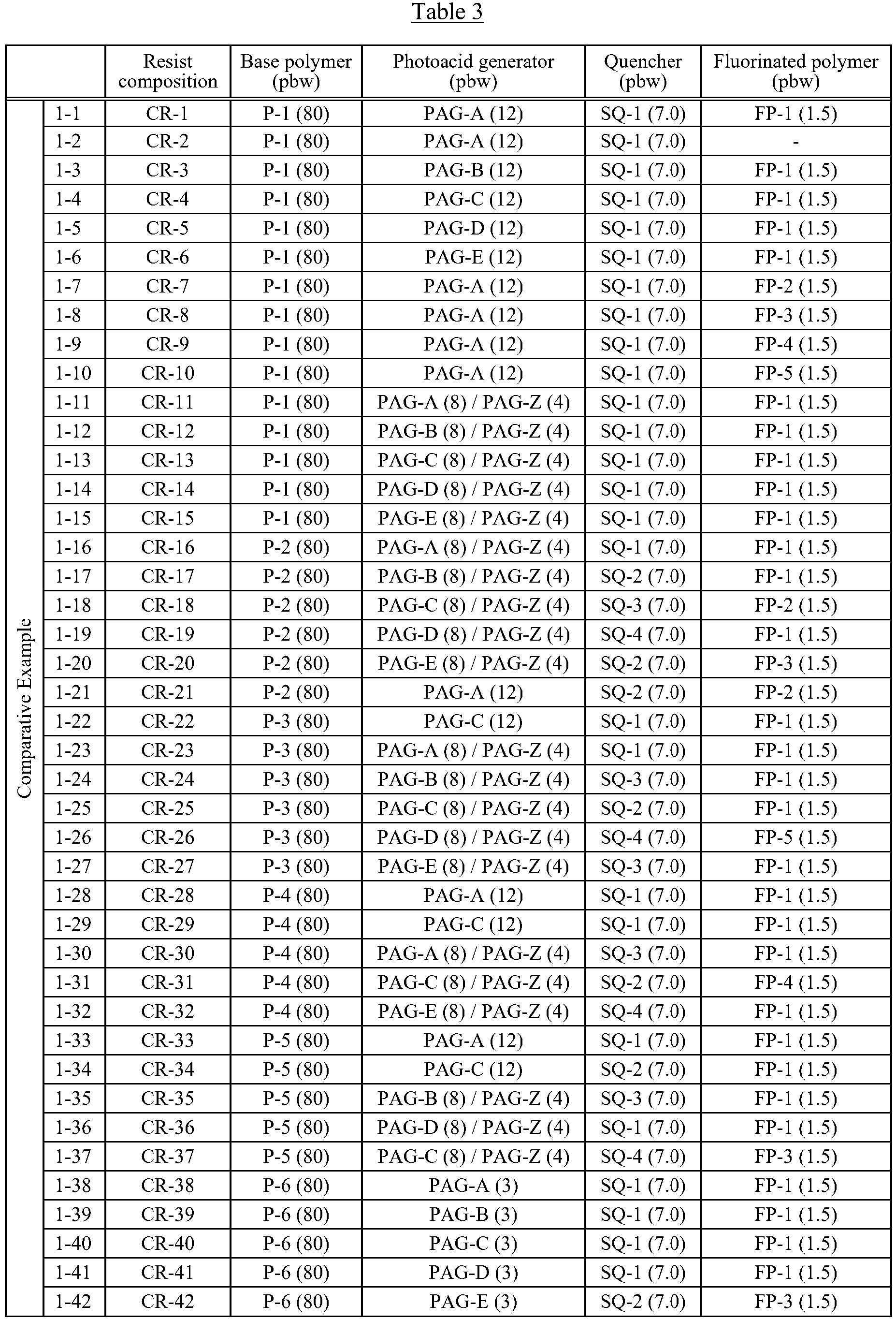

Comparative photoacid generators PAG-A to PAG-E, blending photoacid generator PAG-Z, Quenchers SQ-1 to SQ-4, and Fluorinated Polymers FP-1 to FP-5 in Tables 1 to 3 are identified below.

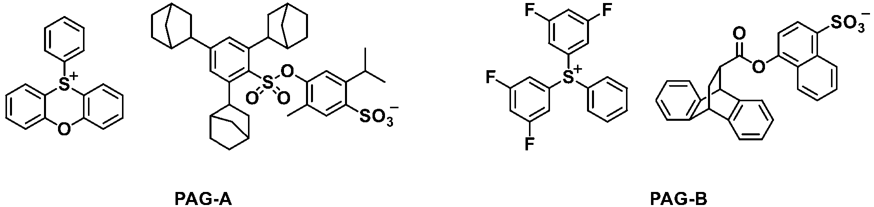

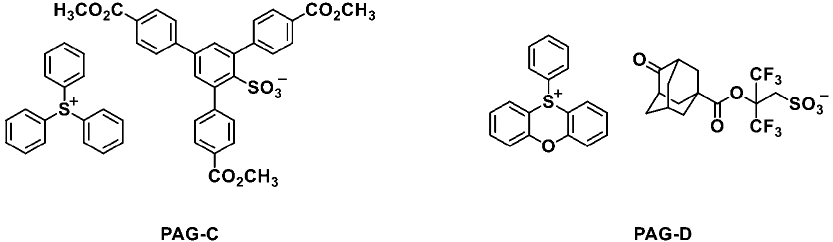

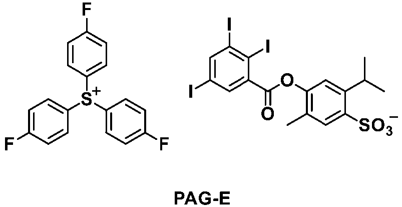

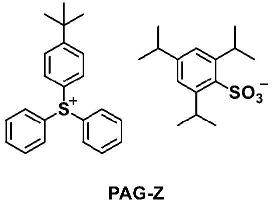

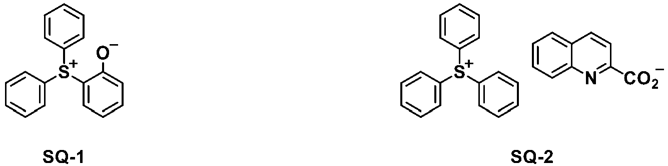

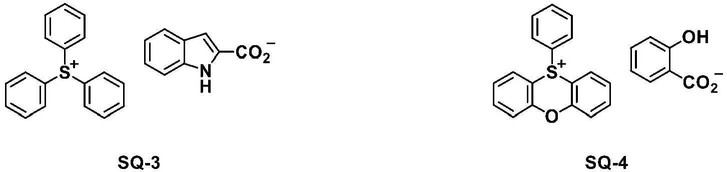

| | | |
|---|---|---|
| **FP-1 (a=0.80, b=0.20, Mw=6000)** | **FP-2 (a=0.80, b=0.20, Mw=6400)** | **FP-3 (a=0.80, b=0.20, Mw=6500)** |
| 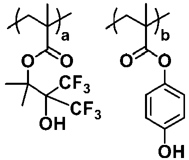 | 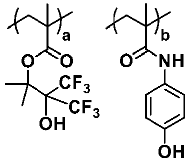 | 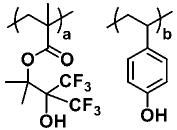 |
| **FP-4 (a=0.80, b=0.20, Mw=8200)** | **FP-5 (a=1.0, Mw=8600)** | |
| 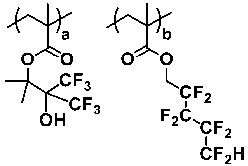 | 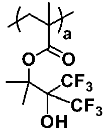 | |

### [4] EB lithography test

### Examples 3-1 to 3-50 and Comparative Examples 2-1 to 2-42

Using a coater/developer system ACT-M (Tokyo Electron Ltd.), each of the resist compositions (R-1 to R-50, CR-1 to CR-42) was spin coated onto a mask blank of reflection type for an EUV lithography mask of 152 mm squares having an outermost surface of chromium compound. The coating was prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

The resist film was exposed to EB using an EB writer system EBM-5000Plus (NuFlare Technology Inc., accelerating voltage 50 kV), then baked (PEB) at 110°C for 600 seconds, and developed in a 2.38 wt% TMAH aqueous solution, thereby yielding a positive pattern.

The resist pattern was evaluated as follows. The patterned mask blank was observed under a top-down scanning electron microscope (TD-SEM). The optimum dose (Eop) was defined as the exposure dose (µC/cm²) which provided a 1:1 resolution at the top and bottom of a 200-nm 1:1 line-and-space (LS) pattern. The resolution (or maximum resolution) was defined as the minimum size at the dose which provided a 1:1 resolution of a 200-nm LS pattern. The edge roughness (LER) of a 200-nm LS pattern was measured under SEM. The resolution (or maximum IS resolution) was defined as the minimum size at the dose which provided a 9:1 resolution of a 200-nm 9:1 LS pattern. The results are shown in Tables 4 to 6.

**Table 4**

| | | Resist composition | Optimum dose (µC/cm²) | L/S resolution (nm) | Maximum IS resolution (nm) | LER (nm) |
|---|---|---|---|---|---|---|
| | 3-1 | R-1 | 205 | 28 | 16 | 2.5 |
| | 3-2 | R-2 | 205 | 28 | 16 | 2.5 |
| | 3-3 | R-3 | 200 | 28 | 16 | 2.6 |
| | 3-4 | R-4 | 210 | 28 | 16 | 2.7 |
| | 3-5 | R-5 | 200 | 30 | 18 | 2.5 |
| | 3-6 | R-6 | 200 | 28 | 18 | 2.6 |
| | 3-7 | R-7 | 205 | 28 | 16 | 2.5 |
| | 3-8 | R-8 | 200 | 28 | 18 | 2.6 |
| | 3-9 | R-9 | 205 | 30 | 16 | 2.7 |
| | 3-10 | R-10 | 200 | 28 | 20 | 2.8 |
| | 3-11 | R-11 | 210 | 28 | 18 | 2.6 |
| | 3-12 | R-12 | 200 | 30 | 16 | 2.7 |
| | 3-13 | R-13 | 205 | 28 | 16 | 2.6 |
| | 3-14 | R-14 | 200 | 28 | 20 | 2.7 |
| Example | 3-15 | R-15 | 205 | 28 | 18 | 2.6 |
| | 3-16 | R-16 | 200 | 28 | 16 | 2.7 |
| | 3-17 | R-17 | 200 | 28 | 16 | 2.8 |
| | 3-18 | R-18 | 205 | 28 | 16 | 2.6 |
| | 3-19 | R-19 | 200 | 28 | 16 | 2.7 |
| | 3-20 | R-20 | 205 | 28 | 18 | 2.7 |
| | 3-21 | R-21 | 200 | 30 | 16 | 2.6 |
| | 3-22 | R-22 | 205 | 28 | 18 | 2.6 |
| | 3-23 | R-23 | 205 | 28 | 16 | 2.7 |
| | 3-24 | R-24 | 205 | 30 | 16 | 2.6 |
| | 3-25 | R-25 | 210 | 28 | 18 | 2.8 |
| | 3-26 | R-26 | 205 | 28 | 16 | 2.7 |
| | 3-27 | R-27 | 205 | 28 | 16 | 2.6 |
| | 3-28 | R-28 | 200 | 28 | 18 | 2.8 |
| | 3-29 | R-29 | 205 | 28 | 18 | 2.6 |
| | 3-30 | R-30 | 205 | 30 | 16 | 2.5 |

**Table 5**

| | | Resist composition | Optimum dose (µC/cm²) | L/S resolution (nm) | Maximum IS resolution (nm) | LER (nm) |
|---|---|---|---|---|---|---|
| | 3-31 | R-31 | 200 | 28 | 16 | 2.6 |
| | 3-32 | R-32 | 200 | 28 | 18 | 2.6 |
| | 3-33 | R-33 | 205 | 30 | 18 | 2.6 |
| | 3-34 | R-34 | 200 | 28 | 18 | 2.8 |
| | 3-35 | R-35 | 210 | 28 | 20 | 2.7 |
| | 3-36 | R-36 | 205 | 26 | 18 | 2.6 |
| | 3-37 | R-37 | 200 | 30 | 18 | 2.6 |
| | 3-38 | R-38 | 205 | 28 | 16 | 2.7 |
| | 3-39 | R-39 | 205 | 28 | 18 | 2.8 |
| Example | 3-40 | R-40 | 200 | 30 | 16 | 2.7 |
| | 3-41 | R-41 | 205 | 28 | 18 | 2.6 |
| | 3-42 | R-42 | 200 | 28 | 16 | 2.7 |
| | 3-43 | R-43 | 205 | 30 | 18 | 2.8 |
| | 3-44 | R-44 | 200 | 28 | 16 | 2.6 |
| | 3-45 | R-45 | 210 | 28 | 18 | 2.8 |
| | 3-46 | R-46 | 205 | 28 | 16 | 2.7 |
| | 3-47 | R-47 | 200 | 30 | 18 | 2.6 |
| | 3-48 | R-48 | 205 | 28 | 18 | 2.7 |
| | 3-49 | R-49 | 210 | 28 | 16 | 2.6 |
| | 3-50 | R-50 | 200 | 30 | 18 | 2.8 |

**Table 6**

| | | Resist composition | Optimum dose (µC/cm²) | L/S resolution (nm) | Maximum IS resolution (nm) | LER (nm) |
|---|---|---|---|---|---|---|
| | 2-1 | CR-1 | 200 | 34 | 22 | 3.3 |
| | 2-2 | CR-2 | 210 | 32 | 24 | 3.4 |
| | 2-3 | CR-3 | 200 | 34 | 22 | 3.4 |
| | 2-4 | CR-4 | 205 | 32 | 22 | 3.8 |
| | 2-5 | CR-5 | 200 | 32 | 24 | 3.6 |
| | 2-6 | CR-6 | 210 | 34 | 22 | 3.4 |
| | 2-7 | CR-7 | 205 | 34 | 24 | 3.4 |
| | 2-8 | CR-8 | 200 | 32 | 22 | 3.6 |
| | 2-9 | CR-9 | 210 | 32 | 22 | 3.5 |
| | 2-10 | CR-10 | 205 | 32 | 24 | 3.6 |
| | 2-11 | CR-11 | 200 | 34 | 22 | 3.5 |
| | 2-12 | CR-12 | 205 | 34 | 24 | 3.6 |
| | 2-13 | CR-13 | 210 | 36 | 22 | 3.3 |
| | 2-14 | CR-14 | 200 | 34 | 22 | 3.4 |
| | 2-15 | CR-15 | 210 | 34 | 24 | 3.4 |
| | 2-16 | CR-16 | 200 | 34 | 22 | 3.6 |
| | 2-17 | CR-17 | 205 | 32 | 24 | 3.4 |
| | 2-18 | CR-18 | 200 | 36 | 22 | 3.6 |
| | 2-19 | CR-19 | 210 | 32 | 24 | 3.5 |
| | 2-20 | CR-20 | 205 | 32 | 22 | 3.4 |
| Comparative Example | 2-21 | CR-21 | 200 | 36 | 24 | 3.6 |
| | 2-22 | CR-22 | 210 | 34 | 22 | 3.5 |
| | 2-23 | CR-23 | 200 | 36 | 24 | 3.6 |
| | 2-24 | CR-24 | 200 | 32 | 22 | 3.6 |
| | 2-25 | CR-25 | 210 | 32 | 24 | 3.4 |
| | 2-26 | CR-26 | 210 | 34 | 22 | 3.6 |
| | 2-27 | CR-27 | 200 | 36 | 22 | 3.6 |
| | 2-28 | CR-28 | 205 | 34 | 22 | 3.6 |
| | 2-29 | CR-29 | 200 | 34 | 24 | 3.5 |
| | 2-30 | CR-30 | 210 | 32 | 22 | 3.6 |
| | 2-31 | CR-31 | 205 | 36 | 24 | 3.6 |
| | 2-32 | CR-32 | 200 | 32 | 22 | 3.5 |
| | 2-33 | CR-33 | 210 | 36 | 24 | 3.6 |
| | 2-34 | CR-34 | 205 | 32 | 24 | 3.4 |
| | 2-35 | CR-35 | 200 | 36 | 24 | 3.5 |
| | 2-36 | CR-36 | 200 | 36 | 22 | 3.6 |
| | 2-37 | CR-37 | 200 | 34 | 22 | 3.2 |
| | 2-38 | CR-38 | 210 | 34 | 22 | 3.4 |
| | 2-39 | CR-39 | 210 | 32 | 24 | 3.3 |
| | 2-40 | CR-40 | 200 | 36 | 22 | 3.2 |
| | 2-41 | CR-41 | 205 | 32 | 24 | 3.4 |
| | 2-42 | CR-42 | 210 | 34 | 24 | 3.3 |

As is evident from Tables 4 to 6, chemically amplified positive resist compositions (R-1 to R-50) within the scope of the invention exhibit satisfactory resolution, LER and pattern rectangularity. In contrast, resist compositions (CR-1 to CR-42) of Comparative Examples fail in optimization of acid diffusion and exhibit poor resolution and LER.

### [4] Evaluation of residue defects

### Examples 5-1 to 5-50 and Comparative Examples 4-1 to 4-42

Each of the resist compositions (R-1 to R-50, CR-1 to CR-42) was applied onto a reflection type mask blank for EUV lithography masks to form a resist film. Using an EB writer system EBM-5000Plus (NuFlare Technology Inc., accelerating voltage 50 kV), the resist film was exposed over its entire surface to EB in its optimum dose. The resist film was then baked (PEB) at 110°C for 600 seconds and developed in a 2.38 wt% TMAH aqueous solution. Using a mask defect inspection system M9650 (Laser Tech), development residues were evaluated. The total number of defects after development is shown in Tables 7 to 9.

**Table 7**

| | | Resist composition | Total number of defects after development |
|---|---|---|---|
| | 5-1 | R-1 | 230 |
| | 5-2 | R-2 | 240 |
| | 5-3 | R-3 | 290 |
| | 5-4 | R-4 | 250 |
| | 5-5 | R-5 | 250 |
| | 5-6 | R-6 | 240 |
| | 5-7 | R-7 | 250 |
| | 5-8 | R-8 | 230 |
| | 5-9 | R-9 | 250 |
| | 5-10 | R-10 | 260 |
| | 5-11 | R-11 | 250 |
| | 5-12 | R-12 | 260 |
| | 5-13 | R-13 | 270 |
| | 5-14 | R-14 | 240 |
| Example | 5-15 | R-15 | 260 |
| | 5-16 | R-16 | 250 |
| | 5-17 | R-17 | 260 |
| | 5-18 | R-18 | 270 |
| | 5-19 | R-19 | 250 |
| | 5-20 | R-20 | 260 |
| | 5-21 | R-21 | 280 |
| | 5-22 | R-22 | 270 |
| | 5-23 | R-23 | 280 |
| | 5-24 | R-24 | 260 |
| | 5-25 | R-25 | 260 |
| | 5-26 | R-26 | 250 |
| | 5-27 | R-27 | 260 |
| | 5-28 | R-28 | 260 |
| | 5-29 | R-29 | 240 |
| | 5-30 | R-30 | 250 |

**Table 8**

| | | Resist composition | Total number of defects after development |
|---|---|---|---|
| | 5-29 | R-29 | 250 |
| | 5-30 | R-30 | 270 |
| | 5-31 | R-31 | 280 |
| | 5-32 | R-32 | 250 |
| | 5-33 | R-33 | 270 |
| | 5-34 | R-34 | 260 |
| | 5-35 | R-35 | 270 |
| | 5-36 | R-36 | 260 |
| | 5-37 | R-37 | 250 |
| | 5-38 | R-38 | 280 |
| Example | 5-39 | R-39 | 260 |
| | 5-40 | R-40 | 270 |
| | 5-41 | R-41 | 260 |
| | 5-42 | R-42 | 240 |
| | 5-43 | R-43 | 230 |
| | 5-44 | R-44 | 250 |
| | 5-45 | R-45 | 260 |
| | 5-46 | R-46 | 270 |
| | 5-47 | R-47 | 250 |
| | 5-48 | R-48 | 270 |
| | 5-49 | R-49 | 260 |
| | 5-50 | R-50 | 250 |

**Table 9**

| | | Resist composition | Total number of defects after development |
|---|---|---|---|
| | 4-1 | CR-1 | 420 |
| | 4-2 | CR-2 | 960 |
| | 4-3 | CR-3 | 680 |
| | 4-4 | CR-4 | 320 |
| | 4-5 | CR-5 | 680 |
| | 4-6 | CR-6 | 970 |
| | 4-7 | CR-7 | 520 |
| | 4-8 | CR-8 | 580 |
| | 4-9 | CR-9 | 560 |
| | 4-10 | CR-10 | 870 |
| | 4-11 | CR-11 | 670 |
| | 4-12 | CR-12 | 450 |
| | 4-13 | CR-13 | 330 |
| | 4-14 | CR-14 | 560 |
| | 4-15 | CR-15 | 510 |
| | 4-16 | CR-16 | 560 |
| | 4-17 | CR-17 | 540 |
| | 4-18 | CR-18 | 310 |
| | 4-19 | CR-19 | 490 |
| | 4-20 | CR-20 | 470 |
| Comparative Example | 4-21 | CR-21 | 460 |
| | 4-22 | CR-22 | 330 |
| | 4-23 | CR-23 | 510 |
| | 4-24 | CR-24 | 450 |
| | 4-25 | CR-25 | 340 |
| | 4-26 | CR-26 | 510 |
| | 4-27 | CR-27 | 520 |
| | 4-28 | CR-28 | 480 |
| | 4-29 | CR-29 | 320 |
| | 4-30 | CR-30 | 480 |
| | 4-31 | CR-31 | 320 |
| | 4-32 | CR-32 | 480 |
| | 4-33 | CR-33 | 460 |
| | 4-34 | CR-34 | 330 |
| | 4-35 | CR-35 | 460 |
| | 4-36 | CR-36 | 500 |
| | 4-37 | CR-37 | 330 |
| | 4-38 | CR-38 | 450 |
| | 4-39 | CR-39 | 520 |
| | 4-40 | CR-40 | 320 |
| | 4-41 | CR-41 | 500 |
| | 4-42 | CR-42 | 510 |

The chemically amplified positive resist compositions (R-1 to R-50) within the scope of the invention show smaller total numbers of defects after development than the comparative resist compositions (CR-1 to CR-42).

It has been demonstrated that the chemically amplified positive resist composition and resist pattern forming process according to the invention are useful in the photolithography for the fabrication of semiconductor devices, especially the processing of photomask blanks of transmission and reflection types.

## Claims

1. An onium salt having the formula (A): 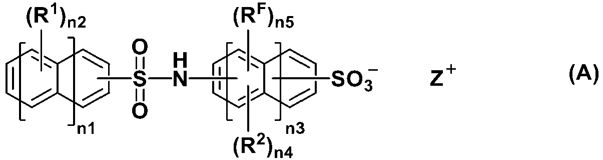 wherein n1 is 0 or 1, n2 is 1, 2, 3, 4 or 5 when n1=0, n2 is 1, 2, 3, 4, 5, 6 or 7 when n1=1, n3 is 0 or 1, n4 is 0, 1, 2, 3 or 4, n5 is 0, 1, 2, 3 or 4, n3 to n5 are in the range: 0 ≤ n4+n5 ≤ 4 when n3=0, and 0 ≤ n4+n5 ≤ 6 when n3=1,
R¹ is each independently iodine or a C₃-C₂₀ branched or cyclic hydrocarbyl group which may contain a heteroatom, at least one R¹ is bonded to a carbon atom adjoining the carbon atom to which S is bonded; when n2 is 2 or more, a plurality of R¹ may be identical or different and a plurality of R¹ may bond together to form a ring with the carbon atoms to which they are attached,
R² is halogen exclusive of fluorine, or a C₁-C₂₀ hydrocarbyl group which may contain a heteroatom exclusive of fluorine; when n4 is 2, 3 or 4, a plurality of R² may be identical or different and a plurality of R² may bond together to form a ring with the carbon atoms to which they are attached,
R^{F} is fluorine, a C₁-C₆ fluorinated alkyl group, C₁-C₆ fluorinated alkoxy group or C₁-C₆ fluorinated alkylthio group; when n5 is 2, 3 or 4, a plurality of R^{F} may be identical or different, and
Z⁺ is an onium cation.

2. The onium salt of claim 1 wherein Z⁺ is a sulfonium cation having the formula (Z-1) or iodonium cation having the formula (Z-2): 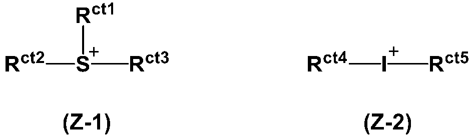 wherein R^{ct1} to R^{ct5} are each independently halogen or a C₁-C₃₀ hydrocarbyl group which may contain a heteroatom, R^{ct1} and R^{ct2} may bond together to form a ring with the sulfur atom to which they are attached.

3. The onium salt of claim 1 wherein Z⁺ is a sulfonium cation having the formula (Z-3): 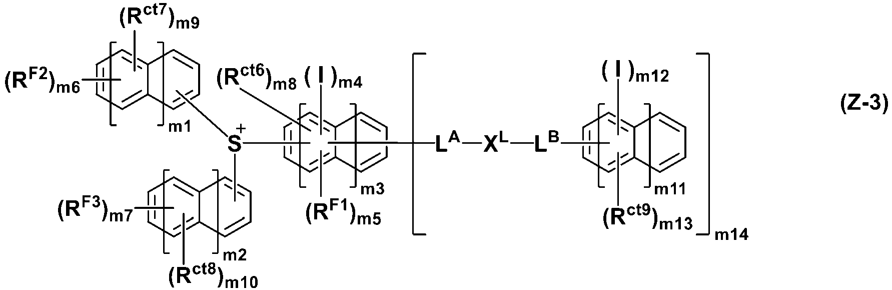 wherein m1 is 0 or 1, m2 is 0 or 1, m3 is 0 or 1, m4 is 0, 1, 2, 3 or 4, m5 is 0, 1, 2, 3 or 4, m6 is 0, 1, 2, 3, 4, 5 or 6, m7 is 0, 1, 2, 3, 4, 5 or 6, m8 is 0, 1 or 2, m9 is 0, 1 or 2, m10 is 0, 1 or 2, m11 is 0 or 1, m12 is 0, 1, 2, 3 or 4, m13 is 0, 1 or 2, m14 is 0, 1 or 2, meeting 0 ≤ m6+m9 ≤ 4 when m1=0, 0 ≤ m6+m9 ≤ 6 when m1=1, 0 ≤ m7+m10 ≤ 4 when m2=0, 0 ≤ m7+m10 ≤ 6 when m2=1, 1 ≤ m4+m5+m8+m14 ≤ 4 when m3=0, 1 ≤ m4+m5+m8+m14 ≤ 6 when m3=1, 0 ≤ m12+m13 ≤ 4 when m11=0, 0 ≤ m12+m13 ≤ 6 when m11=1, and m4+m12 ≥ 1,
R^{F1} to R^{F3} are each independently fluorine, a C₁-C₆ fluorinated saturated hydrocarbyl group, C₁-C₆ fluorinated saturated hydrocarbyloxy group, or C₁-C₆ fluorinated saturated hydrocarbylthio group, with the proviso that a plurality of R^{F1} may be identical or different when m5 is 2,3 or 4, a plurality of R^{F2} may be identical or different when m6 is 2, 3, 4, 5 or 6, and a plurality of R^{F3} may be identical or different when m7 is 2, 3, 4, 5 or 6,
R^{ct6} to R^{ct9} are each independently halogen exclusive of iodine and fluorine, nitro, cyano, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom, with the proviso that two R^{ct6} may be identical or different and two R^{ct6} may bond together to form a ring with the carbon atoms to which they are attached when m8=2, two R^{ct7} may be identical or different and two R^{ct7} may bond together to form a ring with the carbon atoms to which they are attached when m9=2, two R^{ct8} may be identical or different and two R^{ct8} may bond together to form a ring with the carbon atoms to which they are attached when m10=2, two R^{ct9} may be identical or different and two R^{ct9} may bond together to form a ring with the carbon atoms to which they are attached when m13=2,
the aromatic rings directly bonded to S⁺ in the sulfonium cation may bond together to form a ring with S⁺,
L^{A} and L^{B} are each independently a single bond, ether bond, ester bond, amide bond, sulfonate ester bond, sulfonamide bond, carbonate bond or carbamate bond, and
X^{L} is a single bond or a C₁-C₄₀ hydrocarbylene group which may contain a heteroatom.

4. A photoacid generator in the form of the onium salt of any one of claims 1 to 3.

5. A chemically amplified positive resist composition comprising the photoacid generator of claim 4.

6. The resist composition of claim 5, further comprising a base polymer containing a polymer which is decomposed under the action of acid to increase its solubility in alkaline developer,
wherein the polymer comprises repeat units having the formula (B2-1) or (B2-2): 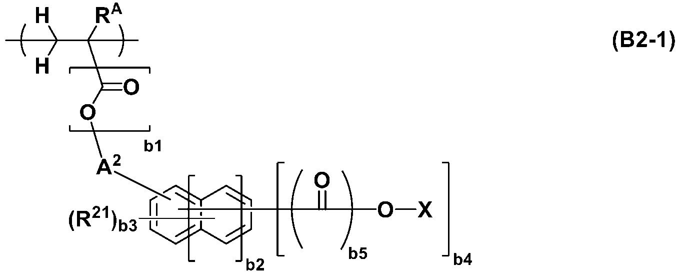 wherein b1 is 0 or 1, b2 is 0, 1 or 2, b3 is an integer meeting 0 ≤ b3 ≤ 5+2(b2)-b4, b4 is 1, 2 or 3, b5 is 0 or 1,
R^{A} is hydrogen, fluorine, methyl or trifluoromethyl,
R²¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
A² is a single bond or C₁-C₁₀ saturated hydrocarbylene group in which some -CH₂-may be replaced by -O-,
X is an acid labile group when b4=1, and X is each independently hydrogen or an acid labile group, at least one being an acid labile group, when b4=2 or 3. 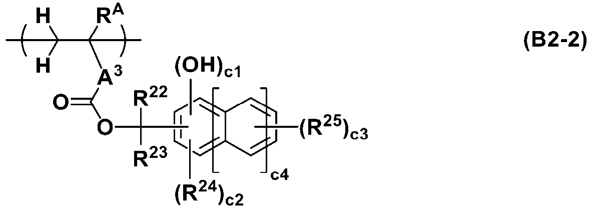 wherein c1 is 0, 1 or 2, c2 is 0, 1 or 2, c3 is 0, 1, 2, 3, 4 or 5, c4 is 0, 1 or 2,
R^{A} is hydrogen, fluorine, methyl or trifluoromethyl,
R²² and R²³ are each independently a C₁-C₁₀ hydrocarbyl group which may contain a heteroatom, R²² and R²³ may bond together to form a ring with the carbon atom to which they are attached,
R²⁴ is each independently fluorine, a C₁-C₅ fluorinated alkyl group or C₁-C₅ fluorinated alkoxy group, a plurality of R²⁴ may be identical or different when c2=2,
R²⁵ is each independently a C₁-C₁₀ hydrocarbyl group which may contain a heteroatom, a plurality of R²⁵ may be identical or different when c3 is 2, 2, 4 or 5, and
A³ is a single bond, phenylene, naphthylene or *-C(=O)-O-A³¹-, A³¹ is a C₁-C₂₀ aliphatic hydrocarbylene group which may contain a hydroxy moiety, ether bond, ester bond or lactone ring, a phenylene group or a naphthylene group, * designates a point of attachment to the carbon atom in the backbone.

7. The resist composition of claim 6 wherein the polymer comprises repeat units having the formula (B 1): 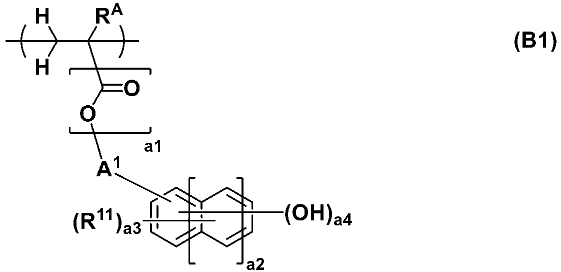 wherein a1 is 0 or 1, a2 is 0, 1 or 2, a3 is an integer meeting 0 ≤ a3 ≤ 5+2(a2)-a4, a4 is 1, 2 or 3,
R^{A} is hydrogen, fluorine, methyl or trifluoromethyl,
R¹¹ is halogen, nitro group, carboxy group, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, a plurality of R¹¹ may be identical or different when a3 is 2 or more, and
A¹ is a single bond or C₁-C₁₀ saturated hydrocarbylene group in which some -CH₂-may be replaced by -O-.

8. The resist composition of claim 6 or 7 wherein the polymer comprises repeat units of at least one type selected from repeat units having the formula (B3), repeat units having the formula (B4), and repeat units having the formula (B5): 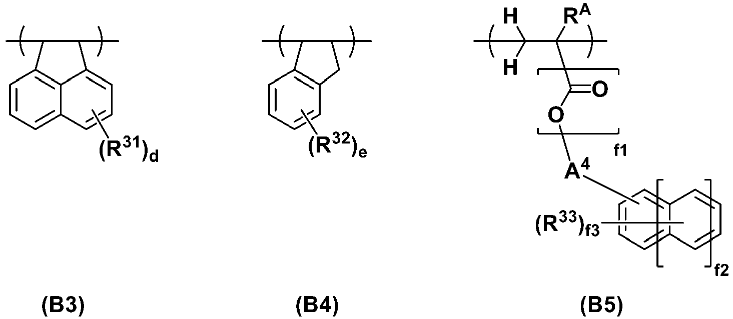 wherein d is 0, 1, 2, 3, 4, 5 or 6, e is 0, 1, 2, 3 or 4, f1 is 0 or 1, f2 is 0, 1 or 2, f3 is 0, 1, 2, 3, 4 or 5,
R^{A} is hydrogen, fluorine, methyl or trifluoromethyl,
R³¹ and R³² are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group; a plurality of R³¹ may be identical or different when d is 2, 3, 4, 5 or 6, and a plurality of R³² may be identical or different when e is 2, 3 or 4,
R³³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro, or cyano, R³³ may also be hydroxy when f2=1 or 2, a plurality of R³³ may be identical or different when f3 is 2, 3, 4 or 5, and
A⁴ is a single bond or C₁-C₁₀ saturated hydrocarbylene group in which some -CH₂-may be replaced by -O-.

9. The resist composition of any one of claims 6 to 8 wherein the polymer comprises repeat units of at least one type selected from repeat units having the formula (B6), repeat units having the formula (B7), repeat units having the formula (B8), repeat units having the formula (B9), and repeat units having the formula (B10): 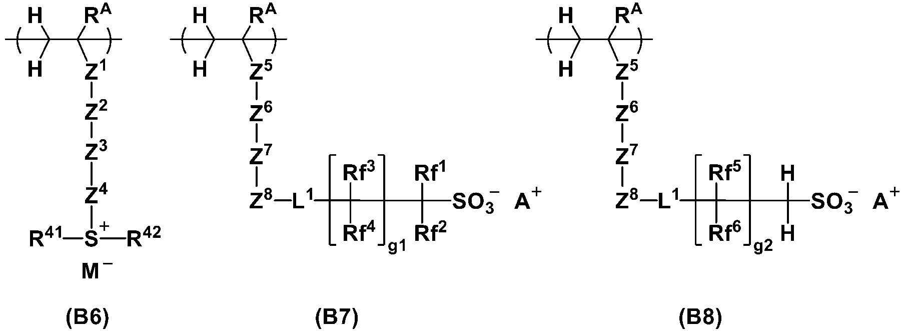 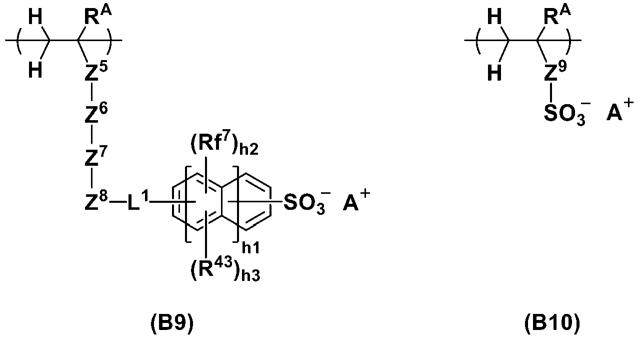 wherein g1 and g2 are each independently 0, 1, 2 or 3, h1 is 0 or 1, h2 is 0, 1, 2, 3 or 4, h3 is 0, 1, 2, 3 or 4, h1 to h3 are in the range: 0 ≤ h2+h3 ≤ 4 when h1=0, and 0 ≤ h2+h3 ≤ 6 when h1=1,
R^{A} is hydrogen, fluorine, methyl or trifluoromethyl,
Z¹ is a single bond or an optionally substituted phenylene group,
Z² is a single bond, **-C(=O)-O-Z²¹-, **-C(=O)-NH-Z²¹-, or **-O-Z²¹-, Z²¹ is a C₁-C₆ aliphatic hydrocarbylene group, phenylene group or a divalent group obtained by combining the foregoing, which may contain halogen, carbonyl moiety, ester bond, ether bond or hydroxy moiety,
Z³ is a single bond, ether bond, ester bond, amide bond, sulfonate ester bond, sulfonamide bond, carbonate bond or carbamate bond,
Z⁴ is a single bond, or a C₁-C₆ aliphatic hydrocarbylene group, phenylene group or a divalent group obtained by combining the foregoing, which may contain halogen, carbonyl moiety, ester bond, ether bond or hydroxy moiety,
Z⁵ is each independently a single bond, an optionally substituted phenylene, naphthylene, or *-C(=O)-O-Z⁵¹-, Z⁵¹ is a C₁-C₁₀ aliphatic hydrocarbylene group which may contain halogen, hydroxy moiety, ether bond, ester bond or lactone ring, or phenylene or naphthylene group,
Z⁶ is a single bond, ether bond, ester bond, amide bond, sulfonate ester bond, sulfonamide bond, carbonate bond or carbamate bond,
Z⁷ is each independently a single bond, ***-Z⁷¹-C(=O)-O-, ***-C(=O)-NH-Z⁷¹-, or ***-O-Z⁷¹-, Z⁷¹ is a C₁-C₂₀ hydrocarbylene group which may contain a heteroatom,
Z⁸ is each independently a single bond, ****-Z⁸¹-C(=O)-O-, ****-C(=O)-NH-Z⁸¹-, or ****-O-Z⁸¹-, Z⁸¹ is a C₁-C₂₀ hydrocarbylene group which may contain a heteroatom,
Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, Z⁹¹ is a C₁-C₆ aliphatic hydrocarbylene group, phenylene group, fluorinated phenylene group or trifluoromethyl-substituted phenylene group, which may contain a carbonyl moiety, ester bond, ether bond or hydroxy moiety,
* is a point of attachment to the carbon atom in the backbone, ** is a point of attachment to Z¹, *** is a point of attachment to Z⁶, **** is a point of attachment to Z⁷,
L¹ is a single bond, ether bond, ester bond, carbonyl group, sulfonate ester bond, sulfonamide bond, carbonate bond or carbamate bond,
Rf¹ and Rf² are each independently fluorine or a C₁-C₆ fluorinated saturated hydrocarbyl group,
Rf and Rf⁴ are each independently hydrogen, fluorine, or a C₁-C₆ fluorinated saturated hydrocarbyl group,
Rf⁵ and Rf⁶ are each independently hydrogen, fluorine, or a C₁-C₆ fluorinated saturated hydrocarbyl group, excluding that all Rf⁵ and Rf⁶ are hydrogen at the same time,
Rf⁹ is fluorine, a C₁-C₆ fluorinated alkyl group, C₁-C₆ fluorinated alkoxy group, or C₁-C₆ fluorinated alkylthio group, a plurality of Rf⁷ may be identical or different when h2 is 2, 3 or 4,
R⁴¹ and R⁴² are each independently a C₁-C₂₀ hydrocarbyl group which may contain a heteroatom, R⁴¹ and R⁴² may bond together to form a ring with the sulfur atom to which they are attached,
R⁴³ is halogen exclusive of fluorine, or a C₁-C₂₀ hydrocarbyl group which may contain a heteroatom; when h3 is 2, 3 or 4, a plurality of R⁴³ may be identical or different and a plurality of R⁴³ may bond together to form a ring with the carbon atoms to which they are attached,
M⁻ is a non-nucleophilic counter ion, and
A⁺ is an onium cation.

10. The resist composition of any one of claims 6 to 9 wherein repeat units having an aromatic ring structure account for at least 60 mol% of the overall repeat units of the polymer in the base polymer.

11. The resist composition of any one of claims 5 to 10, further comprising an organic solvent.

12. The resist composition of any one of claims 5 to 11, further comprising a quencher.

13. The resist composition of any one of claims 5 to 12, further comprising (E) a fluorinated polymer comprising repeat units of at least one type selected from repeat units having the formula (E1), repeat units having the formula (E2), repeat units having the formula (E3) and repeat units having the formula (E4) and optionally repeat units of at least one type selected from repeat units having the formula (E5) and repeat units having the formula (E6): 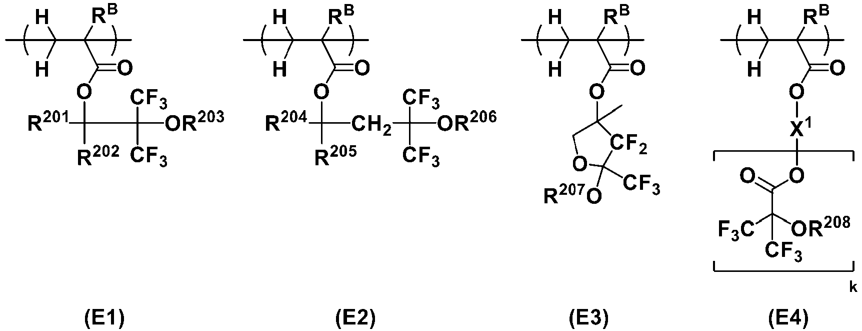 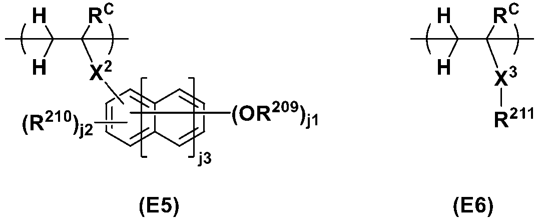 wherein j1 is 1, 2 or 3, j2 is an integer meeting 0 ≤ j2 ≤ 5+2(j3)-j1, j3 is 0 or 1, k is 1, 2 or 3,
R^{B} is each independently hydrogen, fluorine, methyl or trifluoromethyl,
R^{C} is each independently hydrogen or methyl,
R²⁰¹, R²⁰², R²⁰⁴ and R²⁰⁵ are each independently hydrogen or a C₁-C₁₀ saturated hydrocarbyl group,
R²⁰³, R²⁰⁶, R²⁰⁷ and R²⁰⁸ are each independently hydrogen, a C₁-C₁₅ hydrocarbyl group, C₁-C₁₅ fluorinated hydrocarbyl group, or acid labile group, and when R²⁰³, R²⁰⁶, R²⁰⁷ and R²⁰⁸ each are a hydrocarbyl or fluorinated hydrocarbyl group, an ether bond or carbonyl moiety may intervene in a carbon-carbon bond,
R²⁰⁹ is hydrogen or a C₁-C₅ straight or branched hydrocarbyl group in which a heteroatom-containing moiety may intervene in a carbon-carbon bond, a plurality of R²⁰⁹ may be identical or different when j1 is 2 or 3,
R²¹⁰ is a C₁-C₅ straight or branched hydrocarbyl group in which a heteroatom-containing moiety may intervene in a carbon-carbon bond, a plurality of R²¹⁰ may be identical or different when j2 is 2 or more,
R²¹¹ is a C₁-C₂₀ saturated hydrocarbyl group in which at least one hydrogen is substituted by fluorine, and in which some constituent -CH₂- may be replaced by an ester bond or ether bond,
X¹ is a C₁-C₂₀ (k+1)-valent hydrocarbon group or C₁-C₂₀ (k+1)-valent fluorinated hydrocarbon group,
X² is a single bond, *-C(=O)-O- or *-C(=O)-NH-, * designates a point of attachment to the carbon atom in the backbone,
X³ is a single bond, -O-, *-C(=O)=O-X³¹-X³²- or *-C(=O)-NH-X³¹-X³²-, X³¹ is a single bond or C₁-C₁₀ saturated hydrocarbylene group, X³² is a single bond, ester bond, ether bond, or sulfonamide bond, and * designates a point of attachment to the carbon atom in the backbone.

14. The resist composition of any one of claims 5 to 13, further comprising a photoacid generator other than the photoacid generator set forth in claim 6.

15. A resist pattern forming process comprising the steps of:
applying the chemically amplified positive resist composition of any one of claims 5 to 14 onto a substrate to form a resist film thereon,
exposing the resist film patternwise to high-energy radiation, and
developing the exposed resist film in an alkaline developer.
